# EUROPÄISCHE PATENTSCHRIFT

(11) **EP 0 668 267 B1**
(45) Veröffentlichungstag und Bekanntmachung des Hinweises auf die Patenterteilung: **08.04.1998**
(21) Anmeldenummer: 95101136.0
(22) Anmeldetag: 27.01.1995
(51) Int. Cl.: C07D 207/38, C07D 209/54, C07F 9/572, C07D 491/10, C07D 495/10, C07D 401/12, C07D 409/12, C07D 407/12, A01N 43/36, A01N 43/38

(54) **Substituierte 1H-3-Aryl-pyrrolidin-2,4-dion-Derivate**
Substituted 1H-3-aryl-pyrrolidine-2,4-dione derivatives
Dérivés substitués de la 1H-3-aryl-pyrrolidine-2,4-dione

(30) Priorität: 09.02.1994 DE 4404001; 06.09.1994 DE 4431730
(43) Veröffentlichungstag der Anmeldung: 23.08.1995
(73) Patentinhaber: BAYER AG, 51368 Leverkusen (DE)
(72) Erfinder: Fischer, Dr. Reiner, D-40789 Monheim (DE); Bretschneider, Dr. Thomas, D-53797 Lohmar (DE); Krüger, Dr. Bernd-Wieland, D-51467 Bergisch Gladbach (DE); Ruther, Dr. Michael, D-40789 Monheim (DE); Erdelen, Dr. Christoph, D-42799 Leichlingen (DE); Wachendorff-Neumann, Dr. Ulrike, D-40789 Monheim (DE); Santel, Dr. Hans-Joachim, D-51371 Leverkusen (DE); Dollinger, Dr. Markus, D-51381 Leverkusen (DE)

(56) Entgegenhaltungen:
- EP-A- 0 377 893
- EP-A- 0 456 063
- EP-A- 0 521 334
- EP-A- 0 595 130
- EP-A- 0 596 298
- EP-A- 0 613 884
- EP-A- 0 613 885
- WO-A-94/01401

## Beschreibung

Die Erfindung betrifft neue 1H-3-Aryl-pyrrolidin-2,4-dion-Derivate, mehrere Verfahren zu ihrer Herstellung und ihre Verwendung als Schädlingsbekämpfungsmittel, insbesondere als Insektizide, Akarizide und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A-0 262 399 und GB-2 266 888-A werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-415 211) sowie substituierte mono-cyclische 3-Aryl-pyrrolidin-2,4-dion-Derivate (EP-A-377 893 und EP-442 077).

Weiterhin bekannt sind polycyclische 3-Arylpyrrolidin-2,4-dion-Derivate (EP-442 073) sowie 1H-3-Arylpyrrolidin-dion-Derivate (EP-456 063 und EP-521 334).

Es wurden nun neue substituierte 1H-3-Aryl-pyrrolidin-2,4-dion-Derivate der Formel (I) 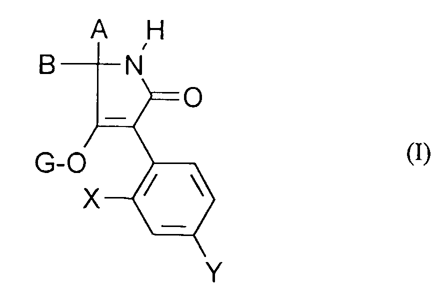 gefunden,
in welcher
- A: für Wasserstoff, jeweils gegebenenfalls durch Halogen substituiertes Alkyl, Alkenyl, Alkoxyalkyl, Alkylthioalkyl, gegebenenfalls durch mindestens ein Heteroatom unterbrochenes, gegebenenfalls substituiertes Cycloalkyl oder jeweils gegebenenfalls durch Halogen, Alkyl, Halogenalkyl, Alkoxy, Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind für einen gesättigten oder ungesättigten gegebenenfalls durch mindestens ein Heteroatom unterbrochenen unsubstituierten oder substituierten Cyclus stehen,
- X: für Halogen oder Alkyl steht,
- Y: für Halogen oder Alkyl steht,
- G: für Wasserstoff (a) oder für eine der Gruppen 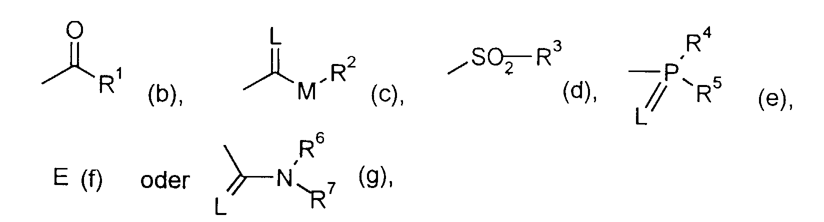 steht,
- E: für ein Metallionäquivalent oder ein Ammoniumion steht,
- L und M: jeweils für Sauerstoff oder Schwefel stehen,
- R¹: für jeweils gegebenenfalls durch Halogen substituiertes Alkyl, Alkenyl, Alkoxyalkyl, Alkylthioalkyl, Polyalkoxyalkyl oder gegebenenfalls durch Halogen oder Alkyl substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Phenoxy oder Phenylthio stehen,
- R⁶ und R⁷: unabhängig voneinander für Wasserstoff, jeweils gegebenenfalls durch Halogen substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, für einen gegebenenfalls durch Sauerstoff oder Schwefel unterbrochenen Cyclus stehen,
mit der Maßgabe, daß X und Y nicht gleichzeitig für Alkyl und nicht gleichzeitig für Halogen stehen.

Unter Einbeziehung der verschiedenen Bedeutungen (a), (b), (c), (d), (e), (f) und (g) der Gruppe G ergeben sich folgende hauptsächlichen Strukturen (Ia) bis (Ig): 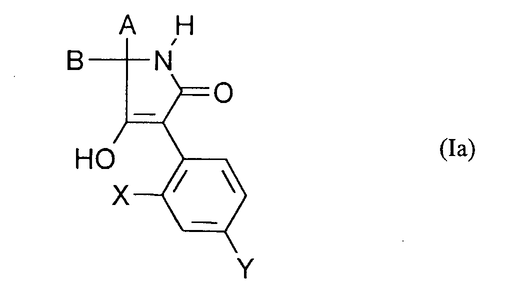 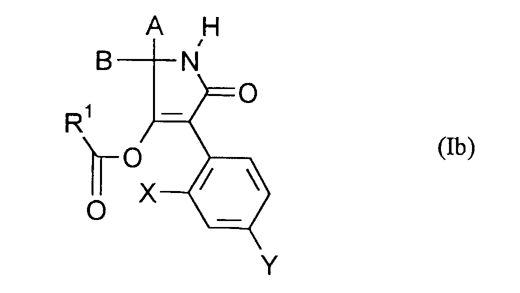 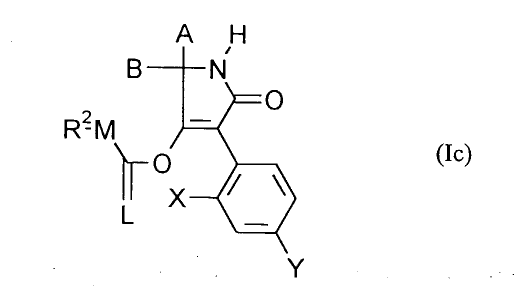 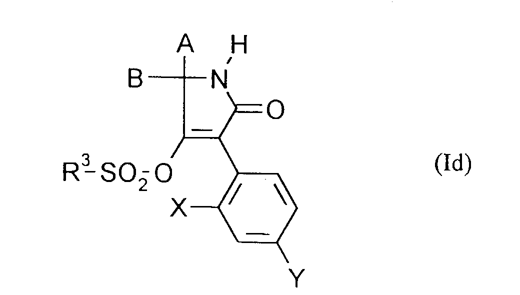 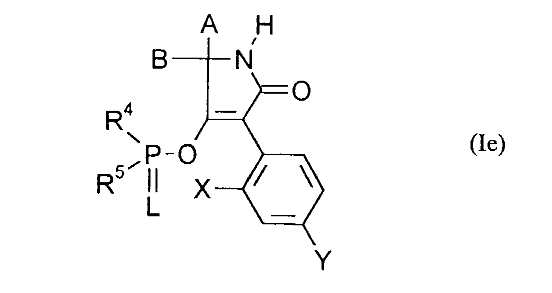 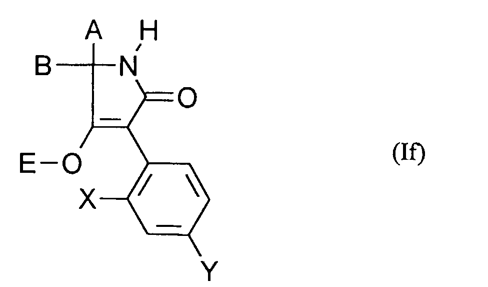 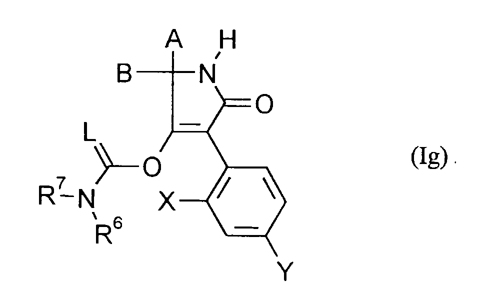 worin
A, B, E, L, M, X, Y, R¹, R², R³, R⁴, R⁵, R⁶ und R⁷ die oben angegebenen Bedeutungen besitzen.

Aufgrund eines oder mehrerer Chiralitätszentren fallen die Verbindungen der Formel (Ia) - (Ig) im allgemeinen als Stereoisomerengemisch an, die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formel (Ia) bis (Ig) gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden, daß man die neuen substituierten 1H-3-Aryl-pyrrolidin-2,4-dion-Derivate der Formel (I) nach einem der im folgenden beschriebenen Verfahren erhält.
(A) Man erhält 1H-3-Aryl-pyrrolidin-2,4-dione bzw. deren Enole der Formel (Ia) 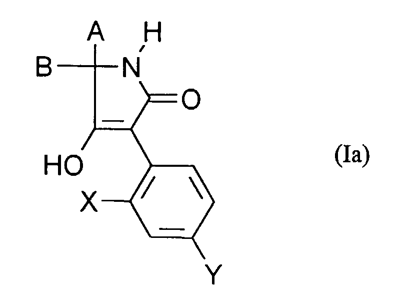 in welcher
   - A, B, X und Y: die oben angegebene Bedeutung haben,
   wenn man
   N-Acylaminosäureester der Formel (II) 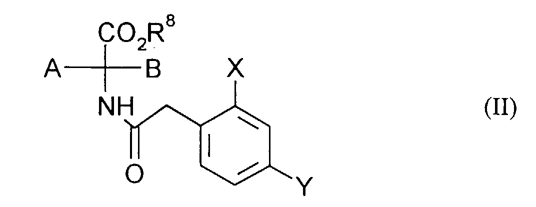 in welcher
   - A, B, X und Y: die oben angegebene Bedeutung haben,
   und
   - R⁸: für Alkyl, inbesondere C₁-C₁₀-Alkyl steht,
   in Gegenwart eines Verdünnungsmittels und in Gegenwart einer Base intramolekular kondensiert;
   oder
(B) man erhält Verbindungen der Formel (Ib) 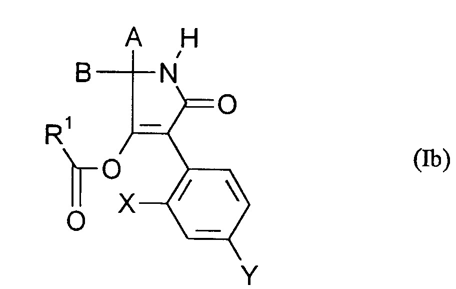 in welcher
   - A, B, X, Y und R¹: die oben angegebene Bedeutung haben,
   wenn man Verbindungen der Formel (Ia), 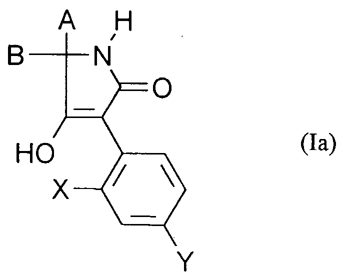 in welcher
   - A, B, X und Y: die oben angegebene Bedeutung haben,

   α) mit Säurehalogeniden der Formel (III) 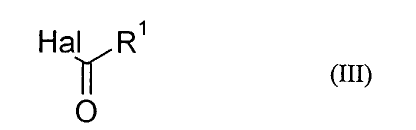 in welcher
      - R¹: die oben angegebene Bedeutung hat und
      - Hal: für Halogen, insbesondere Chlor oder Brom steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
      umsetzt;
   oder
(C) man erhält Verbindungen der Formel (Ic-a) 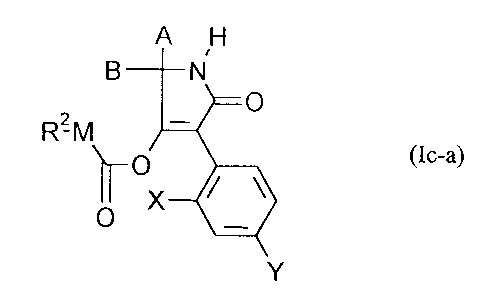 in welcher
   - A, B, X, Y und R²: die oben angegebene Bedeutung haben,
   und
   - M: für Sauerstoff oder Schwefel steht,
   wenn man Verbindungen der Formel (Ia) 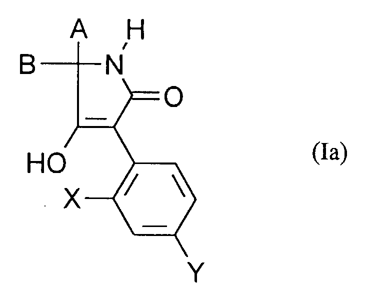 in welcher
   - A, B, X und Y: die oben angegebene Bedeutung haben,
   mit Chlorameisensäureestern oder Chlorameisensäurethiolestern der Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-b) 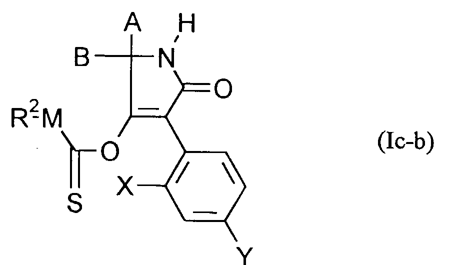 in welcher
   - A, B, R², X und Y: die oben angegebene Bedeutung haben
   und
   - M: für Sauerstoff oder Schwefel steht,
   wenn man Verbindungen der Formel (Ia) 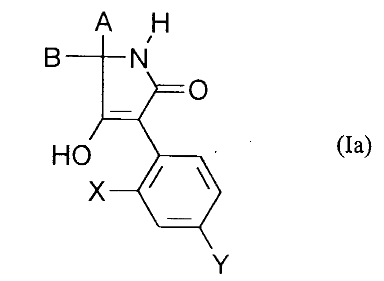 in welcher
   - A, B, X und Y: die oben angegebene Bedeutung haben,

   α) mit Chlormonothioameisensäureestern oder Chlordithioameisensäureestern der Formel (VI) 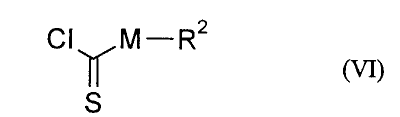 in welcher
      - M und R²: die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der Formel (VII)

      R²-Hal (VII)

      in welcher
      - R²: die oben angegebene Bedeutung hat
      und
      - Hal: für Chlor, Brom oder Iod steht,
      umsetzt;
   oder
(E) man erhält Verbindungen der Formel (Id) 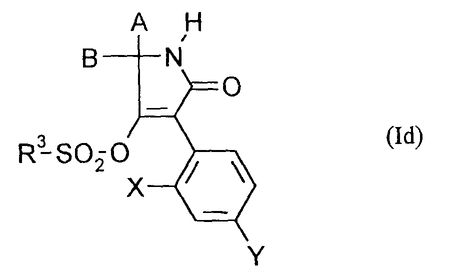 in welcher
   - A, B, X, Y und R³: die oben angegebene Bedeutung haben,
   wenn man Verbindungen der Formel (Ia) 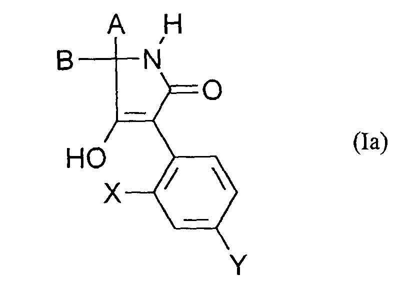 in welcher
   - A, B, X und Y: die oben angegebene Bedeutung haben,
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels,
   umsetzt;
   oder
(F) man erhält 3-Aryl-pyrrolidin-2,4-dione der Formel (Ie) 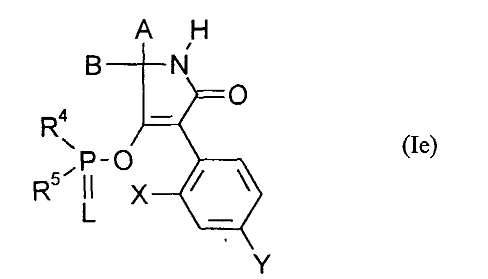 in welcher
   - A, B, L, X, Y, R⁴ und R⁵: die oben angegebene Bedeutung haben,
   wenn man
   1-H-3-Aryl-pyrrolidin-2,4-dione der Formel (Ia) bzw. deren Enole 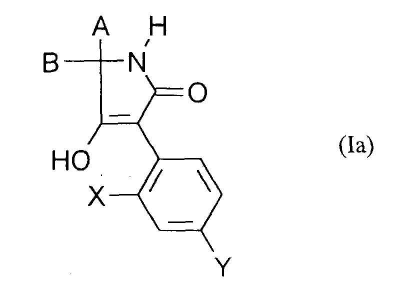 in welcher
   - A, B, X und Y: die oben angegebene Bedeutung haben,
   mit Phosphorverbindungen der Formel (IX) 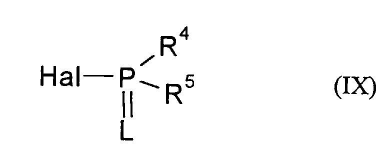 in welcher
   - L, R⁴ und R⁵: die oben angegebene Bedeutung haben
   und
   - Hal: für Halogen, insbesondere Chlor oder Brom steht,
   gegebenenfalls in Gegenwart eines Verdünnungsmittels und gegebenenfalls in Gegenwart eines Säurebindemittels
   umsetzt;
   oder
(G) man erhält Verbindungen der Formel (If) 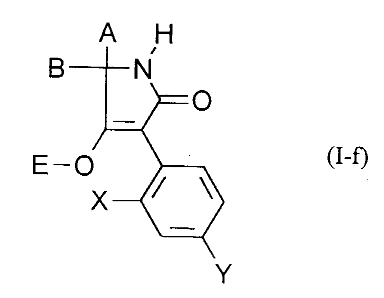 in welcher
   - A, B, X und Y: die oben angegebene Bedeutung haben,
   und
   - E: für ein Metallionäquivalent oder für ein Ammoniumion steht,
   wenn man Verbindungen der Formel (Ia) 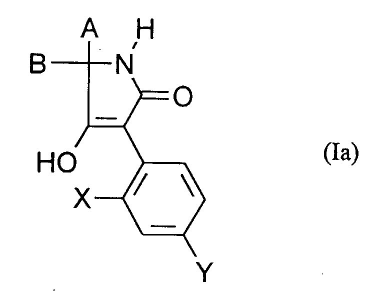 in welcher
   - A, B, X und Y: die oben angegebene Bedeutung haben,
   mit Metallhydroxiden, Metallalkoxiden oder Aminen der Formeln (X) und (XI)

   Me(OR¹⁰)ₜ (X)

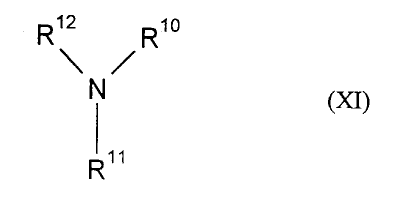

   in welchen
   - Me: für ein ein- oder zweiwertiges Metall wie beispielsweise Lithium, Kalium, Natrium, Calcium oder Magnesium,
   - t: für die Zahl 1 oder 2 und
   - R¹⁰, R¹¹ und R¹²: unabhängig voneinander für Wasserstoff und/oder Alkyl
   stehen,
   gegebenenfalls in Gegenwart eines Verdünnungsmittels, umsetzt.
(H) Ferner wurde gefunden, daß man Verbindungen der Formel (I-g) 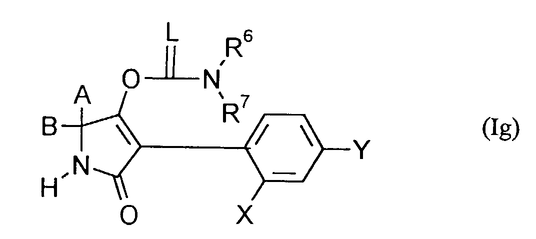 in welcher
   - A, B, L, X, Y, R⁶ und R⁷: die oben angegebene Bedeutung haben,
   erhält, wenn man Verbindungen der Formel (Ia) 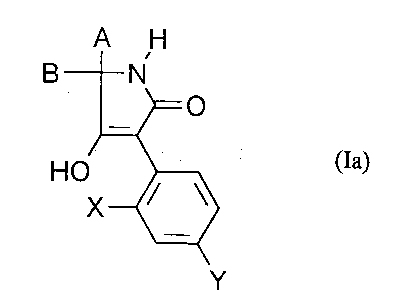 in welcher
   - A, B, X und Y: die oben angegebene Bedeutung haben,

   α) mit Isocyanaten oder Isothiocyanaten der Formel (XII)

      R⁶-N=C=L (XII)

      in welcher
      - L und R⁶: die oben angegebene Bedeutung haben
      gegebenenfalls in Gegenwart eines Verdünnungsmittels und gegebenenfalls in Gegenwart eines Katalysators
      oder
   β) mit Carbamidsäurechloriden oder Thiocarbamidsäurechloriden der Formel (XIII) 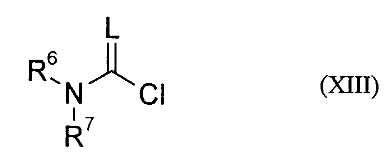 in welcher
      - L, R⁶ und R⁷: die oben angegebene Bedeutung haben
      gegebenenfalls in Gegenwart eines Verdünnungsmittels und gegebenenfalls
      in Gegenwart eines Säurebindemittels, umsetzt.

Weiterhin wurde gefunden, daß sich die neuen 1-H-3-Arylpyrrolidin-2,4-dion-Derivate der Formel (I) durch hervorragende insektizide, akarizide und herbizide Wirkungen auszeichnen. Für die allgemeinen Formeln der vorliegenden Anmeldung gilt:
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₈-Polyalkoxy-C₁-C₈-alkyl, C₁-C₁₀-Alkylthio-C₁-C₆-alkyl, gegebenenfalls durch Halogen, C₁-C₄-Alkyl oder C₁-C₄-Alkoxy substituiertes Cycloalkyl mit 3 bis 8 Ringatomen, das durch Sauerstoff und/oder Schwefel unterbrochen sein kann oder für jeweils gegebenenfalls durch Halogen, C₁-C₆-Alkyl, C₁-C₆-Halogenalkyl, C₁-C₆-Alkoxy und/oder Nitro substituiertes Aryl, 5- bis 6-gliedriges Hetaryl oder Aryl-C₁-C₆-alkyl.
- B: steht bevorzugt für Wasserstoff, C₁-C₁₂-Alkyl oder C₁-C₈-Alkoxyalkyl oder
- A, B: und das Kohlenstoffatom an das sie gebunden sind, stehen bevorzugt für einen gesättigten oder ungesättigten gegebenenfalls durch Sauerstoff oder Schwefel unterbrochenen C₃-C₁₀-Spirocyclus, der gegebenenfalls einfach oder mehrfach durch C₁-C₁₀-Alkyl, C₃-C₁₀-Cycloalkyl, C₁-C₆-Halogenalkyl, C₁-C₁₀-Alkoxy, C₁-C₁₀-Alkylthio, Halogen oder Phenyl substituiert ist oder
- A, B: und das Kohlenstoffatom, an das sie gebunden sind, stehen bevorzugt für einen C₃-C₆-Spirocyclus, der durch eine gegebenenfalls durch ein oder zwei Sauerstoff- und/oder Schwefelatome unterbrochene Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Spirocyclus bildet oder
- A, B: und das Kohlenstoffatom, an das sie gebunden sind, stehen bevorzugt für einen C₃-C₈-Spirocyclus, bei dem zwei Substituenten gemeinsam für einen gegebenenfalls durch C₁-C₆-Alkyl, C₁-C₆-Alkoxy oder Halogen substituierten gesättigten oder ungesättigten 3- bis 8-gliedrigen Cyclus stehen, der durch Sauerstoff oder Schwefel unterbrochen sein kann.
- A: steht besonders bevorzugt für Wasserstoff, jeweils gegebenenfalls durch Fluor und/oder Chlor substituiertes C₁-C₁₀-Alkyl, C₃-C₆-Alkenyl, C₁-C₈-C₁-C₆-Polyalkoxy-C₁-C₆-alkyl, C₁-C₆-Polyalkoxy-C₁-C₆-alkyl, C₁-C₈-Alkylthio-C₁-C₆-alkyl, gegebenenfalls durch Fluor, Chlor, C₁-C₃-Alkyl oder C₁-C₃-Alkoxy substituiertes Cycloalkyl mit 3 bis 7 Ringatomen, das durch 1 bis 2 Sauerstoff- und/oder Schwefelatome unterbrochen sein kann oder jeweils gegebenenfalls durch Fluor, Chlor, Brom, C₁-C₄-Alkyl, C₁-C₄-Halogenalkyl, C₁-C₄-Alkoxy und/oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl.
- B: steht besonders bevorzugt für Wasserstoff, C₁-C₁₀-Alkyl oder C₁-C₆-Alkoxyalkyl oder
- A, B: und das Kohlenstoffatom an das sie gebunden sind, stehen besonders bevorzugt für einen gesättigten oder ungesättigten gegebenenfalls durch Sauerstoff oder Schwefel unterbrochenen C₃-C₉-Spirocyclus, der gegebenenfalls einfach oder mehrfach durch C₁-C₆-Alkyl, C₃-C₈-Cycloalkyl, C₁-C₃-Haloalkyl, C₁-C₆-Alkoxy, C₁-C₆-Alkylthio, Fluor, Chlor oder Phenyl substituiert ist oder
- A,B: und das Kohlenstoffatom, an das sie gebunden sind, stehen besonders bevorzugt für einen C₃-C₆-Spirocyclus, der durch eine gegebenenfalls durch ein oder zwei Sauerstoff- oder Schwefelatome unterbrochene Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind, stehen besonders bevorzugt für einen C₃-C₆-Spirocyclus, bei dem zwei Substituenten gemeinsam für einen gegebenenfalls durch C₁-C₃-Alkyl, C₁-C₃-Alkoxy, Fluor, Chlor oder Brom substituierten gesättigten oder ungesättigten 5- bis 8-gliedrigen Cyclus stehen, der durch Sauerstoff oder Schwefel unterbrochen sein kann.
- A: steht ganz besonders bevorzugt für Wasserstoff, gegebenenfalls durch Fluor und/oder Chlor substituiertes C₁-C₈-Alkyl, C₃-C₄-Alkenyl, C₁-C₆-Alkoxy-C₁-C₄-alkyl, C₁-C₄-Polyalkoxy-C₁-C₄-alkyl, C₁-C₆-Alkylthio-C₁-C₄-alkyl, gegebenenfalls durch Fluor, Chlor, Methyl, Ethyl, Methoxy oder Ethoxy substituiertes Cycloalkyl mit 3 bis 6 Ringatomen, das durch 1 bis 2 Sauerstoff- und/oder Schwefelatomen unterbrochen sein kann oder für jeweils gegebenenfalls durch Fluor, Chlor, Brom, Methyl, Ethyl, Propyl, iso-Propyl, Methoxy, Ethoxy, Trifluormethyl und/oder Nitro substituiertes Phenyl, Furanyl, Pyridyl, Imidazolyl, Pyrazolyl, Triazolyl, Indolyl, Thiazolyl, Thienyl oder Benzyl.
- B: steht ganz besonders bevorzugt für Wasserstoff, C₁-C₈-Alkyl oder C₁-C₄-Alkoxyalkyl oder
- A, B: und das Kohlenstoffatom an das sie gebunden sind, stehen ganz besonders bevorzugt für einen gesättigten oder ungesättigten, gegebenenfalls durch Sauerstoff oder Schwefel unterbrochenen C₃-C₈-Spirocyclus, der gegebenenfalls einfach oder mehrfach durch Methyl, Ethyl, Propyl, Isopropyl, Butyl, iso-Butyl, sec.-Butyl, tert-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert ist oder
- A, B: und das Kohlenstoffatom, an das sie gebunden sind, stehen ganz besonders bevorzugt für einen C₃-C₆-Spirocyclus, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind, stehen ganz besonders bevorzugt für einen C₃-C₆-Spirocyclus, bei dem zwei Substituenten gemeinsam für einen gesättigten oder ungesättigten fünf- oder sechsgliedrigen Cyclus stehen, der durch Sauerstoff oder Schwefel unterbrochen sein kann.
- X: steht bevorzugt für Halogen oder C₁-C₆-Alkyl.
- X: steht besonders bevorzugt für Fluor, Chlor, Brom oder C₁-C₄-Alkyl.
- X: steht ganz besonders bevorzugt für Chlor, Brom, Methyl, Ethyl, Propyl oder iso-Propyl.
- Y: steht bevorzugt für Halogen oder C₁-C₆-Alkyl.
- Y: steht besonders bevorzugt für Fluor, Chlor, Brom oder C₁-C₄-Alkyl.
- Y: steht ganz besonders bevorzugt für Chlor, Brom, Methyl, Ethyl, Propyl oder iso-Propyl.

Dabei gilt, daß X und Y nicht gleichzeitig für Alkyl und nicht gleichzeitig für Halogen stehen.
- G: steht bevorzugt für Wasserstoff (a) oder für eine der Gruppen 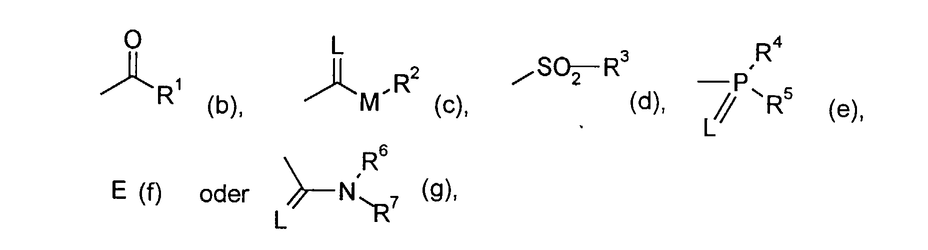 in welchen
E für ein Metallionäquivalent oder ein Ammoniumion steht und
L und M jeweils für Sauerstoff oder Schwefel stehen.
- R¹: steht bevorzugt jeweils für gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₁-C₈-alkyl oder gegebenenfalls durch Halogen oder C₁-C₆-Alkyl substituiertes Cycloalkyl mit 3 bis 8 Ringatomen, das durch mindestens ein Sauerstoff- und/oder Schwefelatom unterbrochen sein kann,
für gegebenenfalls durch Halogen, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl,
für gegebenenfalls durch Halogen und/oder C₁-C₆-Alkyl substituiertes 5- oder 6-gliedriges Hetaryl,
für gegebenenfalls durch Halogen und/oder C₁-C₆-Alkyl substituiertes Phenoxy-C₁-C₆-alkyl oder
für gegebenenfalls durch Halogen, Amino und/oder C₁-C₆-Alkyl substituiertes 5- oder 6-gliedriges Hetaryloxy-C₁-C₆-alkyl.
- R²: steht bevorzugt für jeweils gegebenenfalls durch Halogen substituiertes C₁-C₂₀-Alkyl, C₃-C₂₀-Alkenyl, C₁-C₈-Alkoxy-C₁-C₈-alkyl, C₁-C₈-Polyalkoxy-C₁-C₈-alkyl,
für gegebenenfalls durch Halogen, C₁-C₄-Alkyl und/oder C₁-C₄-Alkoxy substituiertes C₃-C₈-Cycloalkyl, oder
für jeweils gegebenenfalls durch Halogen, Nitro, C₁-C₆-Alkyl, C₁-C₆-Alkoxy und/oder C₁-C₆-Halogenalkyl substituiertes Phenyl oder Benzyl.
- R³, R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₂-C₈-Alkenylthio, C₃-C₇-Cycloalkylthio, für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl und/oder C₁-C₈-Alkoxy substituiertes Phenyl, gegebenenfalls durch Halogen, C₁-C₈-Alkyl, C₁-C₈-Halogenalkyl und/oder C₁-C₈-Alkoxy substituiertes Benzyl oder zusammen für einen gegebenenfalls durch Sauerstoff oder Schwefel unterbrochen C₃-C₆-Alkylenring.
- G: steht besonders bevorzugt für Wasserstoff (a) oder für eine der Gruppen 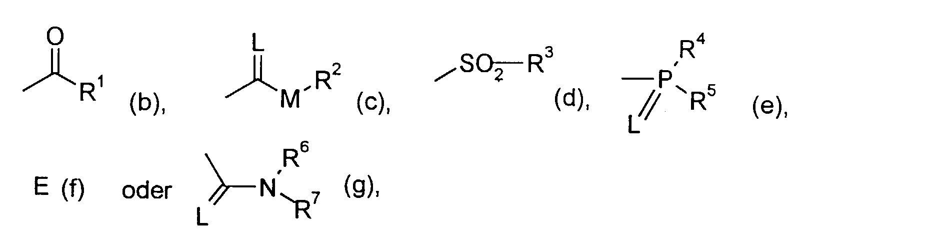 in welchen
E für ein Metallionäquivalent oder ein Ammoniumion steht,
L und M jeweils für Sauerstoff oder Schwefel stehen.
- R¹: steht besonders bevorzugt für jeweils gegebenenfalls durch Fluor und/oder Chlor substituiertes C₁-C₁₆-Alkyl, C₂-C₁₆-Alkenyl, C₁-C₆-Alkoxy-C₁-C₆-alkyl, C₁-C₁₆-Alkylthio-C₁-C₆-alkyl, C₁-C₆-Polyalkoxy-C₁-C₆-alkyl oder gegebenenfalls durch Halogen oder C₁-C₅-Alkyl substituiertes Cycloalkyl mit 3 bis 7 Ringatomen, das durch 1-2 Sauerstoff- und/oder Schwefelatome unterbrochen sein kann,
für gegebenenfalls durch Halogen, Nitro, C₁-C₄-Alkyl, C₁-C₄-Alkoxy; C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl,
für gegebenenfalls durch Fluor, Chlor, Brom und/oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom und/oder C₁-C₄-Alkyl substituiertes Phenoxy-C₁-C₅-alkyl oder
für gegebenenfalls durch Fluor, Chlor, Brom, Amino und/oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Halogen substituiertes C₁-C₁₆-Alkyl, C₃-C₁₆-Alkenyl, C₁-C₆-Alkoxy-C₁-C₆-alkyl, C₁-C₆-Polyalkoxy-C₁-C₆-alkyl,
für gegebenenfalls durch Halogen, C₁-C₃-Alkyl und/oder C₁-C₃-Alkoxy substituiertes C₃-C₇-Cycloalkyl oder
für jeweils gegebenenfalls durch Halogen, Nitro, C₁-C₄-Alkyl, C₁-C₃-Alkoxy und/oder C₁-C₃-Halogenalkyl substituiertes Phenyl oder Benzyl.
- R³, R⁴ und R⁵: stehen unabhängig voneinander besonders bevorzugt für jeweils gegebenenfalls durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₃-C₆-Cycloalkylthio, für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Halogen substituiertes C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Halogen, C₁-C₅-Halogenalkyl, C₁-C₅-Alkyl und/oder C₁-C₅-Alkoxy substituiertes Phenyl, für gegebenenfalls durch Halogen, C₁-C₅-Alkyl, C₁-C₅-Halogenalkyl und/oder C₁-C₅-Alkoxy substituiertes Benzyl oder zusammen für einen gegebenenfalls durch Sauerstoff oder Schwefel unterbrochenen C₃-C₆-Alkylenring.
- G: steht ganz besonders bevorzugt für Wasserstoff (a) oder für eine der Gruppen 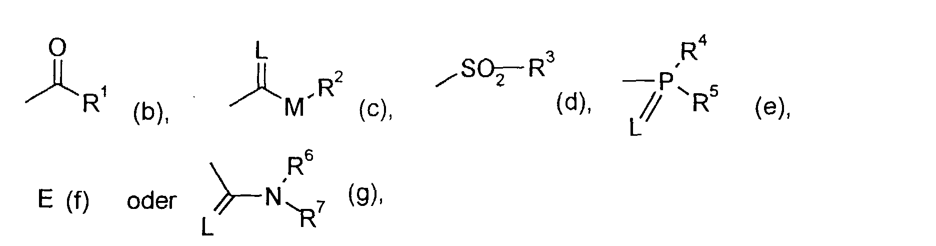 in welcher
E für ein Metallionäquivalent oder ein Ammoniumion steht und
L und M jeweils für Sauerstoff oder Schwefel stehen.
- R¹: steht ganz besonders bevorzugt für jeweils gegebenenfalls durch Fluor und/oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl oder gegebenenfalls durch Fluor, Chlor, Methyl, Ethyl, Propyl, i-Propyl, Butyl, i-Butyl oder tert.-Butyl substituiertes Cycloalkyl mit 3 bis 6 Ringatomen, das durch 1 bis 2 Sauerstoff- und/oder Schwefelatome unterbrochen sein kann,
für gegebenenfalls durch Fluor, Chlor, Brom, Nitro, Methyl, Ethyl, Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl,
für gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl und/oder Ethyl substituiertes Phenoxy-C₁-C₄-alkyl, oder
für gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor und/oder Chlor substituiertes C₁-C₁₄-Alkyl, C₃-C₁₄-Alkenyl, C₁-C₄-Alkoxy-C₁-C₆-alkyl, C₁-C₄-Polyalkoxy-C₁-C₆-alkyl,
für gegebenenfalls durch Fluor, Chlor, Methyl, Ethyl, Propyl, iso-Propyl oder Methoxy substituiertes C₃-C₆-Cycloalkyl,
oder für jeweils gegebenenfalls durch Fluor, Chlor, Nitro, Methyl, Ethyl, Propyl, i-Propyl, Methoxy, Ethoxy, Trifluormethyl substituiertes Phenyl oder Benzyl.
- R³, R⁴ und R⁵: stehen unabhängig voneinander ganz besonders bevorzugt für jeweils gegebenenfalls durch Fluor und/oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio, C₁-C₃-Alkyl substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor, Chlor, Brom substituiertes C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, C₁-C₄-Halogenalkyl, C₁-C₄-Alkyl und/oder C₁-C₄-Alkoxy substituiertes Phenyl, für gegebenenfalls durch Fluor, Chlor, Brom, C₁-C₄-Alkyl, C₁-C₄-Halogenalkyl und/oder C₁-C₄-Alkoxy substituiertes Benzyl, oder zusammen für einen gegebenenfalls durch Sauerstoff oder Schwefel unterbrochenen C₄-C₆-Alkylenring.

In den angegebenen Definitionen können gesättigte oder ungesättigte Alkylreste, auch in Verbindung mit Heteroatomen, wie z.B. Alkoxy oder Alkylthio, soweit möglich jeweils geradkettig oder verzweigt sein.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der allgemeinen Formel (I), in welchen eine Kombination der vorstehend als bevorzugt (vorzugsweise) aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I), in welchen eine Kombination dieser vorstehend als ganz besonders bevorzugt aufgeführten Bedeutungen vorliegt.

Im einzelnen seien außer bei den bei Herstellungsbeispielen genannten Verbindungen die folgenden Verbindungen der Formel (Ia) genannt:

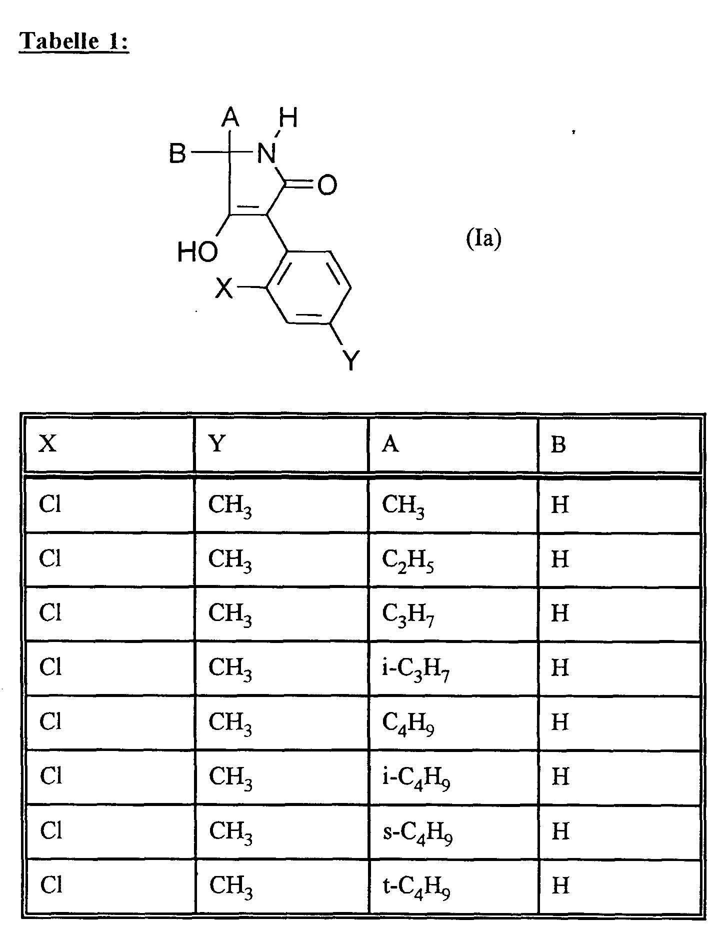

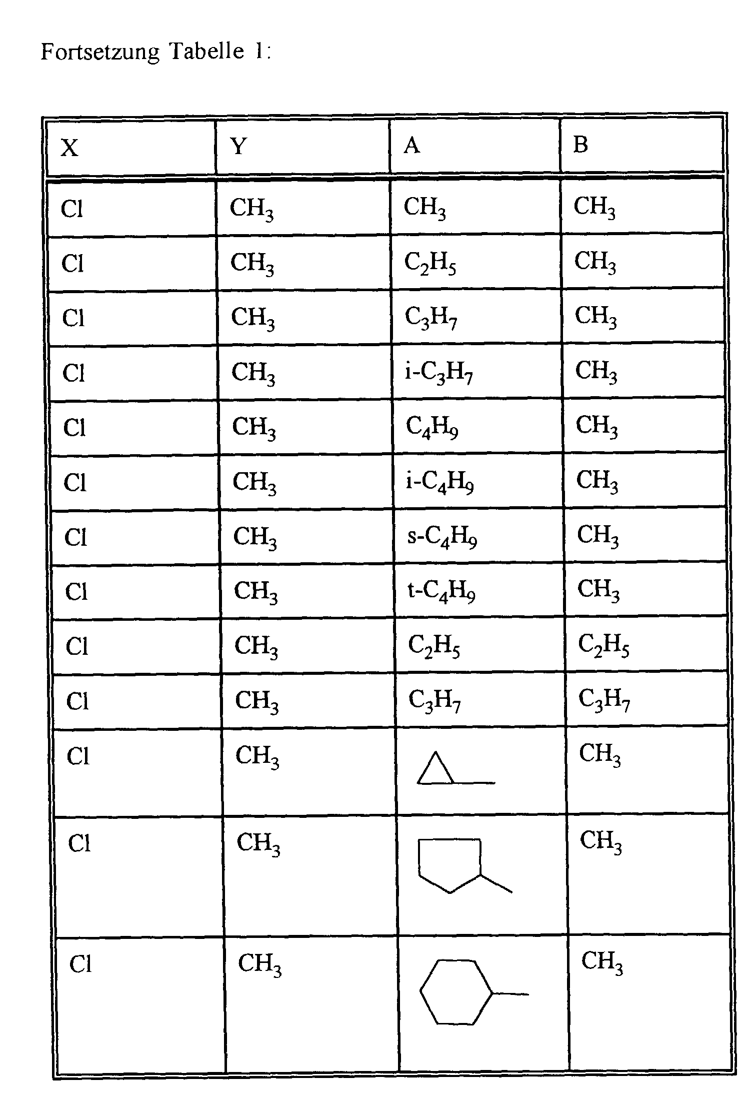

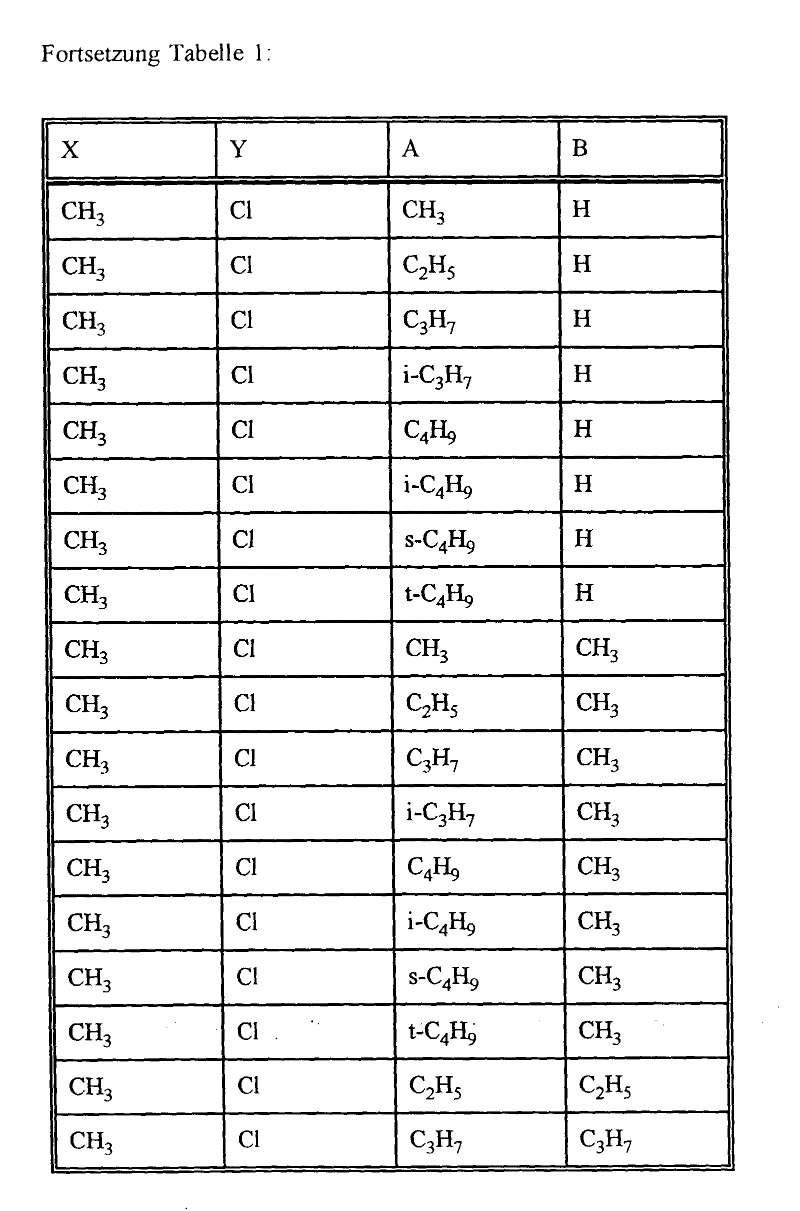

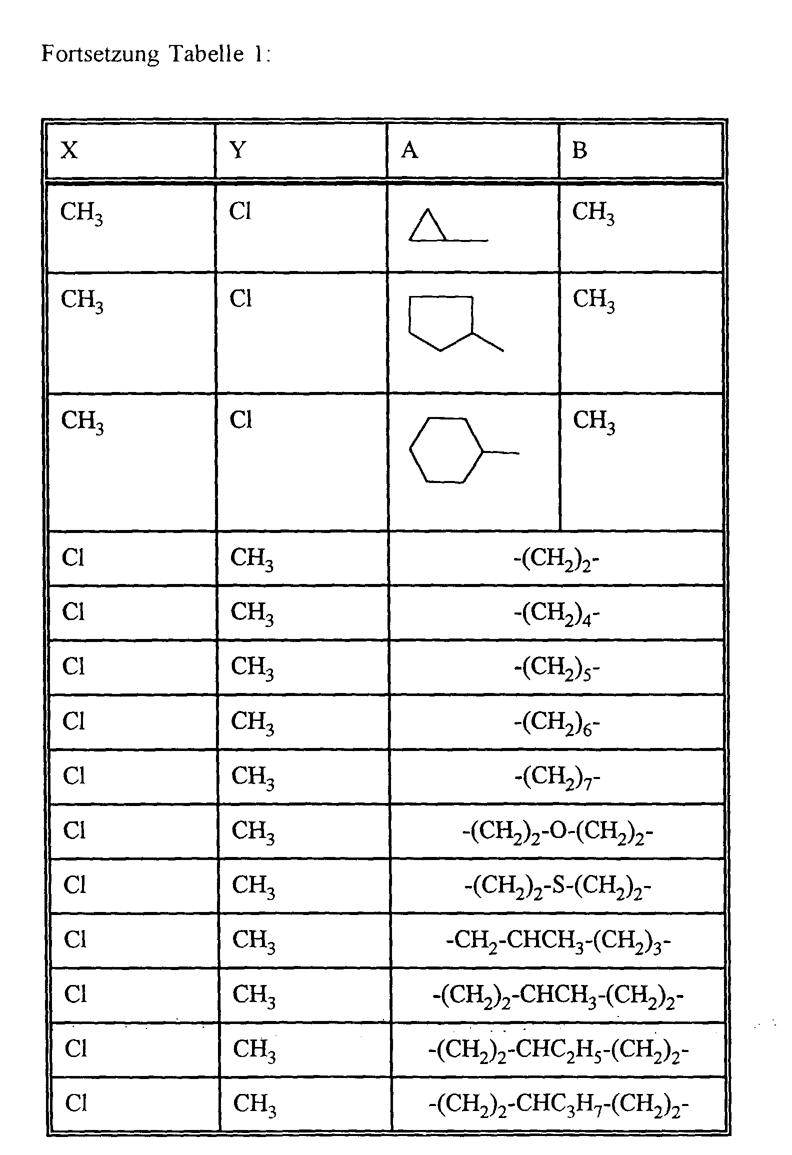

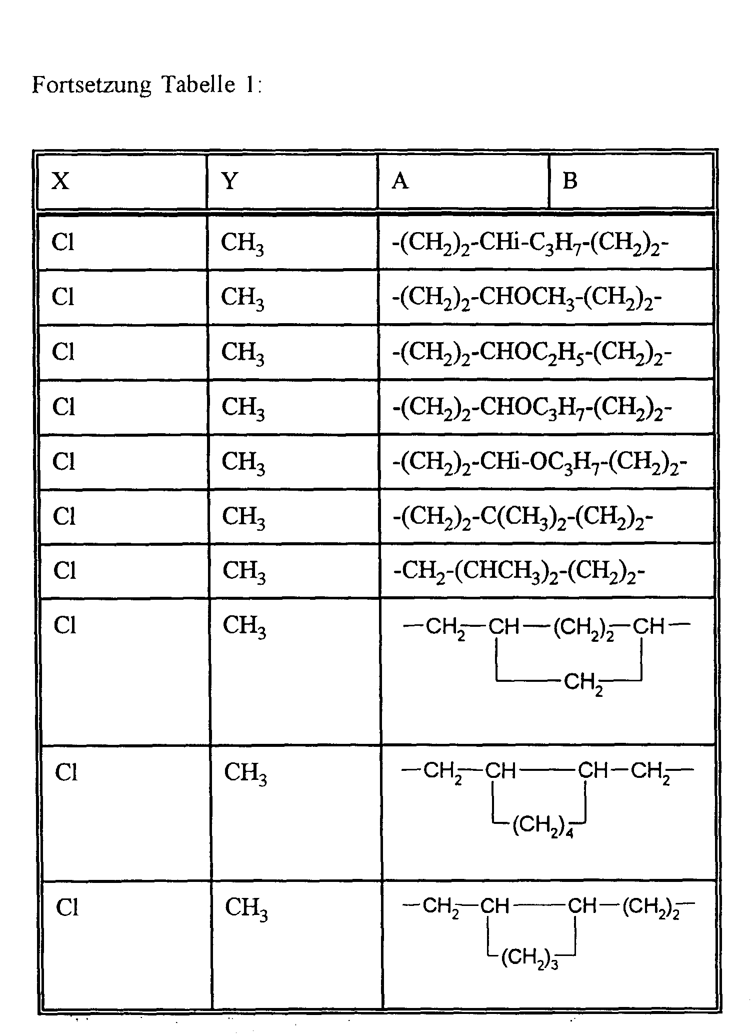

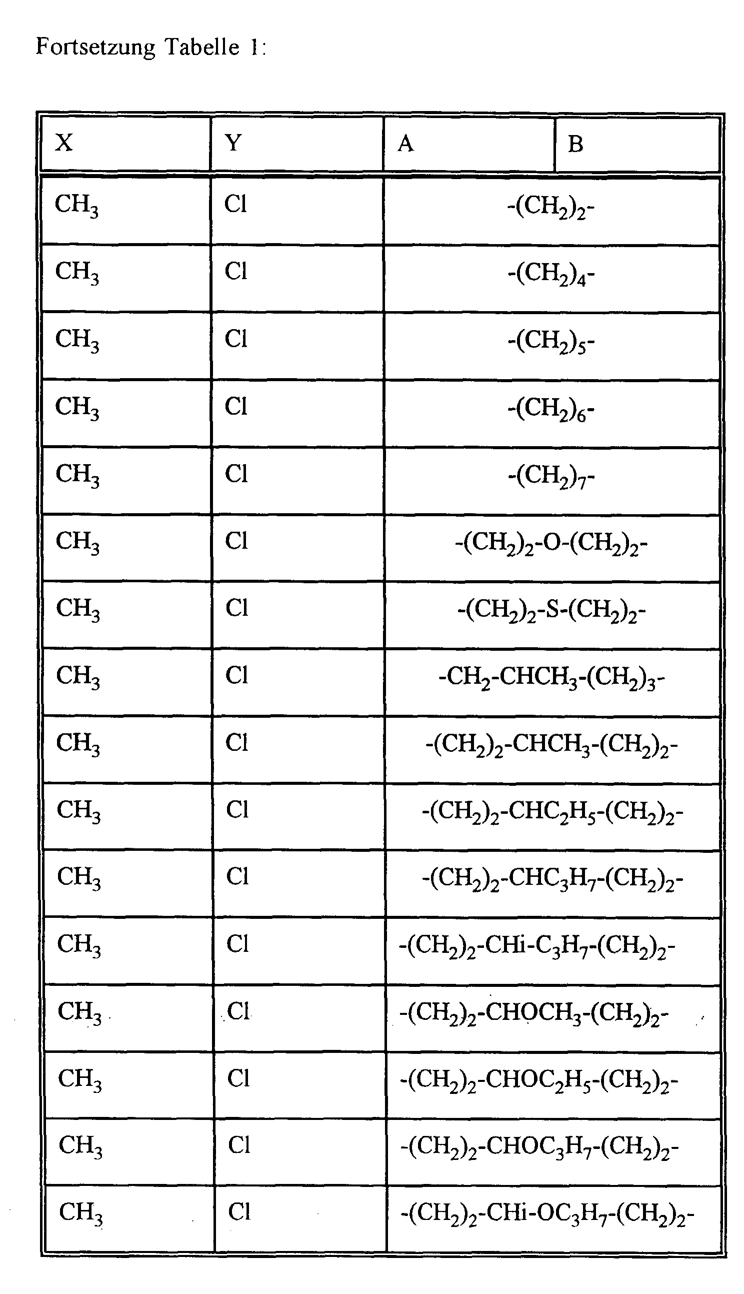

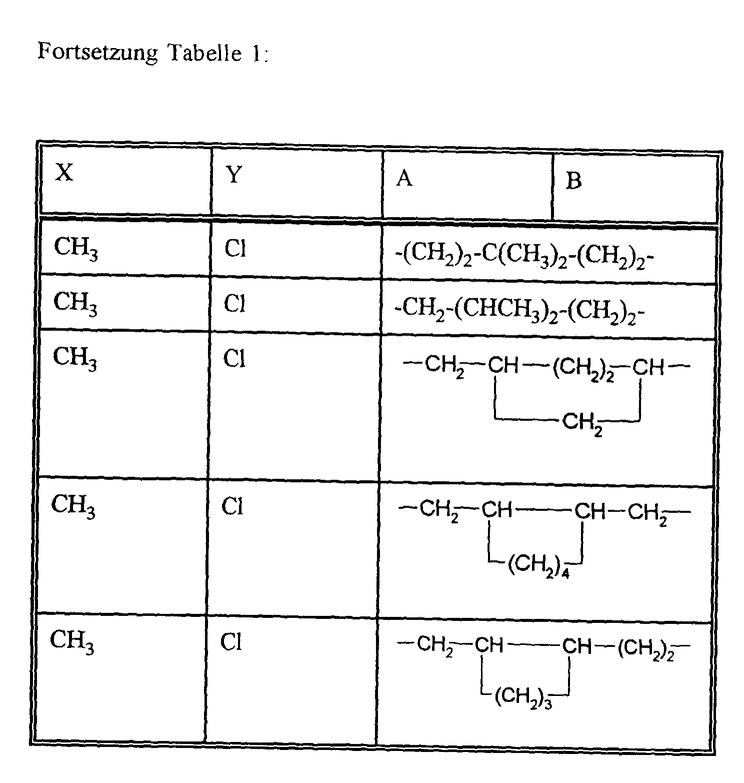

Im einzelnen seien außer den bei den Herstellungsbeispielen genannten Verbindungen die folgenden Verbindungen der Formel (Ib) genannt:

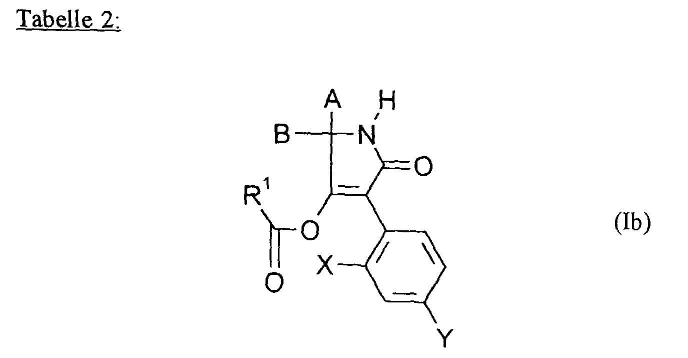

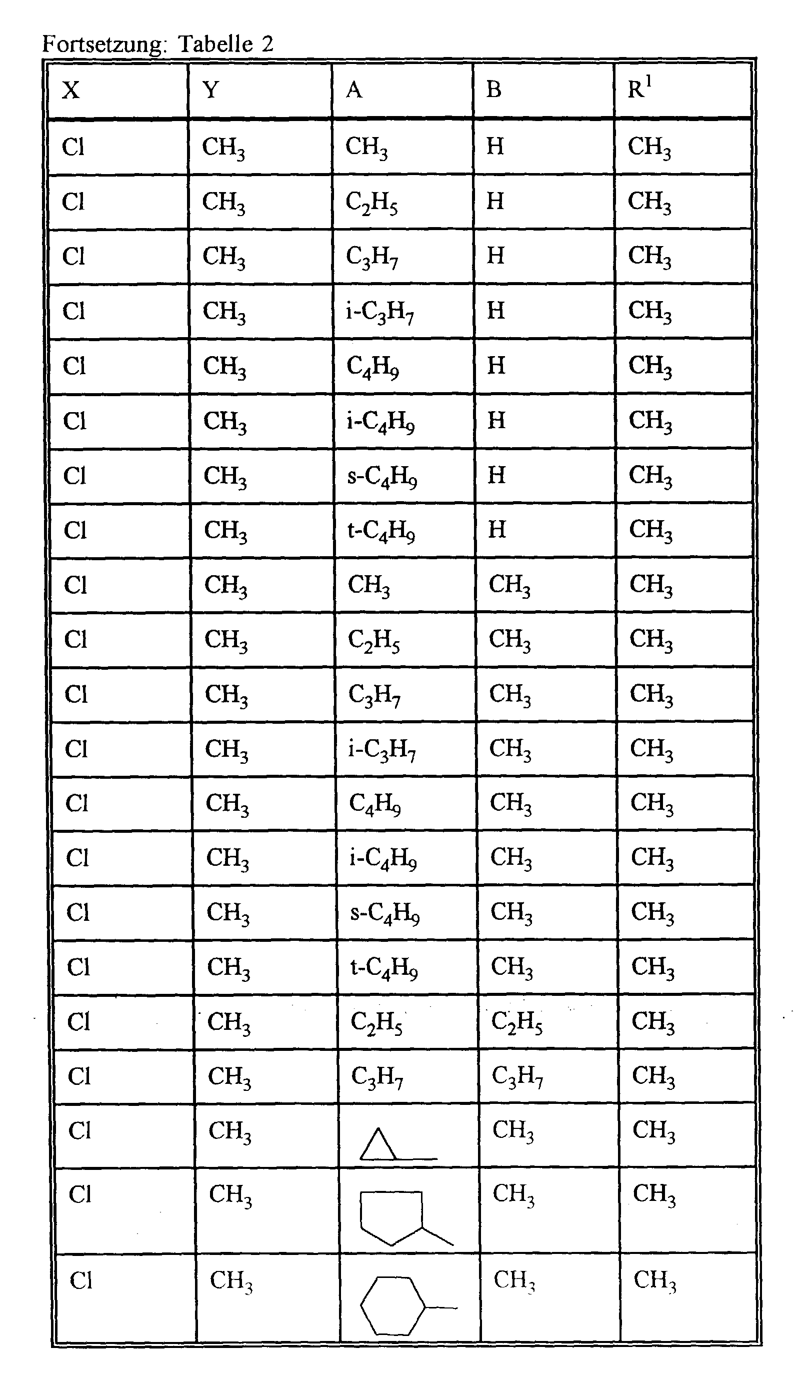

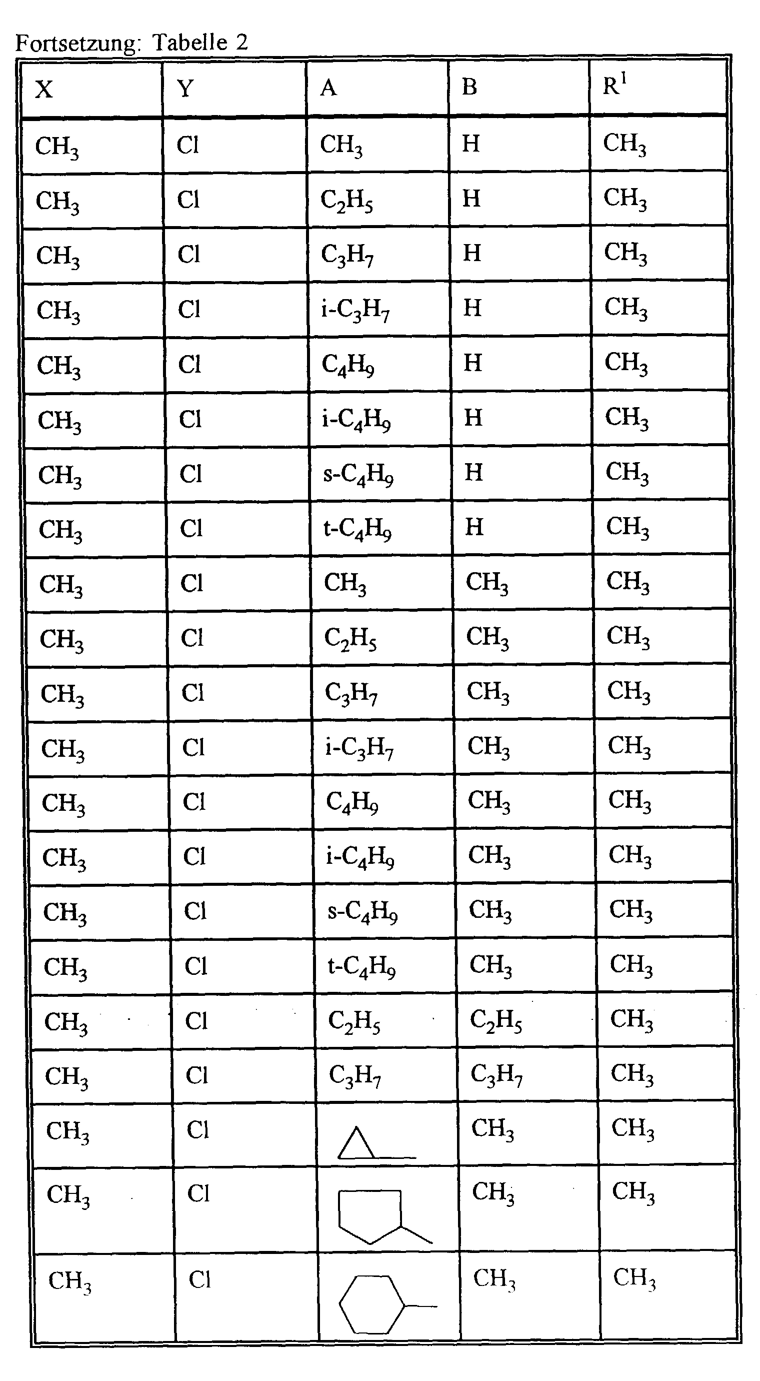

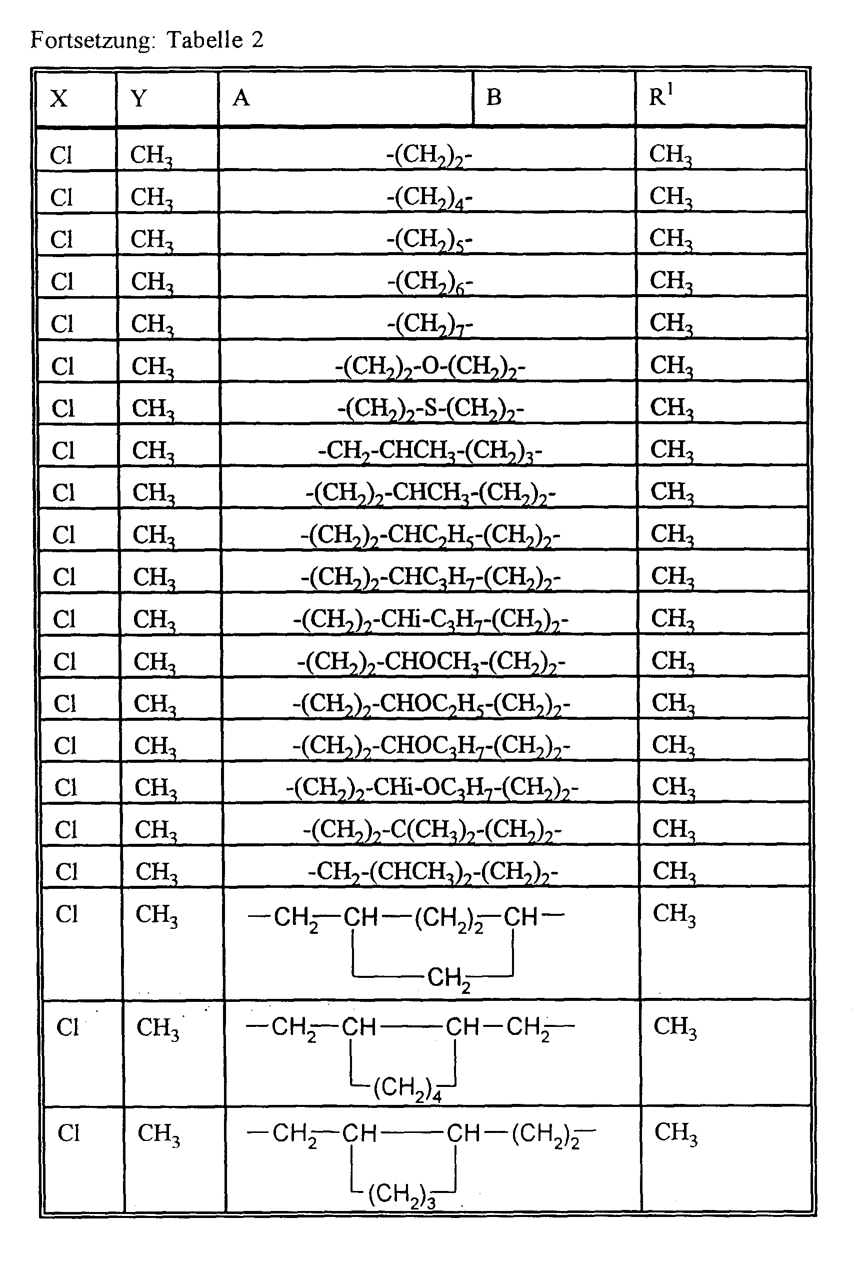

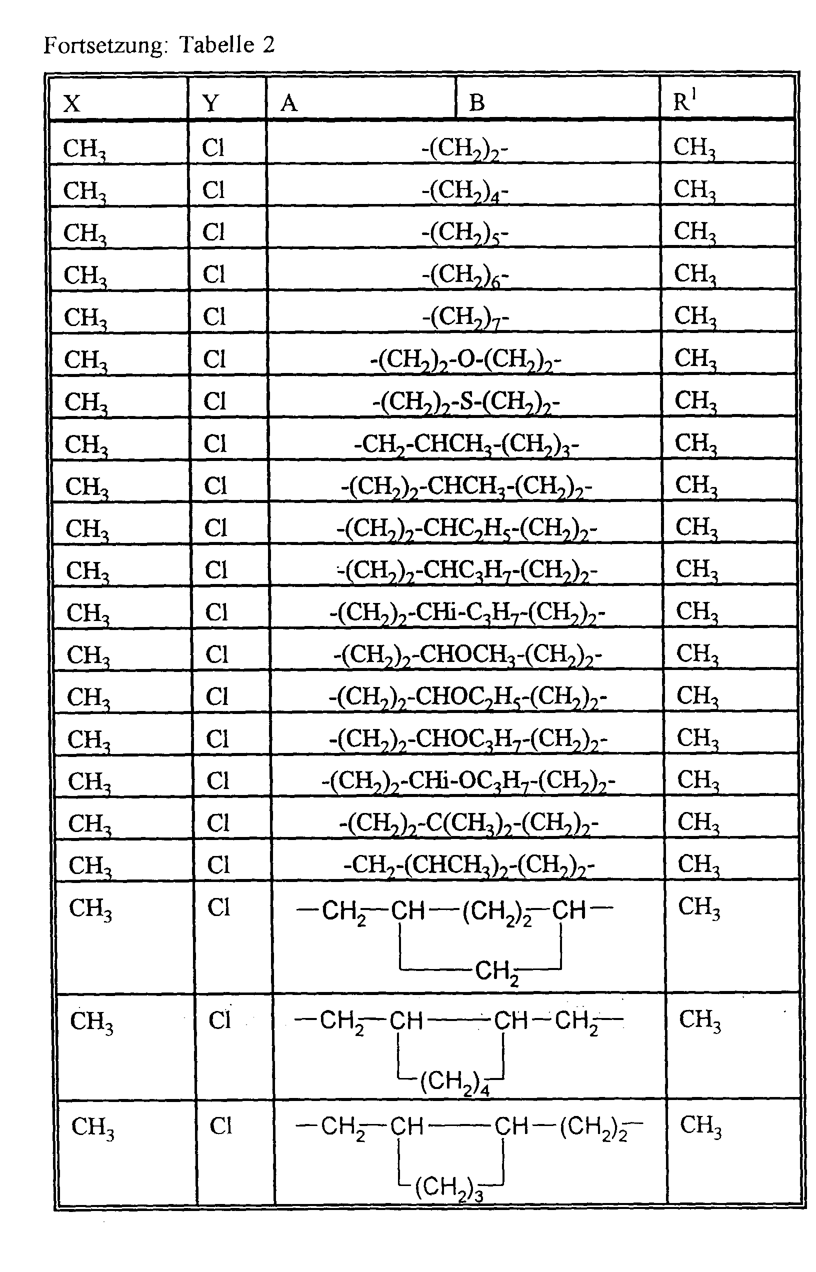

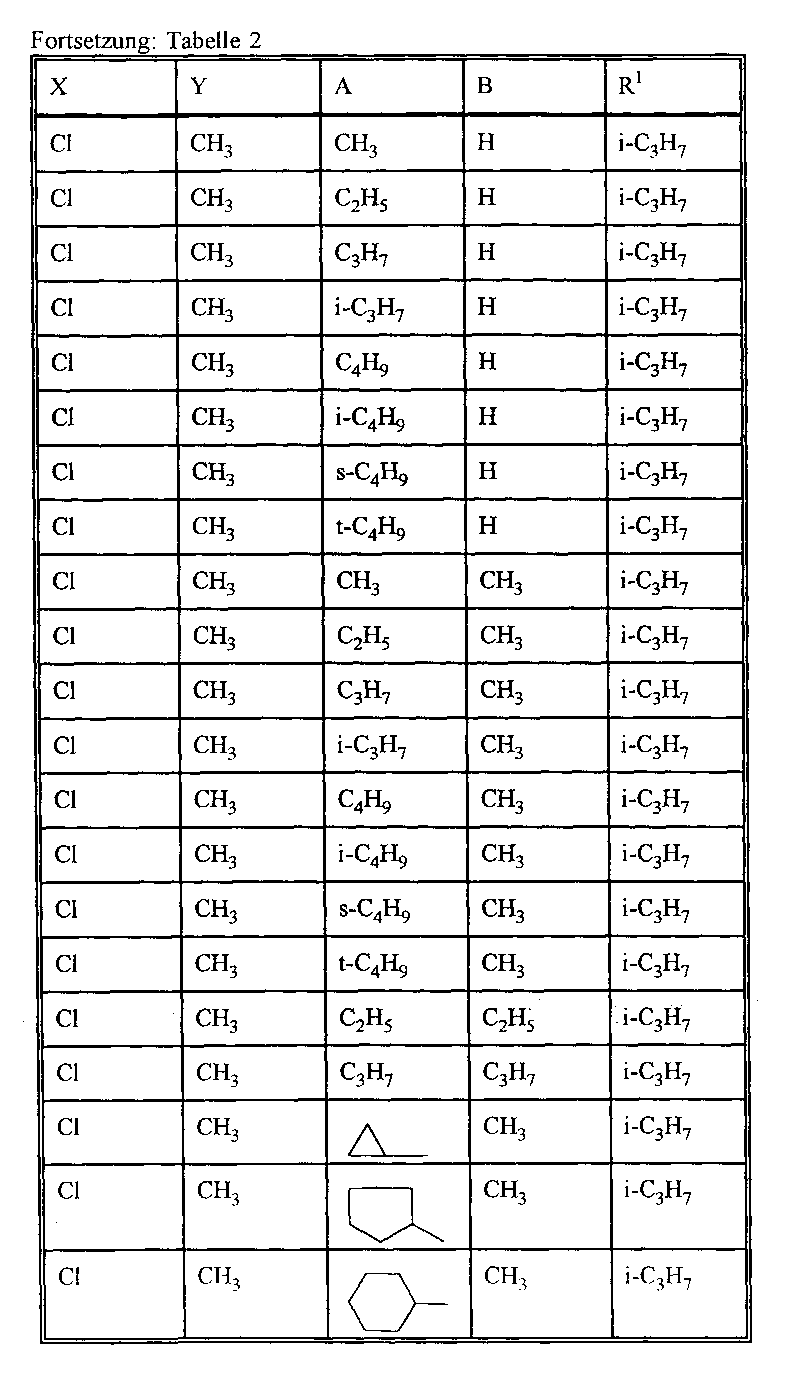

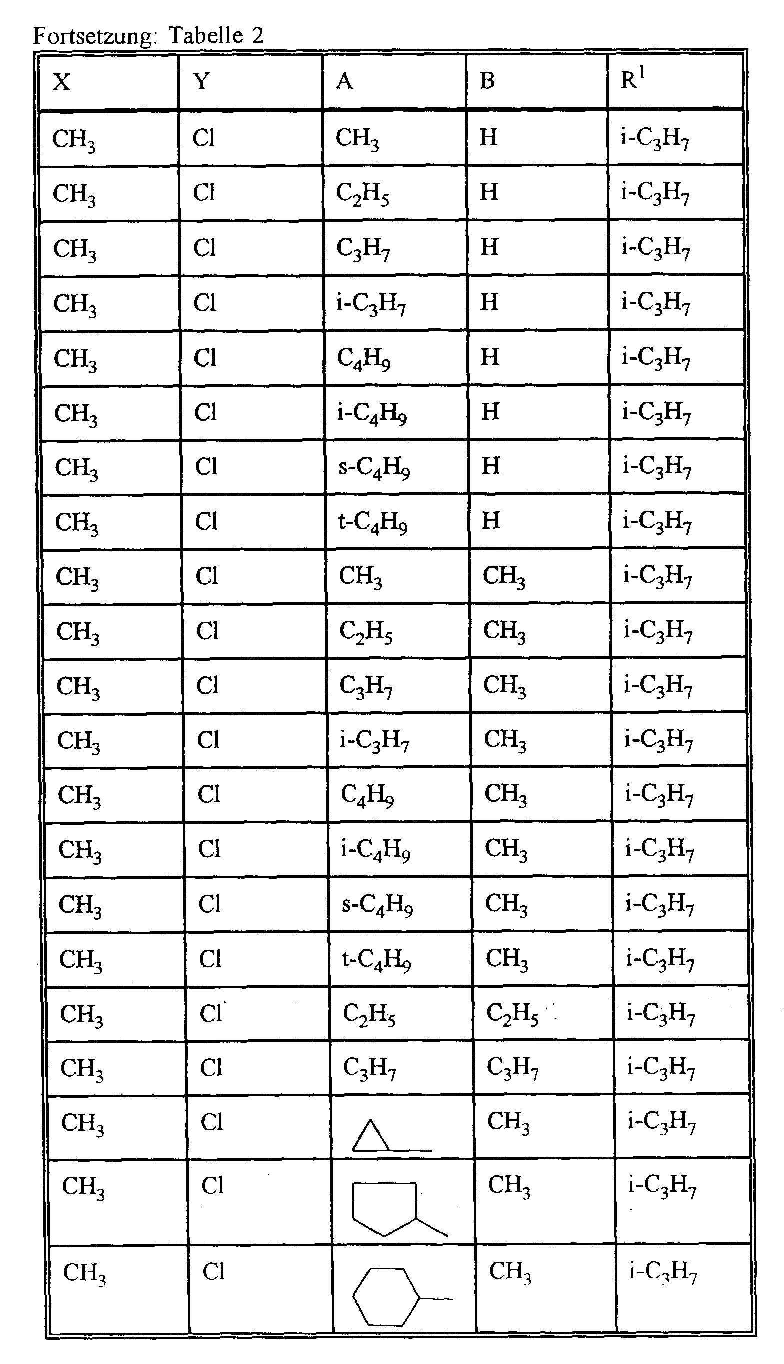

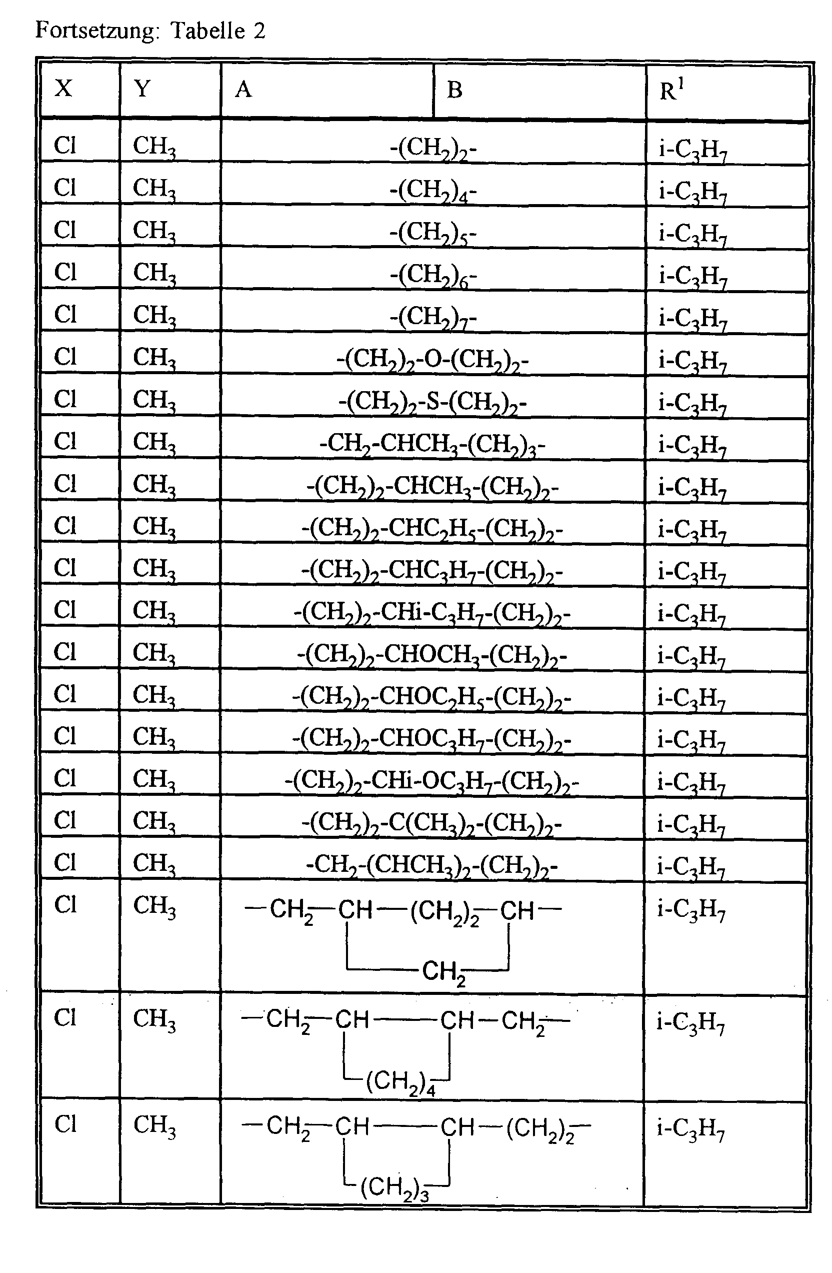

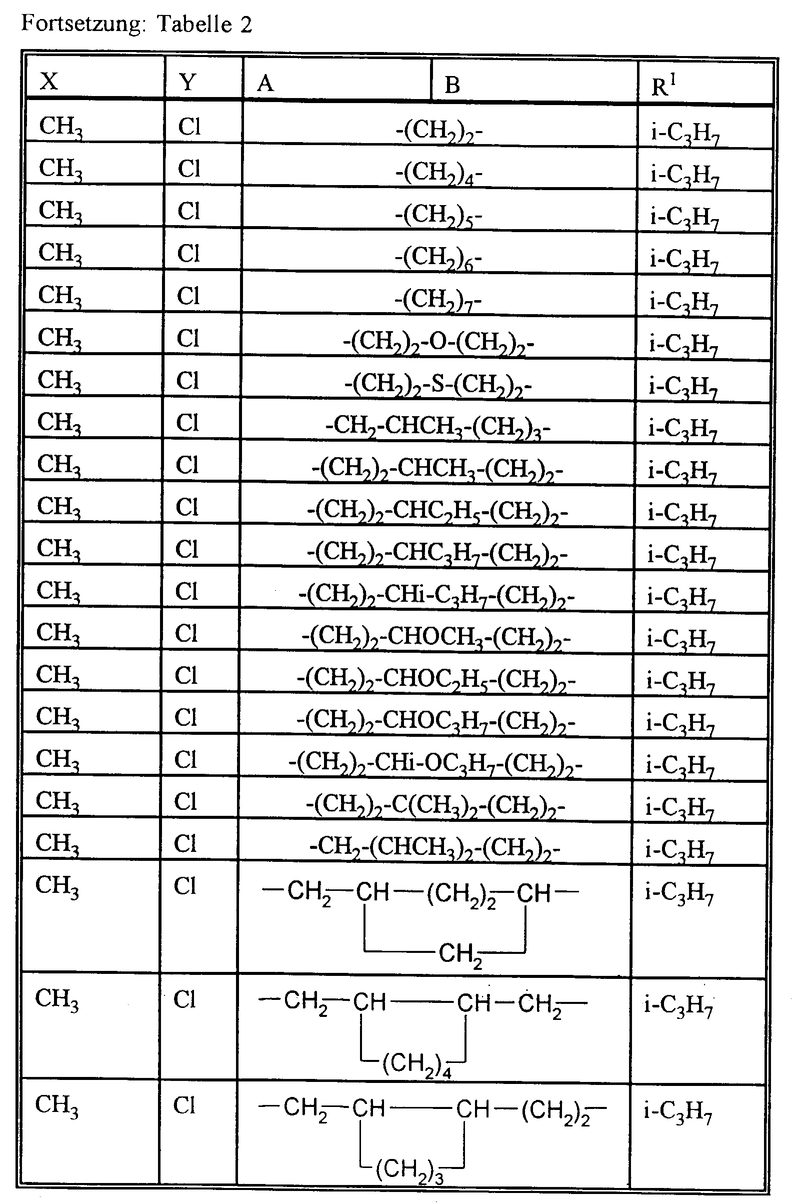

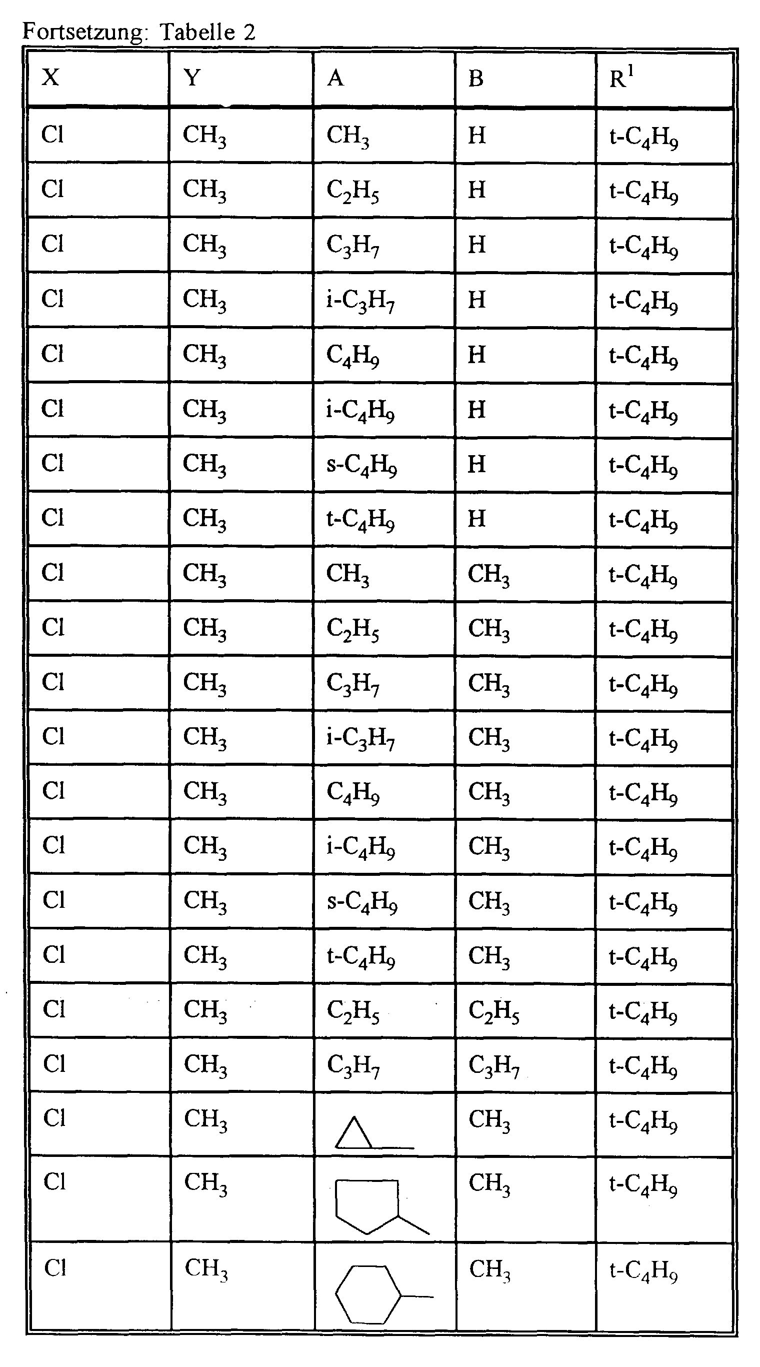

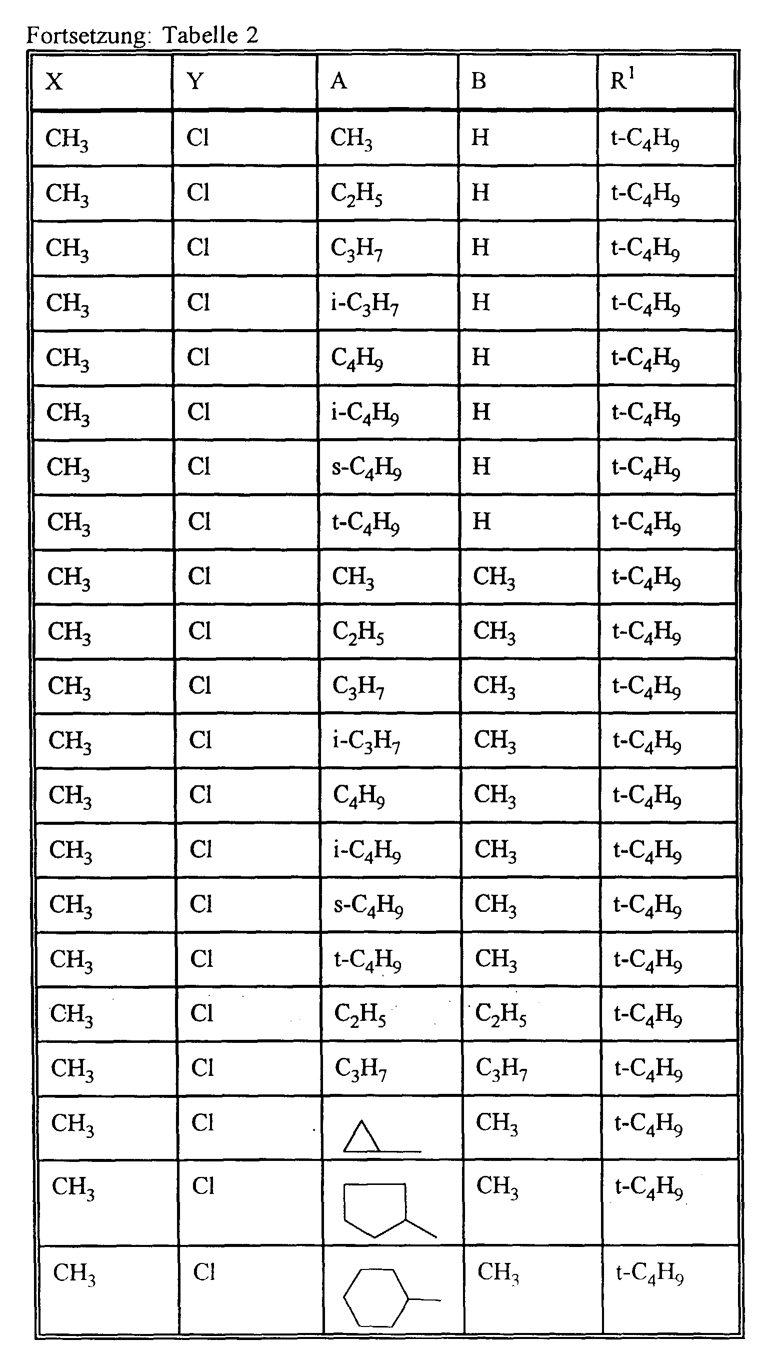

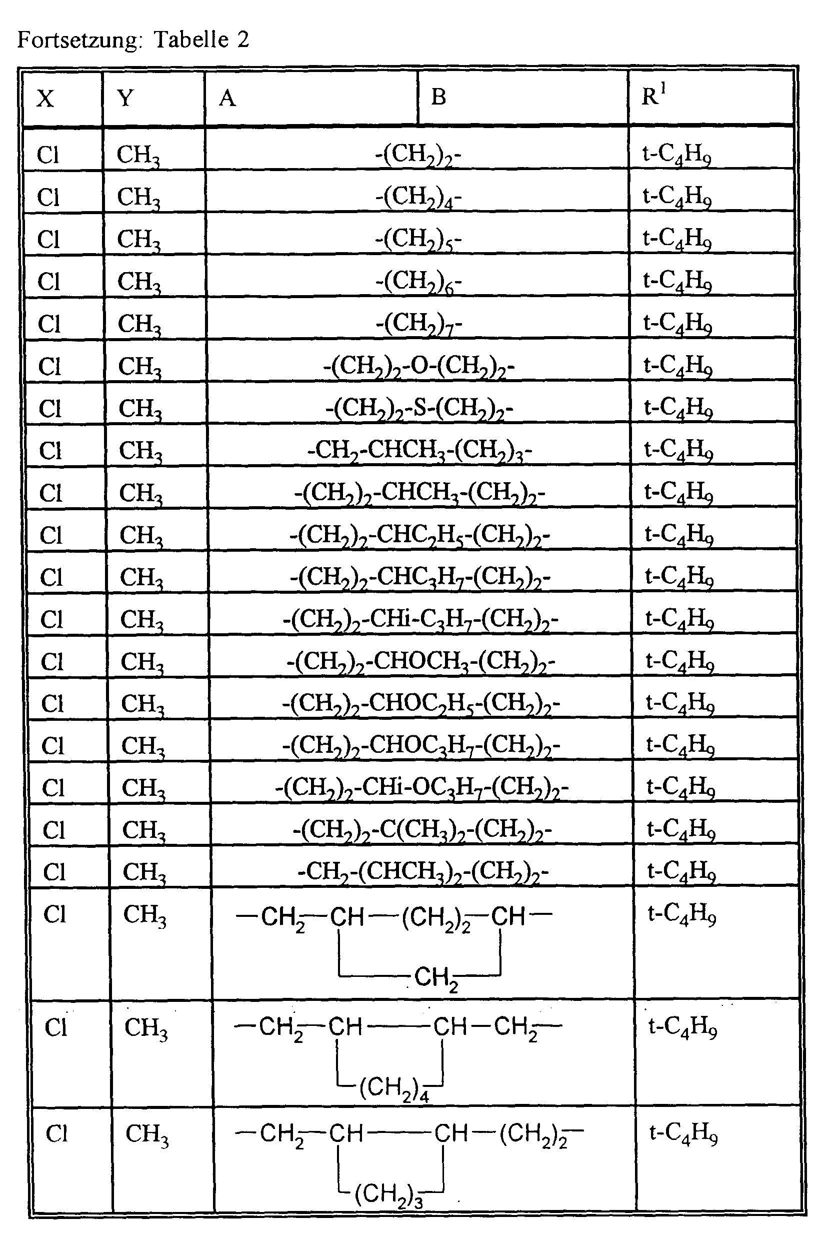

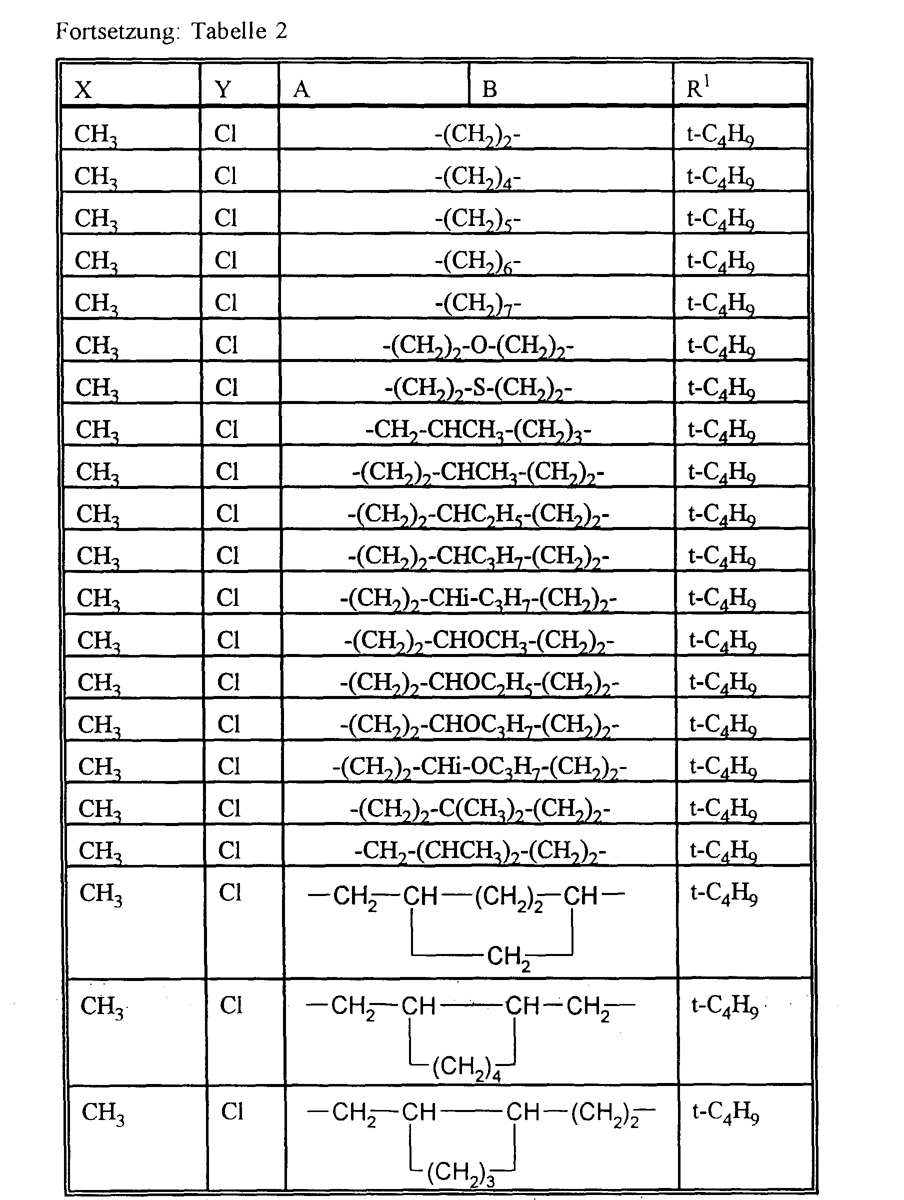

Im einzelnen seien außer den bei den Herstellungsbeispielen genannten Verbindungen die folgenden Verbindungen der Formel (Ic) genannt:

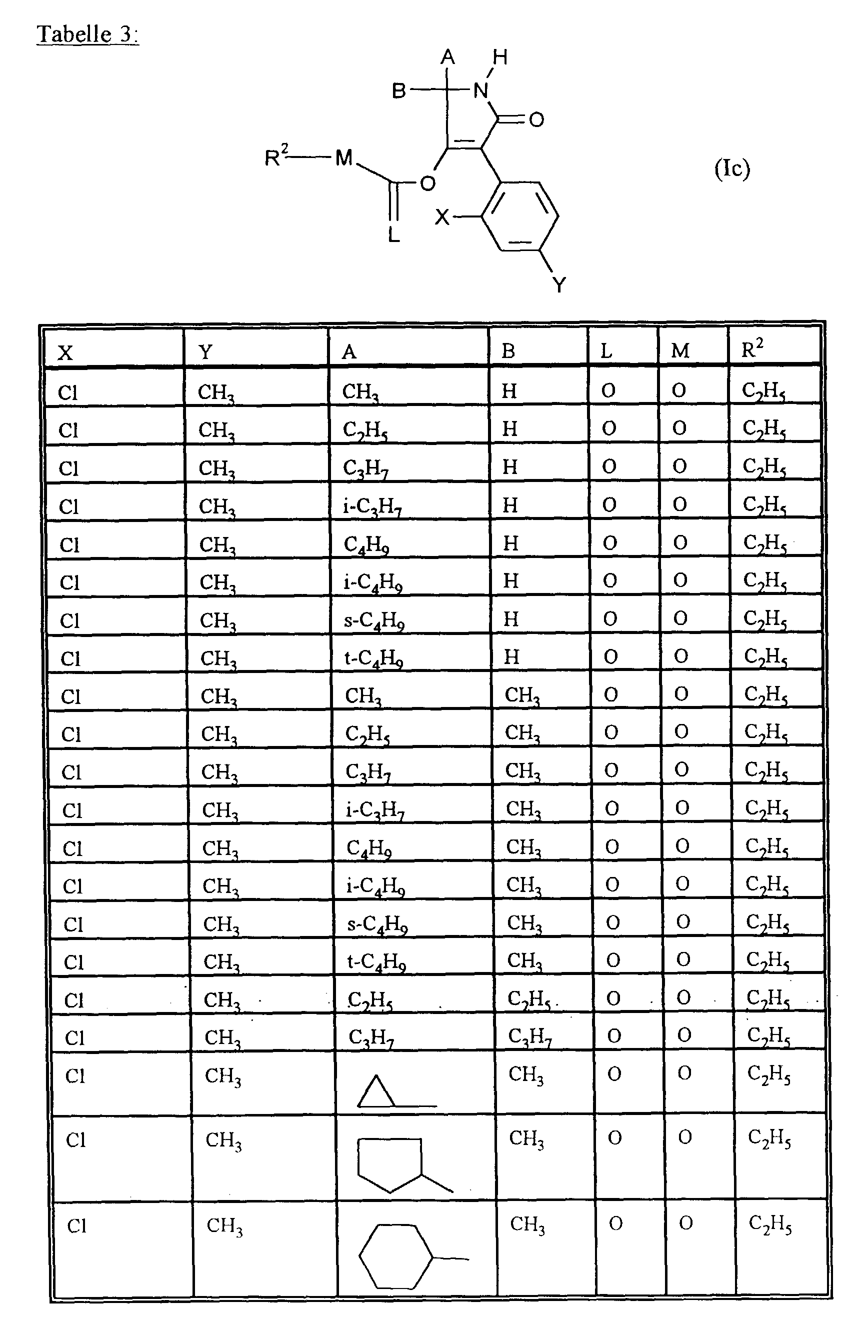

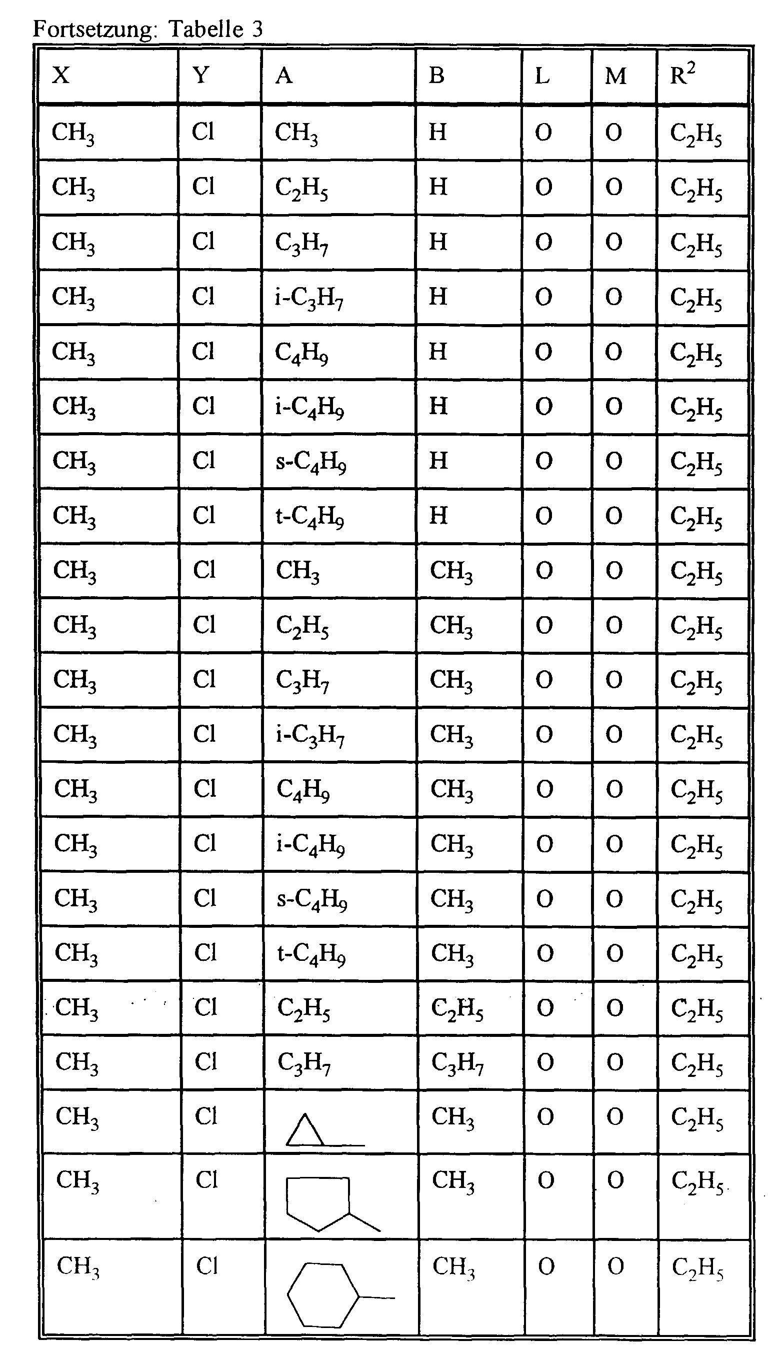

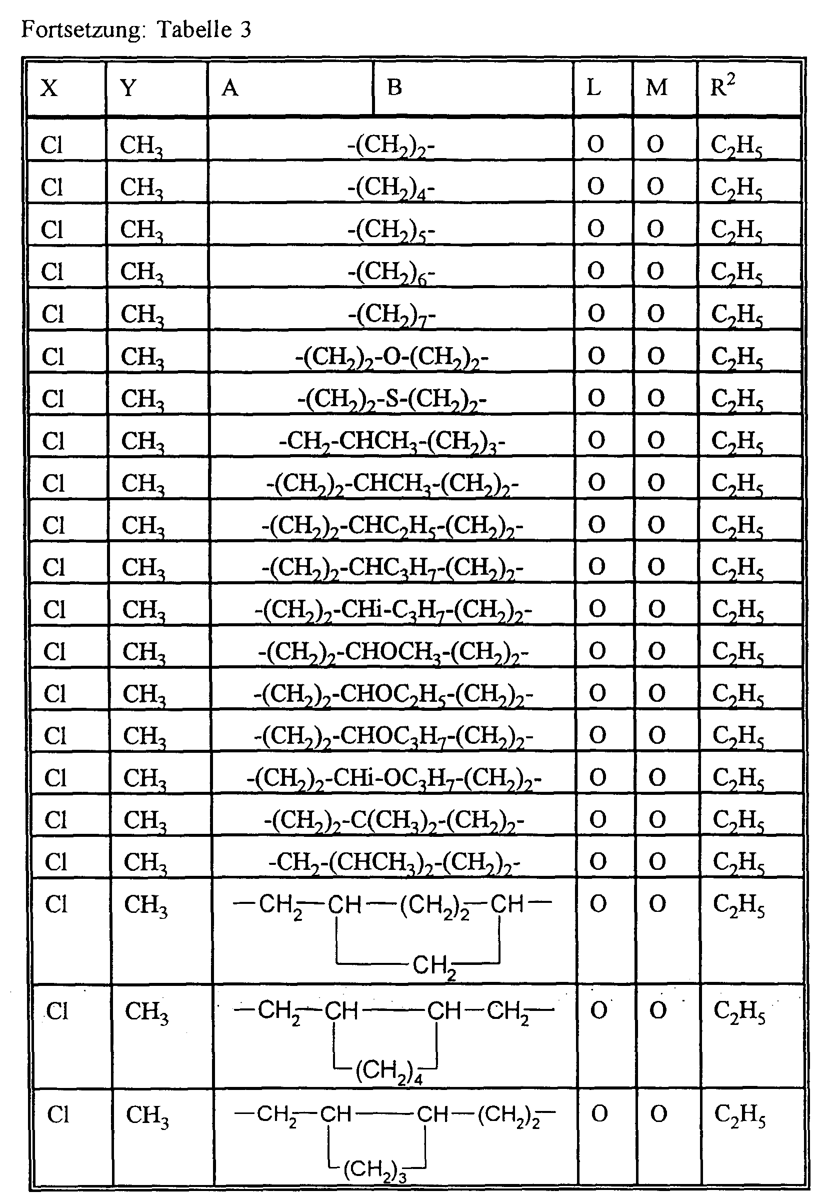

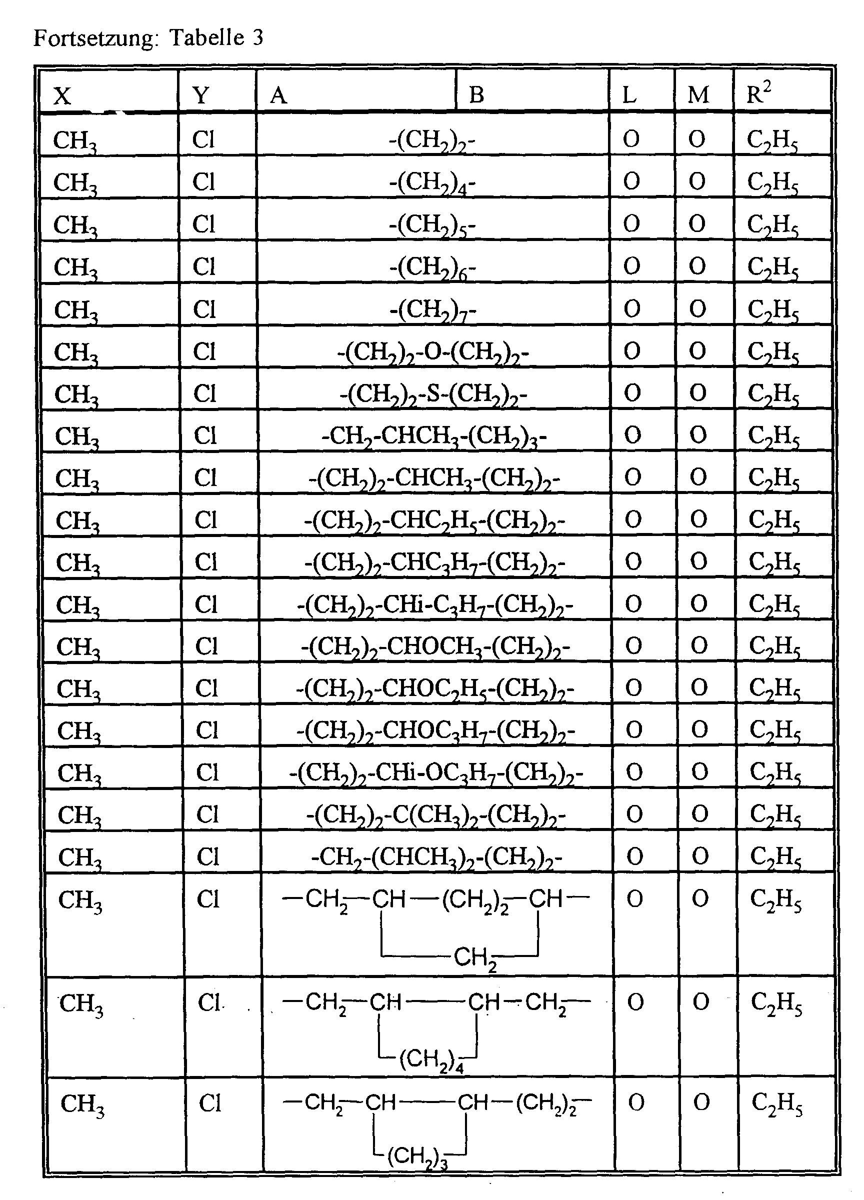

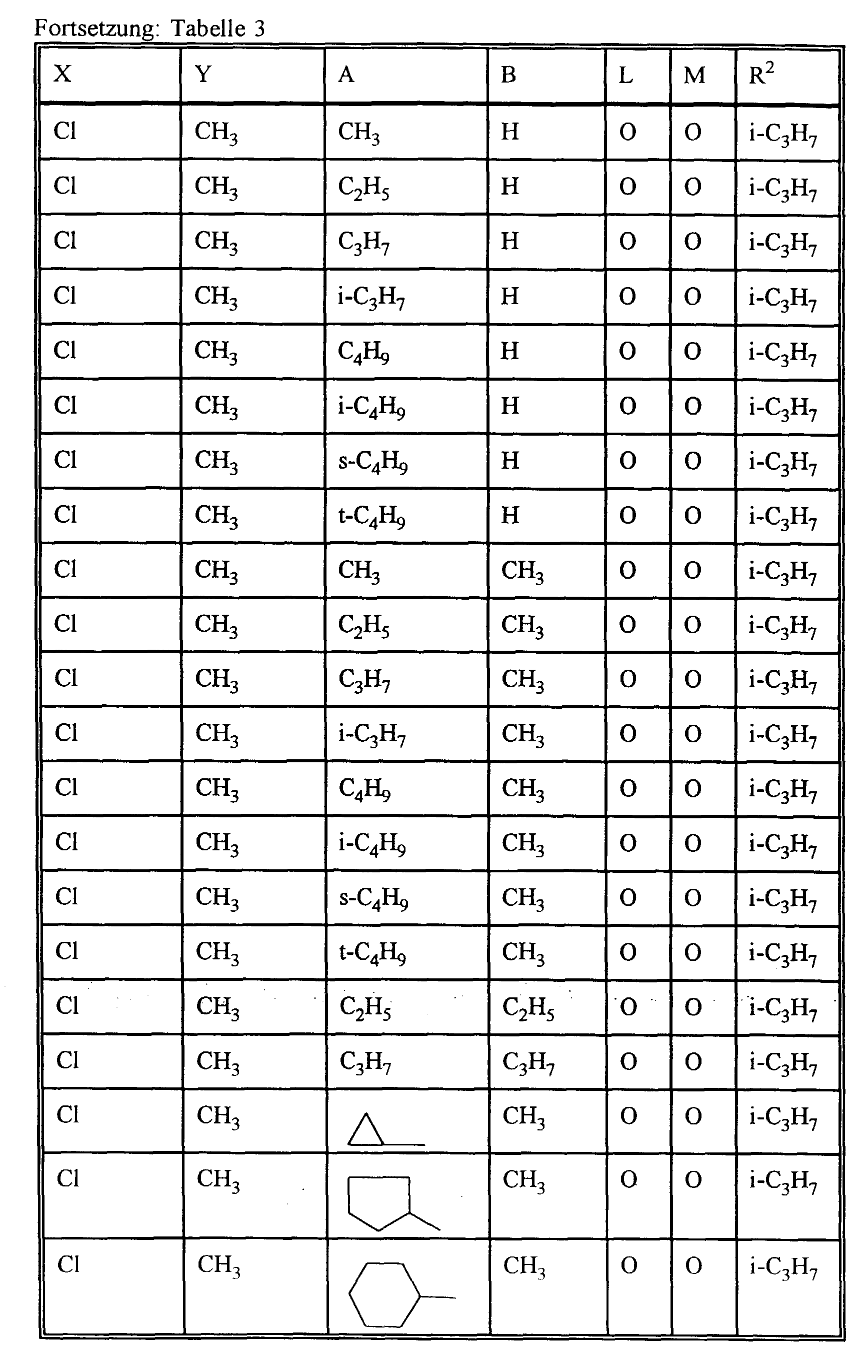

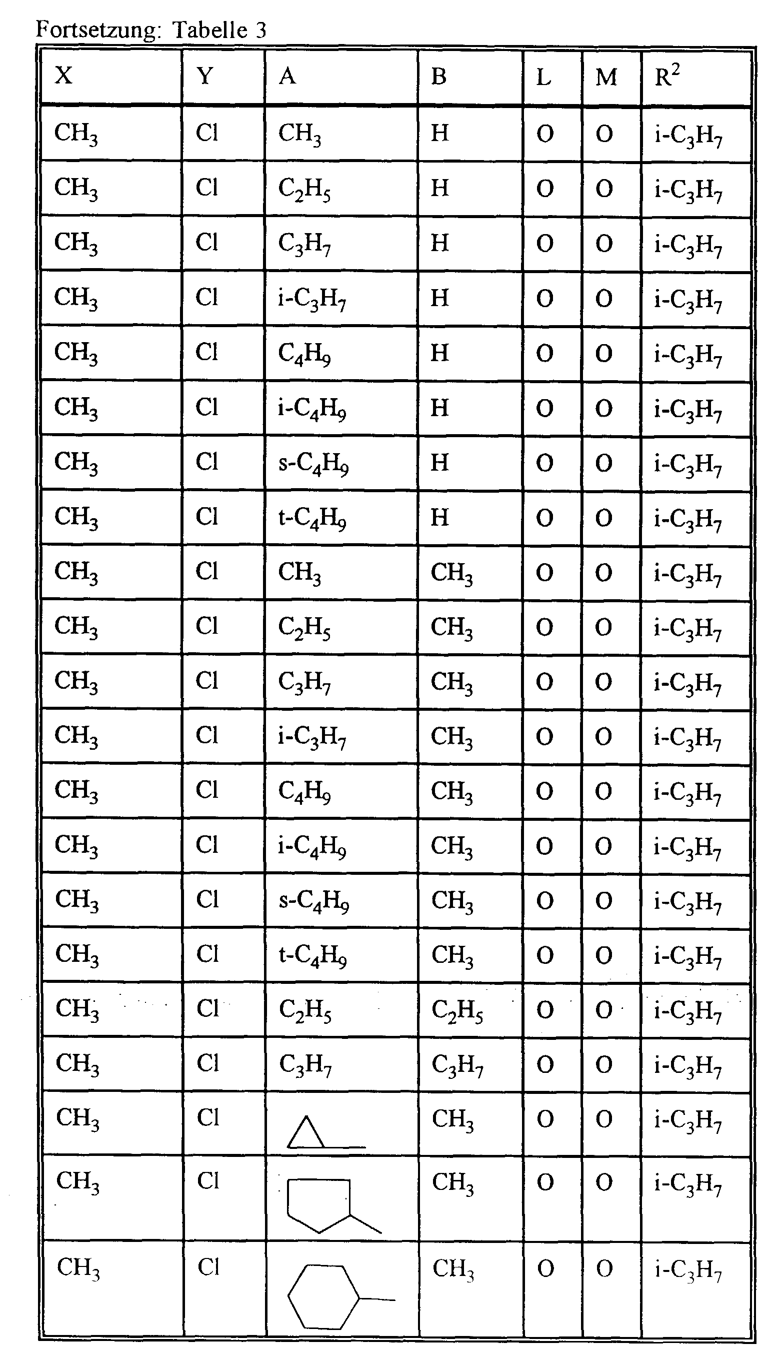

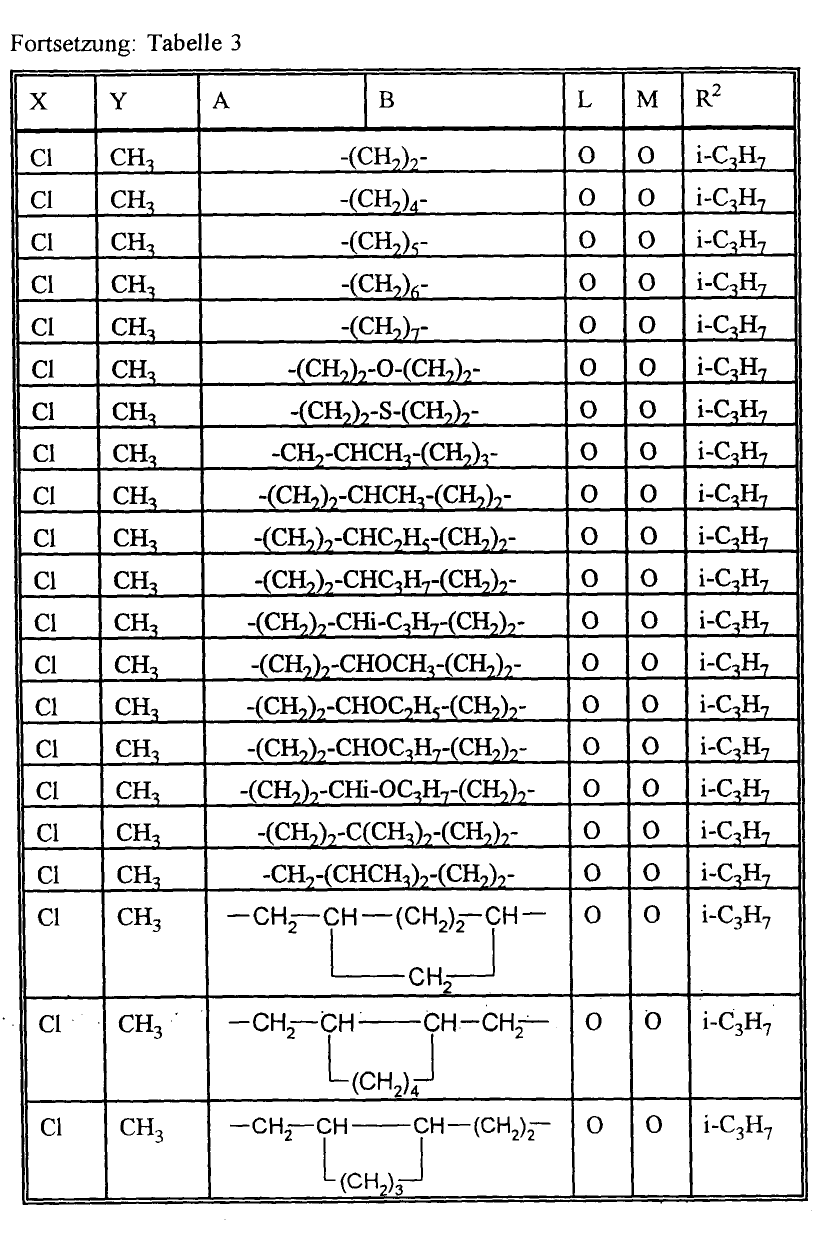

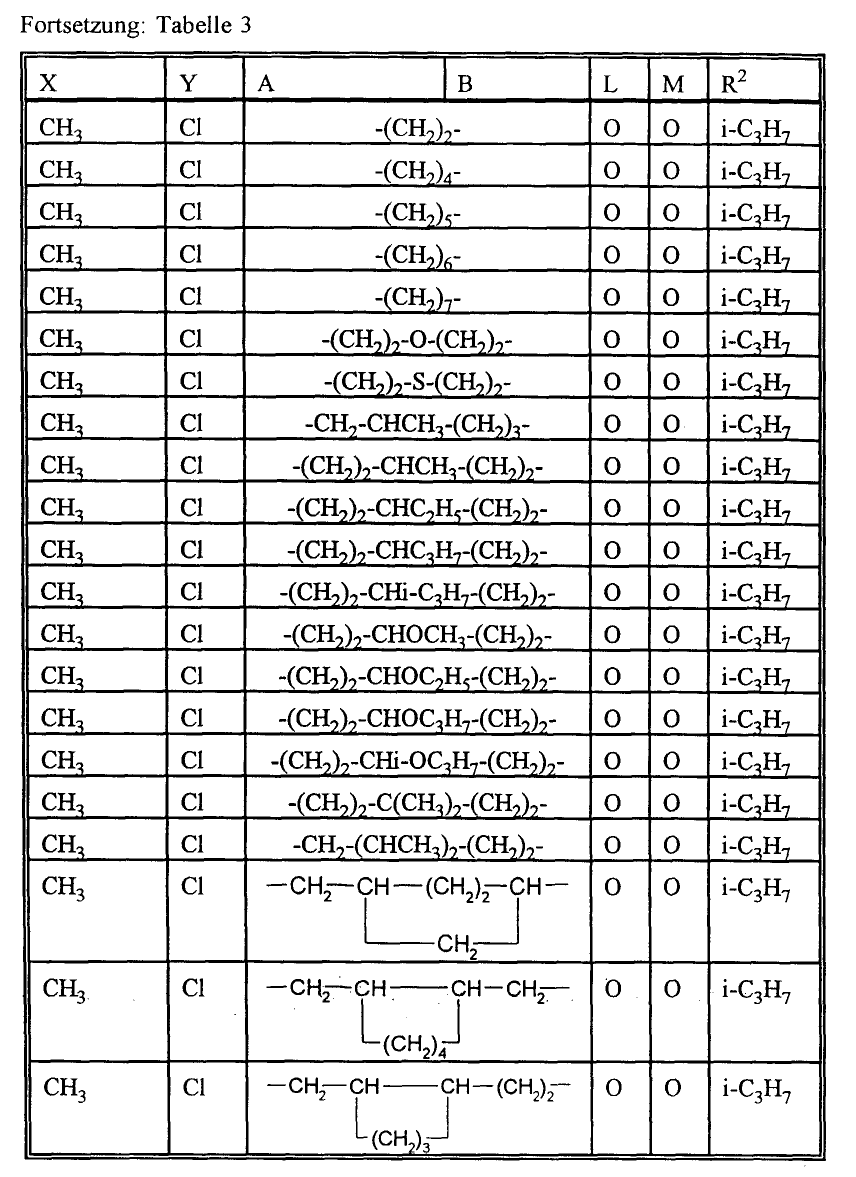

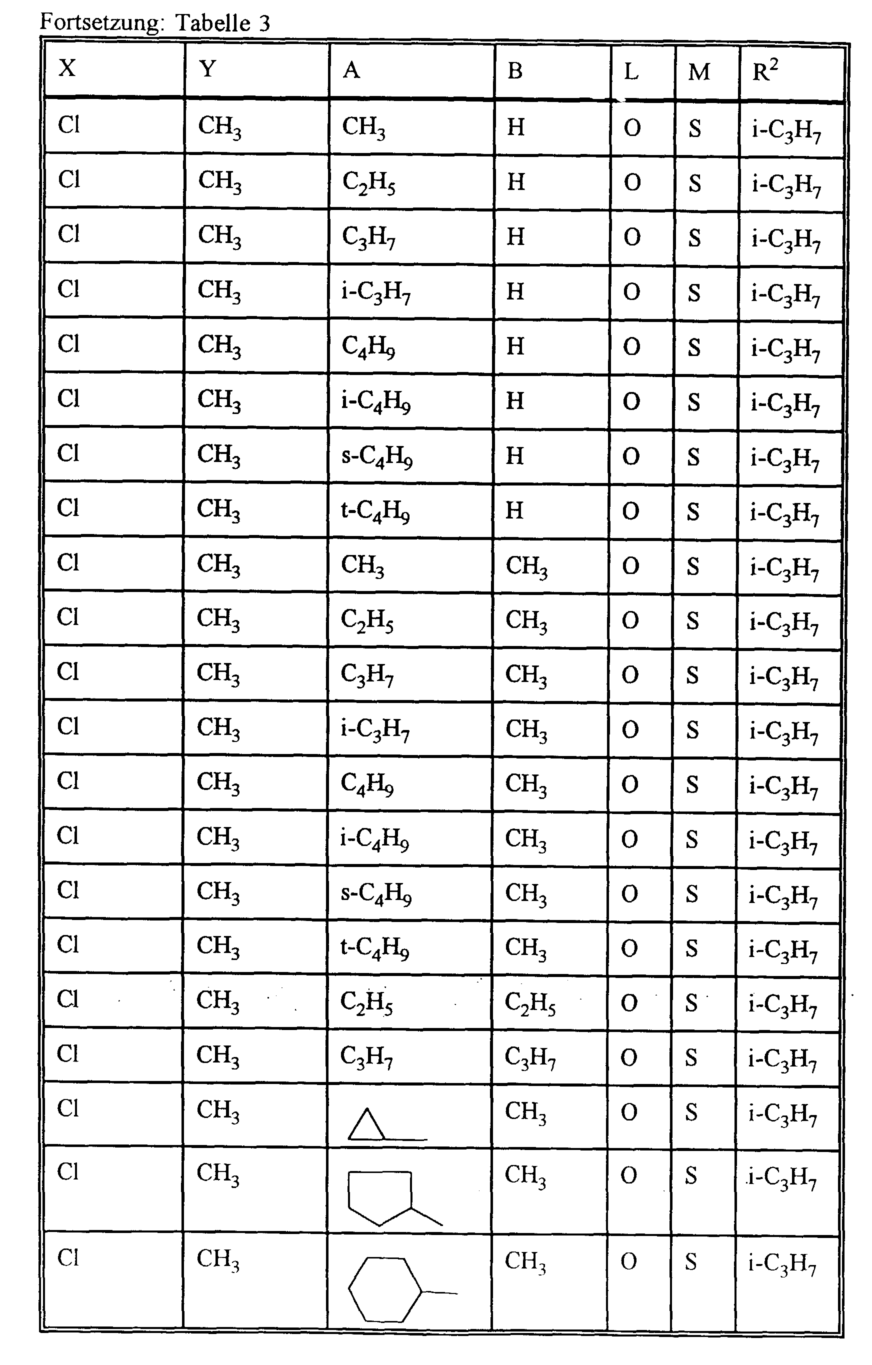

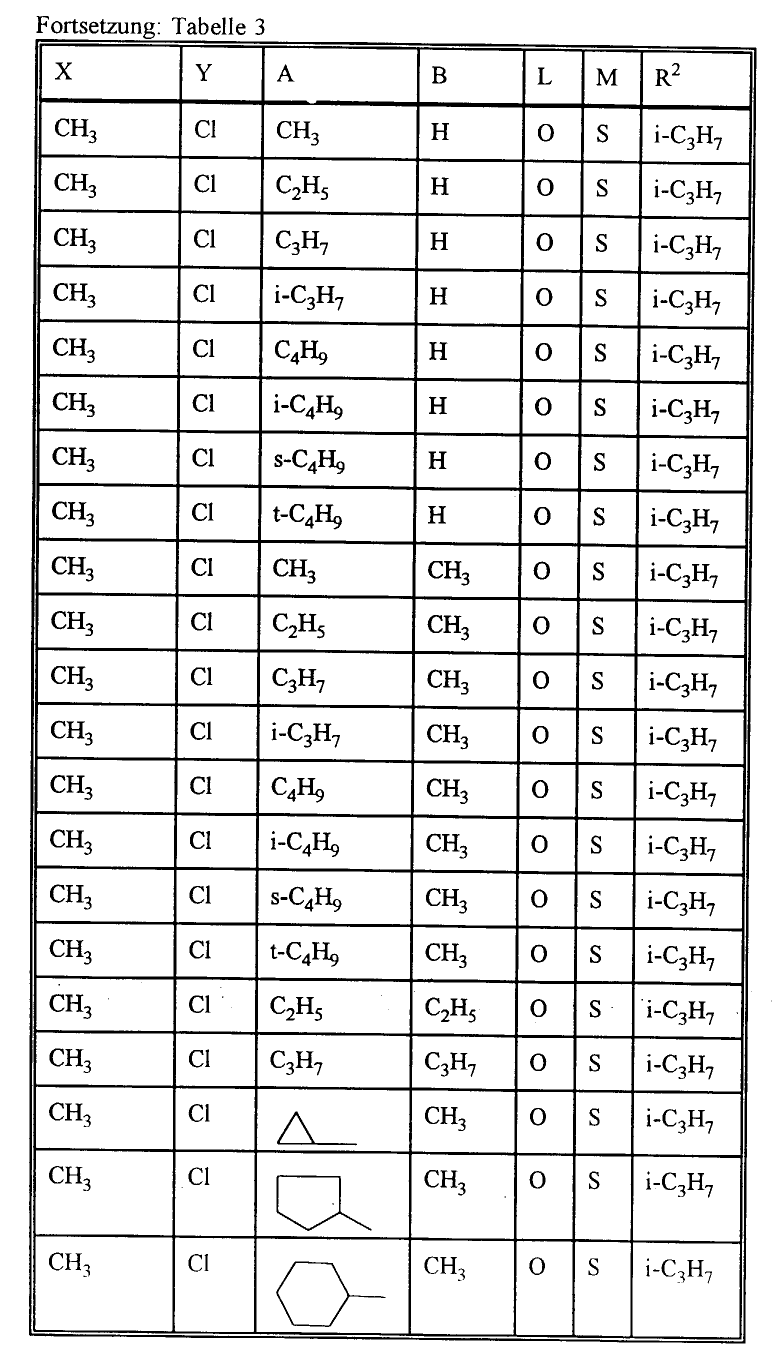

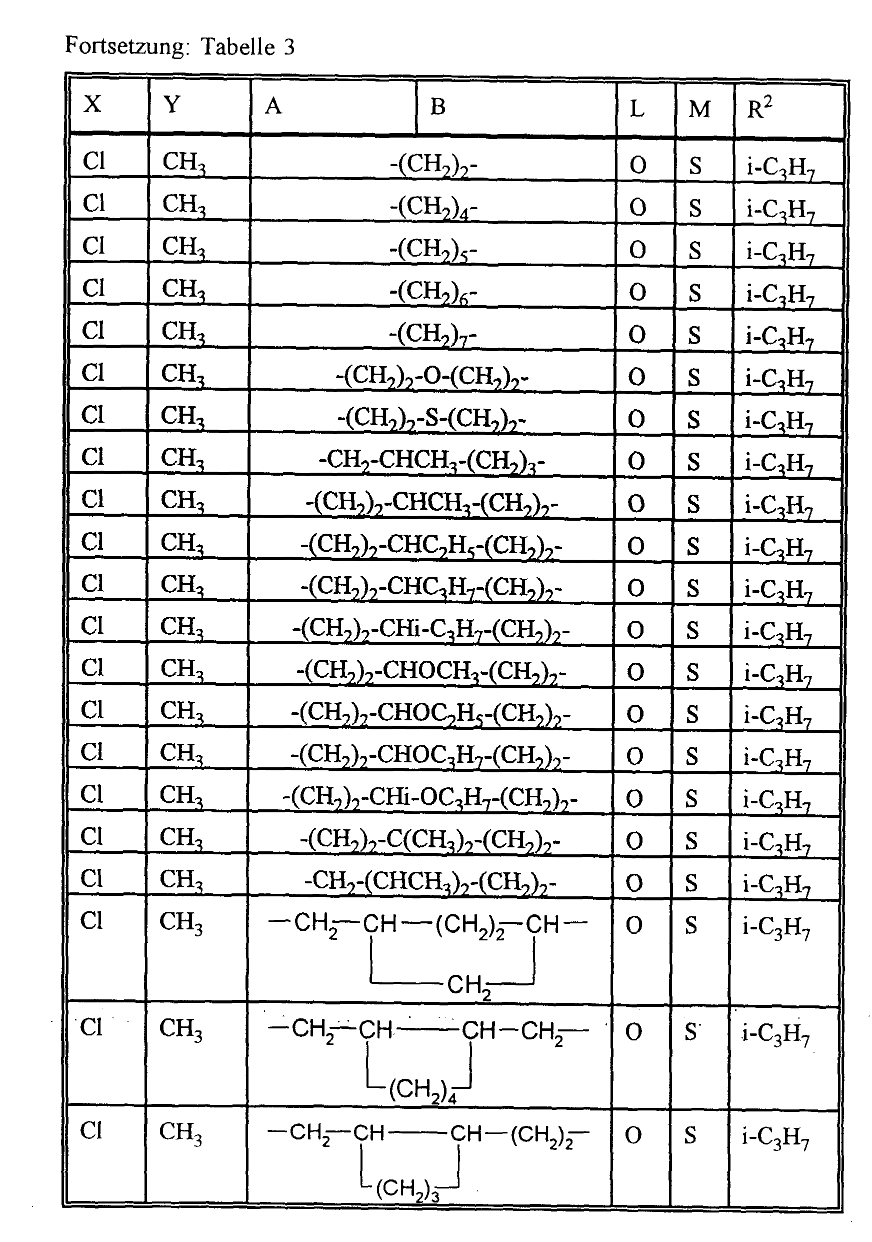

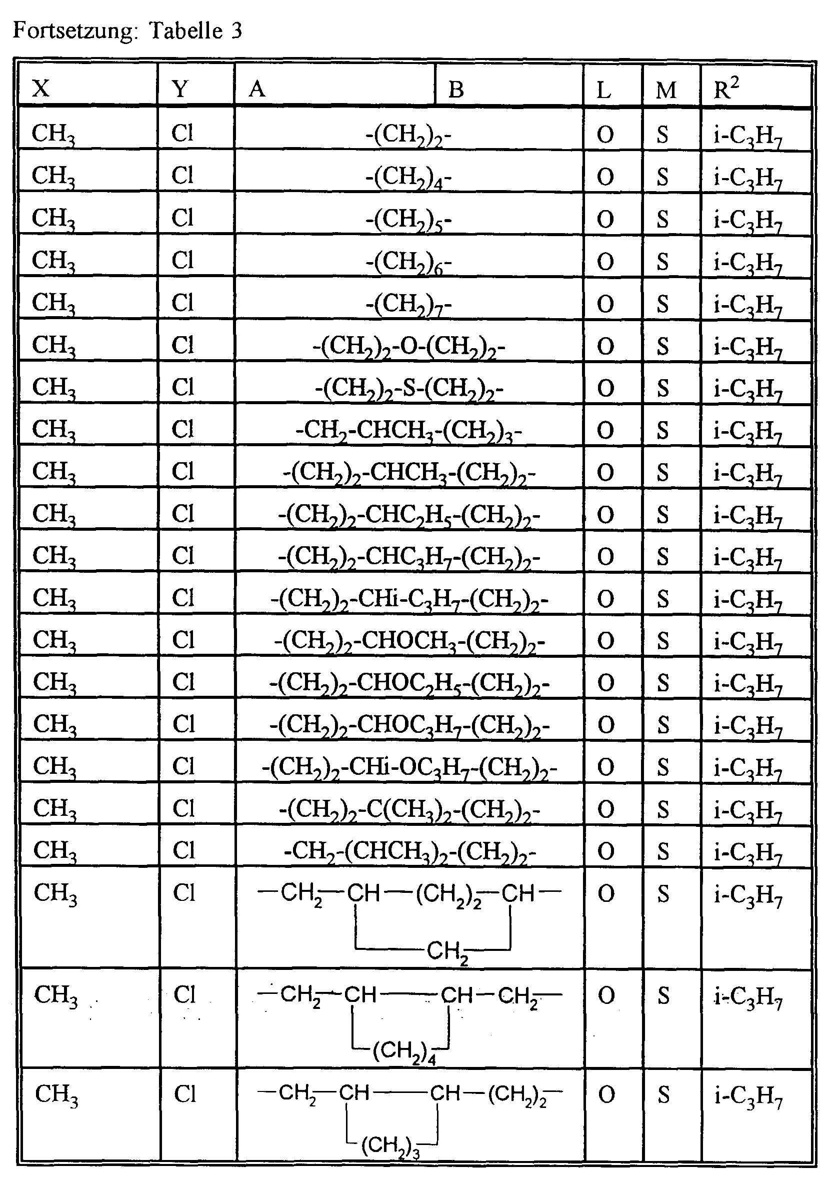

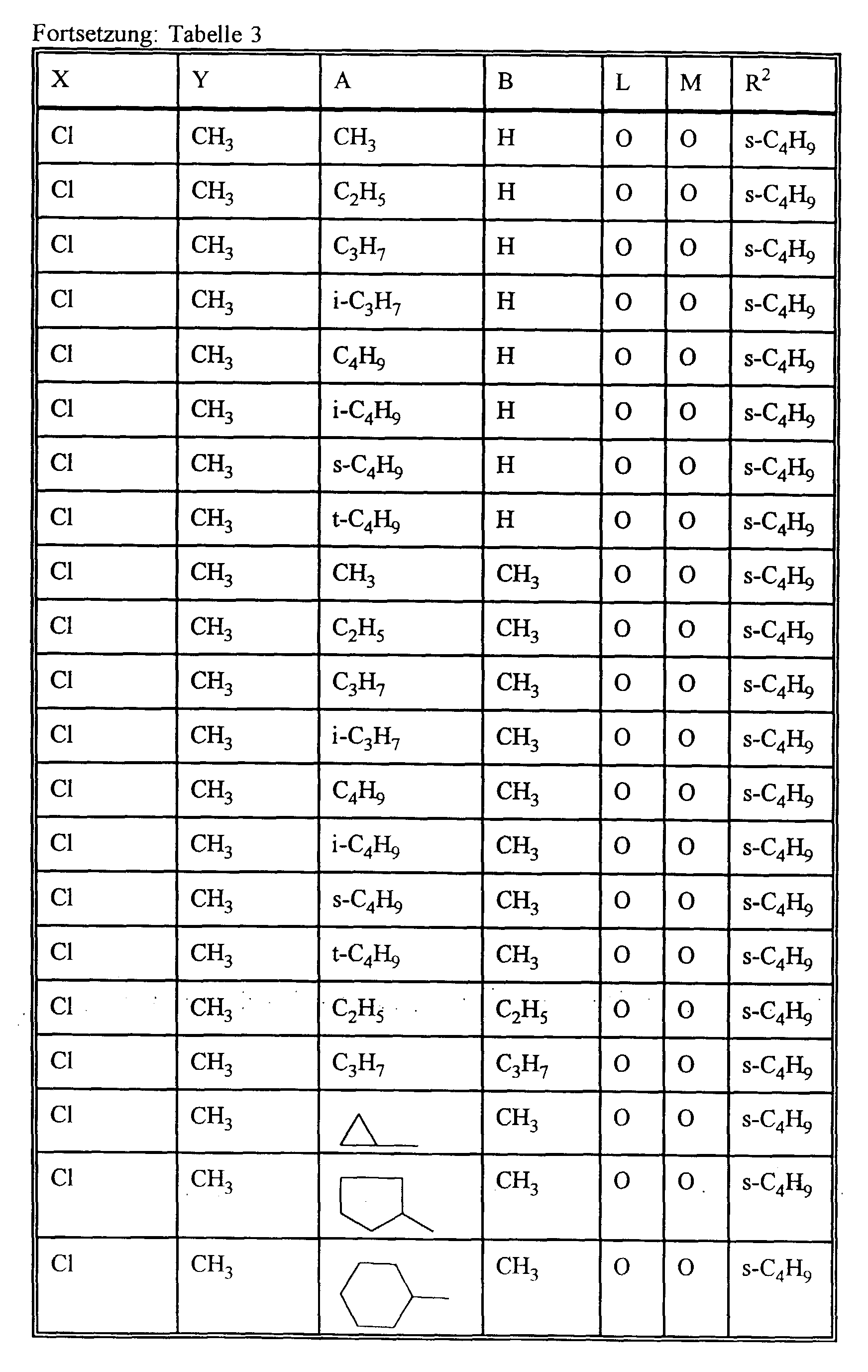

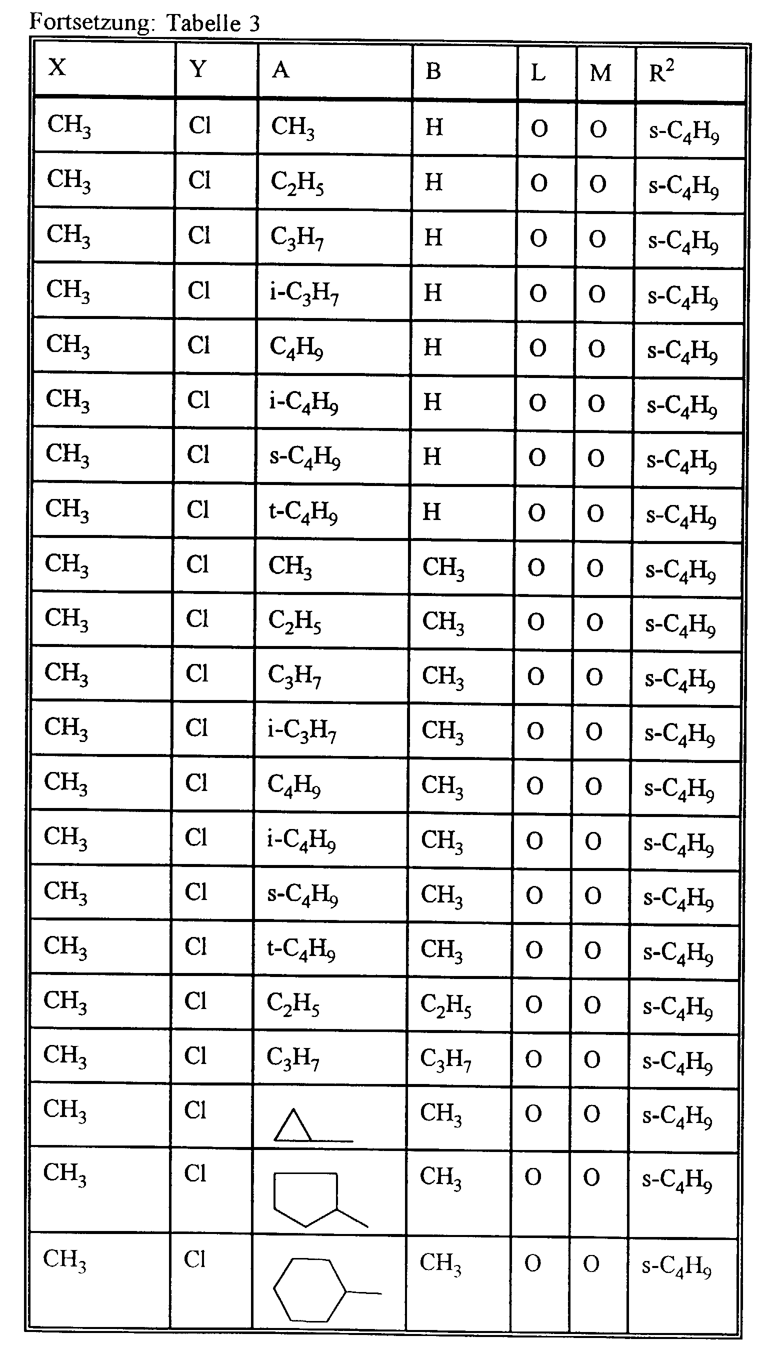

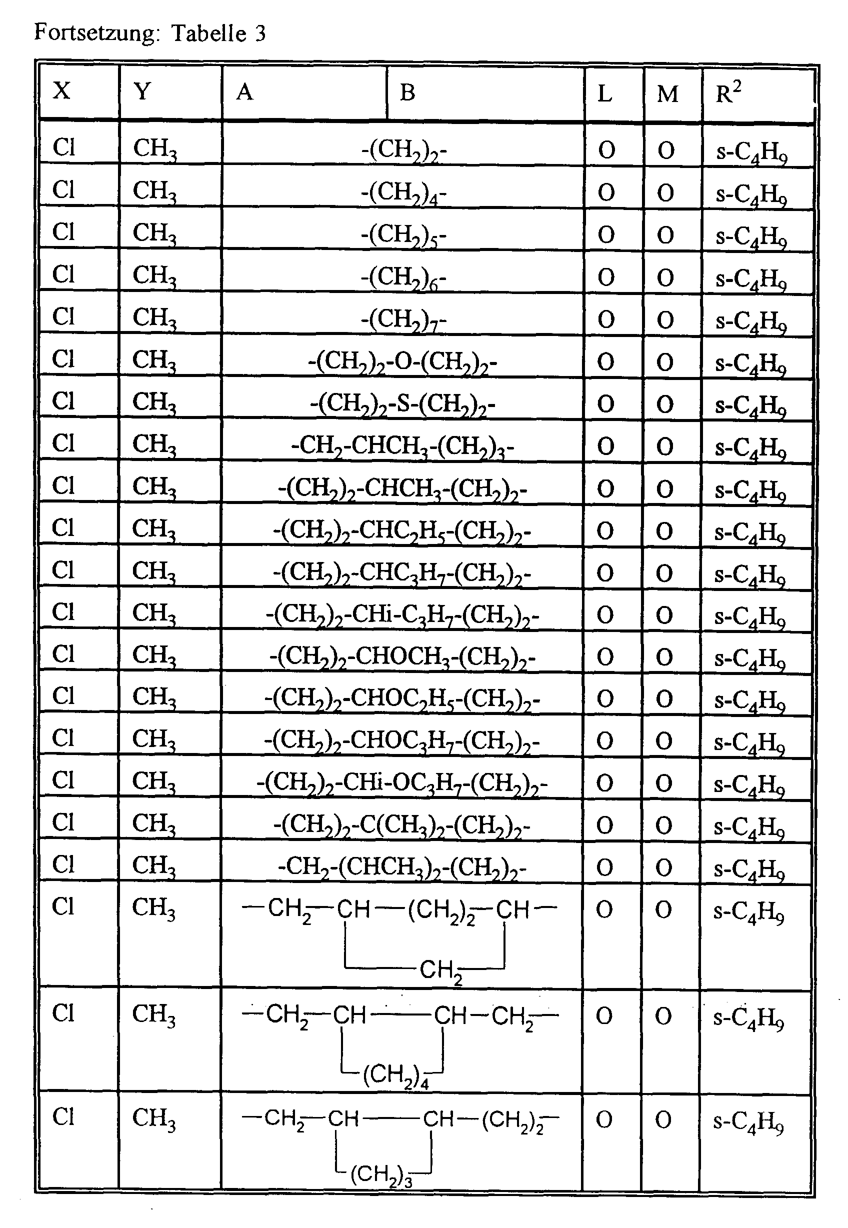

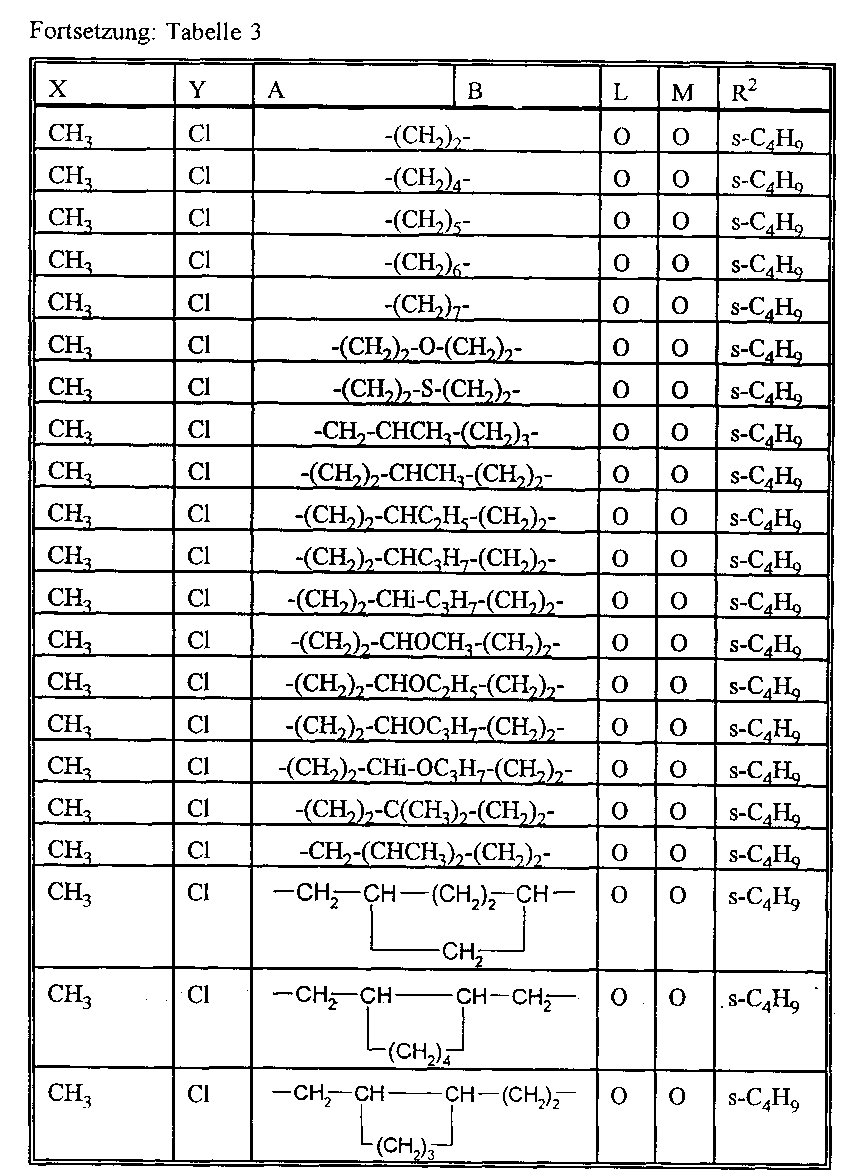

Im einzelnen seien außer den bei den Herstellungsbeispielen genannten Verbindungen die folgenden Verbindungen der Formel (Id) genannt:

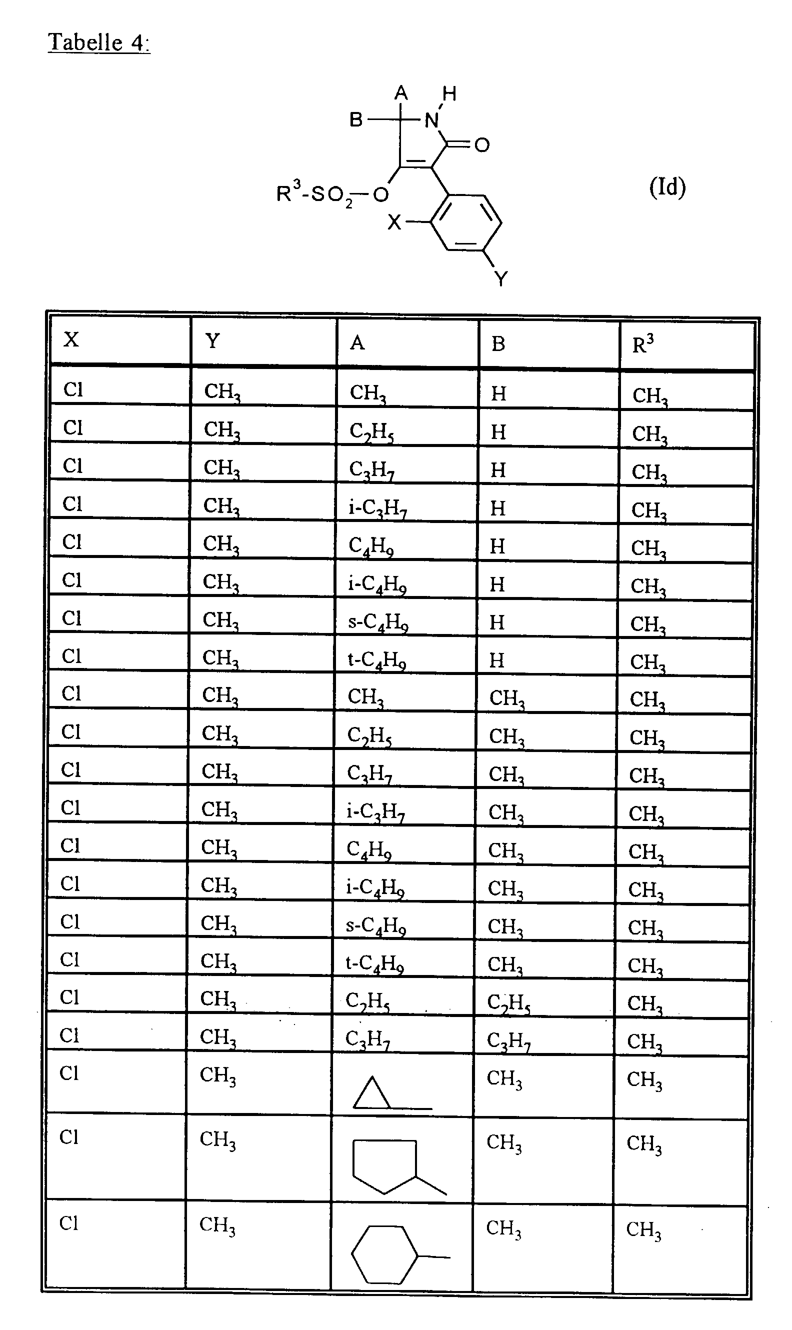

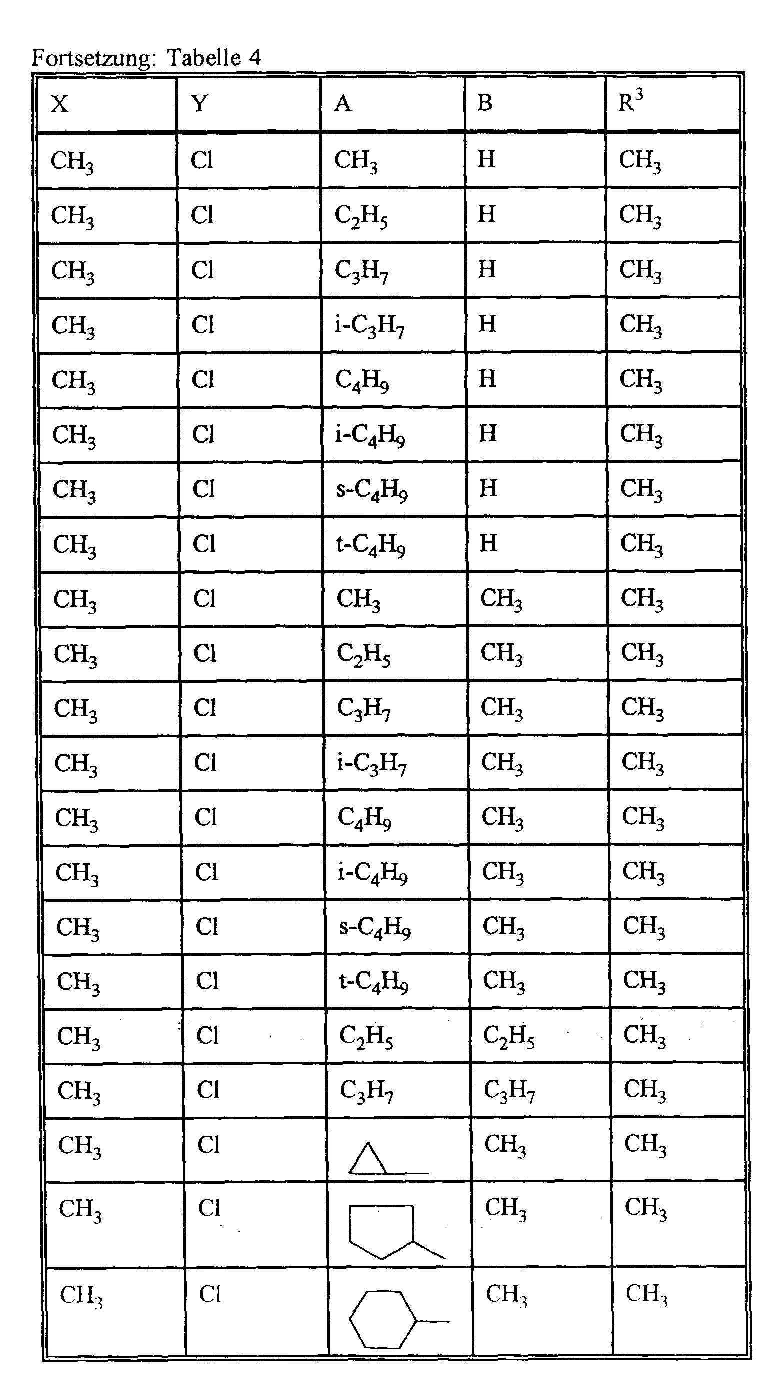

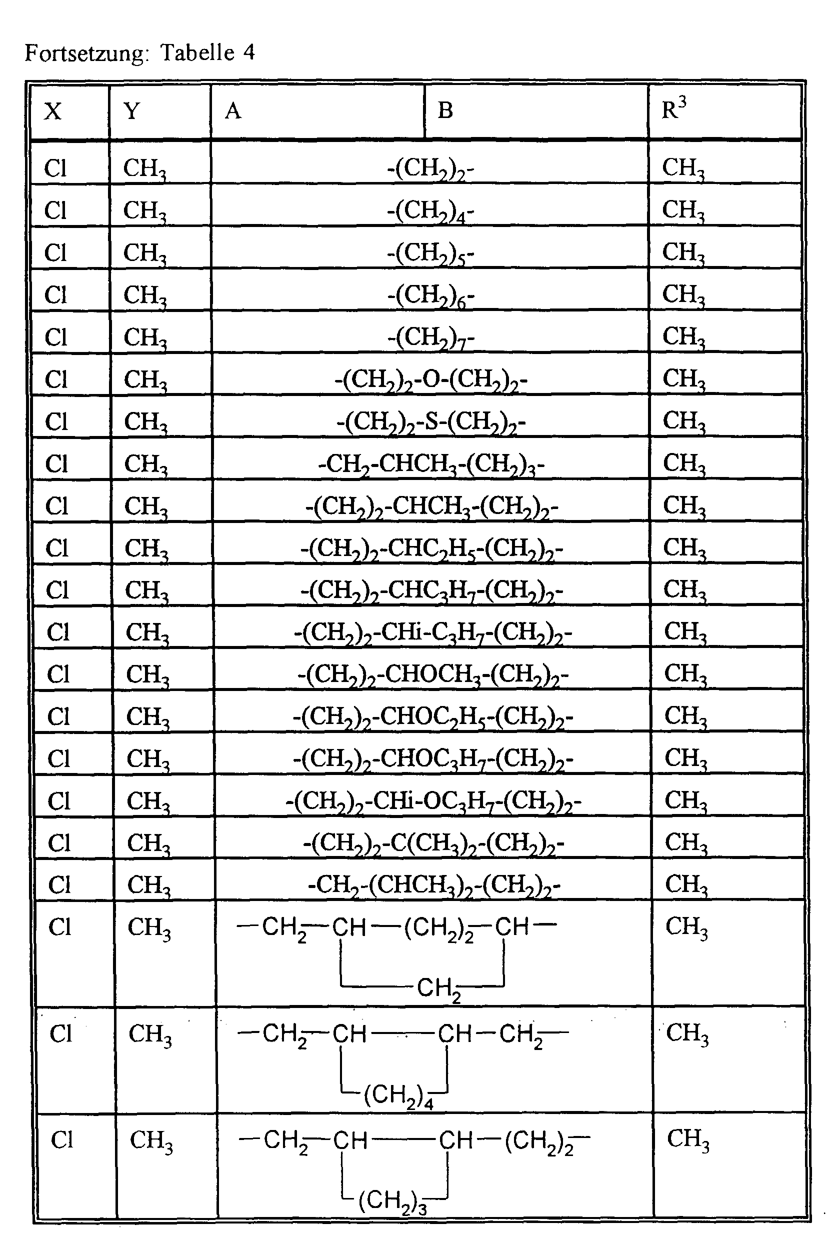

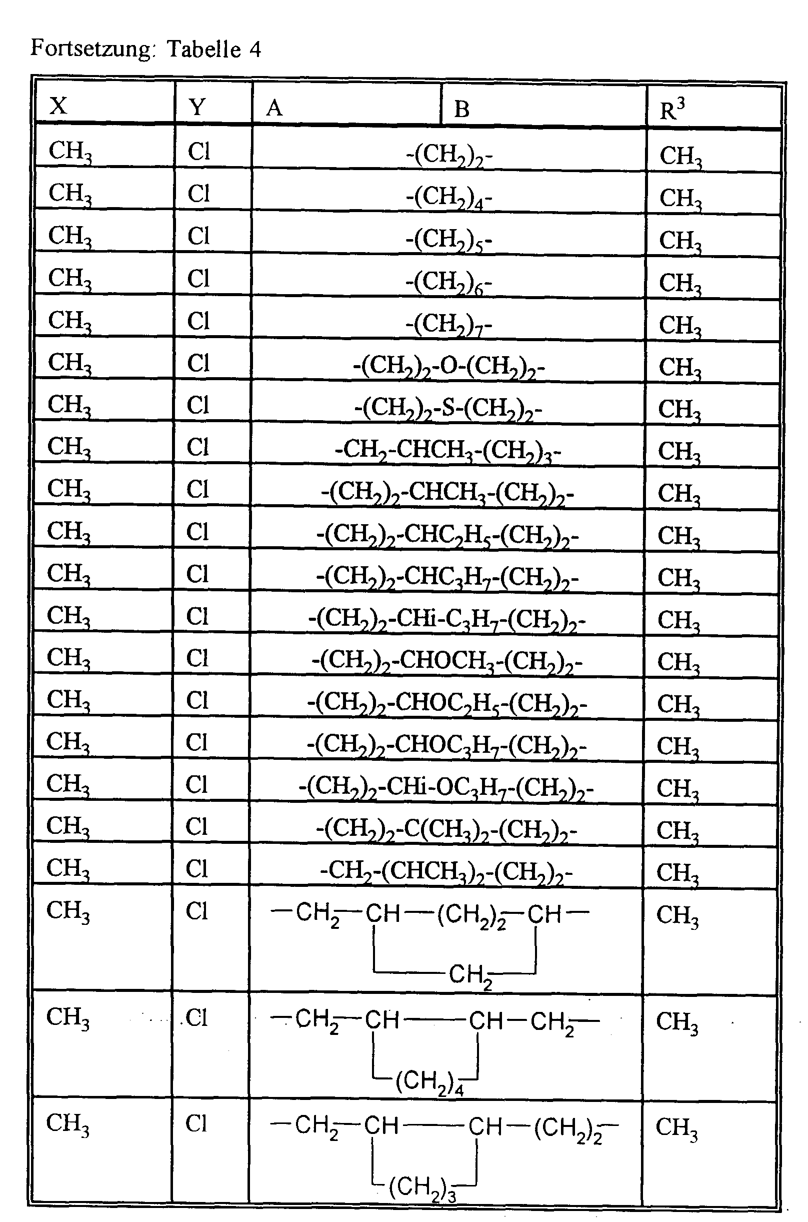

Im einzelnen seien außer den bei den Herstellungsbeispielen genannten Verbindungen die folgenden Verbindungen der Formel (Ie) genannt:

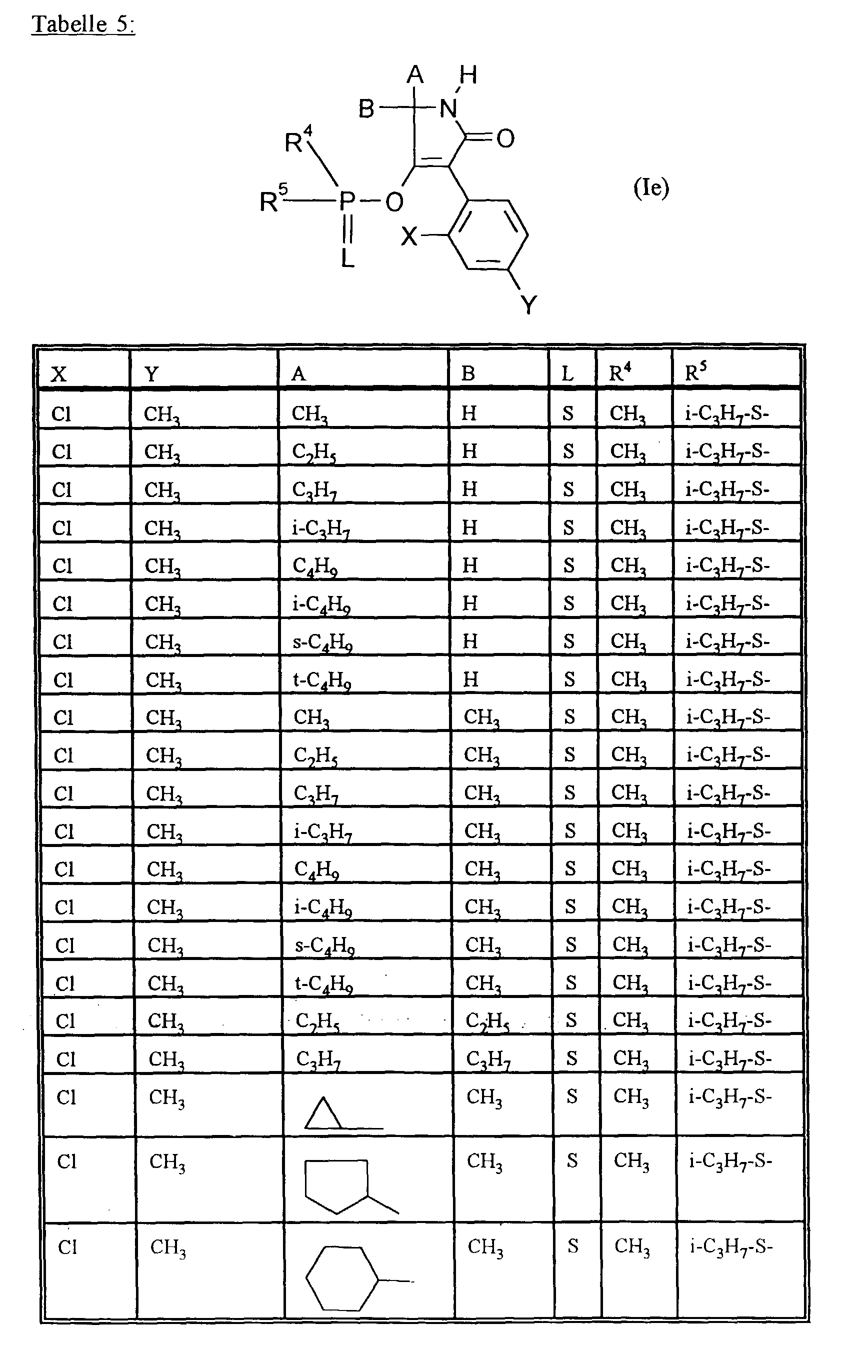

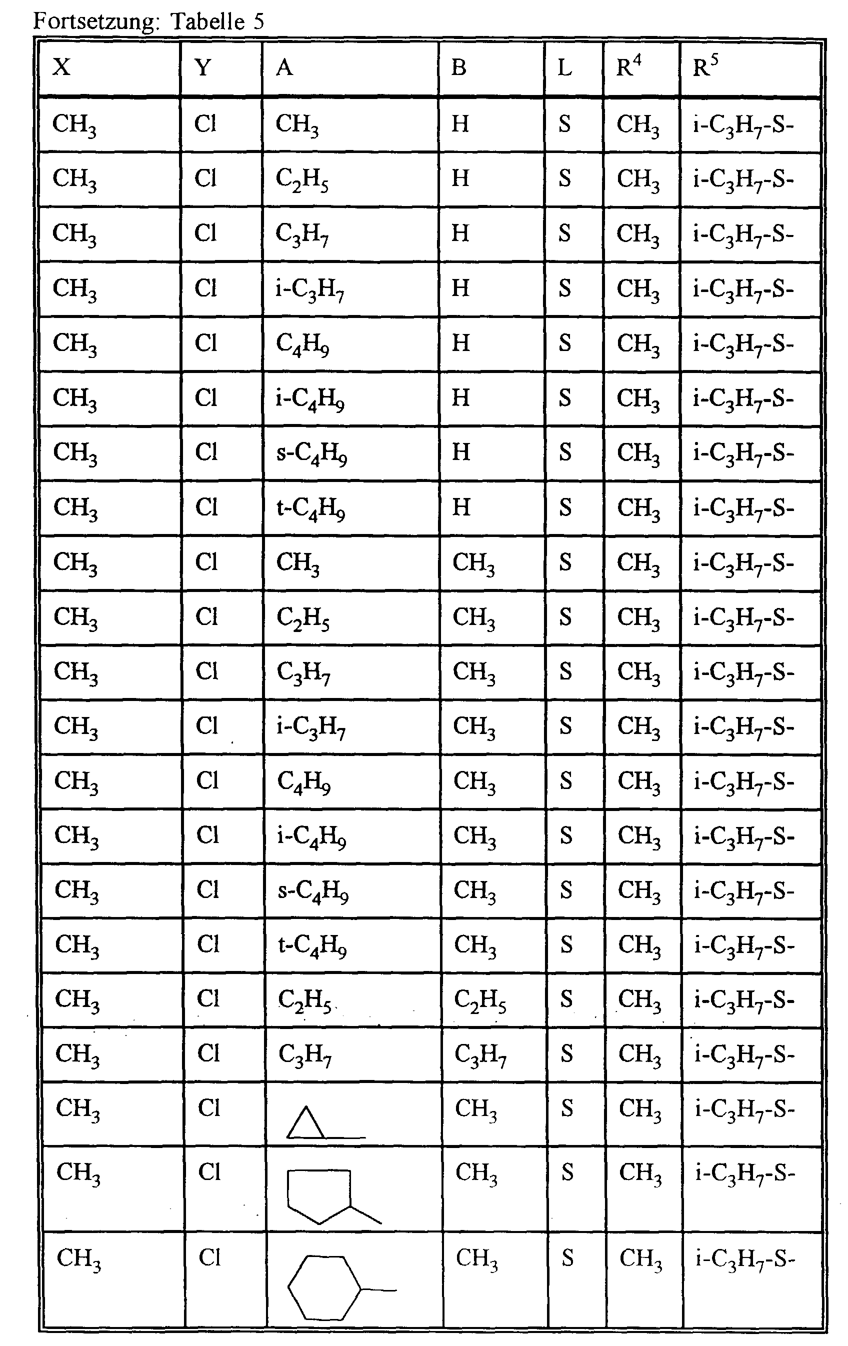

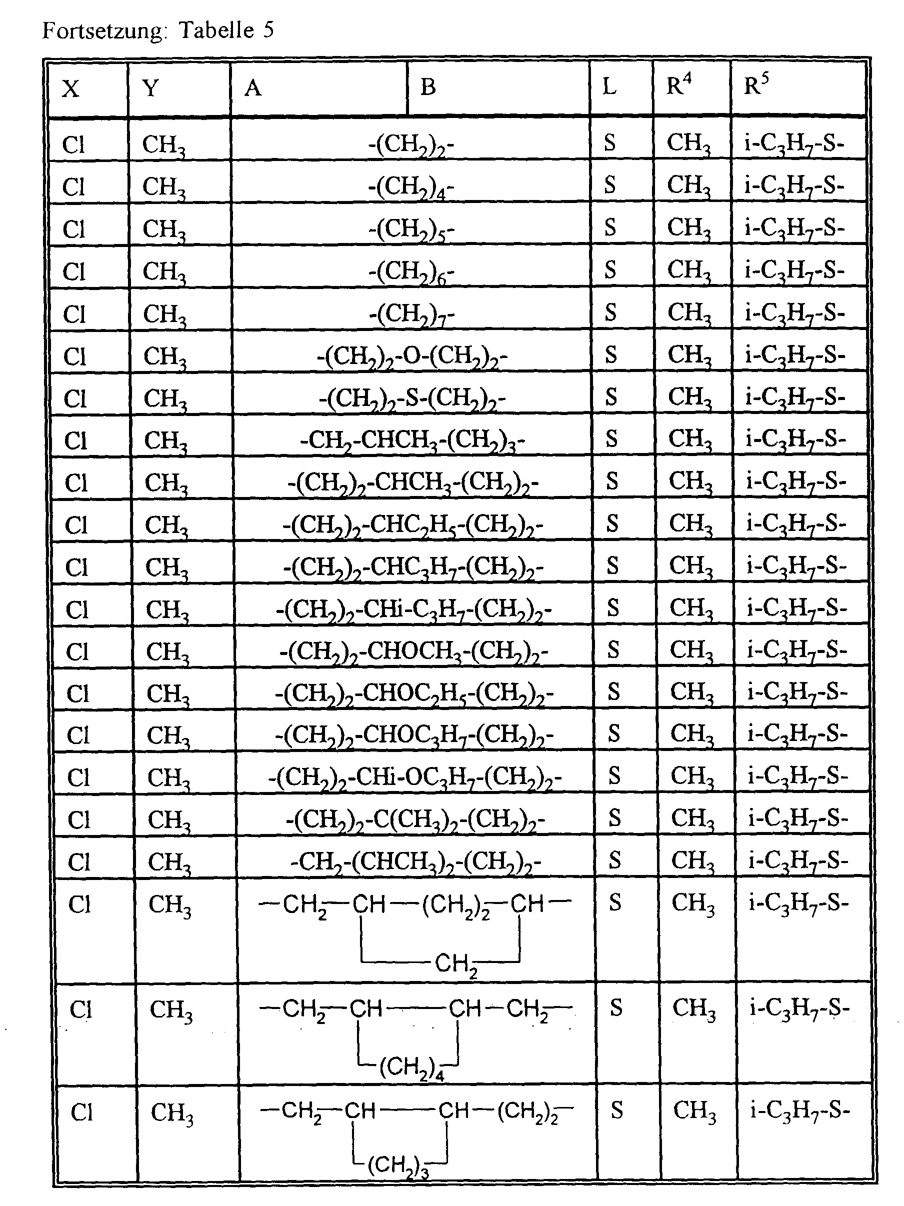

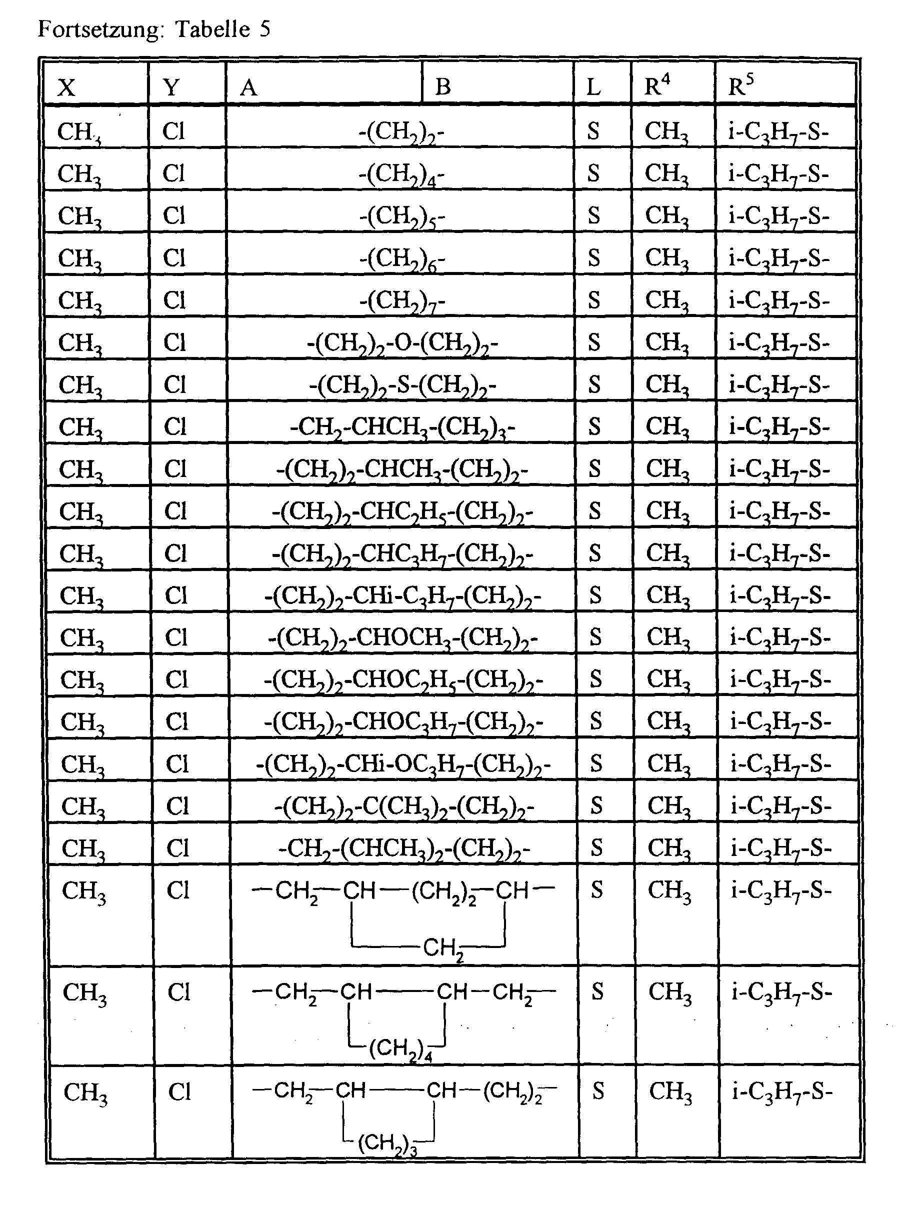

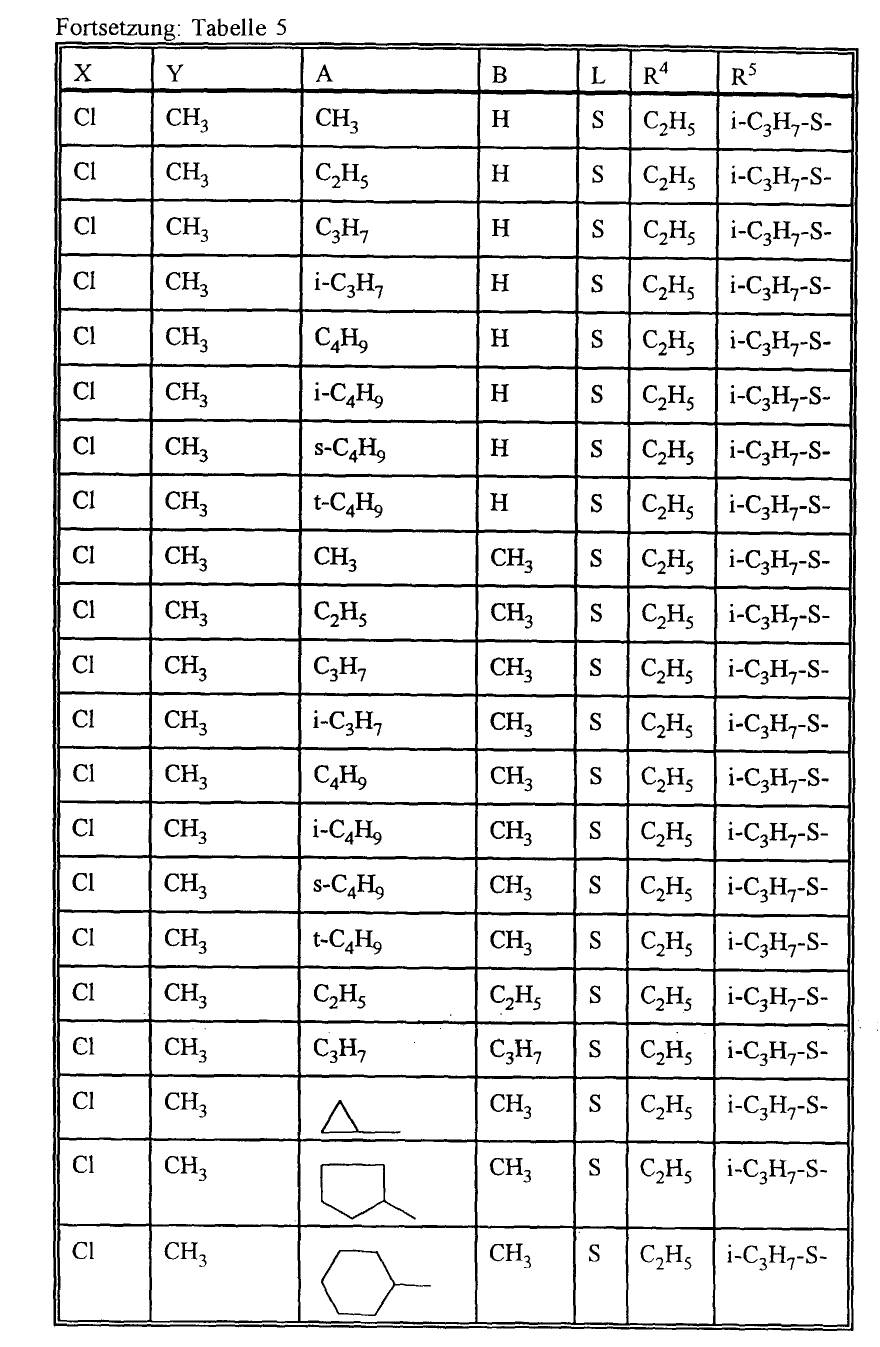

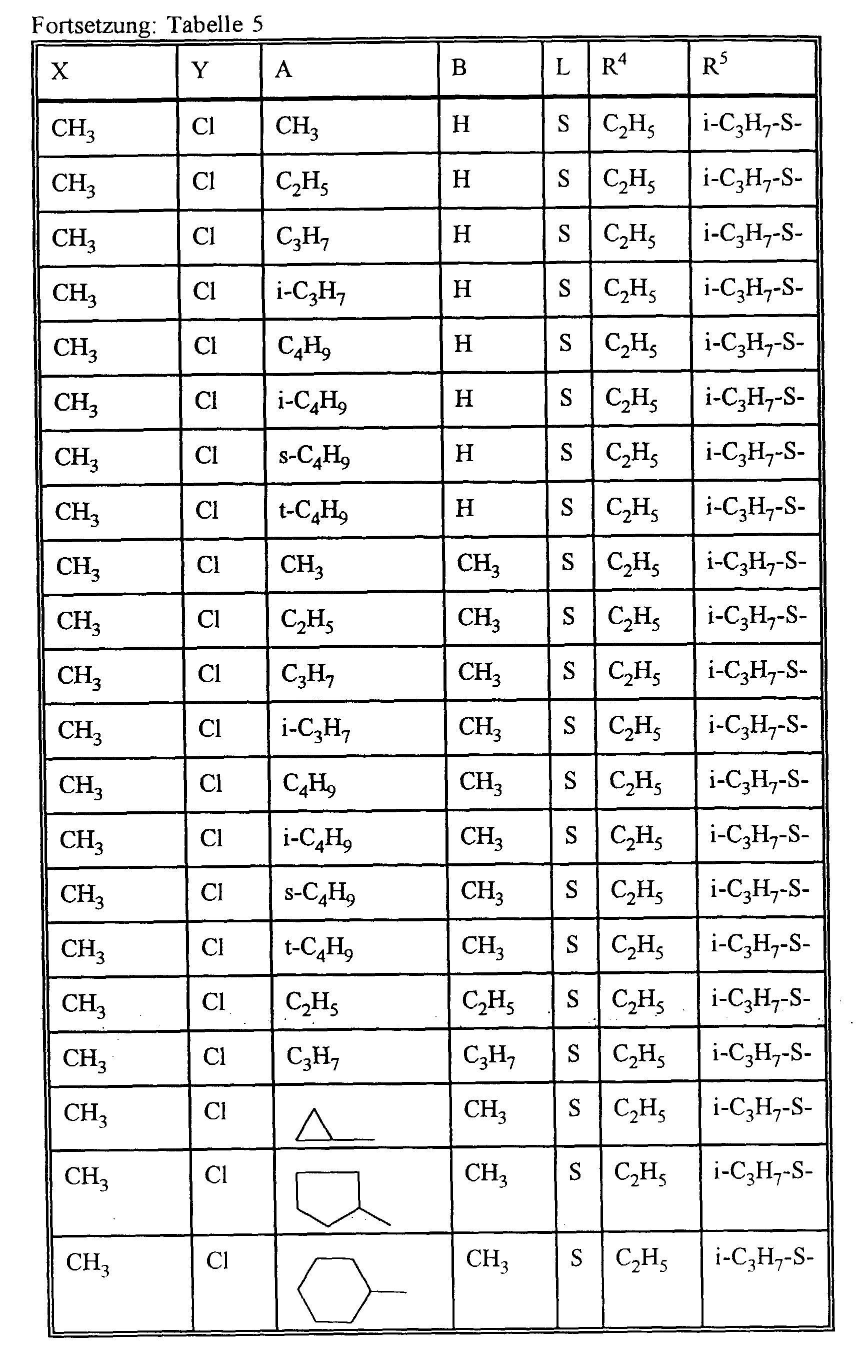

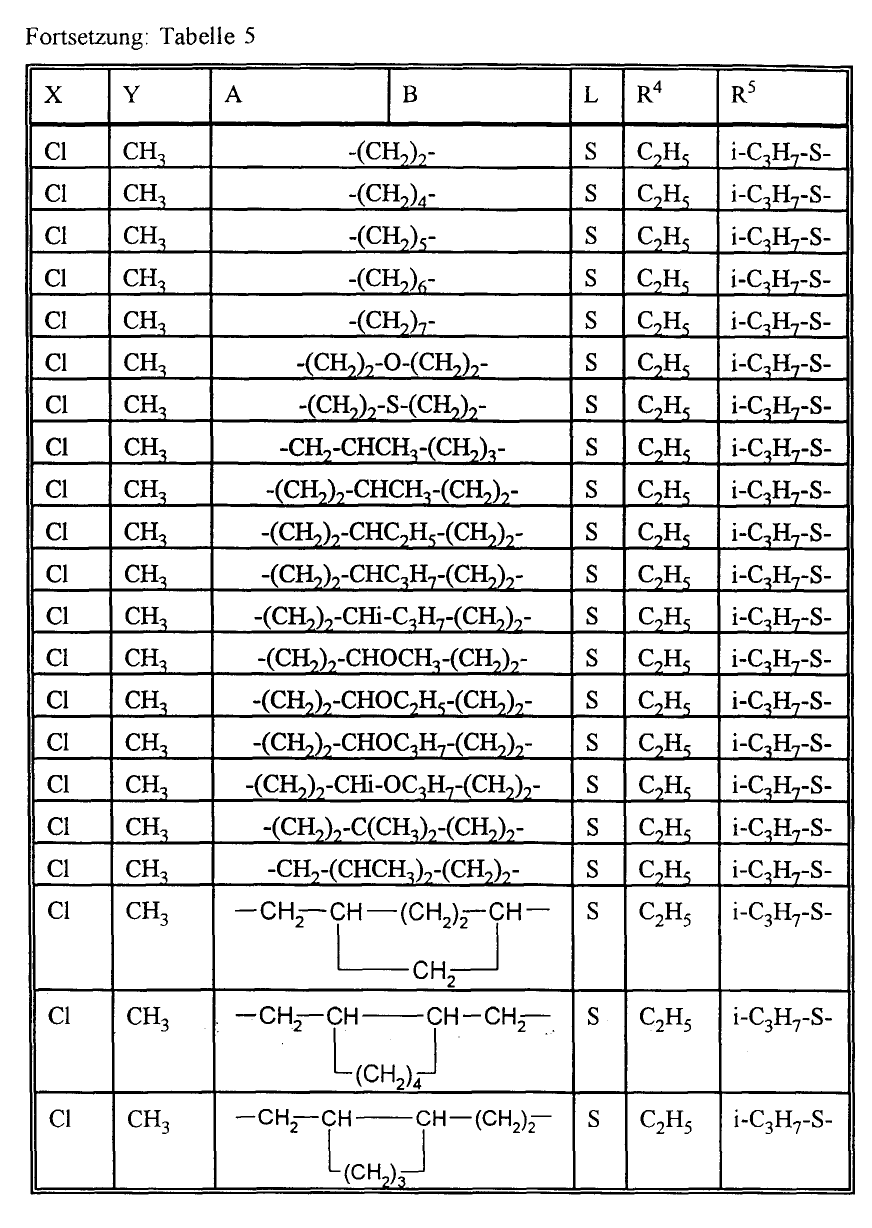

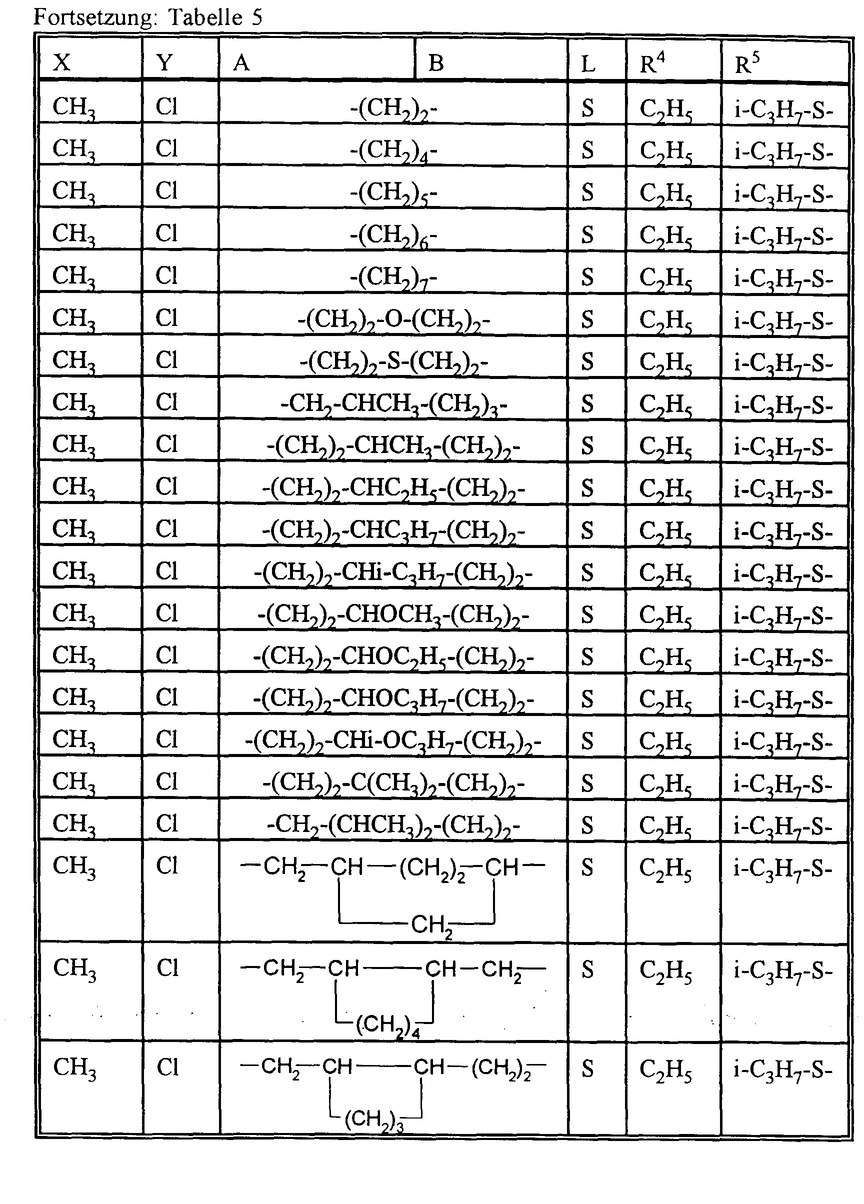

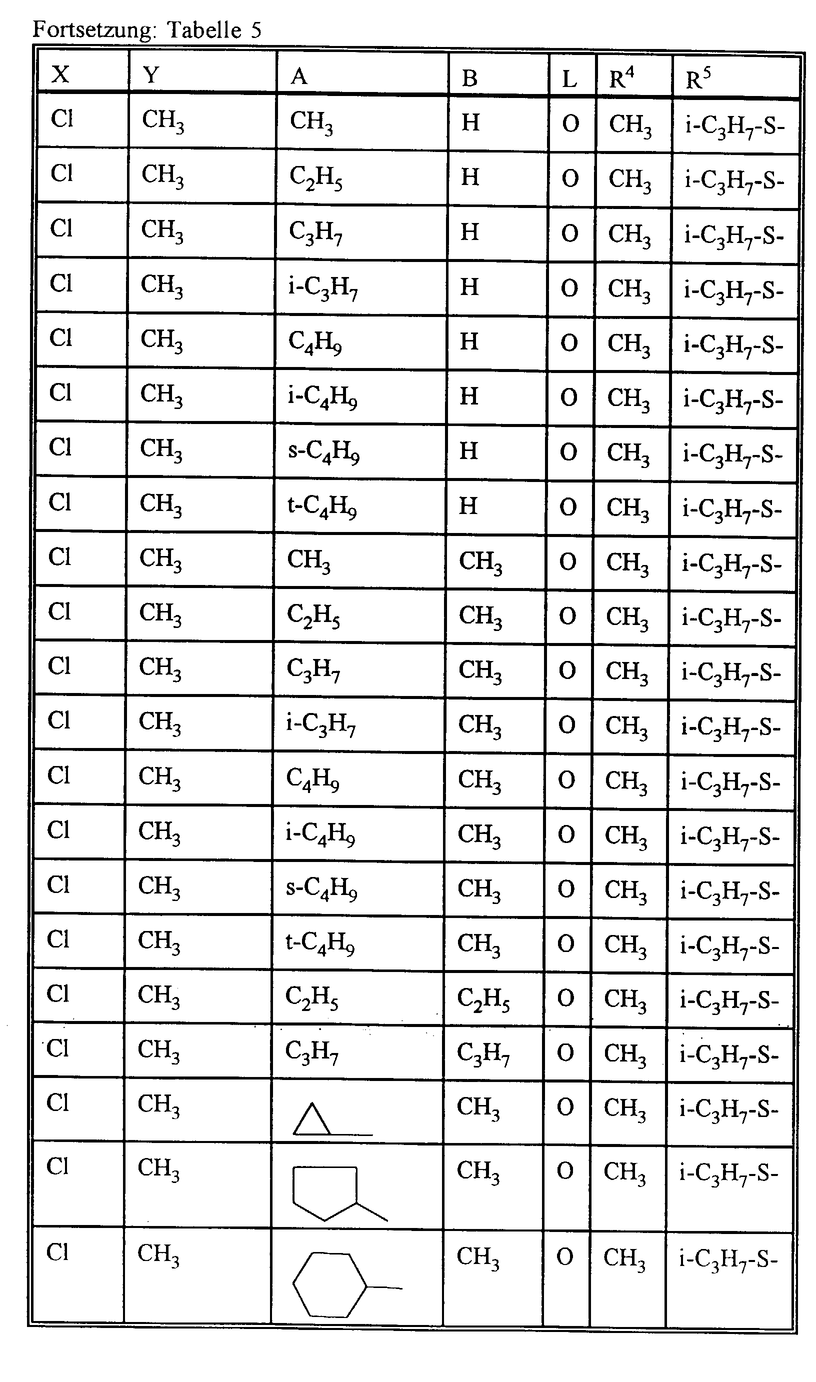

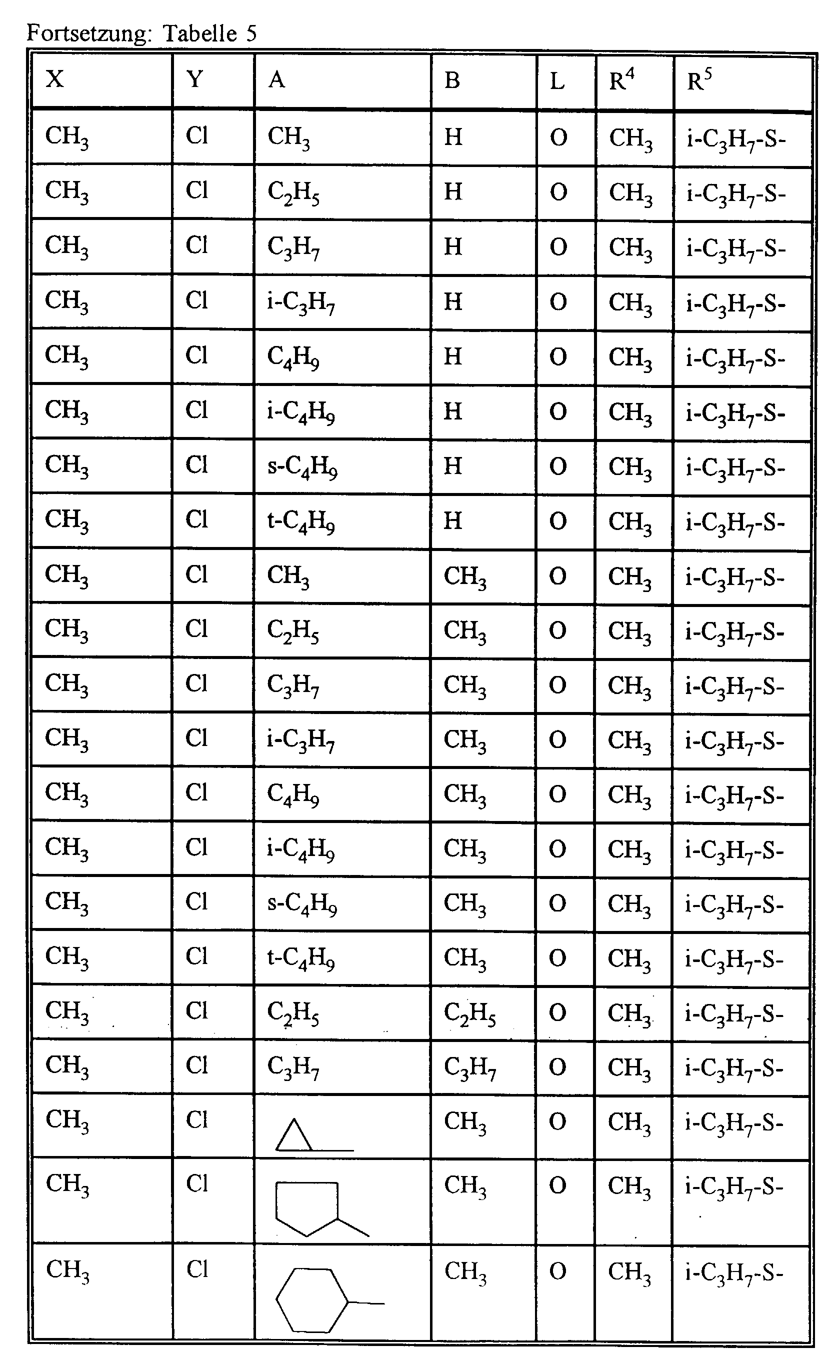

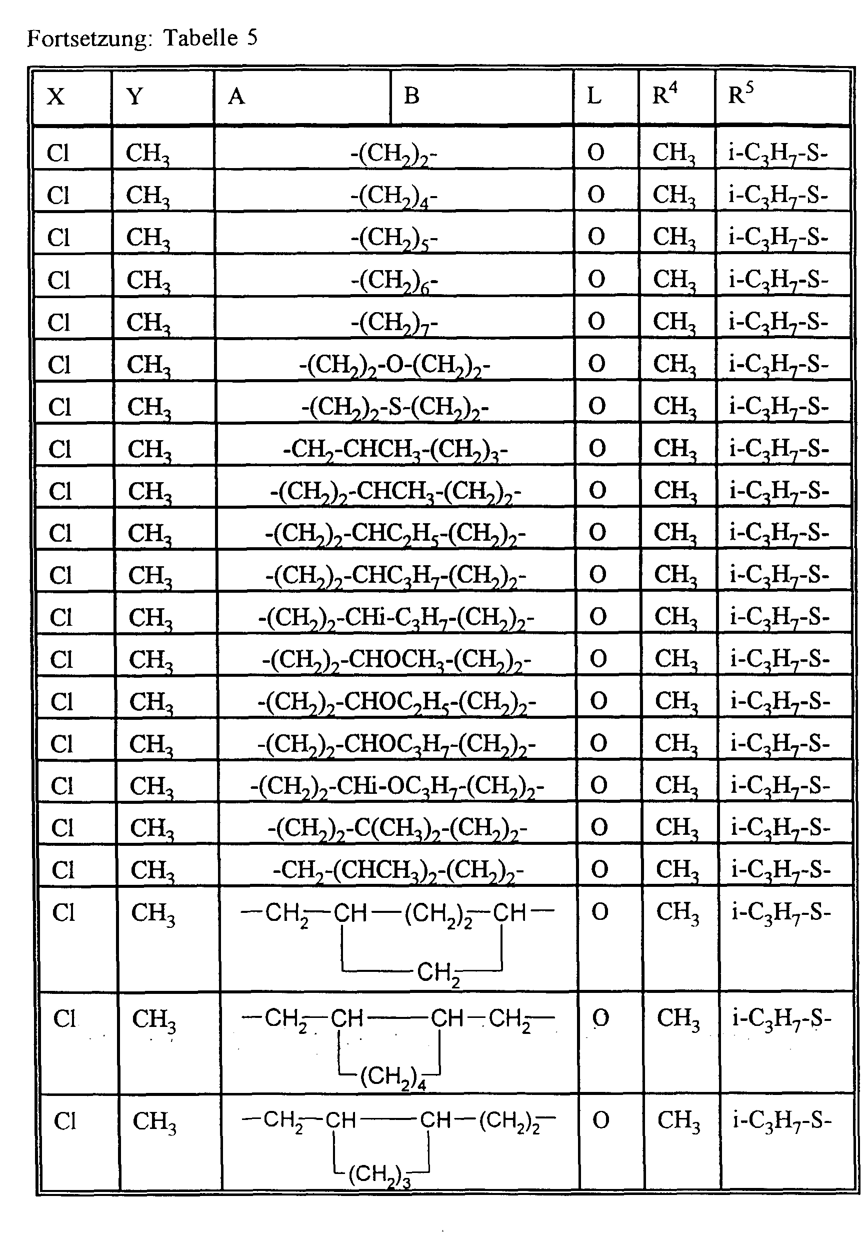

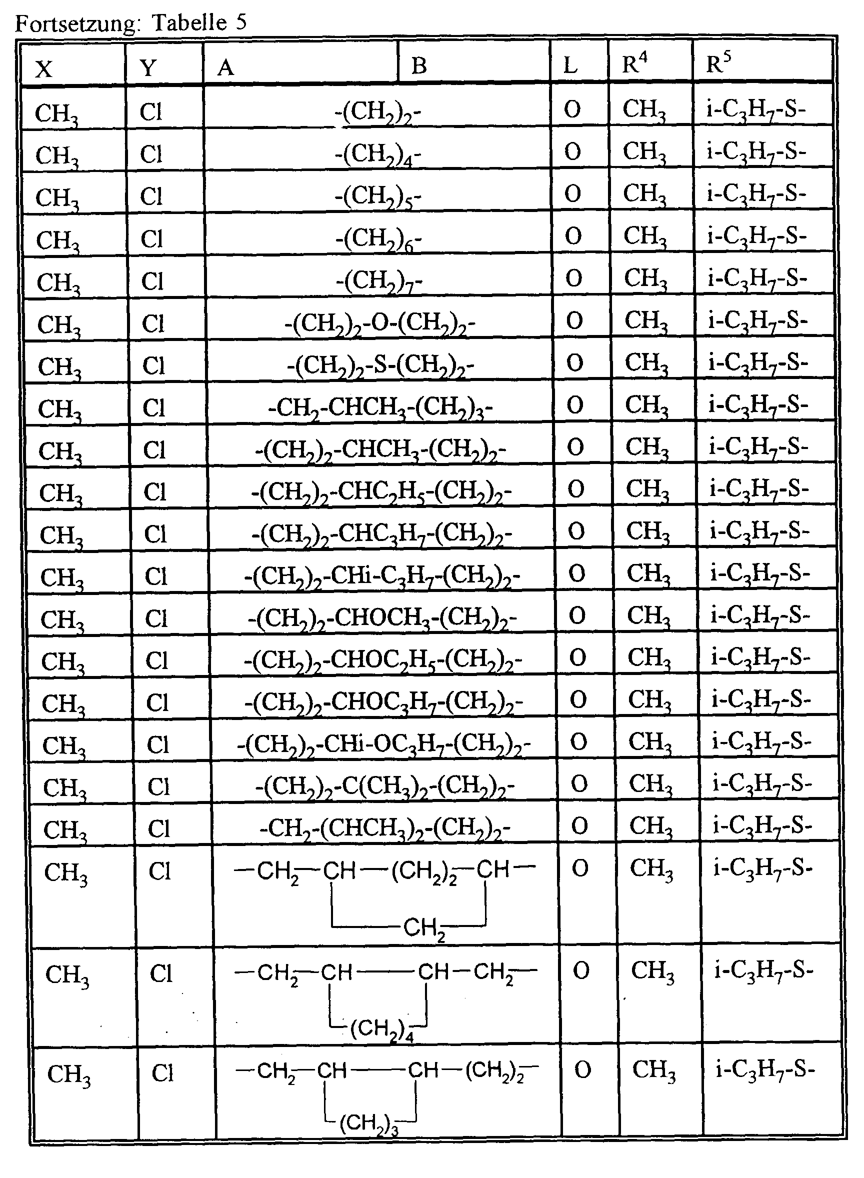

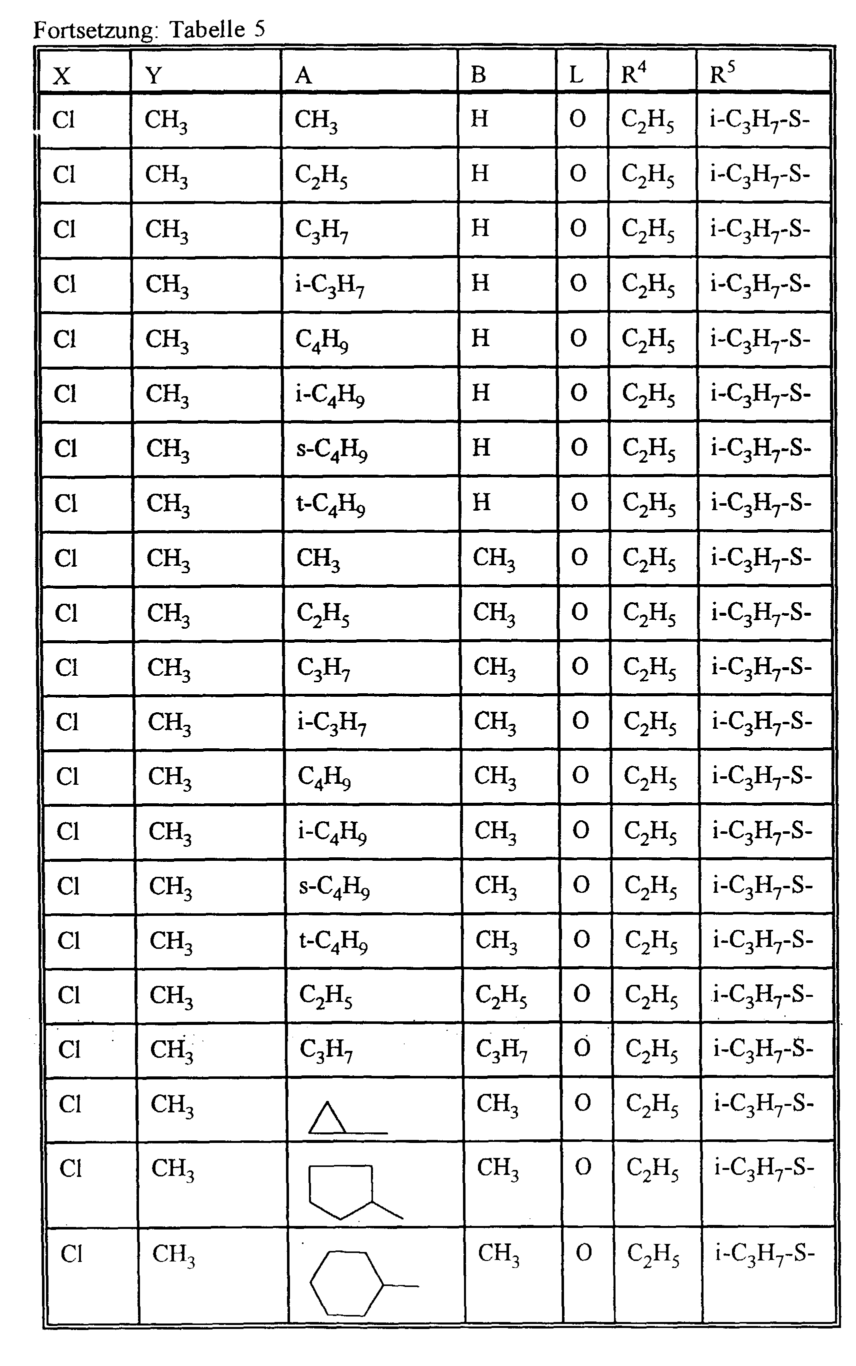

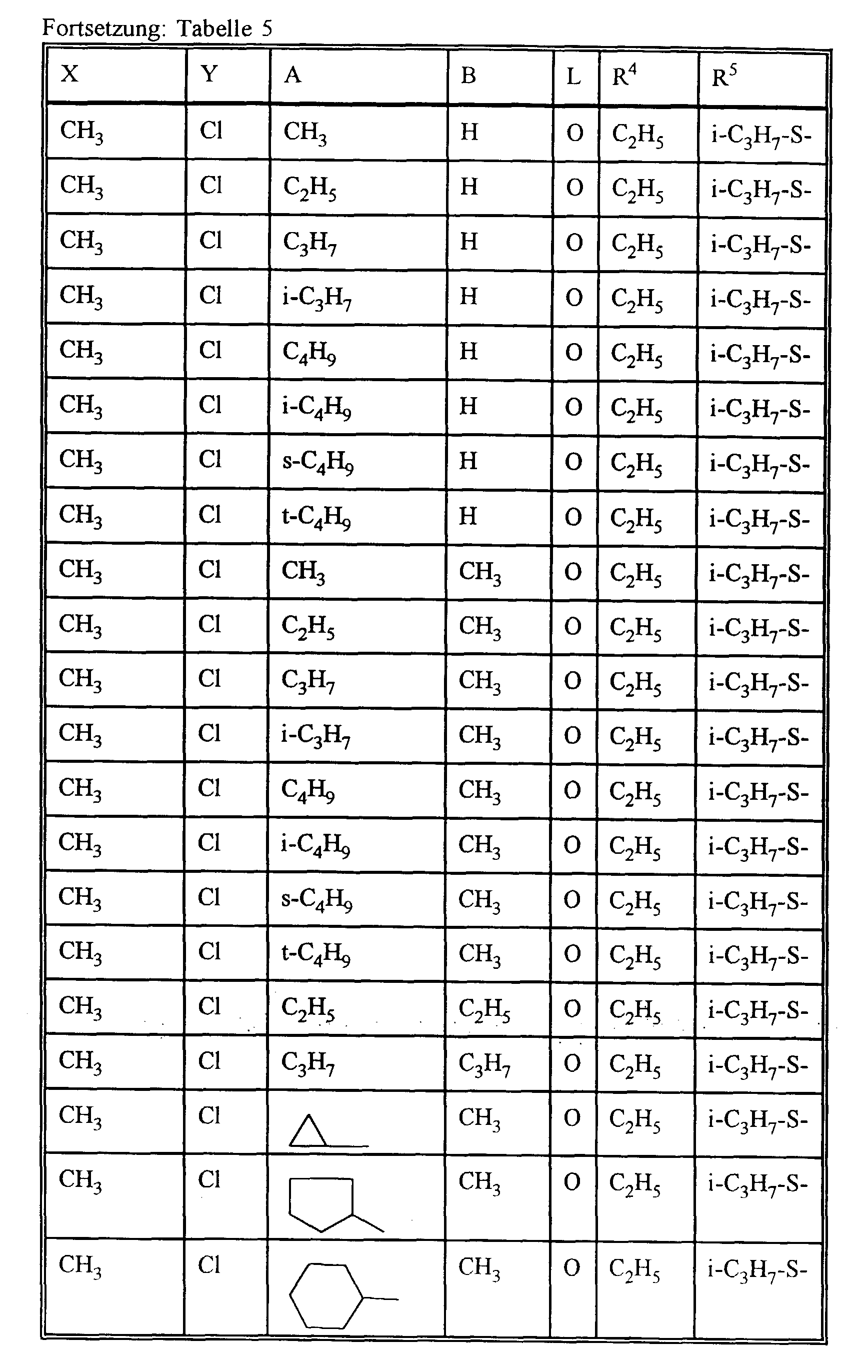

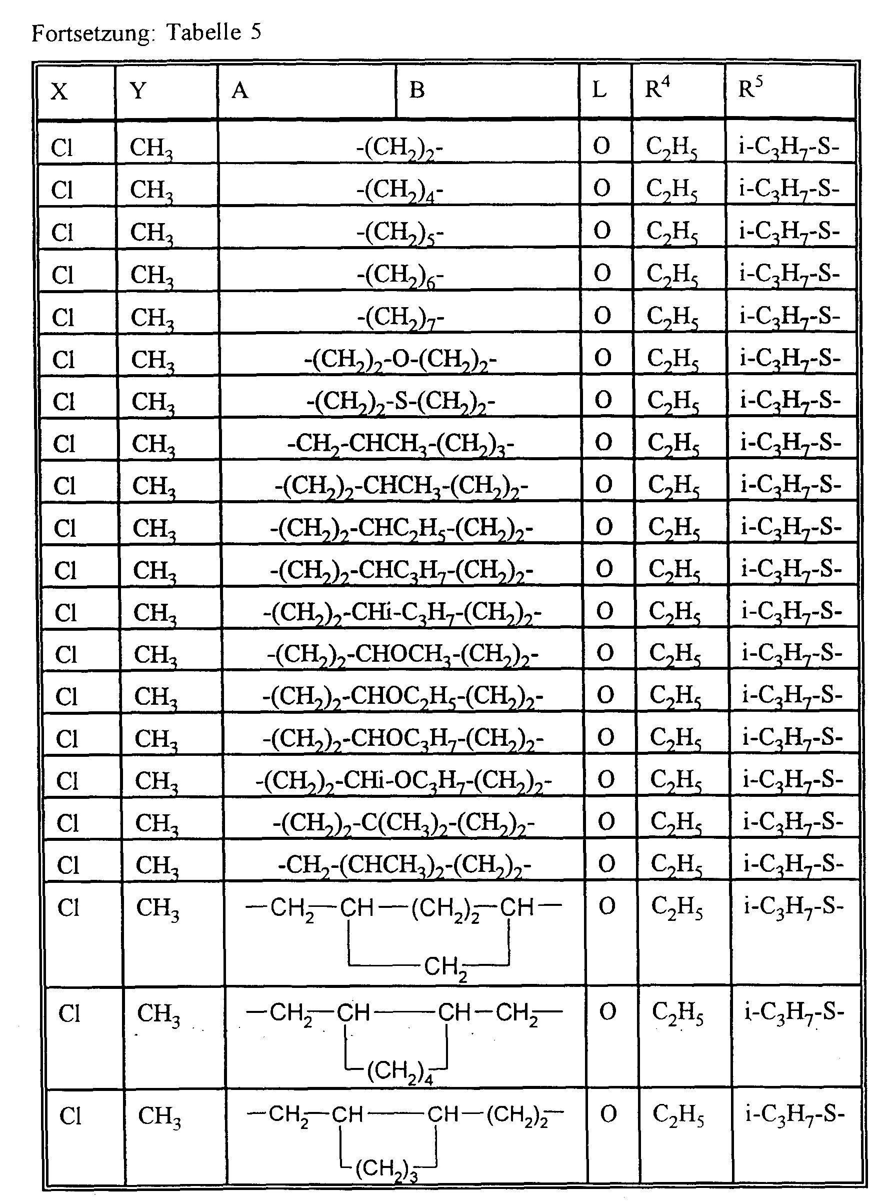

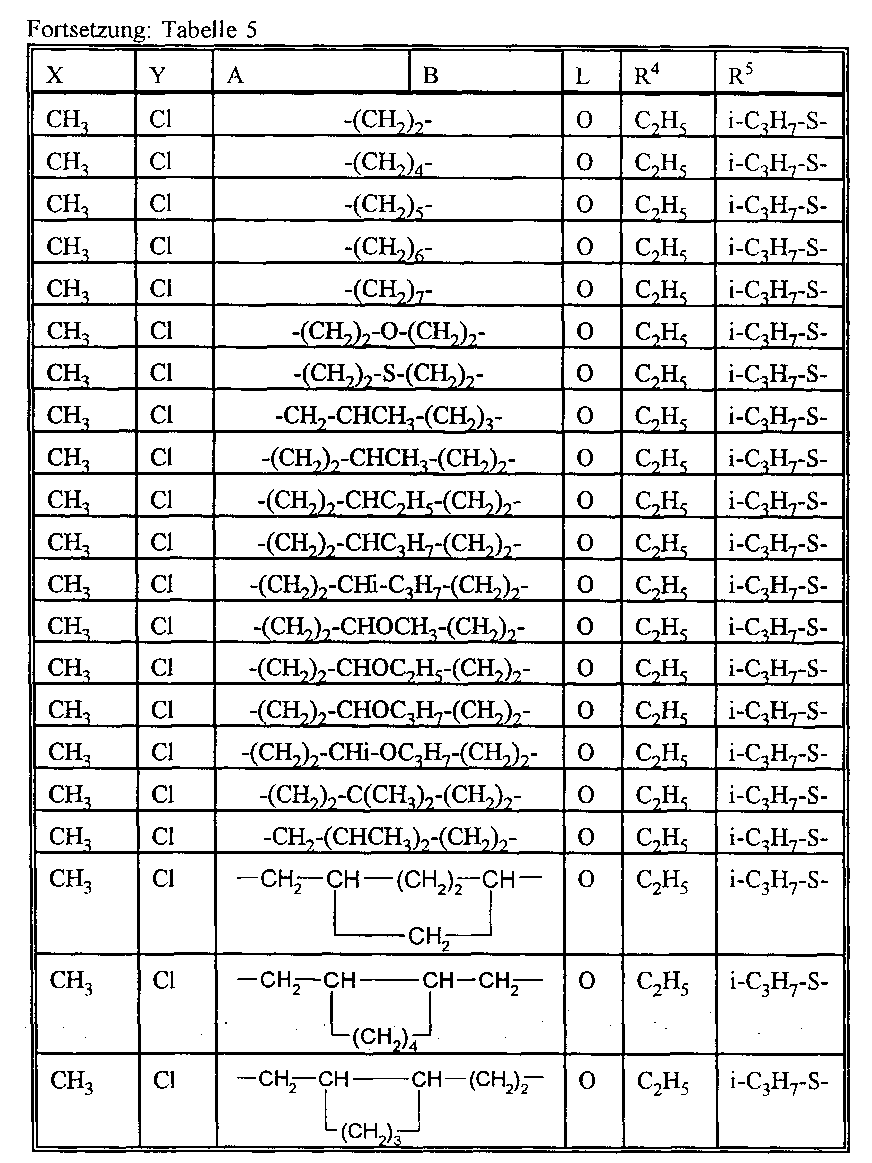

Im einzelnen seien außer den bei den Herstellungsbeispielen genannten Verbindungen die folgenden Verbindungen der Formel (If-a) genannt:

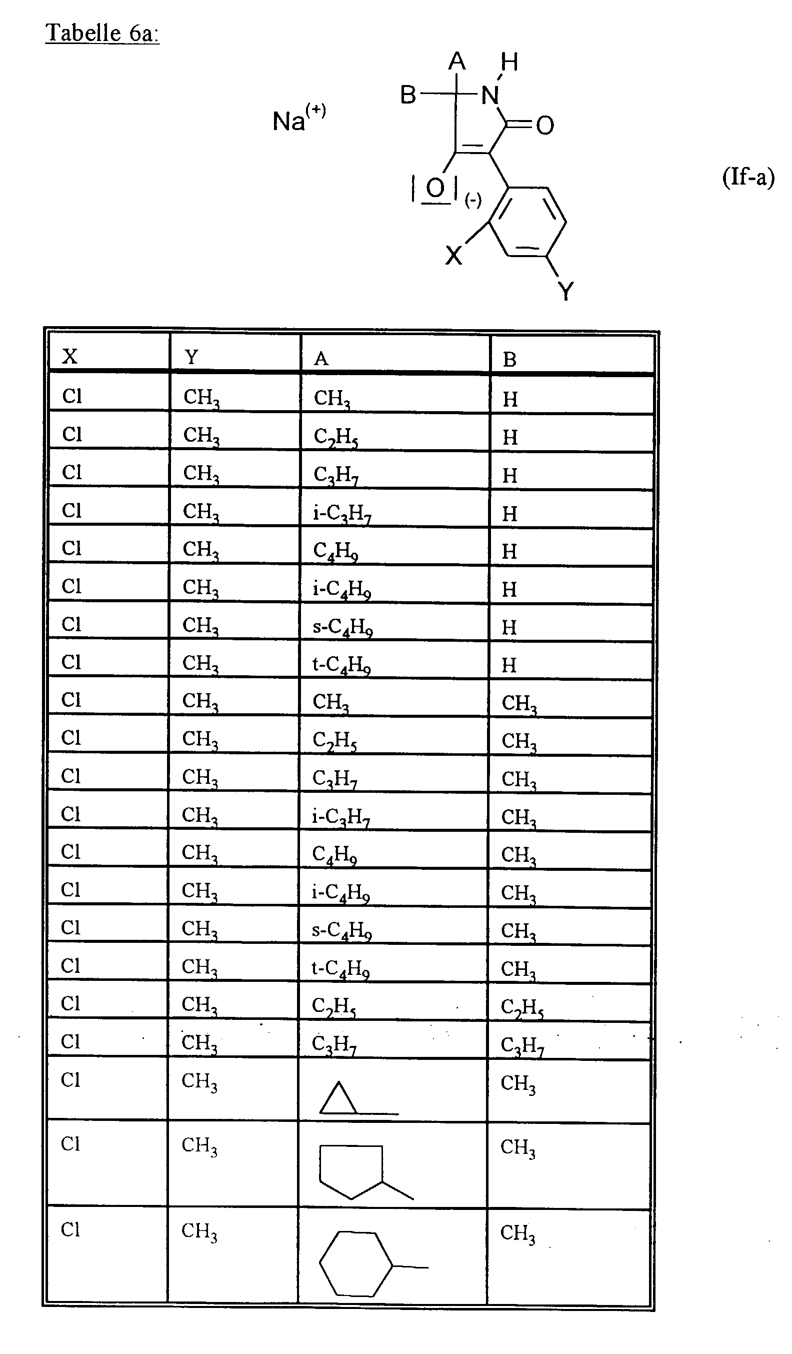

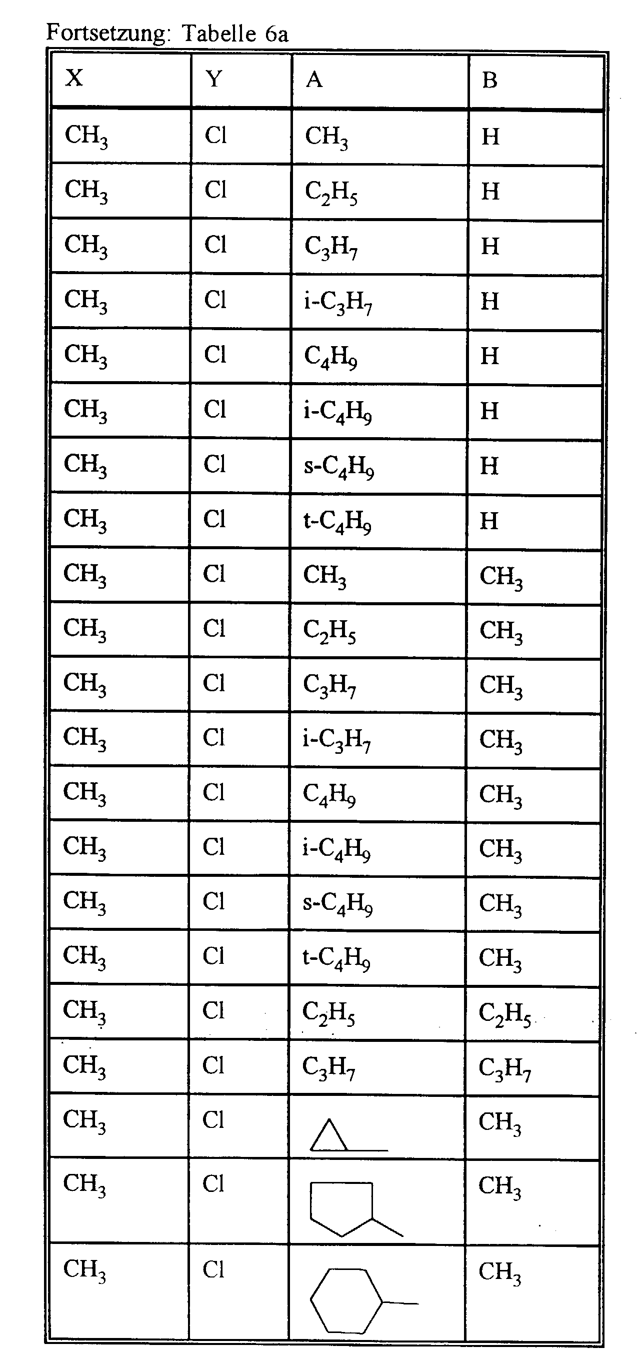

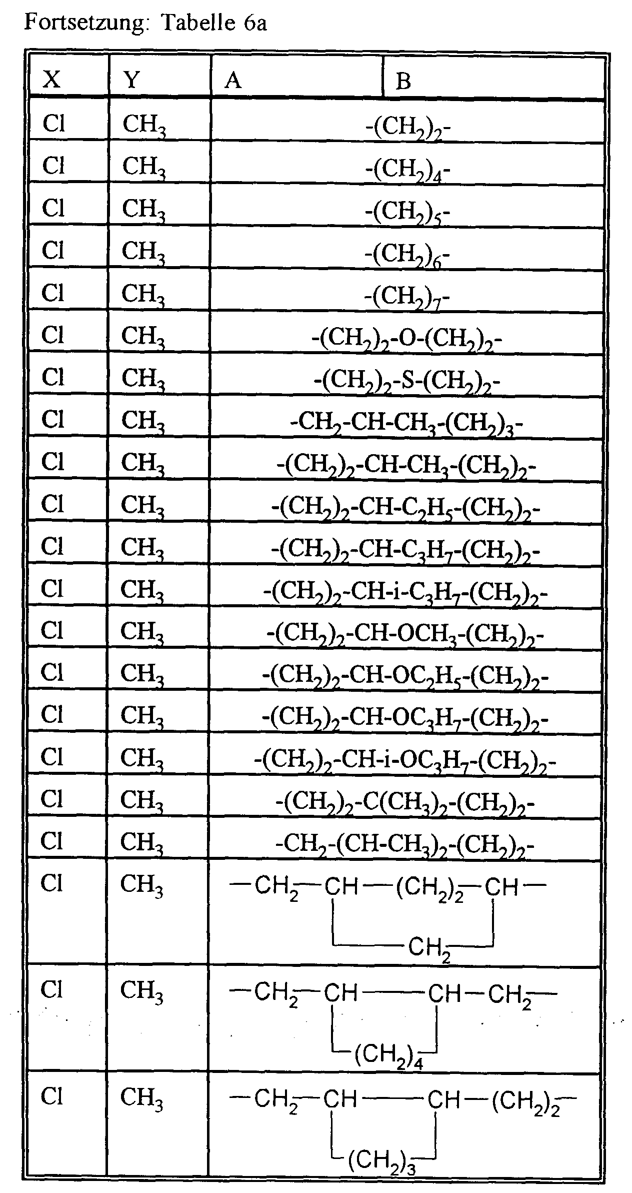

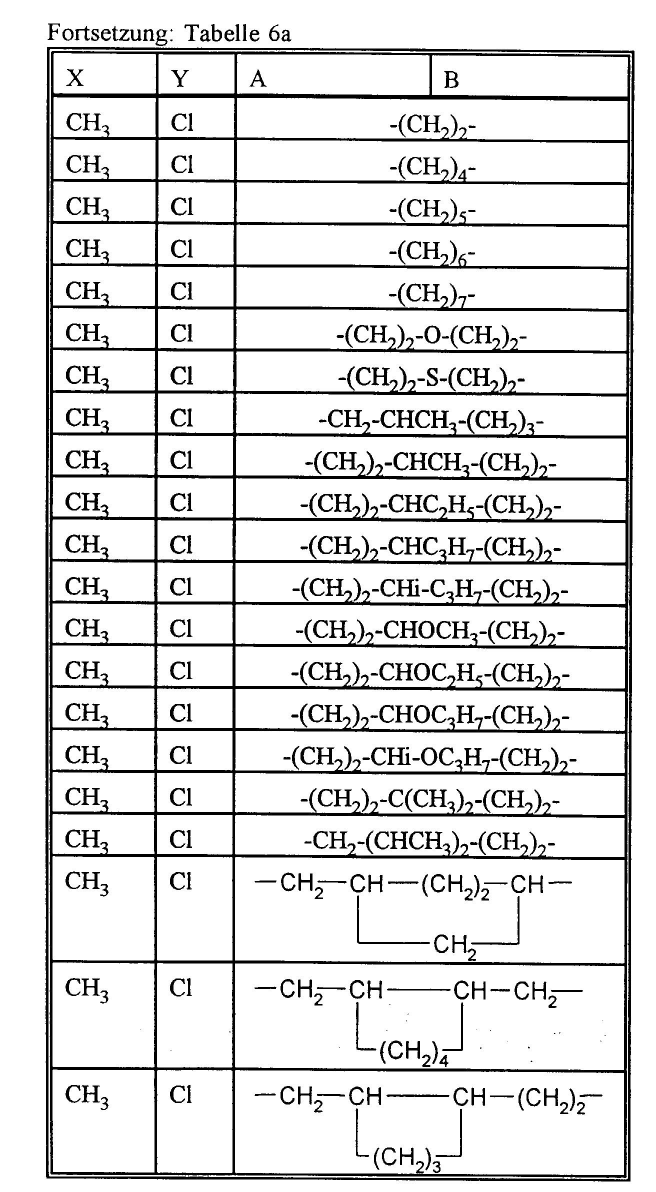

Im einzelnen seien außer den bei den Herstellungsbeispielen genannten Verbindungen die folgenden Verbindungen der Formel (If-b) genannt:

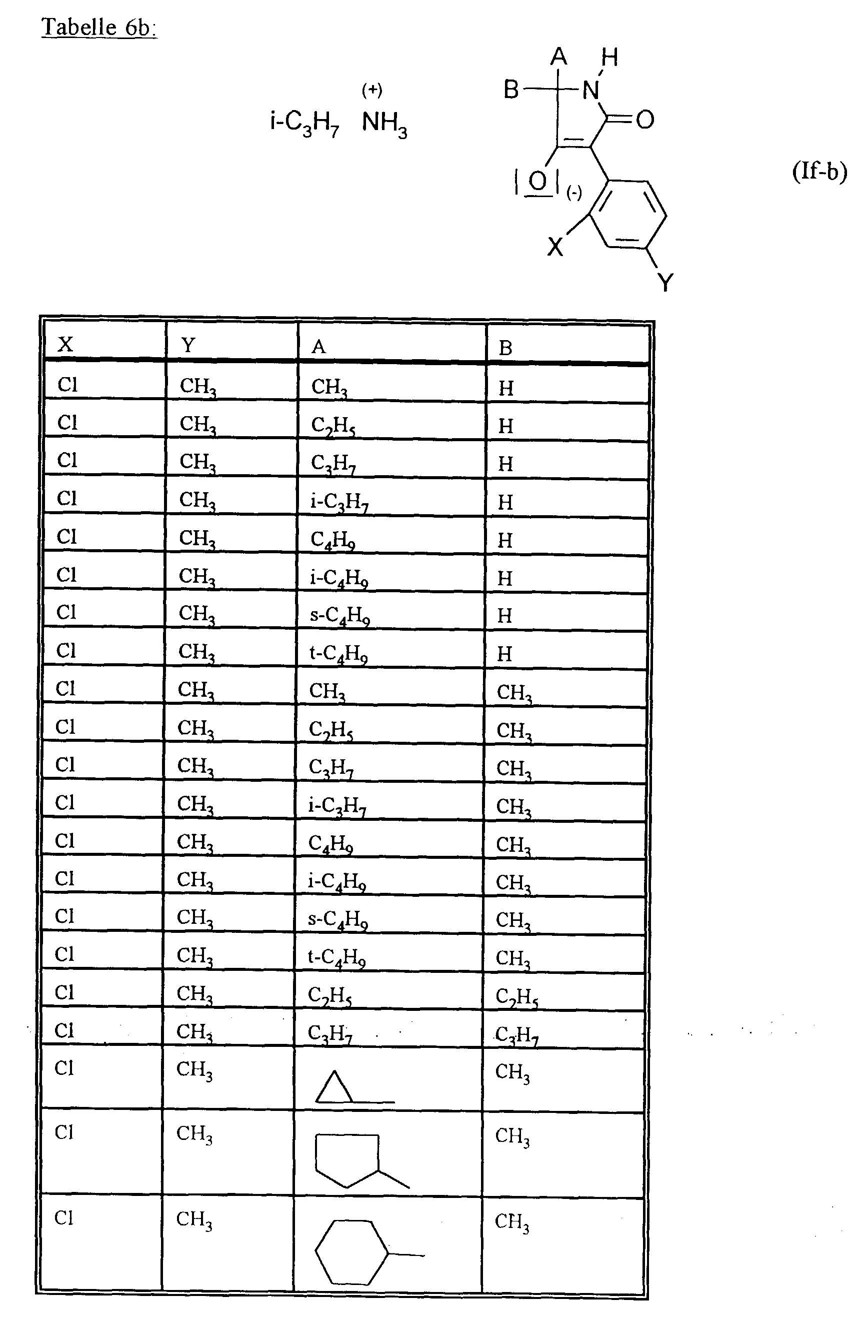

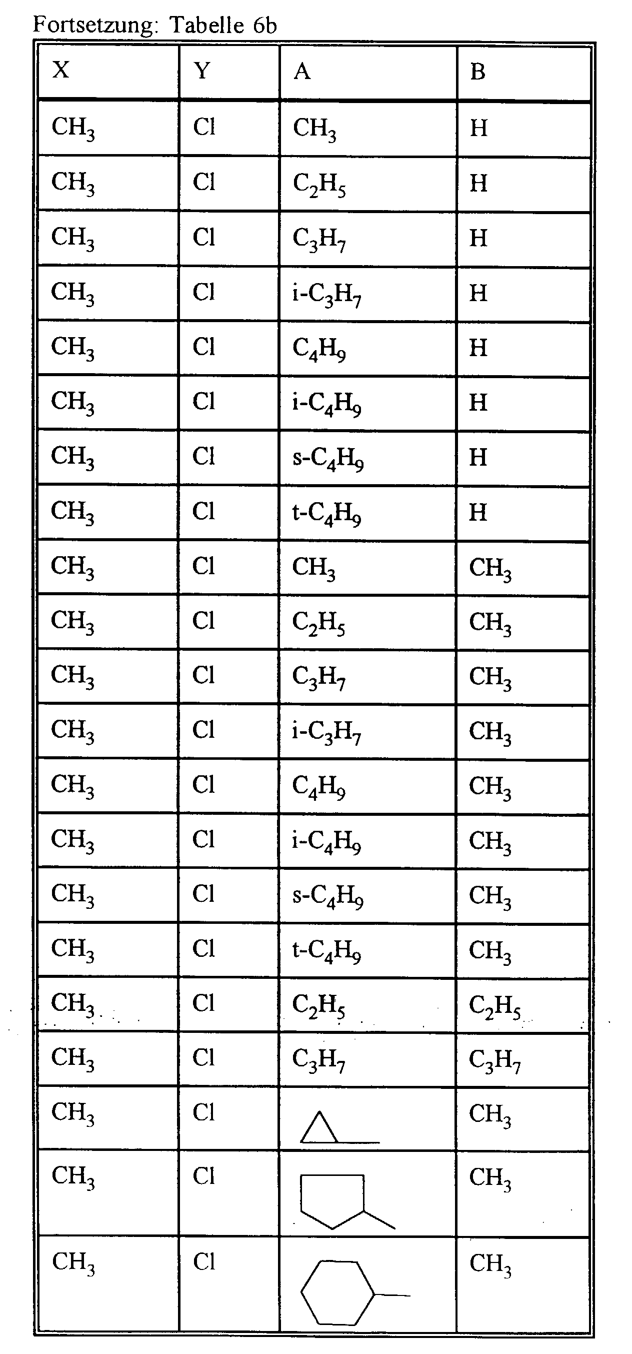

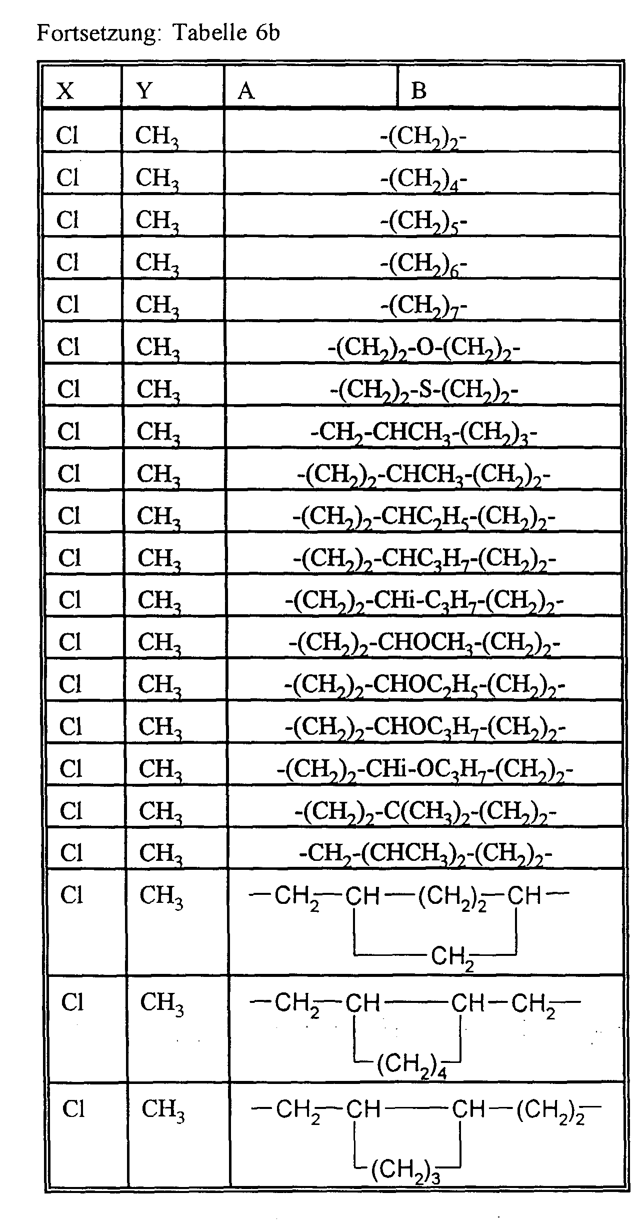

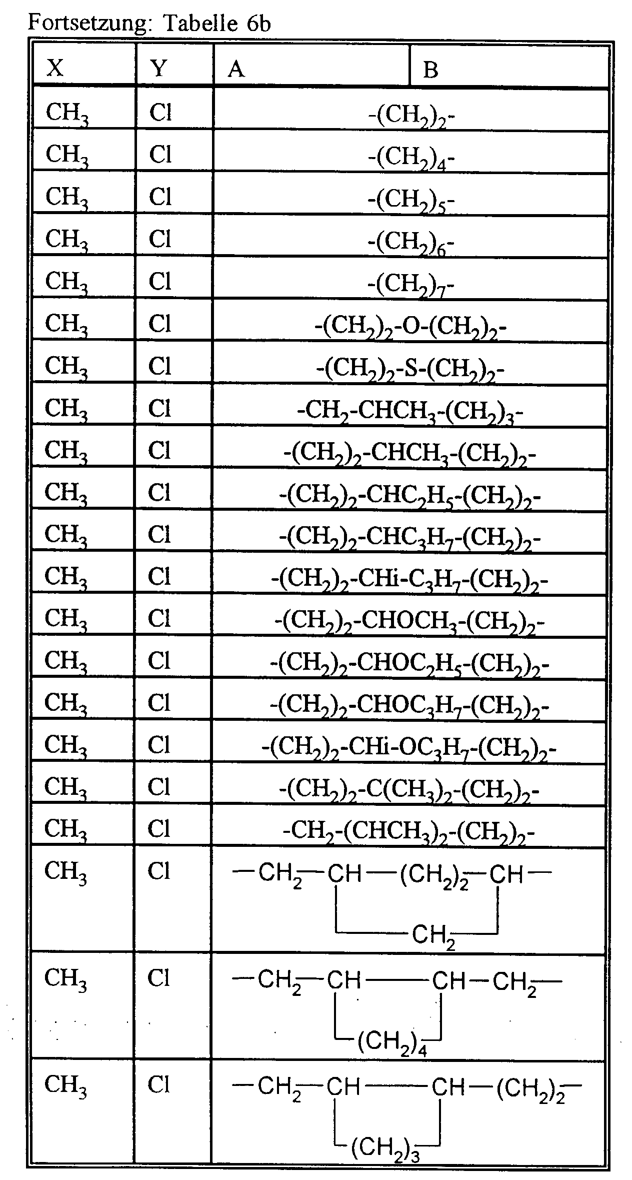

Im einzelnen seien außer den bei den Herstellungsbeispielen genannten Verbindungen die folgenden Verbindungen der Formel (Ig-a) genannt:

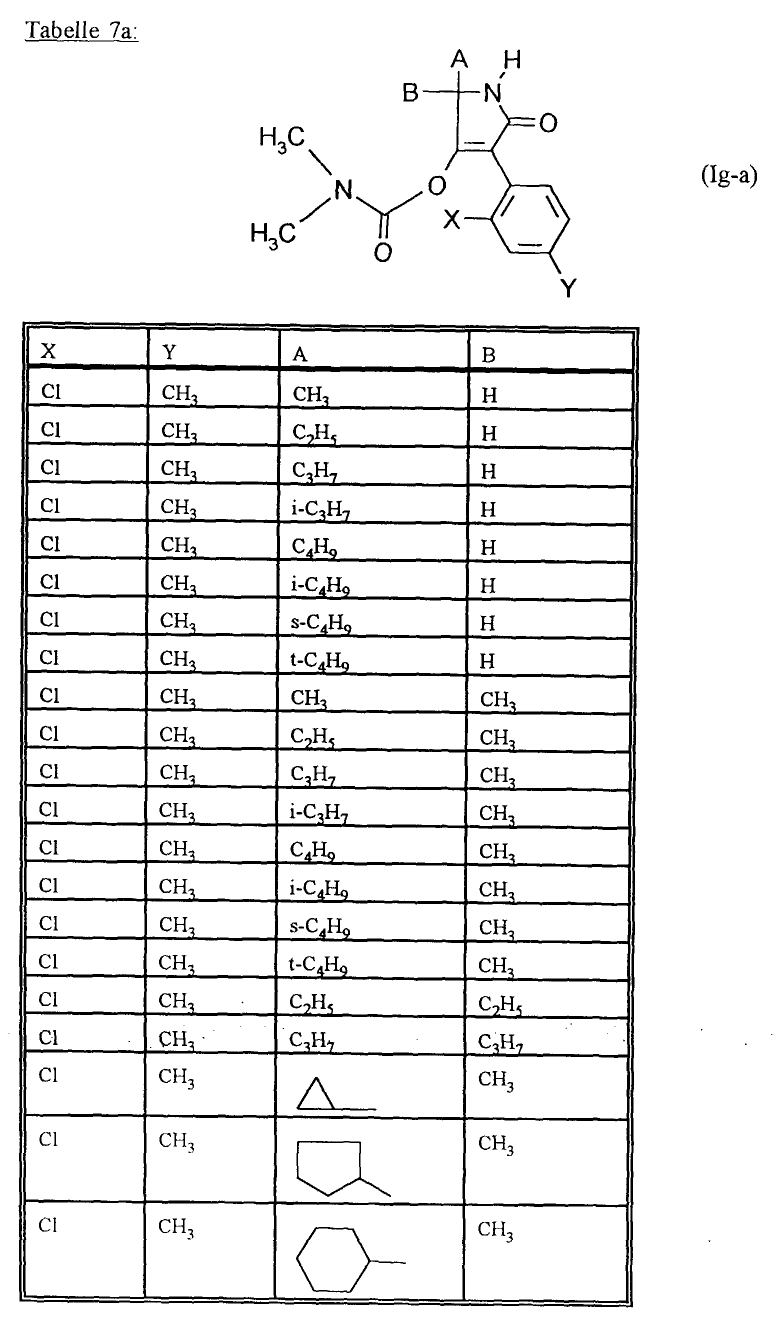

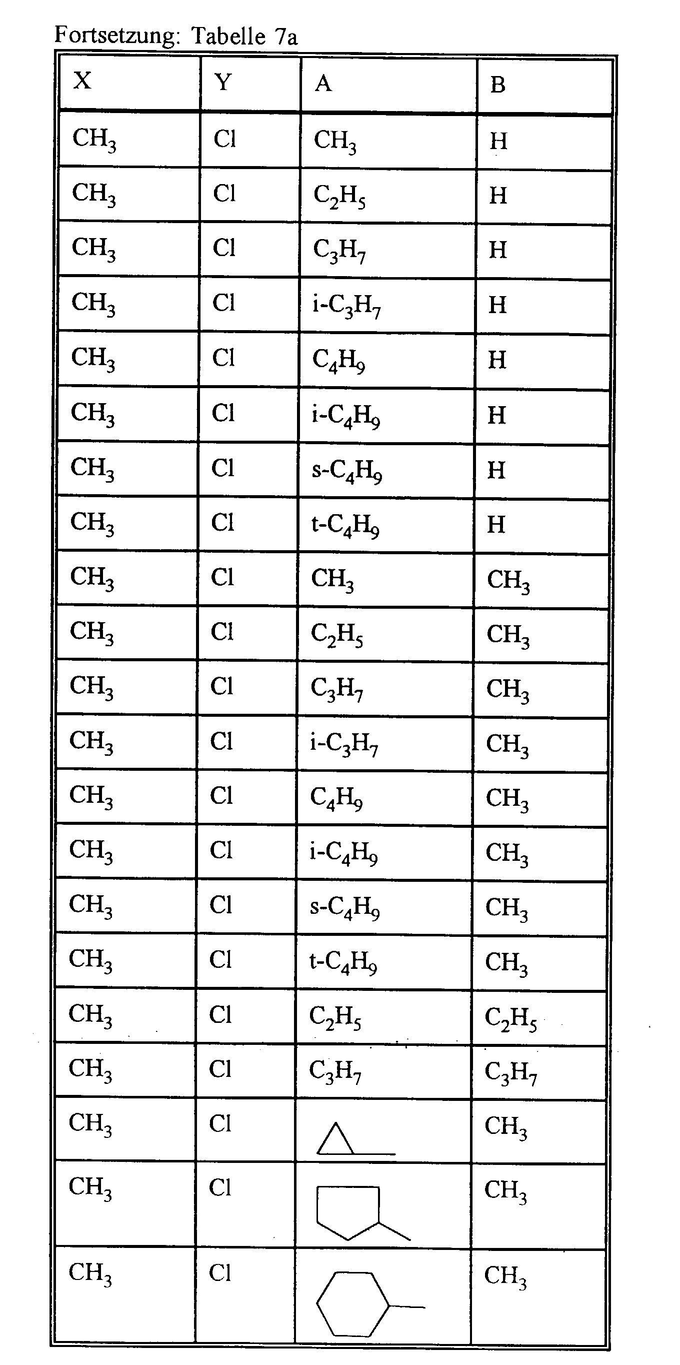

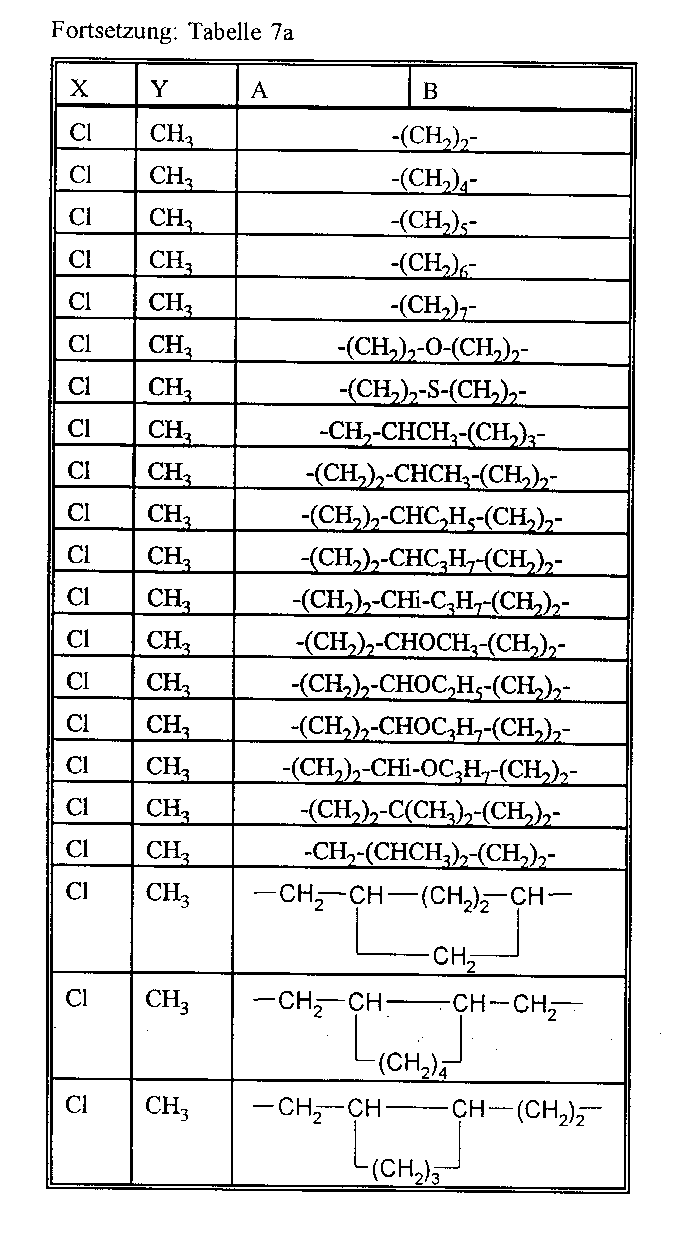

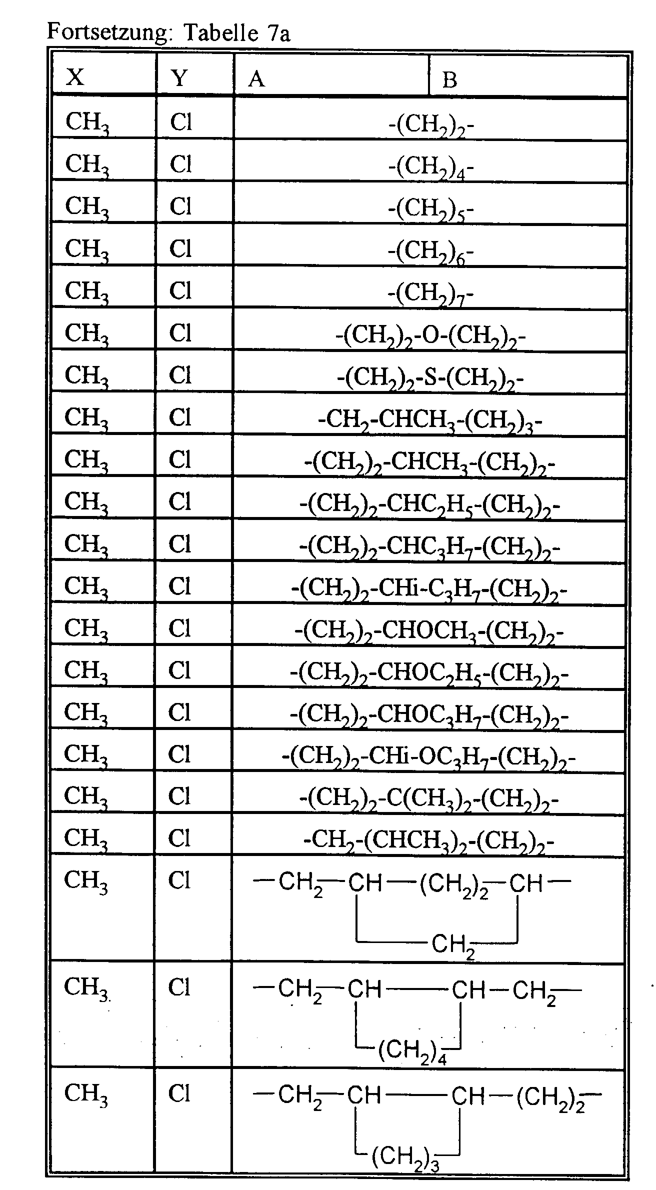

Im einzelnen seien außer den bei den Herstellungsbeispielen genannten Verbindungen die folgenden Verbindungen der Formel (Ig-b) genannt:

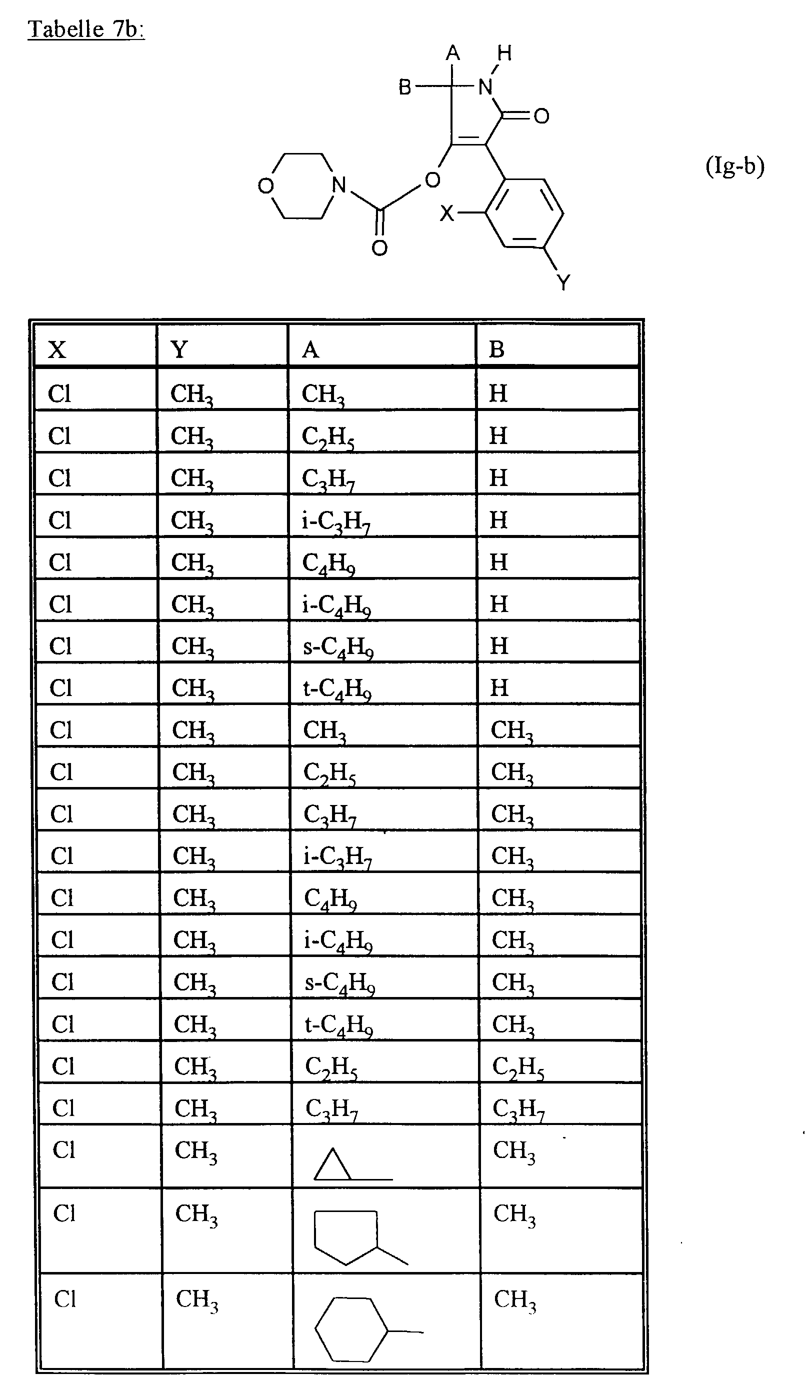

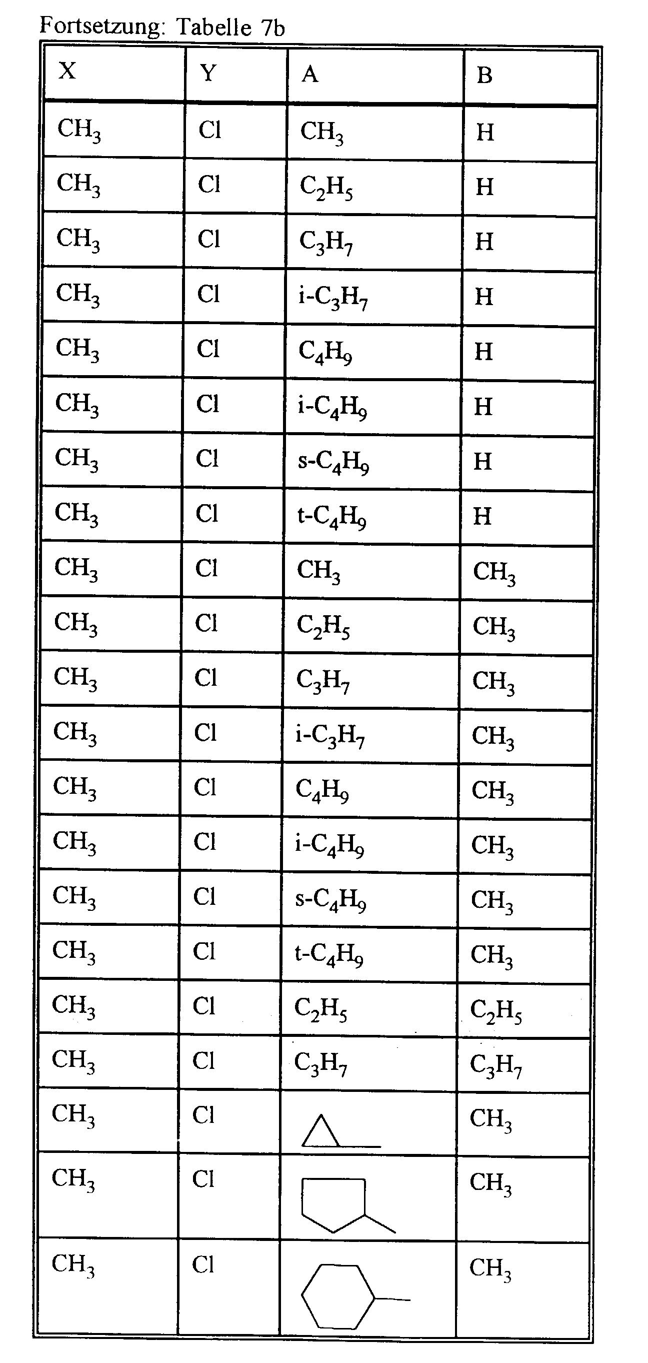

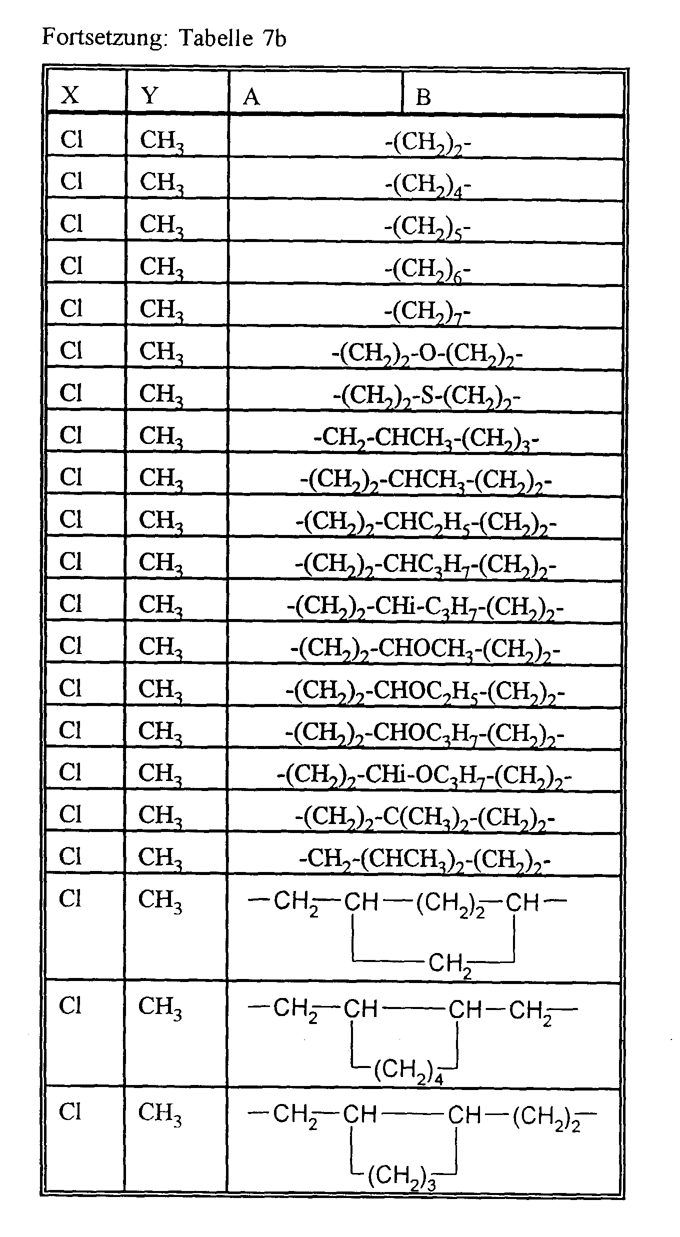

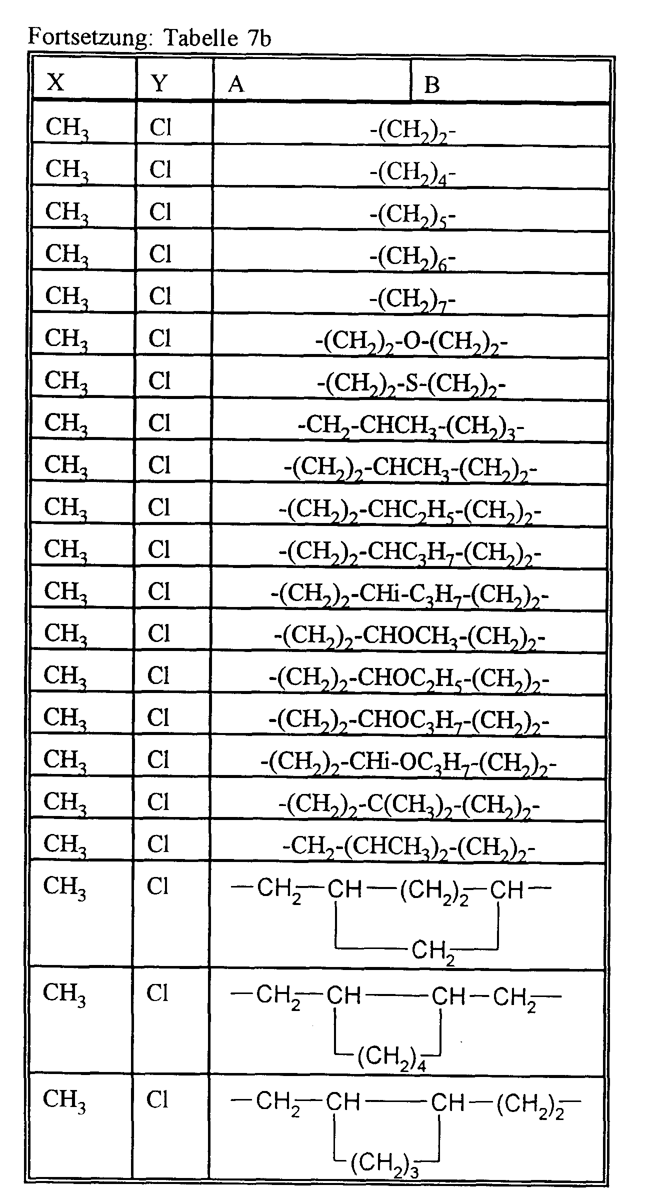

Verwendet man gemäß Verfahren (A) N-(2-Chlor-4-methylphenylacetyl)-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

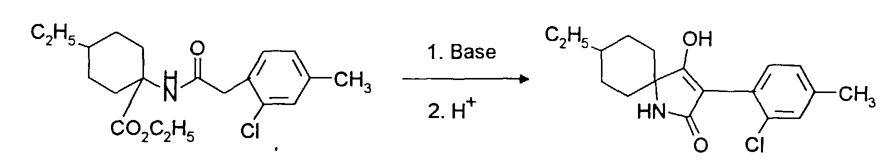

Verwendet man gemäß Verfahren (B_{α}) 3-(2-Methyl-4-chlorphenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

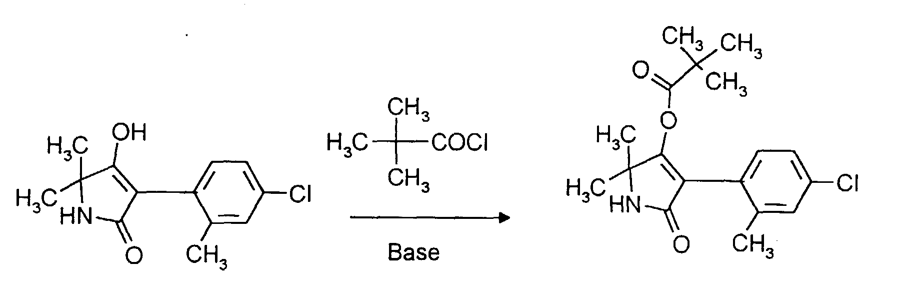

Verwendet man gemäß Verfahren (B_{β}) 3-(2-Brom-4-ethylphenyl)-5-isopropyl-5-methyl-pyrrolidin-2,4-dion und Acetanhydrid als Ausgangsverbindungen, so kann der Verlauf des erfindungsgemäßen Verfahrens durch folgendes Reaktionsschema wiedergegeben werden:

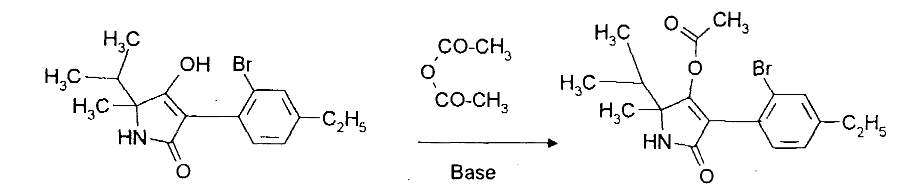

Verwendet man gemäß Verfahren (C) 3-(2-Methyl-4-chlorphenyl)-5,5-diethylpyrrolidin-2,4-dion und Chloramei sensäureethoxyethylester als Ausgangsverbindungen, so kann der Verlauf des erfindungsgemäßen Verfahrens durch folgendes Reaktionsschema wiedergegeben werden:

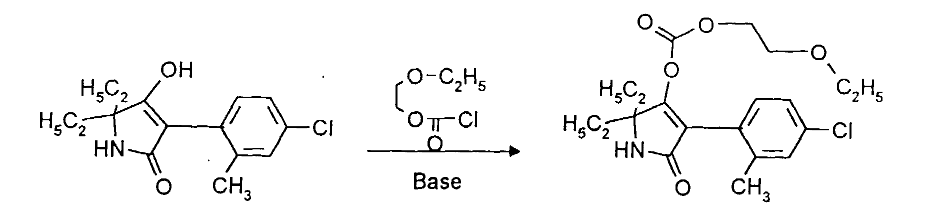

Verwendet man gemäß Verfahren (D_{α}) 3-(2-Chlor-4-methylphenyl)-5,5-pentamethylen-pyrrolidin-2,4-dion und Chlormonothioameisensäuremethylester als Ausgangsprodukte, so kann der Reaktionsverlauf wie folgt wiedergegeben werden:

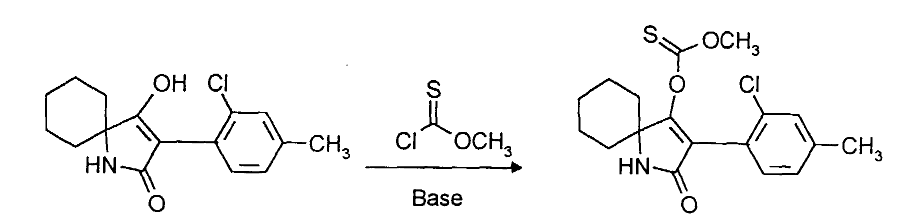

Verwendet man gemäß Verfahren (D_{β}) 3-(2-Brom-4-ethylphenyl)-5,5-ethylmercaptoethyl-pyrrolidin-2,4-dion, Schwefelkohlenstoff und Methyliodid als Ausgangskomponenten, so kann der Reaktionsverlauf wie folgt wiedergegeben werden:

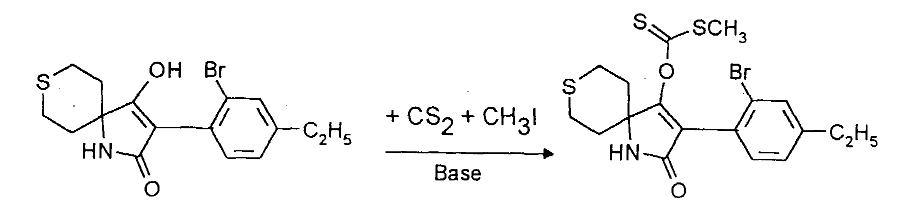

Verwendet man gemäß Verfahren (E) 3-(2-Chlor-4-isopropylphenyl)-5,5-(2-methyl)-pentamethylen-pyrrolidin-2,4-dion und Methansulfonsäurechlorid als Ausgangsprodukt, so kann der Reaktionsverlauf durch folgendes Reaktionsschema wiedergegeben werden:

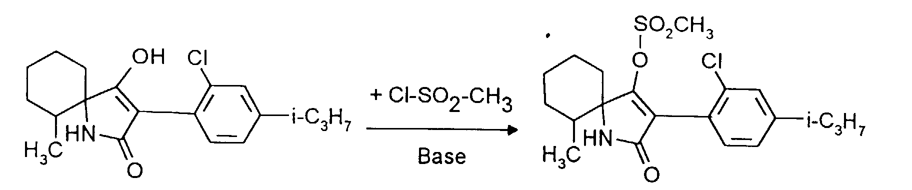

Verwendet man gemäß Verfahren (F) 3-(2-Methyl-4-chlorphenyl)-5-isobutyl-5-methyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

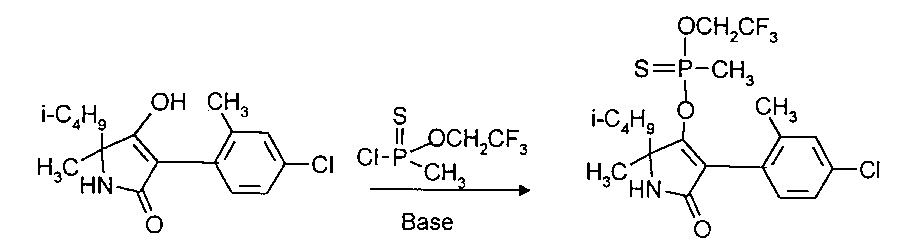

Verwendet man gemäß Verfahren (G) 3-(2-Fluor-4-methylphenyl)-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

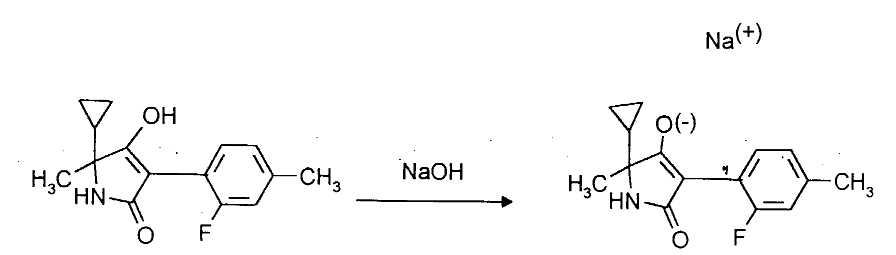

Verwendet man gemäß Verfahren (H_{α}) 3-(2-Chlor-4-ethylphenyl)-5,5-hexamethylen-pyrrolidin-2,4-dion und Ethylisocyanat als Ausgangsprodukte, so kann der Reaktionsverlauf durch folgendes Schema wiedergegeben werden:

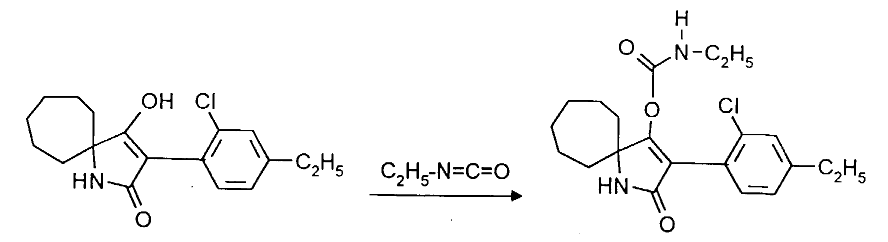

Verwendet man gemäß Verfahren (H_{β}) 3-(2-Methyl-4-chlorphenyl)-5-methyl-pyrrolidin-2,4-dion und Dimethylcarbamidsäurechlorid als Ausgangsprodukte, so kann der Reaktionsverlauf durch folgendes Schema wiedergegeben werden:

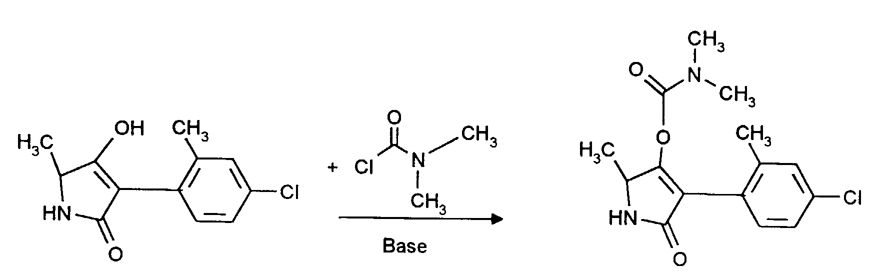

Die bei den erfindungsgemäßen Verfahren (A) als Ausgangsstoffe benötigten Verbindungen der Formel (II) 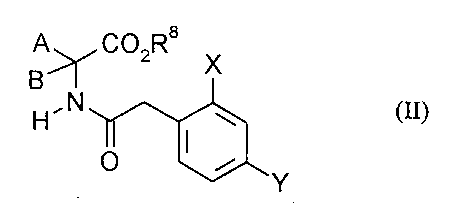 in welcher
- A, B, X, Y und R⁸: die oben angegebene Bedeutung haben,
sind neu.

Man erhält z.B. Acyl-aminosäureester der Formel (II), wenn man Aminosäurederivate der Formel (XIV), 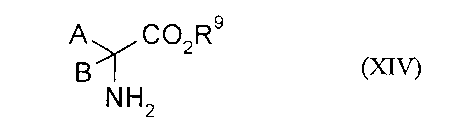 in welcher
- R⁹: für Wasserstoff (XIVa) oder Alkyl, bevorzugt C₁-C₆-Alkyl (XIVb) steht
und
- A und B: die oben angegebene Bedeutung haben,
mit Phenylessigsäurehalogeniden der Formel (XV) 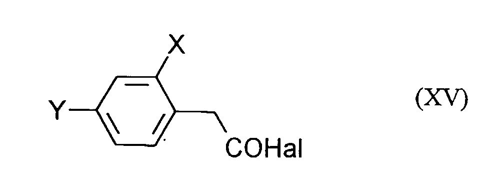 in welcher
- X und Y: die oben angegebene Bedeutung haben und
- Hal: für Chlor oder Brom steht,
acyliert (Chem. Reviews 52, 237-416 (1953); Bhattacharya, Indian J. Chem. 6, 341-5, 1968)
und die dabei für R⁹ = Wasserstoff erhaltenen Acylaminosäuren der Formel (IIa), 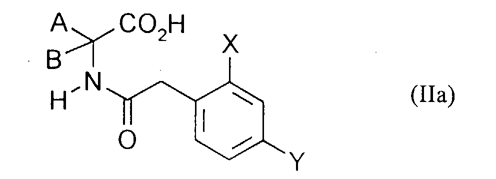 in welcher
- A, B, X und Y: die oben angegebene Bedeutung haben,
verestert (Chem. Ind. (London) 1568 (1968)).

Die substituierten cyclischen Aminocarbonsäuren der Formel (XIVa) sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 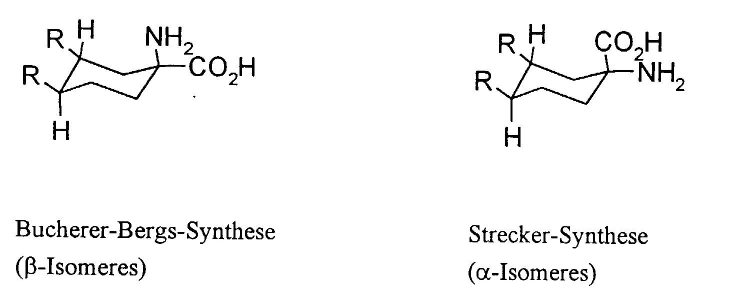 (L. Munday, J. Chem. Soc. 4372 (1961); J.T. Eward, C. Jitrangeri, Can. J. Chem. 53, 3339 (1975).

Weiterhin lassen sich die bei den obigen Verfahren (A) verwendeten Ausgangsstoffe der Formel (II) 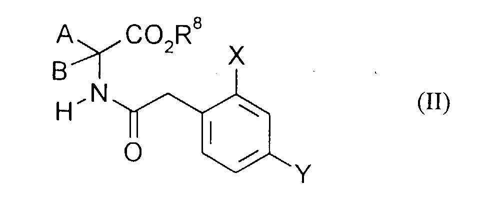 in welcher
- A, B, X, Y und R⁸: die oben angegebene Bedeutung haben,
herstellen, wenn man Aminonitrile der Formel (XVI) 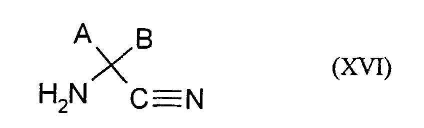 in welcher
- A und B: die oben angegebene Bedeutung haben,
mit Phenylessigsäurehalogeniden der Formel (XV) 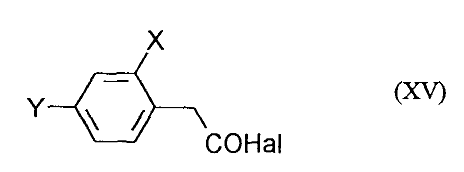 in welcher
- X und Y: die oben angegebene Bedeutung haben und
- Hal: für Chlor oder Brom steht,
zu Verbindungen der Formel (XVII) 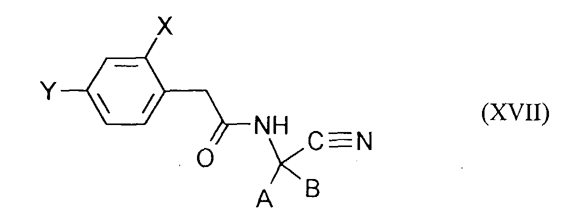 in welcher
- A, B, X und Y: die oben angegebene Bedeutung haben,
umsetzt, und diese anschließend einer schwefelsauren Alkoholyse unterwirft.

Die Verbindungen der Formel (XVII) sind ebenfalls neu.

Beispielhaft aber nicht begrenzend seien außer den bei den Herstellungsbeispielen genannten Zwischenprodukten die folgenden Verbindungen der Formel (II) genannt:
N-(2-Chlor-4-methylphenylacetyl)-alanin-methylester
N-(2-Chlor-4-methylphenylacetyl)-leucin-methylester
N-(2-Chlor-4-methylphenylacetyl)-isoleucin-methylester
N-(2-Chlor-4-methylphenylacetyl)-valin-methylester
N-(2-Chlor-4-methylphenylacetyl)-aminoisobuttersäure-methylester
N-(2-Chlor-4-methylphenylacetyl)-2-ethyl-2-aminobuttersäure-methylester
N-(2-Chlor-4-methylphenylacetyl)-2-methyl-2-aminovaleriansäure-methylester
N-(2-Chlor-4-methylphenylacetyl)-2,3-dimethyl-2-aminovaleriansäure-methylester
N-(2-Chlor-4-methylphenylacetyl)-1-amino-cyclopentancarbonsäure-methylester
N-(2-Chlor-4-methylphenylacetyl)-1-amino-cyclohexancarbonsäure-methylester
N-(2-Chlor-4-methylphenylacetyl)-1-amino-cycloheptancarbonsäure-methylester
N-(2-Chlor-4-methylphenylacetyl)-1-amino-cyclooktancarbonsäure-methylester
N-(4-Chlor-2-methylphenylacetyl)-alanin-methylester
N-(4-Chlor-2-methylphenylacetyl)-leucin-methylester
N-(4-Chlor-2-methylphenylacetyl)-isoleucin-methylester
N-(4-Chlor-2-methylphenylacetyl)-valin-methylester
N-(4-Chlor-2-methylphenylacetyl)-aminoisobuttersäure-methylester
N-(4-Chlor-2-methylphenylacetyl)-2-ethyl-2-aminobuttersäure-methylester
N-(4-Chlor-2-methylphenylacetyl)-2-methyl-2-aminovaleriansäure-methylester
N-(4-Chlor-2-methylphenylacetyl)-2,3-dimethyl-2-aminovaleriansäure-methylester
N-(4-Chlor-2-methylphenylacetyl)-1-amino-cyclopentancarbonsäure-methylester
N-(4-Chlor-2-methylphenylacetyl)-1-amino-cyclohexancarbonsäure-methylester
N-(4-Chlor-2-methylphenylacetyl)-1-amino-cycloheptancarbonsäure-methylester
N-(4-Chlor-2-methylphenylacetyl)-1-amino-cyclooktancarbonsäure-methylester
N-(2-Chlor-4-methyl-phenylacetyl)-1-amino-2-methyl-cyclohexancarbonsäure-methylester,
N-(2-Chlor-4-methyl-phenylacetyl)-1-amino-3-methyl-cyclohexancarbonsäure-methylester,
N-(2-Chlor-4-methyl-phenylacetyl)-1-amino-4-methyl-cyclohexancarbonsäure-methylester,
N-(2-Chlor-4-methyl-phenylacetyl)-1-amino-3,4-dimethyl-cyclohexancarbonsäure-methylester,
N-(2-Chlor-4-methyl-phenylacetyl)-1-amino-4-ethyl-cyclohexancarbonsäure-methylester,
N-(2-Chlor-4-methyl-phenylacetyl)-1-amino-4-isopropyl-cyclohexancarbonsäure-methylester,
N-(2-Chlor-4-methyl-phenylacetyl)-1-amino-4-tert.-butyl-cyclohexancarbonsäure-methylester,
N-(2-Chlor-4-methyl-phenylacetyl)-1-amino-4-methoxy-cyclohexancarbonsäure-methylester,
N-(4-Chlor-2-methyl-phenylacetyl)-1-amino-2-methyl-cyclohexancarbonsäure-methylester,
N-(4-Chlor-2-methyl-phenylacetyl)-1-amino-3-methyl-cyclohexancarbonsäure-methylester,
N-(4-Chlor-2-methyl-phenylacetyl)-1-amino-4-methyl-cyclohexancarbonsäure-methylester,
N-(4-Chlor-2-methyl-phenylacetyl)-1-amino-3,4-dimethyl-cyclohexancarbonsäure-methylester,
N-(4-Chlor-2-methyl-phenylacetyl)-1-amino-4-ethyl-cyclohexancarbonsäure-methylester,
N-(4-Chlor-2-methyl-phenylacetyl)-1-amino-4-isopropyl-cyclohexancarbonsäure-methylester,
N-(4-Chlor-2-methyl-phenylacetyl)-1-amino-4-tert.-butyl-cyclohexancarbonsäure-methylester,
N-(4-Chlor-2-methyl-phenylacetyl)-1-amino-4-methoxy-cyclohexancarbonsäure-methylester,

Beispielhaft, aber nicht begrenzend, seien außer den bei den Herstellungsbeispielen genannten Zwischenprodukten die folgenden Verbindungen der Formel (IIa) genannt:
N-(2-Chlor-4-methylphenylacetyl)-alanin
N-(2-Chlor-4-methylphenylacetyl)-leucin
N-(2-Chlor-4-methylphenylacetyl)-isoleucin
N-(2-Chlor-4-methylphenylacetyl)-valin
N-(2-Chlor-4-methylphenylacetyl)-aminoisobuttersäure
N-(2-Chlor-4-methylphenylacetyl)-2-ethyl-2-aminobuttersäure
N-(2-Chlor-4-methylphenylacetyl)-2-methyl-2-aminovaleriansäure
N-(2-Chlor-4-methylphenylacetyl)-2,3-dimethyl-2-aminovaleriansäure
N-(2-Chlor-4-methylphenylacetyl)-1-amino-cyclopentancarbonsäure
N-(2-Chlor-4-methylphenylacetyl)-1-amino-cyclohexancarbonsäure
N-(2-Chlor-4-methylphenylacetyl)-1-amino-cycloheptancarbonsäure
N-(2-Chlor-4-methylphenylacetyl)-1-amino-cyclooktancarbonsäure
N-(4-Chlor-2-methylphenylacetyl)-alanin
N-(4-Chlor-2-methylphenylacetyl)-leucin
N-(4-Chlor-2-methylphenylacetyl)-isoleucin
N-(4-Chlor-2-methylphenylacetyl)-valin
N-(4-Chlor-2-methylphenylacetyl)-aminoisobuttersäure
N-(4-Chlor-2-methylphenylacetyl)-2-ethyl-2-aminobuttersäure
N-(4-Chlor-2-methylphenylacetyl)-2-methyl-2-aminovaleriansäure
N-(4-Chlor-2-methylphenylacetyl)-2,3-dimethyl-2-aminovaleriansäure
N-(4-Chlor-2-methylphenylacetyl)-1-amino-cyclopentancarbonsäure
N-(4-Chlor-2-methylphenylacetyl)-1-amino-cyclohexancarbonsäure
N-(4-Chlor-2-methylphenylacetyl)-1-amino-cycloheptancarbonsäure
N-(4-Chlor-2-methylphenylacetyl)-1-amino-cyclooktancarbonsäure
N-(2-Chlor-4-methyl-phenylacetyl)-1-amino-2-methyl-cyclohexancarbonsäure
N-(2-Chlor-4-methyl-phenylacetyl)-1-amino-3-methyl-cyclohexancarbonsäure
N-(2-Chlor-4-methyl-phenylacetyl)-1-amino-4-methyl-cyclohexancarbonsäure
N-(2-Chlor-4-methyl-phenylacetyl)-1-amino-3,4-dimethyl-cyclohexancarbonsäure
N-(2-Chlor-4-methyl-phenylacetyl)-1-amino-4-ethyl-cyclohexancarbonsäure
N-(2-Chlor-4-methyl-phenylacetyl)-1-amino-4-isopropyl-cyclohexancarbonsäure
N-(2-Chlor-4-methyl-phenylacetyl)-1-amino-4-tert.-butyl-cyclohexancarbonsäure
N-(2-Chlor-4-methyl-phenylacetyl)-1-amino-4-methoxy-cyclohexancarbonsäure
N-(4-Chlor-2-methyl-phenylacetyl)-1-amino-2-methyl-cyclohexancarbonsäure
N-(4-Chlor-2-methyl-phenylacetyl)-1-amino-3-methyl-cyclohexancarbonsäure
N-(4-Chlor-2-methyl-phenylacetyl)-1-amino-4-methyl-cyclohexancarbonsäure
N-(4-Chlor-2-methyl-phenylacetyl)-1-amino-3,4-dimethyl-cyclohexancarbonsäure
N-(4-Chlor-2-methyl-phenylacetyl)-1-amino-4-ethyl-cyclohexancarbonsäure
N-(4-Chlor-2-methyl-phenylacetyl)-1-amino-4-isopropyl-cyclohexancarbonsäure
N-(4-Chlor-2-methyl-phenylacetyl)-1 -amino-4-tert.-butyl-cyclohexancarbonsäure
N-(4-Chlor-2-methyl-phenylacetyl)-1 -amino-4-methoxy-cyclohexancarbonsäure

Verbindungen der Formel (IIa) sind beispielsweise aus den Phenylessigsäurehalogeniden der Formel (XV) und Aminosäuren der Formel (XIVa) nach Schotten-Baumann (Organikum, 9. Auflage, 446 (1970) VEB Deutscher Verlag der Wissenschaften, Berlin) erhältlich.

Die Phenylessigsäurehalogenide der Formel (XV) sind allgemein bekannte Verbindungen der organischen Chemie oder lassen sich nach bekannten Verfahren herstellen.

Die zur Durchführung der erfindungsgemäßen Verfahren (B), (C), (D), (E), (F), (G) und (H) als Ausgangsstoffe benötigten Verbindungen der Formel (Ia) sind durch das erfindungsgemäße Verfahren (A) erhältlich.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX), Metallhydroxide, Metallalkoxide oder Amine der Formel (X) und (XI) und Isocyanate der Formel (XII) oder Carbamidsäurechlorid der Formel (XIII) sind allgemein bekannte Verbindungen der organischen bzw. anorganischen Chemie.

Das Verfahren (A) ist dadurch gekennzeichnet, daß man Verbindungen der Formel (II) in welcher A, B, X, Y und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Als Verdünnungsmittel können vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (Da) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid -eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{β}) setzt man pro Mol Ausgangsverbindung der Formel (Ia) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid (VIII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Halogenalkane, Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallhydroxiden bzw. Metallalkoxiden der Formel (X) oder Aminen der Formel (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Bei Herstellungsverfahren (H_{α}) setzt man pro Mol Ausgangsverbindung der Formel (Ia) ca. 1 Mol Isocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H_{β}) setzt man pro Mol Ausgangsverbindung der Formel (Ia) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werdern, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten einsetzen, wie beispielsweise gegen die Larven des Meerrettichblattkäfers (Phaedon cochleariae) oder gegen die Larven der grünen Reiszikade (Nephotettix cincticeps) und gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzanlid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301/5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxyphenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

### Herstellungsbeispiele:

### Beispiel (Ia-1):

12,42 g Kalium-t-butylat werden in 35 ml trockenem Tetrahydrofuran vorgelegt und unter Rückfluß mit einer Lösung von 16 g N-(4-Chlor-2-methylphenyl)-acetyl-1-amino-cyclohexan-carbonsäure-methylester in 100 ml trockenem Toluol versetzt und 90 Minuten am Rückfluß gekocht. Nach Abkühlen wird die Reaktionslösung mit 150 ml Wasser versetzt, die wäßrige Phase abgetrennt. Die organische Phase wird erneut mit 75 ml Wasser gewaschen. Die wäßrigen Phasen werden vereinigt, mit 16 ml konzentrierter Salzsäure angesäuert und der Niederschlag abgesaugt und getrocknet. Es werden erhalten 11,7 g (81 % der Theorie), Fp.162°C.

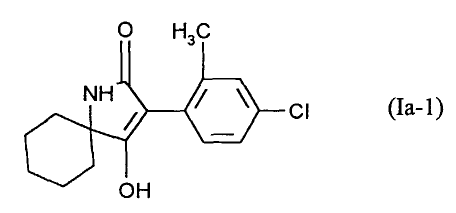

Analog werden die folgenden Verbindungen erhalten:

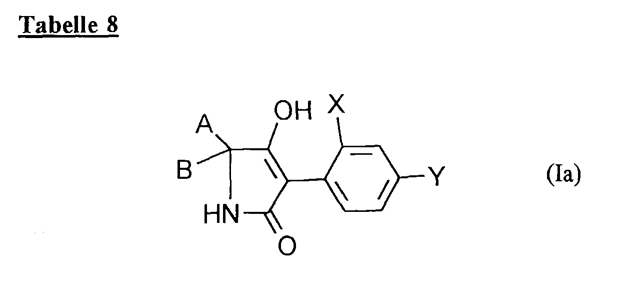

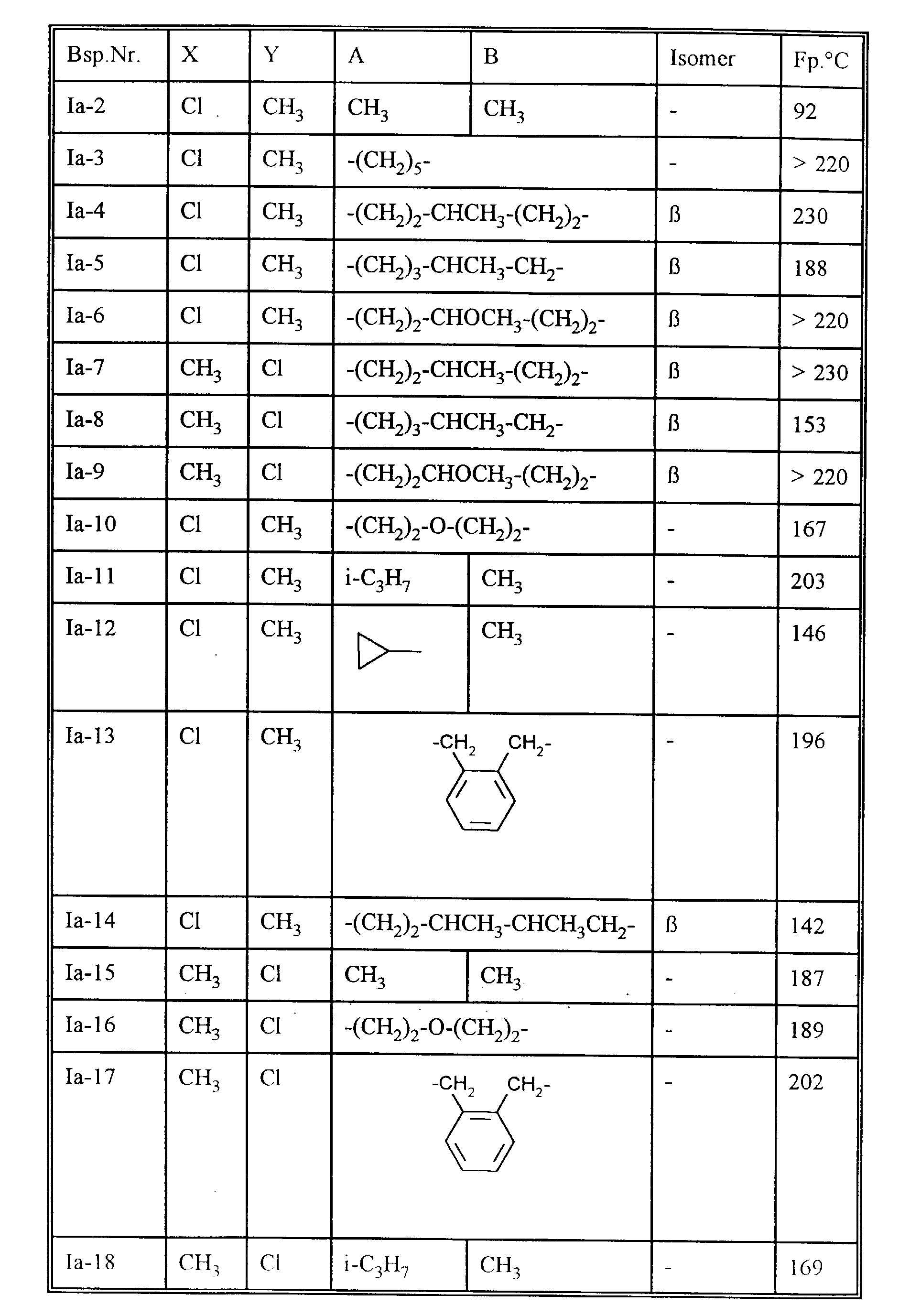

### Beispiel (Ib-1)

4,38 g der Verbindung des Beispiels Ia-1 werden in 70 ml trockenem Methylenchlorid mit 2,1 ml Triethylamin versetzt und bei 0 bis 10°C 1,58 ml Isobuttersäurechlorid in 5 ml trockenem Methylenchlorid zugegeben. Die Reaktionslösung wird zweimal mit 50 ml 0,5 N Natronlauge gewaschen, über Magnesiumsulfat getrocknet und das Lösungsmittel abdestilliert. Es bleiben 2,6 g (47 % der Theorie), Fp. 186°C.

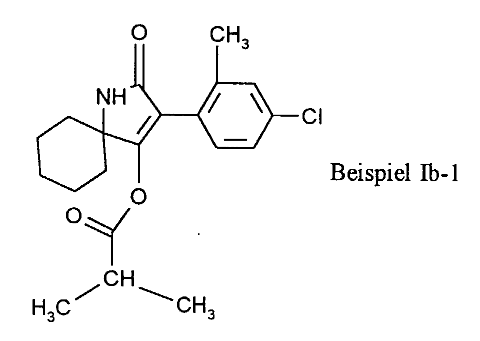

Analog und gemäß den allgemeinen Angaben zur Herstellung werden die folgenden Verbindungen erhalten:

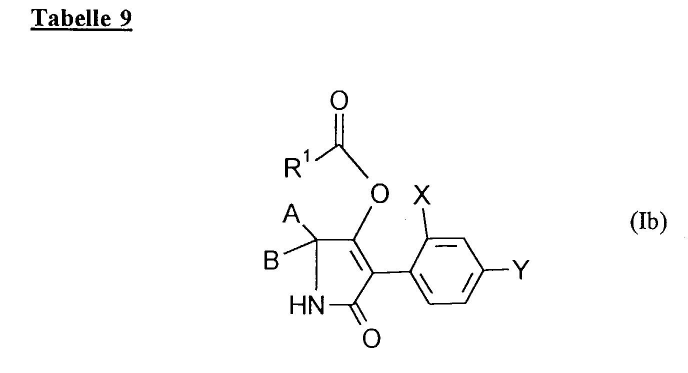

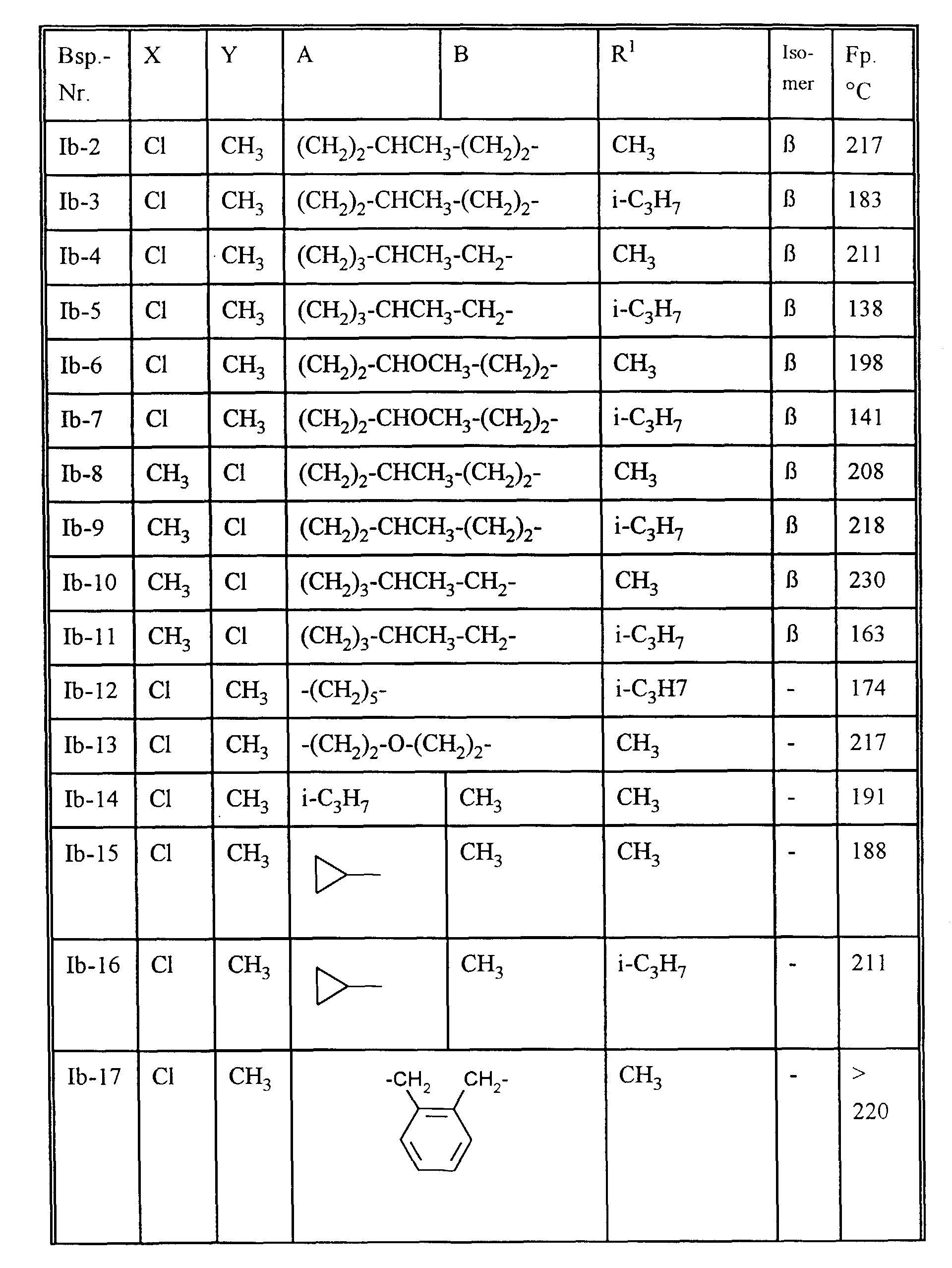

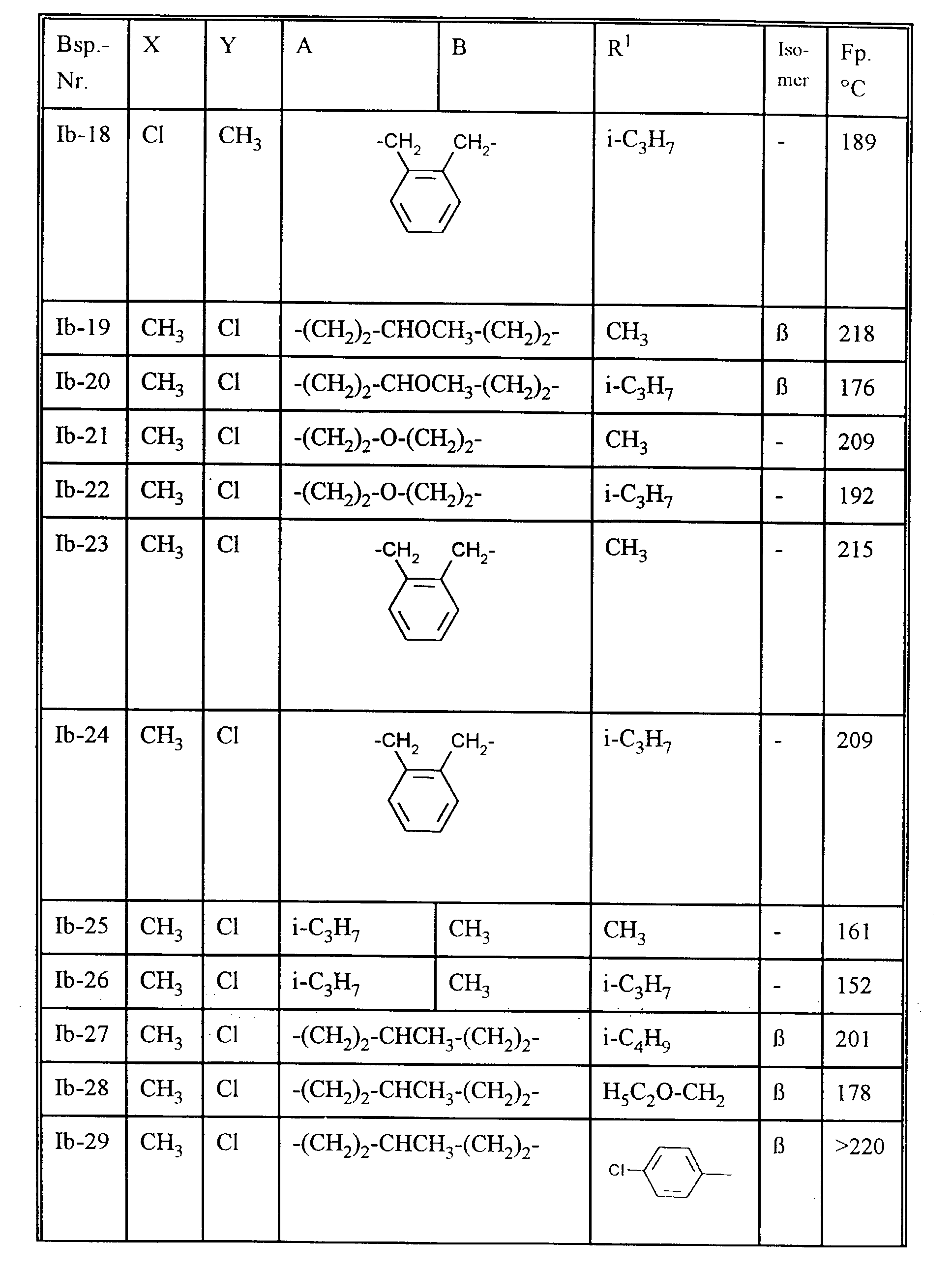

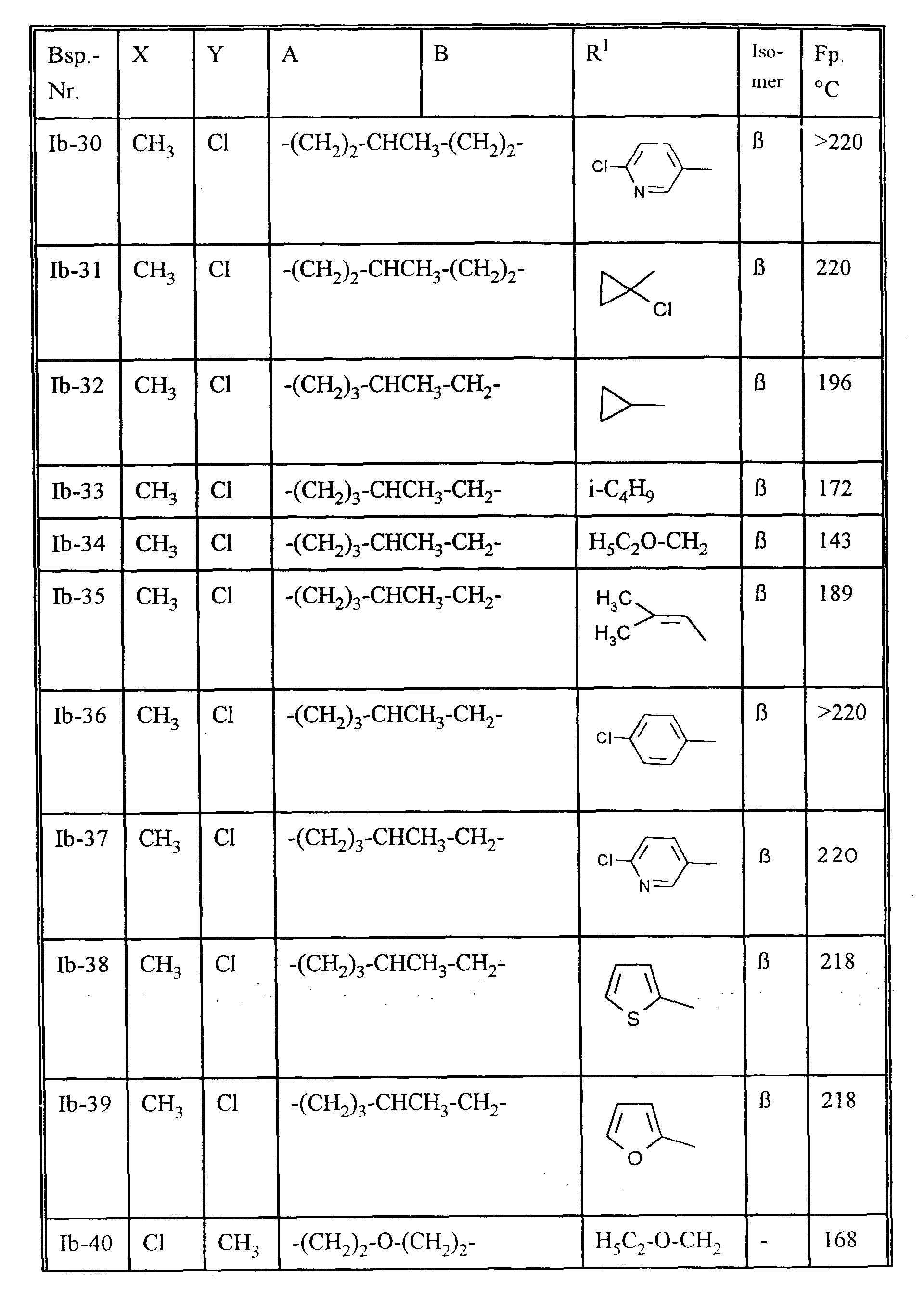

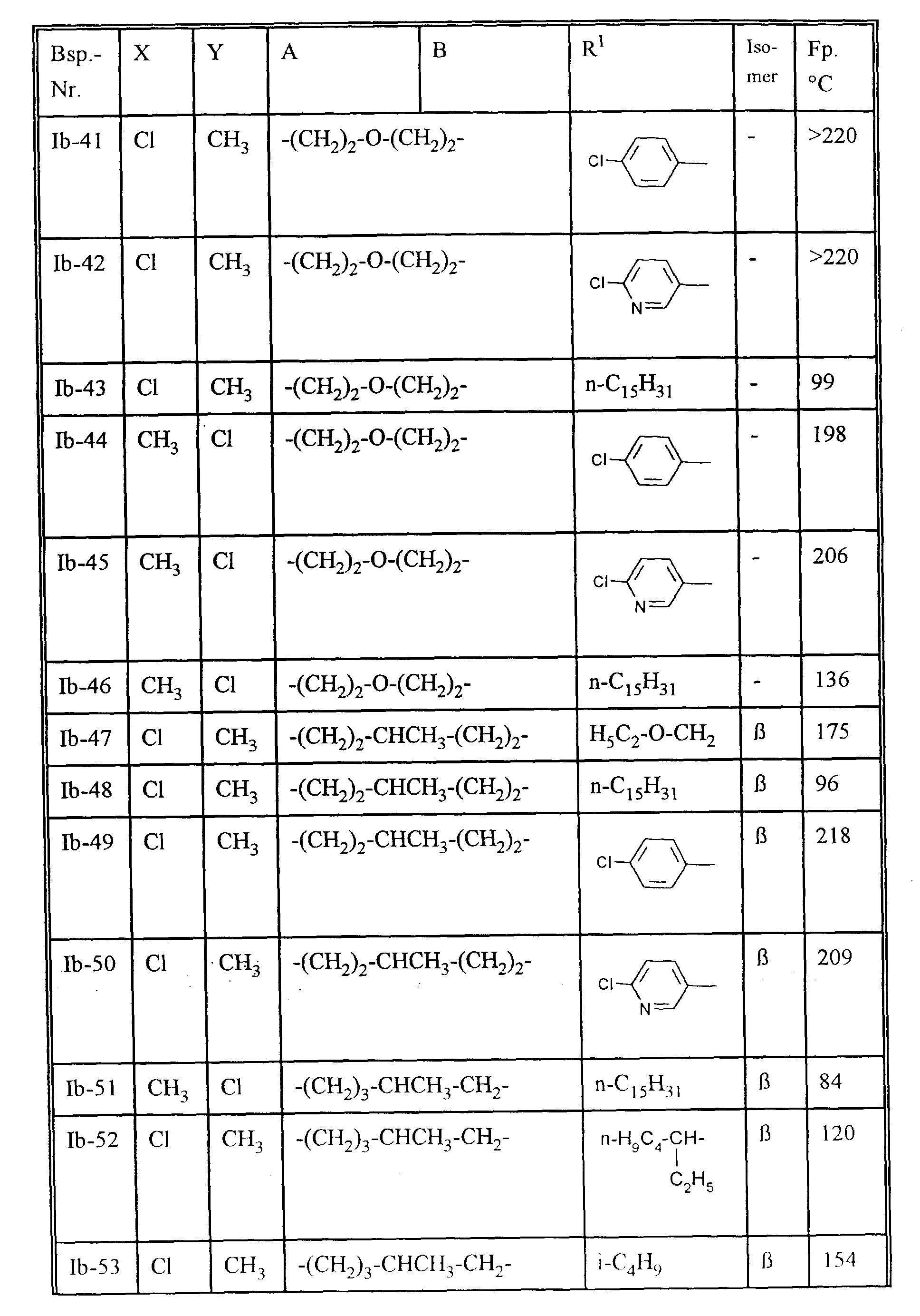

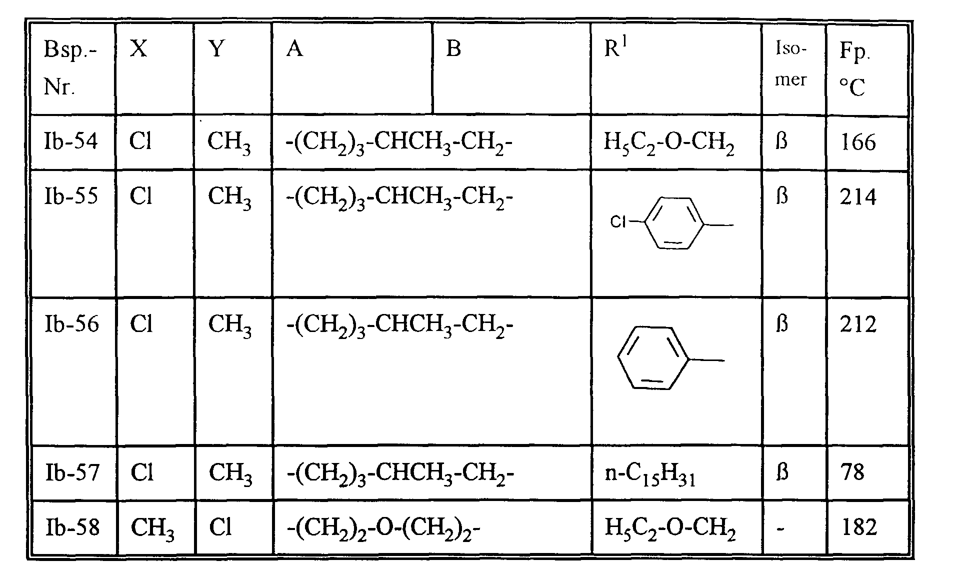

### Beispiel (Ic-1)

4,36 g der Verbindung des Beispiels Ia-1 werden in 70 ml trockenem Methylenchlorid mit 2,1 ml Triethylamin versetzt und bei 0 bis 10°C 1,5 ml Chlorameisensäure-ethylester in 5 ml trockenem Methylenchlorid zugegeben. Die Reaktionslösung wird zweimal mit 50 ml 0,5 N Natronlauge gewaschen, über Magnesiumsulfat getrocknet und das Lösungsmittel abdestilliert. Es bleiben 4,1 g (75 % der Theorie), Fp. 184°C.

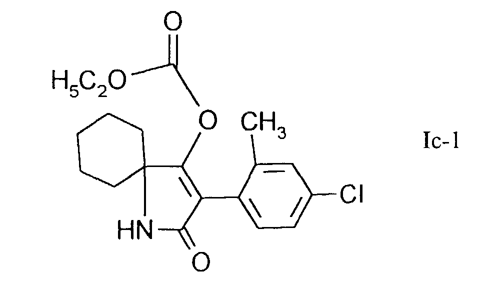

Analog und gemäß den allgemeinen Angaben zur Herstellung werden folgende Verbindungen erhalten:

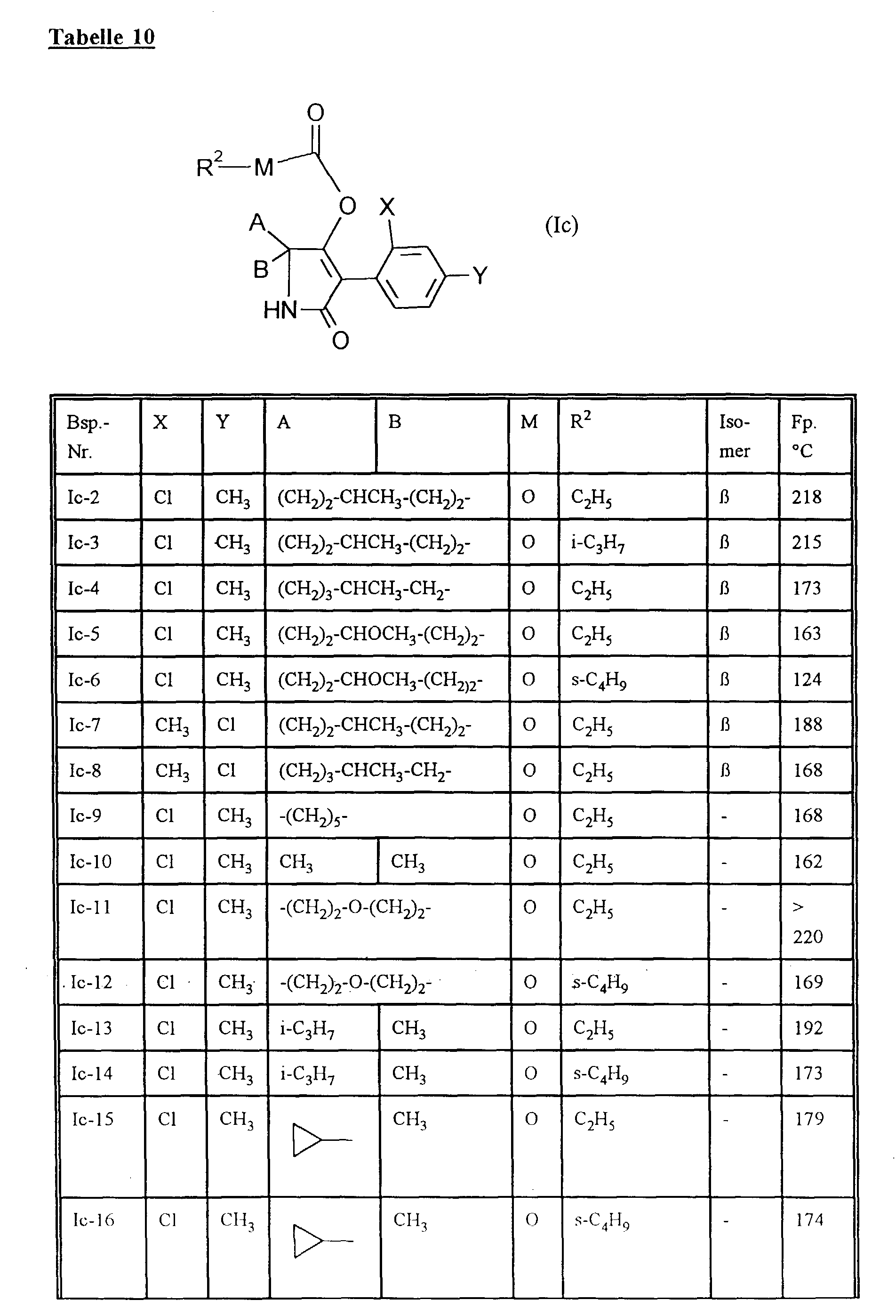

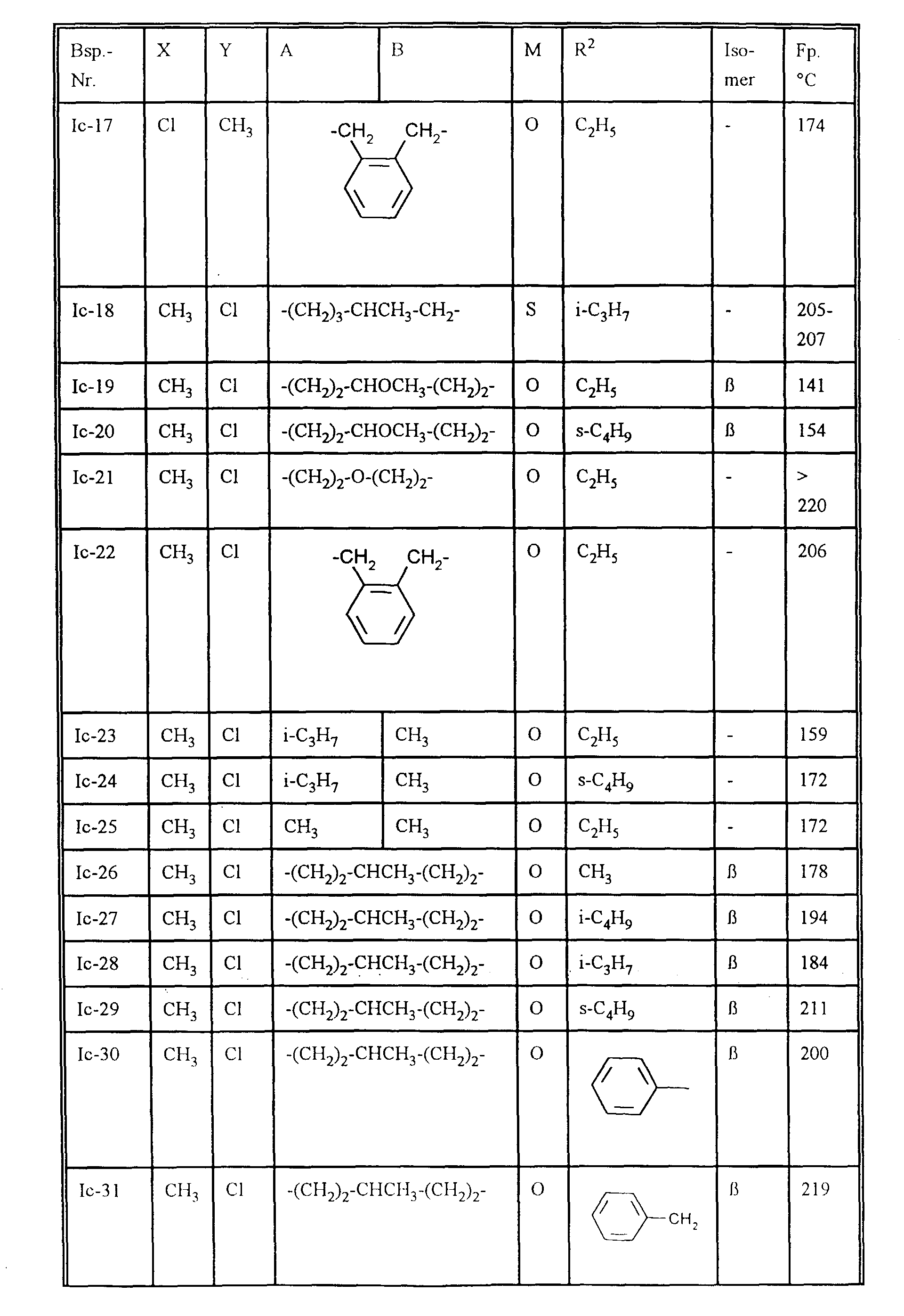

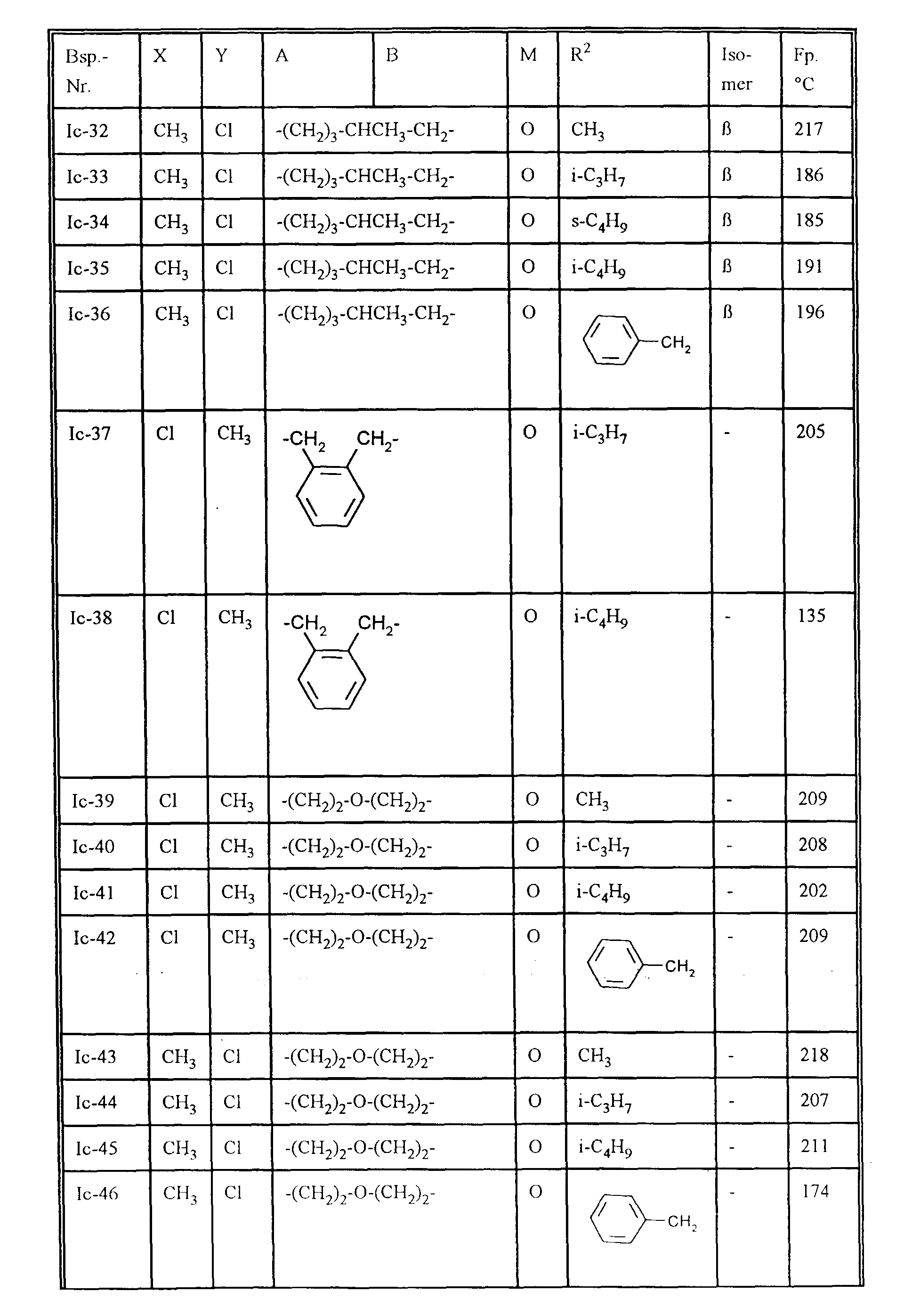

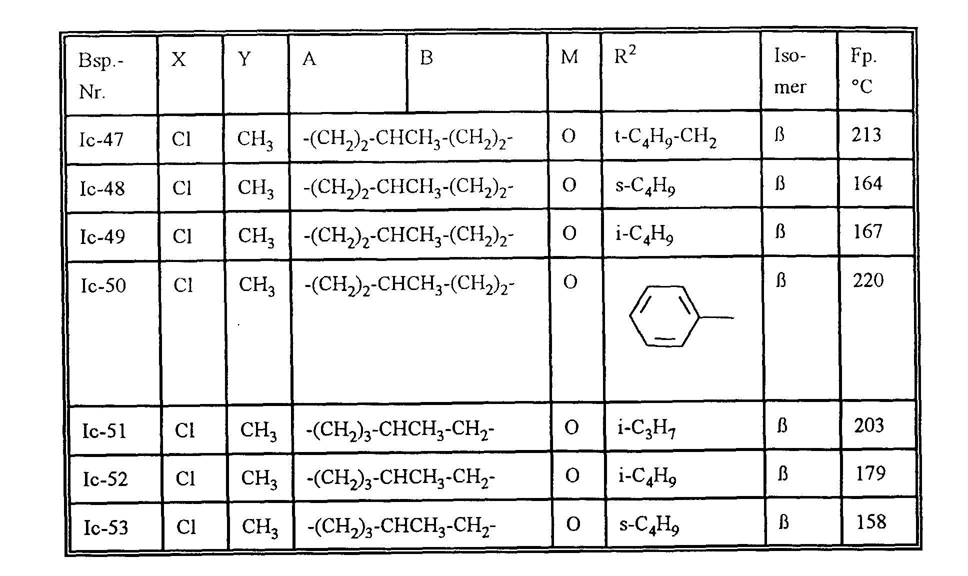

### Beispiel Id-1

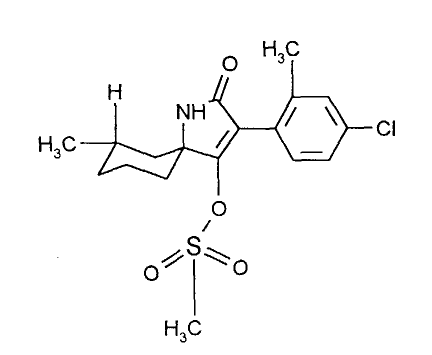

3,05 g der Verbindung des Beispiels Ia-8 werden in 70 ml trockenem Methylenchlorid mit 1,4 ml Triethylamin versetzt und bei 0 bis 10°C 1,15 ml Methansulfonsäurechlorid in 5 ml trockenem Methylenchlorid zugegeben. Die Reaktionslösung wird zweimal mit 50 ml 0,5 N Natronlauge gewaschen, über Magnesiumsulfat getrocknet und das Lösungsmittel abdestilliert. Es bleiben 3 g (78 % der Theorie), Fp. 198°C.

### Beispiel Ie-1

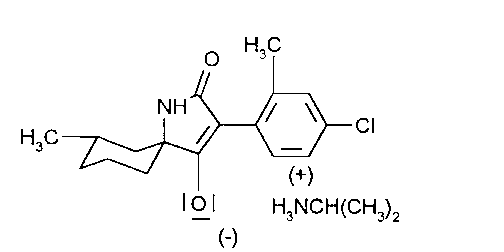

3,06 g (10 mmol) der Verbindung Ia-8 werden in 50 ml wasserfreiem Methylenchlorid suspendiert und mit 1,02 ml (12 mmol) wasserfreiem Isopropylamin versetzt. Nach 15 Min. wird das Lösungsmittel im Vakuum abgedampft. Man erhält 3,6 g (△ 98 % der Theorie) der Verbindung Ie-1 vom Schmp. 152°C.

### Beispiel Ig-1

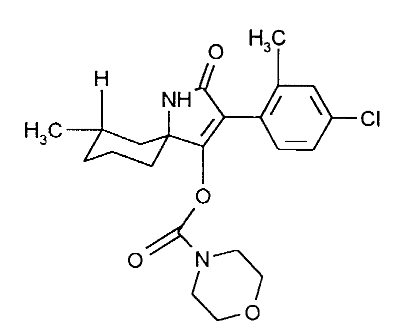

4,59 g der Verbindung des Beispiels Ia-8 werden in 50 ml trockenem Tetrahydrofuran mit 2,28 g Diazabicycloundecen versetzt und bei 0 bis 10°C 1,76 ml Morpholincarbamidsäurechlorid in 5 ml trockenem Tetrahydrofuran zugegeben und anschließend 3 h unter Rückfluß erwärmt. Die Reaktionslösung wird zweimal mit 50 ml 0,5 N Natronlauge gewaschen, über Natriumsulfat getrocknet und das Lösungsmittel abdestilliert. Es bleiben 2,6 g (47 % der Theorie), Fp. 182°C.

### Herstellung der Ausgangsverbindungen:

### Beispiel (II-1)

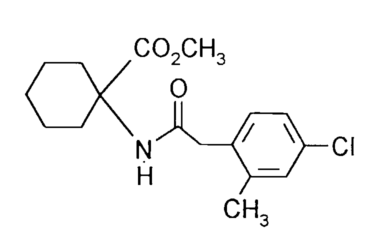

14,5 g (75 mmol) 1-Amino-cyclohexancarbonsäure-methylester-hydrochlorid werden in 180 ml absolutem Tetrahydrofuran vorgelegt, mit 21 ml Triethylamin versetzt, bei 0 bis 10°C 15,2 g (75 mmol) 4-Chlor-2-methyl-phenylessigsäurechlorid in 20 ml absolutem Tetrahydrofuran zugetropft und 1 Stunde bei Raumtemperatur nachgerührt. Man gießt das Reaktionsgemisch in 500 ml Eiswasser + 200 ml HCl, saugt das ausgefallene Produkt ab und trocknet es. Nach Umkristallisieren aus Methyl-tert.-butylether/n-Hexan erhält man 16 g (△ 65 % der Theorie) des oben gezeigten Produkts vom Schmelzpunkt 151°C.

### Beispiel (II-2)

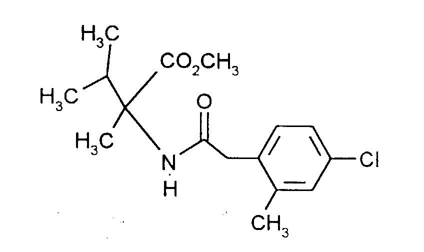

Zu 124,4 g (1,27 Mol) konzentrierter Schwefelsäure tropft man 70,4 g (0,253 Mol) N-(2-Chlor-4-methylphenylacetyl)-2-amino-2,3-dimethyl-buttersäurenitril in 500 ml Methylenchlorid, so daß die Lösung mäßig siedet. Nach zwei Stunden werden 176 ml absolutes Methanol zugetropft und 6 unter Rückfluß erwärmt. Die Reaktionsmischung wird auf 1,25 kg Eis gegossen und mit Methylenchlorid extrahiert. Die vereinigten Methylenchloridphasen werden mit gesättigter Natriumhydrogencarbonatlösung gewaschen, getrocknet, das Lösungsmittel im Vakuum abgedampft und der Rückstand aus Methyl-tert.-butylether/n-Hexan umkristallisiert.
Auf diese Weise erhält man 69,6 g (△ 88 % der Theorie) der Verbindung II-2 vom Schmp. 96°C.

Analog den Beispielen (II-1) und (II-2) erhält man die in Tabelle 11 gezeigten Beispiele.

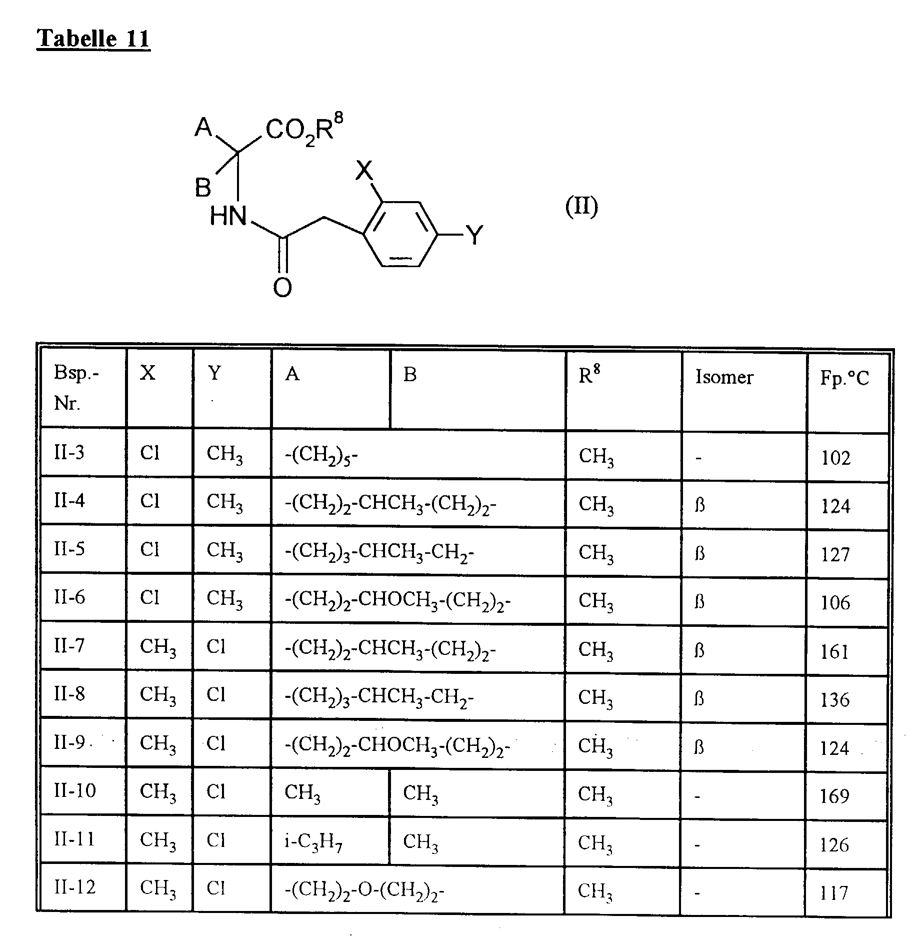

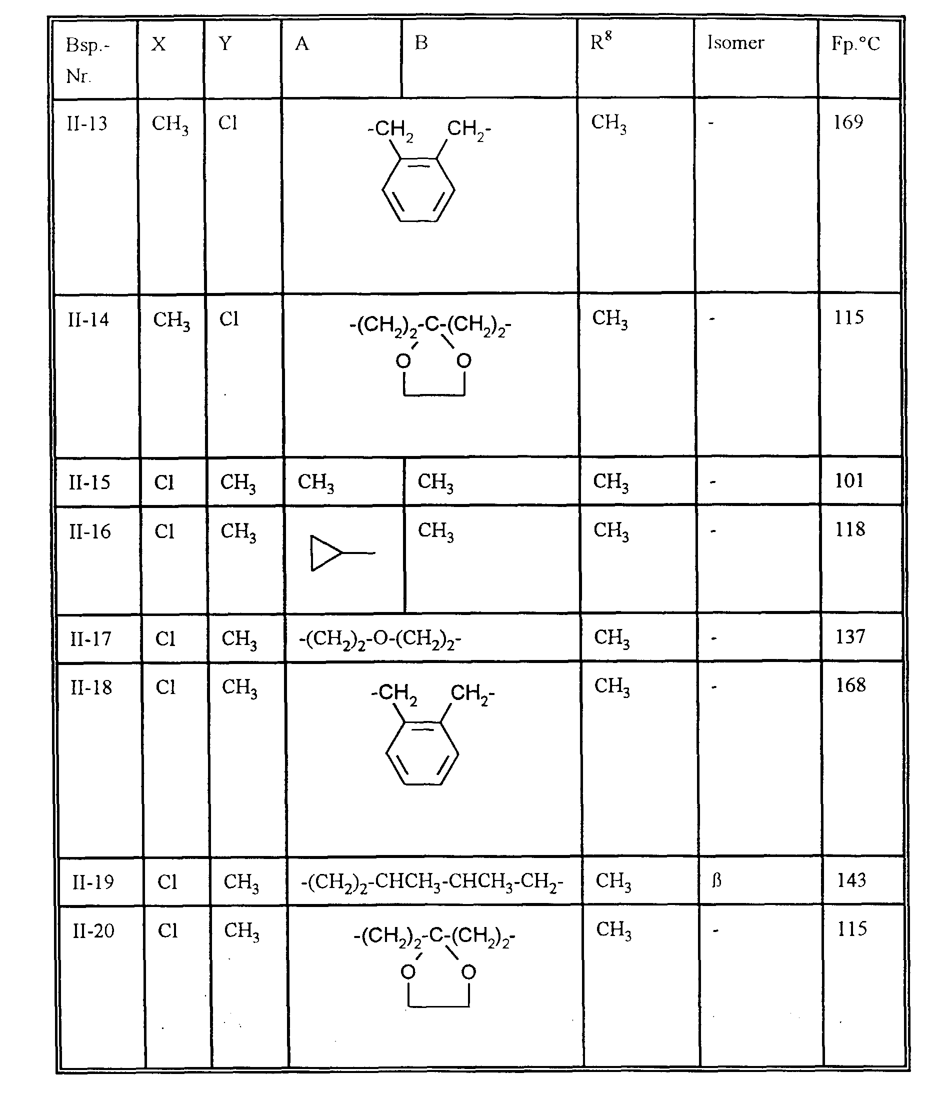

### Beispiel (XVII-1)

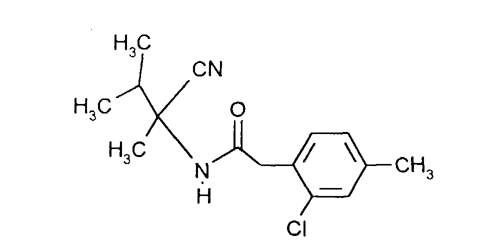

33,6 g (0,3 Mol) 2-Amino-2,3-dimethyl-buttersäurenitril werden in 450 ml absolutem Tetrahydrofuran vorgelegt, mit 42 ml Triethylamin versetzt und bei 0 bis 10°C 60,9 g 2-Chlor-4-methyl-phenylessigsäurechlorid zugetropft. Man rührt eine Stunde bei Raumtemperatur nach, rührt den Ansatz in 1,3 1 Eiswasser und 200 ml 1 N HCl ein, saugt den Niederschlag ab, trocknet und kristallisiert aus Methyl-tert.-butylether/n-Hexan um. Auf diese Weise erhält man 70,4 g (△ 84 % der Theorie) des oben gezeigten Produktes vom Schmp. 112°C.

Analog erhält man die in Tabelle 12 aufgeführten Verbindungen der Formel (XVII).

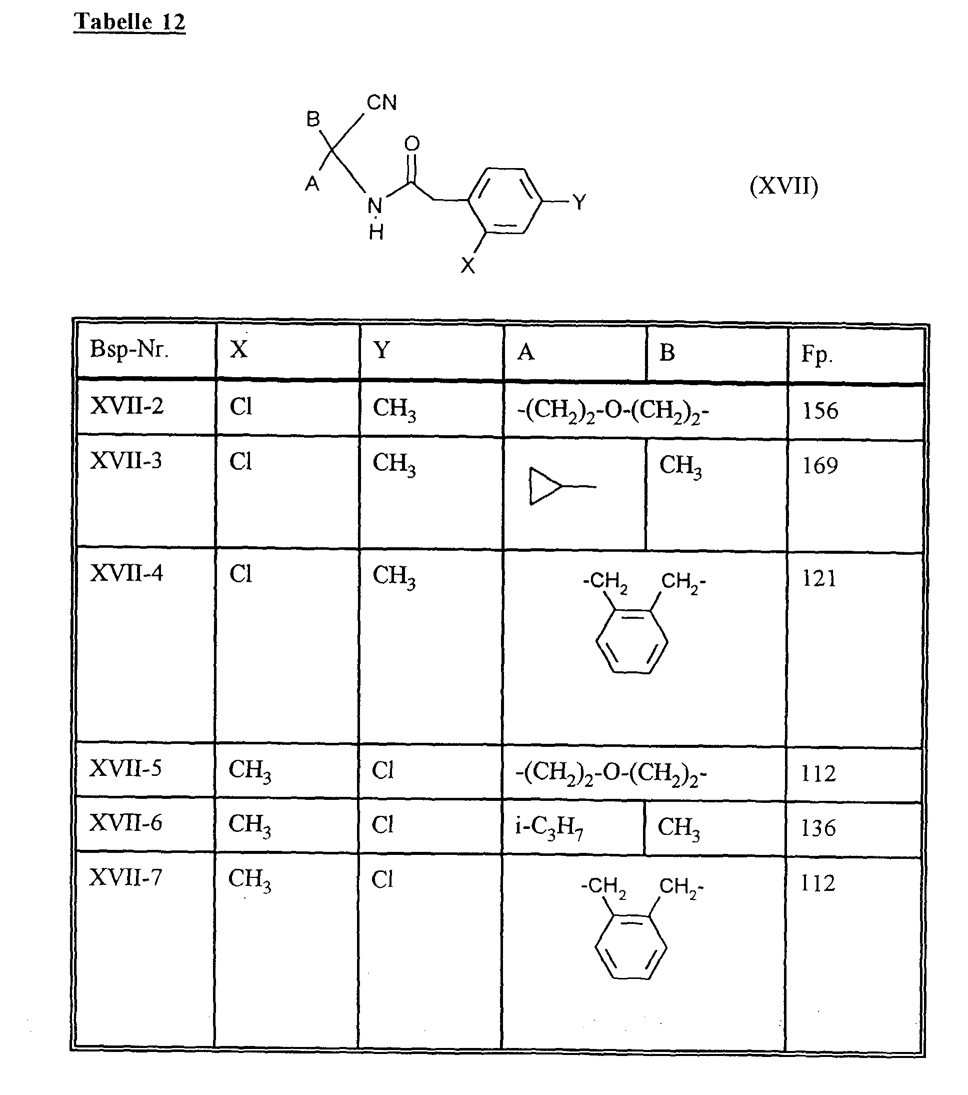

### Anwendungsbeispiele

### Beispiel A

| Phaedon-Larven-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica olearacea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test bewirkten z.B. die Verbindungen gemäß den Herstellungsbeispielen Ia-4 und Ia-5 bei einer beispielhaften Wirkstoffkonzentration von 0,01 % eine Abtötung von 100 % nach 7 Tagen.

### Beispiel B

| Plutella-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica olearacea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Wirkung in % bestimmt. Dabei bedeutet 100 %, daß alle Raupen abgetötet wurden; 0 % bedeutet, daß keine Raupen abgetötet wurden.

Bei diesem Test bewirkten z.B. die Verbindungen gemäß den Herstellungsbeispielen Ia-4 und. Ia-7 bei einer beispielhaften Wirkstoffkonzentration von 0,01 % eine Abtötung von 100 % nach 7 Tagen.

Bei einer beispielhaften Wirkstoffkonzentration von 0,01% bewirkten z.B. die Verbindungen gemäß den Herstellungsbeispielen Ib-9 und Ib-11 eine Abtötung von 100 % nach 3 Tagen.

Die Verbindungen gemäß den Herstellungsbeispielen Ia-7 (0,01 %, 7 Tage), Ib-8 (0,01 %, 3 Tage) und Ic-7 (0,01 %, 3 Tage) bewirkten bei den in Klammern angegebenen Wirkstoffkonzentrationen und Zeiten eine Abtötung von 100 %.

### Beispiel C

| Nephotettix-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Wirkung in % bestimmt. Dabei bedeutet 100 %, daß alle Zikaden abgetötet wurden; 0 % bedeutet, daß keine Zikaden abgetötet wurden.

Bei diesem Test bewirkten z.B. die Verbindungen gemäß den Herstellungsbeispielen Ia-4, Ia-5, Ib-8, Ib-9 und Ib-11 bei einer beispielhaften Wirkstoffkonzentration von 0,01 % eine Abtötung von 100 % nach 6 Tagen.

### Beispiel D

| Myzus-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Wirkung in % bestimmt. Dabei bedeutet 100 %, daß alle Blattläuse abgetötet wurden; 0 % bedeutet, daß keine Blattläuse abgetötet wurden.

Bei diesem Test zeigten z.B. die Verbindungen gemäß den Herstellungsbeispielen Ia-4, Ia-7 und Ia-8 bei einer beispielhaften Wirkstoffkonzentration von 0,01 % einen Abtötungsgrad von mindestens 70 % nach 6 Tagen.

Bei einer beispielhaften Wirkstoffkonzentration von 0,1% bewirkte z.B. die Verbindung gemäß dem Herstellungsbeispiel Ib-11 eine Abtötung von 90 % nach 6 Tagen.

### Beispiel E

| Panonychus-Test | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Ca. 30 cm hohe Pflaumenbäumchen (Prunus domestica), die stark von allen Entwicklungsstadien der Obstbaumspinnmilbe (Panonychus ulmi) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. die Verbindungen gemäß den Herstellungsbeispielen Ia-8, Ib-8, Ib-9, Ic-7, Ib-10, Ib-11, Ic-8, Ia-4 und Ia-5 bei einer beispielhaften Wirkstoffkonzentration von 0,02 % einen Abtötungsgrad von mindestens 95 % nach 7 Tagen.

### Beispiel F

| Tetranychus-Test (OP-resistent/Spritzbehandlung) | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die Verbindungen gemäß den Herstellungsbeispielen Ia-7, Ia-8, Ib-8, Ib-9, Ic-7, Ib-10, Ib-11, Ic-8, Ia-4 und Ia-5 bei einer beispielhaften Wirkstoffkonzentration von 0,02 % eine Abtötung von mindestens 95 % nach 7 Tagen.

### Beispiel G

| Pre-emergence-Test | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung

In diesem Test zeigen beispielsweise die Verbindungen gemäß Herstellungsbeispiel (Ic-14) bei einer beispielhaften Aufwandmenge von 250 g/ha und einer sehr guten Verträglichkeit durch Beta vulgaris eine Wirkung von mindestens 95 % gegenüber folgenden Testpflanzen: Alopecurus myosuroides, Digitaria sanguinalis, Echinocloa colonum, Lolium perenne und Setaria viridis.

## Patentansprüche

1. 1H-3-Aryl-pyrrolidin-2,4-dion-Derivate der Formel (I) 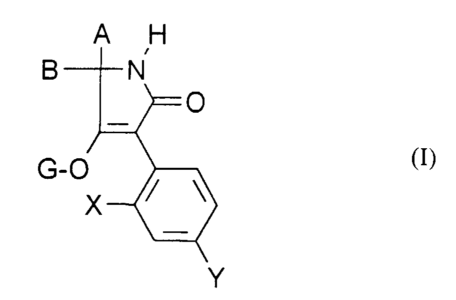 in welcher
A für Wasserstoff, jeweils gegebenenfalls durch Fluor und/oder Chlor substituiertes C₁-C₁₀-Alkyl, C₃-C₆-Alkenyl, C₁-C₈-Alkoxy-C₁-C₆-alkyl, C₁-C₆-Polyalkoxy-C₁-C₆-alkyl, C₁-C₈-Alkylthio-C₁-C₆-alkyl, gegebenenfalls durch Fluor, Chlor, C₁-C₃-Alkyl, C₁-C₃-Alkoxy substituiertes Cycloalkyl mit 3 bis 7 Ringatomen, das durch 1 oder 2 Sauerstoff- und/oder Schwefelatome unterbrochen sein kann oder jeweils gegebenenfalls durch Fluor, Chlor, Brom, C₁-C₄-Alkyl, C₁-C₄-Halogenalkyl, C₁-C₄-Alkoxy und/oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl steht,
B für Wasserstoff, C₁-C₁₂-Alkyl oder C₁-C₈-Alkoxyalkyl steht, oder
A, B und das Kohlenstoffatom an das sie gebunden sind, für einen gesättigten oder ungesättigten gegebenenfalls durch Sauerstoff oder Schwefel unterbrochenen C₃-C₁₀-Spirocyclus stehen, der gegebenenfalls einfach oder mehrfach durch C₁-C₁₀-Alkyl, C₃-C₁₀-Cycloalkyl, C₁-C₆-Halogenalkyl, C₁-C₁₀-Alkoxy, C₁-C₁₀-Alkylthio, Halogen oder Phenyl substituiert ist oder
A, B und das Kohlenstoffatom, an das sie gebunden sind, für einen C₃-C₆-Spirocyclus stehen, der durch eine gegebenenfalls durch ein oder zwei Sauerstoff- und/oder Schwefelatome unterbrochene Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Spirocyclus bildet oder
A, B und das Kohlenstoffatom, an das sie gebunden sind, für einen C₃-C₈-Spirocyclus stehen, bei dem zwei Substituenten gemeinsam für einen gegebenenfalls durch C₁-C₆-Alkyl, C₁-C₆-Alkoxy oder Halogen substituierten gesättigten oder ungesättigten 3- bis 8-gliedrigen Cyclus stehen, der durch Sauerstoff oder Schwefel unterbrochen sein kann,
X für Halogen oder C₁-C₆-Alkyl steht,
Y für Halogen oder C₁-C₆-Alkyl steht,
G für Wasserstoff (a) oder für eine der Gruppen 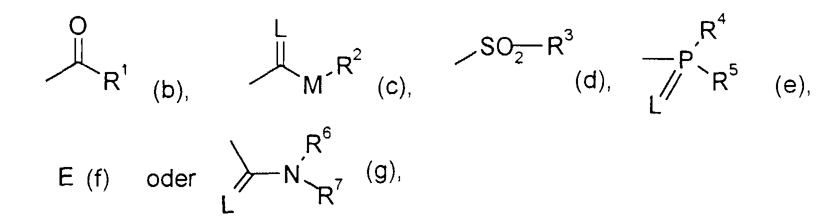 steht,
in welchen
E für ein Metallionäquivalent oder ein Ammoniumion steht und
L und M jeweils für Sauerstoff oder Schwefel stehen,
R¹ für jeweils gegebenenfalls durch Fluor und/oder Chlor substituiertes C₁-C₁₆-Alkyl, C₂-C₁₆-Alkenyl, C₁-C₆-Alkoxy-C₁-C₆-alkyl, C₁-C₁₆-Alkylthio-C₁-C₆-alkyl, C₁-C₆-Polyalkoxy-C₁-C₆-alkyl oder gegebenenfalls durch Halogen oder C₁-C₅-Alkyl substituiertes Cycloalkyl mit 3 bis 7 Ringatomen, das durch 1 oder 2 Sauerstoff- und/oder Schwefelatome unterbrochen sein kann,
für gegebenenfalls durch Halogen, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl,
für gegebenenfalls durch Fluor, Chlor, Brom und/oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom und/oder C₁-C₄-Alkyl substituiertes Phenoxy-C₁-C₅-alkyl oder
für gegebenenfalls durch Fluor, Chlor, Brom, Amino und/oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Halogen substituiertes C₁-C₂₀-Alkyl, C₃-C₂₀-Alkenyl, C₁-C₈-Alkoxy-C₁-C₈-alkyl, C₁-C₈-Polyalkoxy-C₁-C₈-alkyl,
für gegebenenfalls durch Halogen, C₁-C₄-Alkyl und/oder C₁-C₄-Alkoxy substituiertes C₃-C₈-Cycloalkyl, oder
für jeweils gegebenenfalls durch Halogen, Nitro, C₁-C₆-Alkyl, C₁-C₆-Alkoxy und/oder C₁-C₆-Halogenalkyl substituiertes Phenyl oder Benzyl steht,
R³, R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₂-C₈-Alkenylthio, C₃-C₇-Cycloalkylthio, für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen und
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₂-C₈-alkyl, für gegebenenfalls durch Halogen, C₁-C₈-Halogenalkoxy, C₁-C₈-Alkyl und/oder C₁-C₈-Alkoxy substituiertes Phenyl, gegebenenfalls durch Halogen, C₁-C₈-Alkyl, C₁-C₈-Halogenalkyl und/oder C₁-C₈-Alkoxy substituiertes Benzyl oder zusammen für einen gegebenenfalls durch Sauerstoff oder Schwefel unterbrochen C₃-C₆-Alkylenrest stehen,
mit der Maßgabe, daß X und Y nicht gleichzeitig für Alkyl und nicht gleichzeitig für Halogen stehen.

2. 1H-Aryl-pyrrolidin-2,4-dion-Derivate der Formel (I) gemäß Anspruch 1,
in welcher
A für Wasserstoff, jeweils gegebenenfalls durch Fluor und/oder Chlor substituiertes C₁-C₁₀-Alkyl, C₃-C₆-Alkenyl, C₁-C₈-Alkoxy-C₁-C₆-alkyl, C₁-C₆-Polyalkoxy-C₁-C₆-alkyl, C₁-C₈-Alkylthio-C₁-C₆-alkyl, gegebenenfalls durch Fluor, Chlor, C₁-C₃-Alkyl, C₁-C₃-Alkoxy substituiertes Cycloalkyl mit 3 bis 7 Ringatomen, das durch 1 oder 2 Sauerstoff- und/oder Schwefelatome unterbrochen sein kann oder jeweils gegebenenfalls durch Fluor, Chlor, Brom, C₁-C₄-Alkyl, C₁-C₄-Halogenalkyl, C₁-C₄-Alkoxy und/oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl steht,
B für Wasserstoff, C₁-C₁₀-Alkyl oder C₁-C₆-Alkoxyalkyl steht oder
A, B und das Kohlenstoffatom an das sie gebunden sind, für einen gesättigten oder ungesättigten gegebenenfalls durch Sauerstoff oder Schwefel unterbrochenen C₃-C₉-Spirocyclus stehen, der gegebenenfalls einfach oder mehrfach durch C₁-C₆-Alkyl, C₃-C₈-Cycloalkyl, C₁-C₃-Haloalkyl, C₁-C₆-Alkoxy, C₁-C₆-Alkylthio, Fluor, Chlor oder Phenyl substituiert ist oder
A, B und das Kohlenstoffatom, an das sie gebunden sind, für einen C₃-C₆-Spirocyclus stehen, der durch eine gegebenenfalls durch ein oder zwei Sauerstoff- oder Schwefelatome unterbrochene Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind, für einen C₃-C₆-Spirocyclus stehen, bei dem zwei Substituenten gemeinsam für einen gegebenenfalls durch C₁-C₃-Alkyl, C₁-C₃-Alkoxy, Fluor, Chlor oder Brom substituierten gesättigten oder ungesättigten 5- bis 8-gliedrigen Cyclus stehen, der durch Sauerstoff oder Schwefel unterbrochen sein kann,
X für Fluor, Chlor, Brom oder C₁-C₄-Alkyl steht,
Y für Fluor, Chlor, Brom oder C₁-C₄-Alkyl steht,
G für Wasserstoff (a) oder für eine der Gruppen 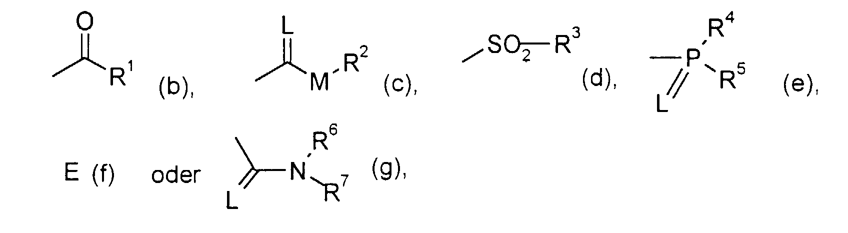 steht,
in welchen
E für ein Metallionäquivalent oder ein Ammoniumion steht und
L und M jeweils für Sauerstoff oder Schwefel stehen,
R¹ für jeweils gegebenenfalls durch Fluor und/oder Chlor substituiertes C₁-C₁₆-Alkyl, C₂-C₁₆-Alkenyl, C₁-C₆-Alkoxy-C₁-C₆-alkyl, C₁-C₁₆-Alkylthio-C₁-C₆-alkyl, C₁-C₆-Polyalkoxy-C₁-C₆-alkyl oder gegebenenfalls durch Halogen oder C₁-C₅-Alkyl substituiertes Cycloalkyl mit 3 bis 7 Ringatomen, das durch 1 oder 2 Sauerstoff- und/oder Schwefelatome unterbrochen sein kann,
für gegebenenfalls durch Halogen, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl,
für gegebenenfalls durch Fluor, Chlor, Brom und/oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom und/oder C₁-C₄-Alkyl substituiertes Phenoxy-C₁-C₅-alkyl oder
für gegebenenfalls durch Fluor, Chlor, Brom, Amino und/oder C₁-C₄-Alkyl substituiertes Pyridyloxy-C₁-C₅-alkyl, Pyrimidyloxy-Cₗ-C₅-alkyl oder Thiazolyloxy-C₁-C₅-alkyl steht,
R² für jeweils gegebenenfalls durch Halogen substituiertes C₁-C₁₆-Alkyl, C₃-C₁₆-Alkenyl, C₁-C₆-Alkoxy-C₁-C₆-alkyl, C₁-C₆-Polyalkoxy-C₁-C₆-alkyl,
für gegebenenfalls durch Halogen, C₁-C₃-Alkyl und/oder C₁-C₃-Alkoxy substituiertes C₃-C₇-Cycloalkyl oder
für jeweils gegebenenfalls durch Halogen, Nitro, C₁-C₄-Alkyl, C₁-C₃-Alkoxy und/oder C₁-C₃-Halogenalkyl substituiertes Phenyl oder Benzyl steht,
R³, R⁴ und R⁵ unabhängig voneinander für jeweils gegebenenfalls durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₃-C₆-Cycloalkylthio, für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Phenoxy oder Phenylthio stehen und
R⁶ und R⁷ unabhängig voneinander für Wasserstoff, für jeweils gegebenenfalls durch Halogen substituiertes C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Halogen, C₁-C₅-Halogenalkyl, C₁-C₅-Alkyl und/oder C₁-C₅-Alkoxy substituiertes Phenyl, für gegebenenfalls durch Halogen, C₁-C₅-Alkyl, C₁-C₅-Halogenalkyl und/oder C₁-C₅-Alkoxy substituiertes Benzyl, oder zusammen für einen gegebenenfalls durch Sauerstoff oder Schwefel unterbrochenen C₃-C₆-Alkylenrest stehen,
mit der Maßgabe, daß X und Y nicht gleichzeitig für Alkyl und nicht gleichzeitig für Halogen stehen.

3. 1H-3-Aryl-pyrrolidin-2,4-dion Derivate der Formel (I) gemäß Anspruch 1,
in welcher
A für Wasserstoff, gegebenenfalls durch Fluor und/oder Chlor substituiertes C₁-C₈-Alkyl, C₃-C₄-Alkenyl, C₁-C₆-Alkoxy-C₁-C₄-alkyl, C₁-C₄-Polyalkoxy-C₁-C₄-alkyl, C₁-C₆-Alkylthio-C₁-C₄-alkyl, gegebenenfalls durch Fluor, Chlor, Methyl, Ethyl, Methoxy oder Ethoxy substituiertes Cycloalkyl mit 3 bis 6 Ringatomen, das durch 1 oder 2 Sauerstoff- und/oder Schwefelatomen unterbrochen sein kann oder für jeweils gegebenenfalls durch Fluor, Chlor, Brom, Methyl, Ethyl, Propyl, iso-Propyl, Methoxy, Ethoxy, Trifluormethyl und/oder Nitro substituiertes Phenyl, Furanyl, Pyridyl, Imidazolyl, Pyrazolyl, Triazolyl, Indolyl, Thiazolyl, Thienyl oder Benzyl steht,
B für Wasserstoff, C₁-C₈-Alkyl oder C₁-C₄-Alkoxyalkyl steht oder
A, B und das Kohlenstoffatom an das sie gebunden sind, für einen gesättigten oder ungesättigten gegebenenfalls durch Sauerstoff oder Schwefel unterbrochenen C₃-C₈-Spirocyclus stehen, der gegebenenfalls einfach oder mehrfach durch Methyl, Ethyl, Propyl, Isopropyl, Butyl, iso-Butyl, sec.-Butyl, tert-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert ist oder
A, B und das Kohlenstoffatom, an das sie gebunden sind, für einen C₃-C₆-Spirocyclus stehen, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind, für einen C₃-C₆-Spirocyclus stehen, bei dem zwei Substituenten gemeinsam für einen gesättigten oder ungesättigten fünf- oder sechsgliedrigen Cyclus stehen, der durch Sauerstoff oder Schwefel unterbrochen sein kann,
X für Chlor, Brom, Methyl, Ethyl, Propyl oder iso-Propyl steht,
Y für Chlor, Brom, Methyl, Ethyl, Propyl oder iso-Propyl steht,
G für Wasserstoff (a) oder für eine der Gruppen 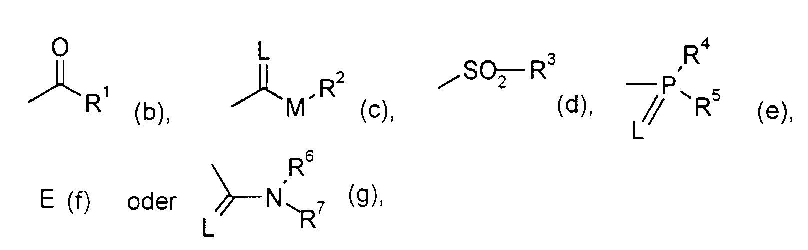 steht,
in welchen
E für ein Metallionäquivalent oder ein Ammoniumion steht,
L und M jeweils für Sauerstoff oder Schwefel stehen,
R¹ für jeweils gegebenenfalls durch Fluor und/oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl oder gegebenenfalls durch Fluor, Chlor, Methyl, Ethyl, Propyl, i-Propyl, Butyl, i-Butyl oder t-Butyl substituiertes Cycloalkyl mit 3 bis 6 Ring-atomen, das durch 1 oder 2 Sauerstoff- und/oder Schwefelatome unterbrochen sein kann,
für gegebenenfalls durch Fluor, Chlor, Brom, Nitro, Methyl, Ethyl, Propyl, i-Propyl, Methoxy, Ethoxy, Trifluormethyl, Trifluormethoxy, Methylthio, Ethylthio, Methylsulfonyl, Ethylsulfonyl substituiertes Phenyl,
für gegebenenfalls durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl steht,
für gegebenenfalls durch Fluor, Chlor, Brom, Methyl oder Ethyl substituiertes Furanoyl, Thienyl, Pyridyl, Pyrimidyl, Thiazolyl oder Pyrazolyl,
für gegebenenfalls durch Fluor, Chlor, Methyl und/oder Ethyl substituiertes Phenoxy-C₁-C₄-alkyl, oder
für gegebenenfalls durch Fluor, Chlor, Amino, Methyl, Ethyl, substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor und/oder Chlor substituiertes C₁-C₁₄-Alkyl, C₃-C₁₄-Alkenyl, C₁-C₄-Alkoxy-C₁-C₆-alkyl, C₁-C₄-Polyalkoxy-C₁-C₆-alkyl,
für gegebenenfalls durch Fluor, Chlor, Methyl, Ethyl, Propyl, iso-Propyl oder Methoxy substituiertes C₃-C₆-Cycloalkyl,
oder für jeweils gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für jeweils gegebenenfalls durch Fluor und/oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Alkylthio, C₁-C₂-Fluoralkylthio, C₁-C₂-Alkyl substituiertes Phenyl, Phenoxy oder Phenylthio stehen und
R⁶ und R⁷ unabhängig voneinander für Wasserstoff, für jeweils gegebenenfalls durch Fluor, Chlor, Brom substituiertes C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, C₁-C₄-Halogenalkyl, C₁-C₄-Alkyl und/oder C₁-C₄-Alkoxy substituiertes Phenyl, für gegebenenfalls durch Fluor, Chlor, Brom, C₁-C₄-Alkyl, C₁-C₄-Halogenalkyl und/oder C₁-C₄-Alkoxy substituiertes Benzyl, oder zusammen für einen gegebenenfalls durch Sauerstoff oder Schwefel unterbrochenen C₄-C₆-Alkylenrest stehen,
mit der Maßgabe, daß X und Y nicht gleichzeitig für Alkyl und nicht gleichzeitig für Halogen stehen.

4. 1H-3-Aryl-pyrrolidin-2,4-dion-Derivate der Formel (I) gemäß Anspruch 1, dadurch gekennzeichnet, daß es sich um eine der folgenden Strukturen (Ia) bis (Ig) handelt: 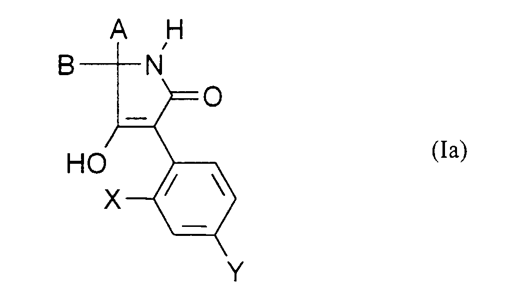 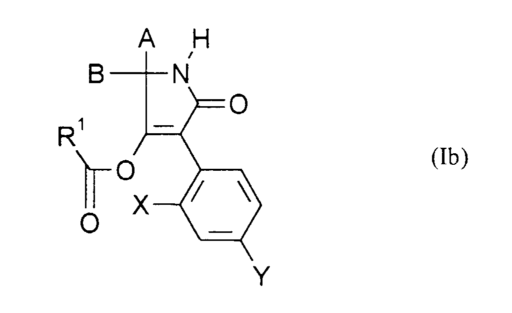 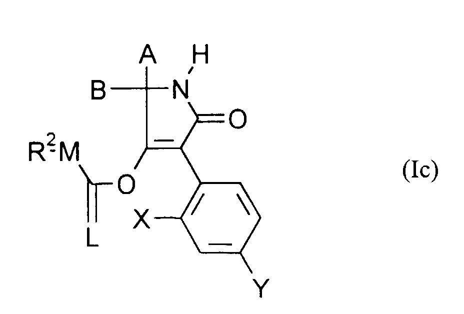 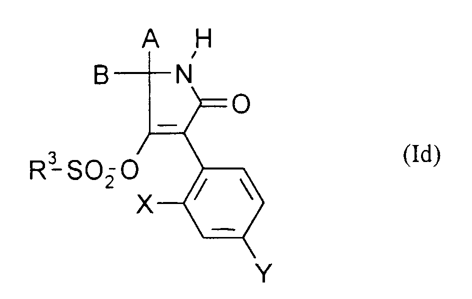 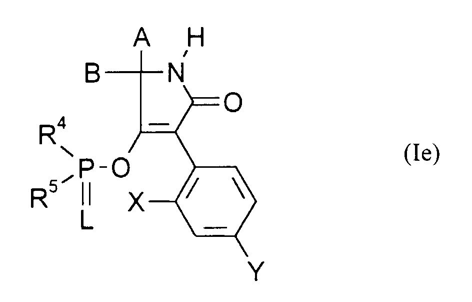 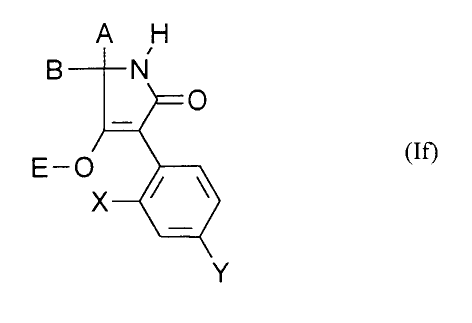 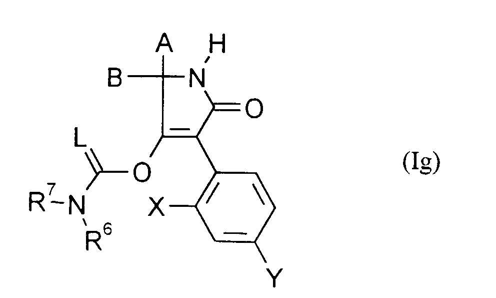 worin
A, B, E, L. M, X, Y, R¹, R², R³, R⁴, R⁵, R⁶ und R⁷ die in Anspruch 1 angegebenen Bedeutungen besitzen.

5. Verfahren zur Herstellung von substituierten 1H-3-Aryl-pyrrolidin-2,4-dion-Derivaten der Formel (I) gemäß Anspruch 1, dadurch gekennzeichnet, daß man
(A) N-Acylaminosäureester der Formel (II) 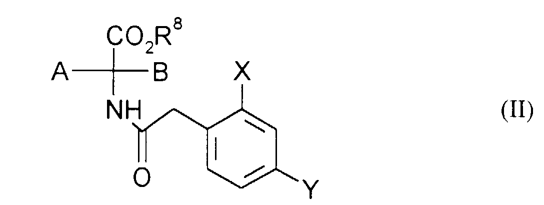 in welcher
A, B, X und Y die in Anspruch 1 angegebene Bedeutung haben,
und
R⁸ für Alkyl steht,
in Gegenwart eines Verdünnungsmittels und in Gegenwart einer Base intramolekular kondensiert;
und anschließend
die so erhaltenen Verbindungen der Formel (Ia), 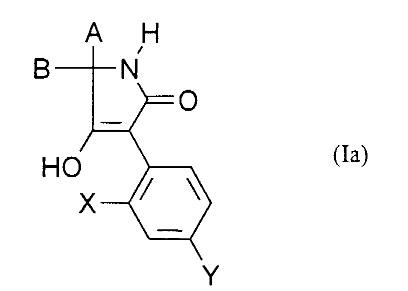 in welcher
A, B, X und Y die oben angegebenen Bedeutungen haben,
(B)
α) mit Säurehalogeniden der Formel (III) 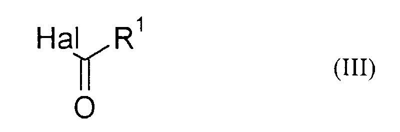 in welcher
R¹ die in Anspruch 1 angegebenen Bedeutungen hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) mit Chlorameisensaureestern oder Chlorameisensäurethiolestern der Formel (V)
R²-M-CO-Cl (V)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt;
oder
(D)
α) mit Chlormonothioameisensäureestern oder Chlordithioameisensäureestern der Formel (VI) 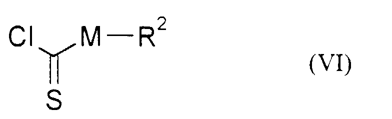 in welcher
M und R² die oben angegebenen Bedeutungen haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der Formel (VII)
R²-Hal (VII)
in welcher
R² die oben angegebene Bedeutung hat
und
Hal für Chlor, Brom oder Iod steht,
umsetzt;
oder
(E) mit Sulfonsäurechloriden der Formel (VIII)
R³-SO₂-Cl (VIII)
in welcher
R³ die in Anspruch 1 angegebene Bedeutung hat,
gegebenenfalls in Gegenwart eines Verdünnungsmittels und gegebenenfalls in Gegenwart eines Säurebindemittels,
umsetzt; oder
(F) mit Phosphorverbindungen der Formel (IX) 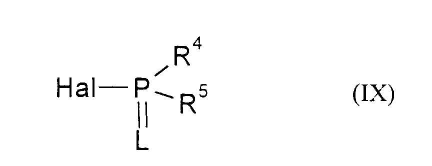 in welcher
L, R⁴ und R² die in Anspruch 1 angegebenen Bedeutungen haben
und
Hal für Halogen steht,
gegebenenfalls in Gegenwart eines Verdünnungsmittels und gegebenenfalls in Gegenwart eines Säurebindemittels
umsetzt;
oder
(G) mit Metallhydroxiden, Metallalkoxiden oder Aminen der Formeln (X) und (XI)
Me(OR¹⁰)ₜ (X) 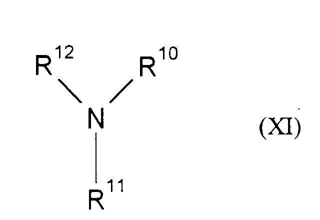
in welchen
Me für ein- oder zweiwertige Metallionen,
t für die Zahl 1 oder 2 und
R¹⁰, R¹¹ und R¹² unabhängig voneinander für Wasserstoff und/oc Alkyl
stehen,
gegebenenfalls in Gegenwart eines Verdünnungsmittels, umsetzt, oder
(H)
α) mit Isocyanaten oder Isothiocyanaten der Formel (XII)
R⁶-N=C=L (XII)
in welcher
L und R⁶ die in Anspruch 1 angegebenen Bedeutungen haben
gegebenenfalls in Gegenwart eines Verdünnungsmittels und gegebenenfalls in Gegenwart eines Katalysators
oder
β) mit Carbamidsäurechloriden oder Thiocarbamidsäurechloriden der Formel (XIII) 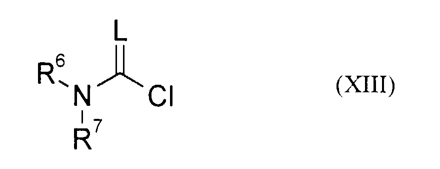 in welcher
L, R⁶ und R⁷ die in Anspruch 1 angegebenen Bedeutungen haben
gegebenenfalls in Gegenwart eines Verdünnungsmittels und gegebenenfalls in Gegenwart eines Säurebindemittels,
umsetzt.

6. Verbindungen der Formel (II) 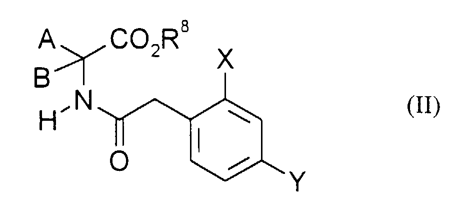 in welcher
A, B, X und Y die in Anspruch 1 angegebenen Bedeutungen haben und
R⁸ für Alkyl steht.

7. Verfahren zur Herstellung der Acyl-aminosäureester der Formel (II), gemäß Anspruch 6, dadurch gekennzeichnet, daß man Aminosäurederivate der Formel (XIV), 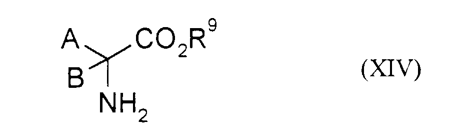 in welcher
R⁹ für Wasserstoff (XIVa) oder Alkyl (XIVb) steht
und
A und B die in Anspruch 1 angegebenen Bedeutungen haben,
mit Phenylessigsäurehalogeniden der Formel (XV) 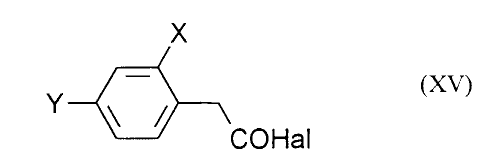 in welcher
X und Y die oben angegebenen Bedeutungen haben und
Hal für Chlor oder Brom steht,
acyliert und die dabei für R⁹ = Wasserstoff erhaltenen Acylaminosäuren der Formel (IIa), 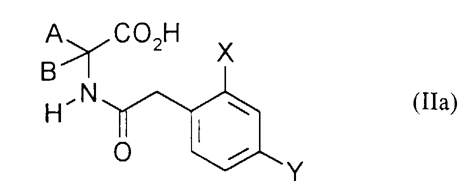 in welcher
A, B, X und Y die oben angegebenen Bedeutungen haben,
verestert,
oder daß man Aminonitrile der Formel (XVI) 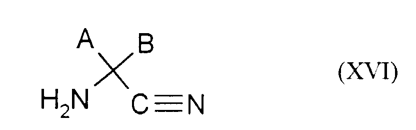 in welcher
A und B die oben angegebenen Bedeutungen haben,
mit Phenylessigsäurehalogeniden der Formel (XV) 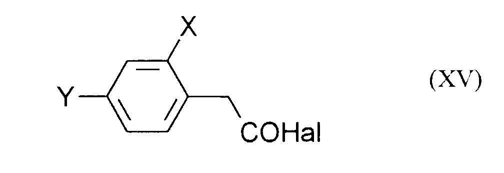 in welcher
X und Y die oben angegebenen Bedeutungen haben und
Hal für Chlor oder Brom steht,
zu Verbindungen der Formel (XVII) 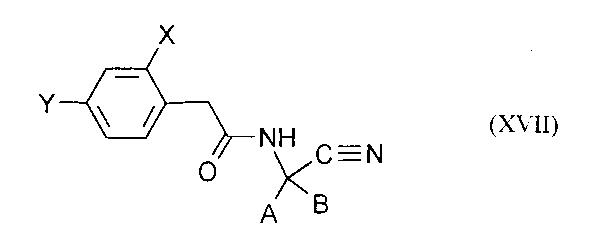 in welcher
A, B, X und Y die oben angegebenen Bedeutungen haben,
umsetzt, und diese anschließend einer schwefelsauren Alkoholyse unterwirft.

8. Verbindungen der Formel (XVII) 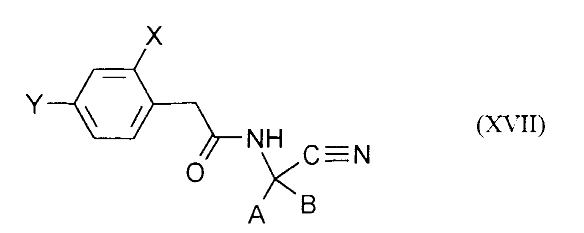 in welcher
A, B, X und Y die in Anspruch 1 angegebenen Bedeutungen haben.

9. Verfahren zur Herstellung von Verbindungen der Formel (XVII) gemäß Anspruch 8, dadurch gekennzeichnet, daß man Aminonitrile der Formel (XVI) 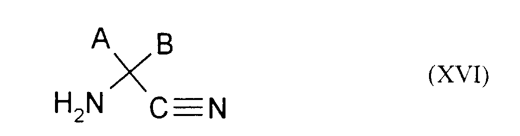 in welcher
A und B die in Anspruch 1 angegebenen Bedeutungen haben,
mit Phenylessigsäurehalogeniden der Formel (XV) 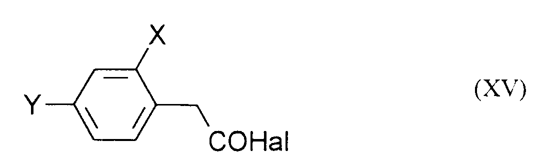 in welcher
X und Y die in Anspruch 1 angegebenen Bedeutungen haben
und
Hal für Chlor oder Brom steht,
umsetzt.

10. Schädlingsbekämpfungsmittel und Herbizide, gekennzeichnet durch einen Gehalt an mindestens einem 1H-3-Aryl-pyrrolidin-2,4-dion-Derivat der Formel (I) gemäß Anspruch 1.

11. Verwendung von 1H-3-Aryl-pyrrolidin-2,4-dion-Derivaten der Formel (I) gemäß Anspruch 1 zur Bekämpfung von Schädlingen und unerwünschtem Pflanzenbewuchs.

12. Verfahren zur Bekämpfung von Schädlingen, dadurch gekennzeichnet, daß man 1H-3-Aryl-pyrrolidin-2,4-dion-Derivate der Formel (I) gemäß Anspruch 1 auf Schädlinge, unerwünschten Pflanzenbewuchs und/oder ihren Lebensraum einwirken läßt.

13. Verfahren zur Herstellung von Schädlingsbekämpfungsmitteln und Herbiziden, dadurch gekennzeichnet, daß man 1H-3-Aryl-pyrrolidin-2,4-dion-Derivate der Formel (I) gemäß Anspruch 1 mit Streckmitteln und/oder oberflächenaktiven Mitteln vermischt.

## Claims

1. 1H-3-aryl-pyrrolidine-2,4-dione derivative of the formula (I) 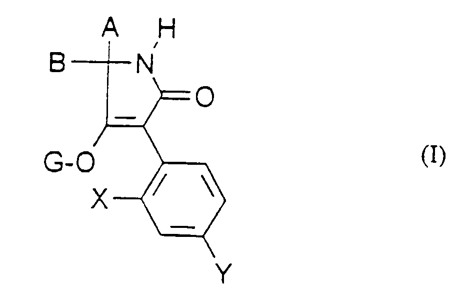 in which
A represents hydrogen, or C₁-C₁₀-alkyl, C₃-C₆-alkenyl, C₁-C₈-alkoxy-C₁-C₆-alkyl, C₁-C₆-polyalkoxy-C₁-C₆-alkyl or C₁-C₈-alkylthio-C₁-C₆-alkyl, each of which is optionally substituted by fluorine and/or chlorine, or cycloalkyl having 3 to 7 ring atoms which is optionally substituted by fluorine, chlorine, C₁-C₃-alkyl or C₁-C₃-alkoxy and which can be interrupted by 1 or 2 oxygen and/or sulphur atoms, or represents phenyl, furanyl, pyridyl, imidazolyl, triazolyl, pyrazolyl, indolyl, thiazolyl, thienyl or phenyl-C₁-C₄-alkyl, each of which is optionally substituted by fluorine, chlorine, bromine, C₁-C₄-alkyl, C₁-C₄-halogenoalkyl, C₁-C₄-alkoxy and/or nitro,
B represents hydrogen, C₁-C₁₂-alkyl or C₁-C₈-alkoxyalkyl, or
A, B and the carbon atom to which they are bonded represent a saturated or unsaturated C₃-C₁₀-spirocycle which is optionally interrupted by oxygen or sulphur and optionally monosubstituted or polysubstituted by C₁-C₁₀-alkyl, C₃-C₁₀-cycloalkyl, C₁-C₆-halogenoalkyl, C₁-C₁₀-alkoxy, C₁-C₁₀-alkylthio, halogen or phenyl, or
A, B and the carbon atom to which they are bonded represent a C₃-C₆-spirocycle which is substituted by an alkylenediyl group which is optionally interrupted by one or two oxygen atoms and/or sulphur atoms, or which is substituted by an alkylenedioxy or by an alkylenedithio group, this alkylenediyl, alkylenedioxy or alkylenedithio group together with the carbon atom to which it is bonded forming a further five- to eight-membered spirocycle, or
A, B and the carbon atom to which they are bonded represent a C₃-C₈-spirocycle in which two substituents together represent a saturated or unsaturated 3- to 8-membered cycle which is optionally substituted by C₁-C₆-alkyl, C₁-C₆-alkoxy or halogen and which can be interrupted by oxygen or sulphur,
X represents halogen or C₁-C₆-alkyl,
Y represents halogen or C₁-C₆-alkyl,
G represents hydrogen (a) or one of the groups 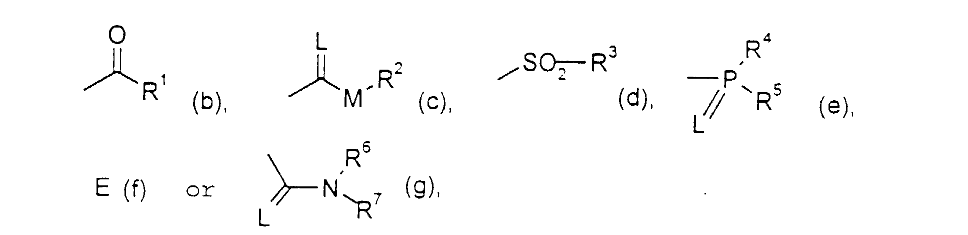 in which
E represents a metal ion equivalent or an ammonium ion and
L and M in each case represent oxygen or sulphur,
R¹ represents C₁-C₁₆-alkyl, C₂-C₁₆-alkenyl, C₁-C₆-alkoxy-C₁-C₆-alkyl, C₁-C₁₆-alkylthio-C₁-C₆-alkyl or C₁-C₆-polyalkoxy-C₁-C₆-alkyl, each of which is optionally substituted by fluorine and/or chlorine, or cycloalkyl having 3 to 7 ring atoms which is optionally substituted by halogen or C₁-C₅-alkyl and which can be interrupted by 1 or 2 oxygen and/or sulphur atoms,
phenyl which is optionally substituted by halogen, nitro, C₁-C₄-alkyl, C₁-C₄-alkoxy, C₁-C₃-halogenoalkyl, C₁-C₃-halogenoalkoxy, C₁-C₄-alkylthio or C₁-C₄-alkylsulphonyl,
phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
pyrazolyl, thiazolyl, pyridyl, pyrimidyl, furanyl or thienyl, each of which is optionally substituted by fluorine, chlorine, bromine and/or C₁-C₄-alkyl,
phenoxy-C₁-C₅-alkyl which is optionally substituted by fluorine, chlorine, bromine and/or C₁-C₄-alkyl, or
pyridyloxy-C₁-C₅-alkyl, pyrimidyloxy-C₁-C₅-alkyl or thiazolyloxy-C₁-C₅-alkyl, each of which is optionally substituted by fluorine, chlorine, bromine, amino and/or C₁-C₄-alkyl,
R² represents C₁-C₂₀-alkyl, C₃-C₂₀-alkenyl, C₁-C₈-alkoxy-C₁-C₈-alkyl or C₁-C₈-polyalkoxy-C₁-C₈-alkyl, each of which is optionally substituted by halogen,
C₁-C₈-cycloalkyl which is optionally substituted by halogen, C₁-C₄-alkyl and/or C₁-C₄-alkoxy, or
phenyl or benzyl, each of which is optionally substituted by halogen, nitro, C₁-C₆-alkyl, C₁-C₆-alkoxy and/or C₁-C₆-halogenoalkyl,
R³, R⁴ and R⁵ independently of one another represent C₁-C₈-alkyl, C₁-C₈-alkoxy, C₁-C₈-alkylamino, di-(C₁-C₈)-alkylamino, C₁-C₈-alkylthio, C₂-C₈-alkenylthio or C₃-C₇-cycloalkylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl and
R⁶ and R⁷ independently of one another represent hydrogen, or C₁-C₈-alkyl, C₃-C₈-cycloalkyl, C₁-C₈-alkoxy, C₃-C₈-alkenyl or C₁-C₈-alkoxy-C₁-C₈-alkyl, each of which is optionally substituted by halogen, or represent phenyl which is optionally substituted by halogen, C₁-C₈-halogenoalkyl, C₁-C₈-alkyl and/or C₁-C₈-alkoxy, or represent benzyl which is optionally substituted by halogen, C₁-C₈-alkyl, C₁-C₈-halogenoalkyl and/or C₁-C₈-alkoxy, or together represent a C₃-C₆-alkylene ring which is optionally interrupted by oxygen or sulphur,
with the proviso that X and Y do not simultaneously represent alkyl and not simultaneously halogen.

2. 1H-Aryl-pyrrolidine-2,4-dione derivative of the formula (I) according to Claim 1,
in which
A represents hydrogen, or C₁-C₁₀-alkyl, C₃-C₆-alkenyl, C₁-C₈-alkoxy-C₁-C₆-alkyl, C₁-C₆-polyalkoxy-C₁-C₆-alkyl or C₁-C₆-alkylthio-C₁-C₆-alkyl, each of which is optionally substituted by fluorine and/or chlorine, or cycloalkyl having 3 to 7 ring atoms which is optionally substituted by fluorine, chlorine, C₁-C₃-alkyl or C₁-C₃-alkoxy and which can be interrupted by 1 or 2 oxygen and/or sulphur atoms, or represents phenyl, furanyl, pyridyl, imidazolyl, triazolyl, pyrazolyl, indolyl, thiazolyl, thienyl or phenyl-C₁-C₄-alkyl, each of which is optionally substituted by fluorine, chlorine, bromine, C₁-C₄-alkyl, C₁-C₄-halogenoalkyl, C₁-C₄-alkoxy and/or nitro,
B represents hydrogen, C₁-C₁₀-alkyl or C₁-C₆-alkoxyalkyl, or
A, B and the carbon atom to which they are bonded represent saturated or unsaturated C₃-C₉-spirocycle which is optionally interrupted by oxygen or sulphur and optionally monosubstituted or polysubstituted by C₁-C₆-alkyl, C₃-C₈-cycloalkyl, C₁-C₃-haloalkyl, C₁-C₆-alkoxy, C₁-C₆-alkylthio, fluorine, chlorine or phenyl, or
A, B and the carbon atom to which they are bonded represent a C₃-C₆-spirocycle which is substituted by an alkylenediyl group which is optionally interrupted by one or two oxygen or sulphur atoms or which is substituted by an alkylenedioxy or by an alkylenedithio group, this alkylenediyl, alkylenedioxy or alkylenedithio group together with the carbon atom to which it is bonded forming a further five- to seven-membered spirocycle, or
A, B and the carbon atom to which they are bonded represent a C₃-C₆-spirocycle in which two substituents together represent a saturated or unsaturated 5- to 8-membered cycle which is optionally substituted by C₁-C₃-alkyl, C₁-C₃-alkoxy, fluorine, chlorine or bromine and which can be interrupted by oxygen or sulphur,
X represents fluorine, chlorine, bromine or C₁-C₄-alkyl,
Y represents fluorine, chlorine, bromine or C₁-C₄-alkyl,
G represents hydrogen (a) or one of the groups 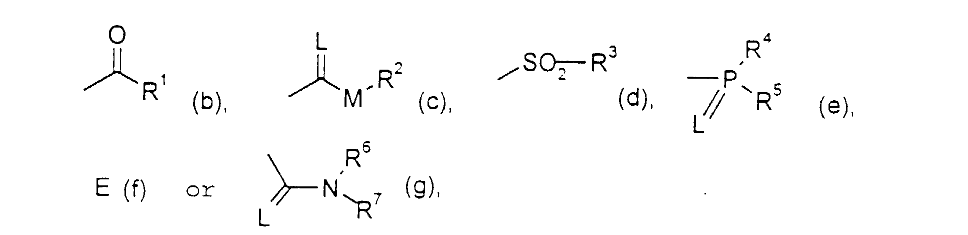 in which
E represents a metal ion equivalent or an ammonium ion, and
L and M in each case represent oxygen or sulphur,
R¹ represents C₁-C₁₆-alkyl, C₂-C₁₅-alkenyl, C₁-C₆-alkoxy-C₁-C₆-alkyl, C₁-C₁₆-alkylthio-C₁-C₆-alkyl or C₁-C₆-polyalkoxy-C₁-C₆-alkyl, each of which is optionally substituted by fluorine and/or chlorine, or cycloalkyl having 3 to 7 ring atoms which is optionally substituted by halogen or C₁-C₅-alkyl and which can be interrupted by 1 or 2 oxygen and/or sulphur atoms,
phenyl which is optionally substituted by halogen, nitro, C₁-C₄-alkyl, C₁-C₄-alkoxy, C₁-C₃-halogenoalkyl, C₁-C₃-halogenoalkoxy, C₁-C₄-alkylthio or C₁-C₄-alkylsulphonyl,
phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
pyrazolyl, thiazolyl, pyridyl, pyrimidyl, furanyl or thienyl, each of which is optionally substituted by fluorine, chlorine, bromine and/or C₁-C₄-alkyl,
phenoxy-C₁-C₅-alkyl which is optionally substituted by fluorine, chlorine, bromine and/or C₁-C₄-alkyl, or
pyridyloxy-C₁-C₅-alkyl, pyrimidyloxy-C₁-C₅-alkyl or thiazolyloxy-C₁-C₅-alkyl, each of which is optionally substituted by fluorine, chlorine, bromine, amino and/or C₁-C₄-alkyl,
R² represents C₁-C₁₆-alkyl, C₃-C₁₆-alkenyl, C₁-C₆-alkoxy-C₁-C₆-alkyl or C₁-C₆-polyalkoxy-C₁-C₆-alkyl, each of which is optionally substituted by halogen,
C₃-C₇-cycloalkyl which is optionally substituted by halogen, C₁-C₃-alkyl and/or C₁-C₃-alkoxy, or
phenyl or benzyl, each of which is optionally substituted by halogen, nitro, C₁-C₄-alkyl, C₁-C₃-alkoxy and/or C₁-C₃-halogenoalkyl,
R³, R⁴ and R⁵ independently of one another represent C₁-C₆-alkyl, C₁-C₆-alkoxy, C₁-C₆-alkylamino, di-(C₁-C₆)-alkylamino, C₁-C₆-alkylthio, C₃-C₄-alkenylthio or C₃-C₆-cycloalkylthio, each of which is optionally substituted by halogen, or phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylchio, C₁-C₃-halogenoalkylthio, C₁-C₃-alkyl or C₁-C₃-halogenoalkyl and
R⁶ and R⁷ independently of one another represent hydrogen, or represent C₁-C₈-alkyl, C₃-C₈-cycloalkyl, C₁-C₆-alkoxy, C₃-C₆-alkenyl or C₁-C₆-alkoxy-C₁-C₆-alkyl, each of which is optionally substituted by halogen, or represent phenyl which is optionally substituted by halogen, C₁-C₅-halogenoalkyl, C₁-C₅-alkyl and/ or C₁-C₅-alkoxy, or represent benzyl which is optionally substituted by halogen, C₁-C₅-alkyl, C₁-C₅-halogenoalkyl and/or C₁-C₅-alkoxy, or together represent a C₃-C₆-alkylene radical which is optionally interrupted by oxygen or sulphur,
with the proviso that X and Y do not simultaneously represent alkyl and not simultaneously halogen.

3. 1H-3-aryl-pyrrolidine-2,4-dione derivative of the formula (I) according to Claim 1,
in which
A represents hydrogen, or C₁-C₈-alkyl, C₃-C₄-alkenyl, C₁-C₆-alkoxy-C₁-C₄-alkyl, C₁-C₄-polyalkoxy-C₁-C₄-alkyl or C₁-C₆-alkylthio-C₁-C₄-alkyl, each of which is optionally substituted by fluorine and/or chlorine, or cycloalkyl having 3 to 6 ring atoms which is optionally substituted by fluorine, chlorine, methyl, ethyl, methoxy or ethoxy and which can be interrupted by 1 or 2 oxygen and/or sulphur atoms, or represents phenyl, furanyl, pyridyl, imidazolyl, pyrazolyl, triazolyl, indolyl, thiazolyl, thienyl or benzyl, each of which is optionally substituted by fluorine, chlorine, bromine, methyl, ethyl, propyl, iso-propyl, methoxy, ethoxy, trifluoromethyl and/or nitro,
B represents hydrogen, C₁-C₈-alkyl or C₁-C₄-alkoxyalkyl, or
A, B and the carbon atom to which they are bonded represent a saturated or unsaturated C₃-C₈-spirocycle which is optionally interrupted by oxygen or sulphur and optionally monosubstituted or polysubstituted by methyl, ethyl, propyl, isopropyl, butyl, iso-butyl, sec-butyl, tert-butyl, cyclohexyl, trifluoromethyl, methoxy, ethoxy, propoxy, iso-propoxy, butoxy, iso-butoxy, sec-butoxy, tert-butoxy, methylthio, ethylthio, fluorine, chlorine or phenyl, or
A, B and the carbon atom to which they are bonded represent a C₃-C₆-spirocycle which is substituted by an alkylenediyl group which is optionally interrupted by an oxygen or sulphur atom, or represent an alkylenedioxy group, this alkylenediyl or alkylenedioxy group together with the carbon atom to which it is bonded forming a further five- to seven-membered spirocycle, or
A, B and the carbon atom to which they are bonded represent a C₃-C₆-spirocycle in which two substituents together represent a saturated or unsaturated five- or six-membered cycle which can be interrupted by oxygen or sulphur,
X represents chlorine, bromine, methyl, ethyl, propyl or iso-propyl,
Y represents chlorine, bromine, methyl, ethyl, propyl or iso-propyl,
G represents hydrogen (a) or one of the groups 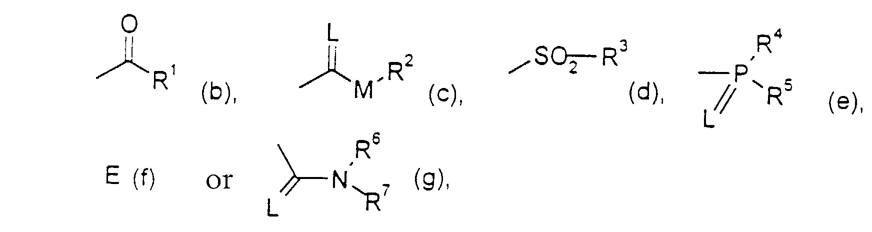 in which
E represents a metal ion equivalent or an ammonium ion and
L and M in each case represent oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or C₁-C₄-polyalkoxy-C₁-C₄-alkyl, each of which is optionally substituted by fluorine and/or chlorine, or cycloalkyl having 3 to 6 ring atoms which is optionally substituted by fluorine, chlorine, methyl, ethyl, propyl, i-propyl, butyl, i-butyl or butyl and which can be interrupted by 1 or 2 oxygen and/or sulphur atoms,
phenyl which is optionally substituted by fluorine, chlorine, bromine, nitro, methyl, ethyl, propyl, i-propyl, methoxy, ethoxy, trifluoromethyl, trifluoromethoxy, methylthio, ethylthio, methylsulphonyl or ethylsulphonyl,
phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
furanyl, thienyl, pyridyl, pyrimidyl, thiazolyl or pyrazolyl, each of which is optionally substituted by fluorine, chlorine, bromine, methyl or ethyl,
phenoxy-C₁-C₄-alkyl, each of which is optionally substituted by fluorine, chlorine, methyl and/or ethyl, or
pyridyloxy-C₁-C₄-alkyl, pyrimidyloxy-C₁-C₄-alkyl or thiazolyloxy-C₁-C₄-alkyl, each of which is optionally substituted by fluorine, chlorine, amino, methyl or ethyl,
R² represents C₁-C₁₄-alkyl, C₃-C₁₄-alkenyl, C₁-C₄-alkoxy-C₁-C₆-alkyl or C₁-C₄-polyalkoxy-C₁-C₆-alkyl, each of which is optionally substituted by fluorine and/or chlorine,
C₃-C₆-cycloalkyl which is optionally substituted by fluorine, chlorine, methyl, ethyl, propyl, iso-propyl or methoxy,
or phenyl or benzyl, each of which is optionally substituted by fluorine, chlorine, nitro, methyl, ethyl, propyl, i-propyl, methoxy, ethoxy or trifluoromethyl,
R³, R⁴ and R⁵ independently of one another represent C₁-C₄-alkyl, C₁-C₄-alkoxy, C₁-C₄-alkylamino, di-(C₁-C₄)-alkylamino or C₁-C₄-alkylthio, each of which is optionally substituted by fluorine and/or chlorine, or represent phenyl, phenoxy or phenylthio, each of which is optionally substituted by fluorine, chlorine, bromine, nitro, cyano, C₁-C₂-alkoxy, C₁-C₂-fluoroalkoxy, C₁-C₂-alkylthio, C₁-C₂-fluoroalkylthio or C₁-C₃-alkyl, and
R⁶ and R⁷ independently of one another represent hydrogen, or represent C₁-C₄-alkyl, C₃-C₆-cycloalkyl, C₁-C₄-alkoxy, C₃-C₄-alkenyl or C₁-C₄-alkoxy-C₁-C₄-alkyl, each of which is optionally substituted by fluorine, chlorine, or bromine, or represent phenyl which is optionally substituted by fluorine, chlorine, bromine, C₁-C₄-halogenoalkyl, C₁-C₄-alkyl and/or C₁-C₄-alkoxy, or represent benzyl which is optionally substituted by fluorine, chlorine, bromine, C₁-C₄-alkyl, C₁-C₄-halogenoalkyl and/or C₁-C₄-alkoxy, or together represent a C₄-C₆-alkylene radical which is optionally interrupted by oxygen or sulphur,
with the proviso that X and Y do not simultaneously represent alkyl and not simultaneously halogen.

4. 1H-3-aryl-pyrrolidine-2,4-dione derivative of the formula (I) according to Claim 1, characterized in that it is one of the structures (Ia) to (Ia) below: 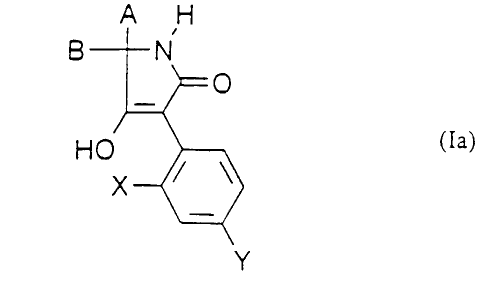 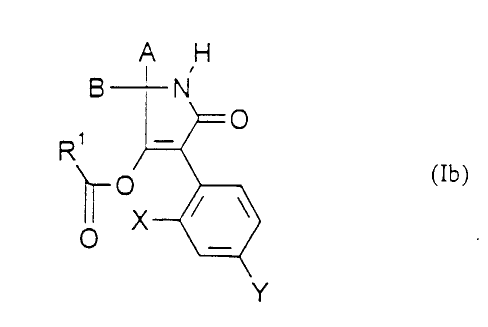 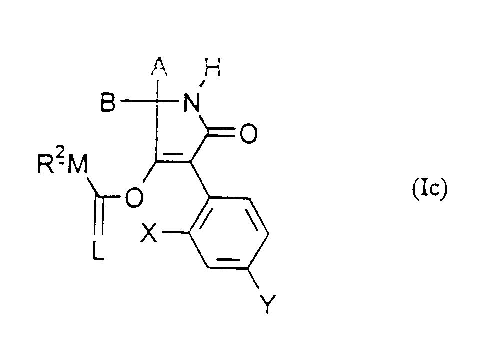 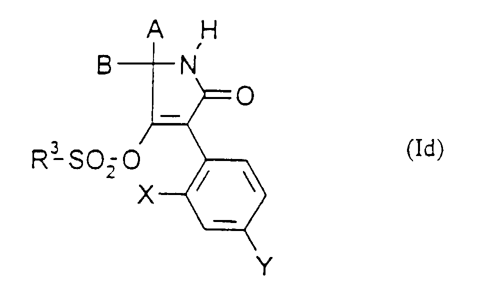 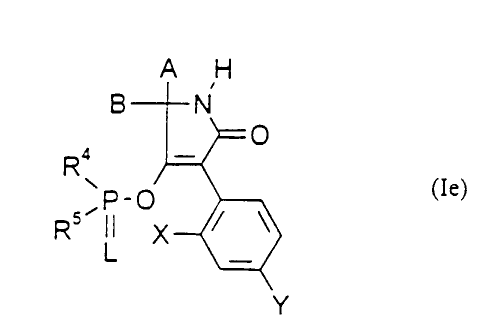 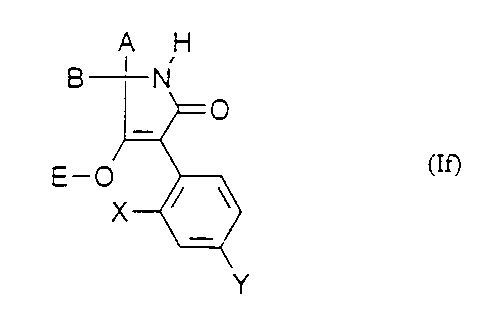 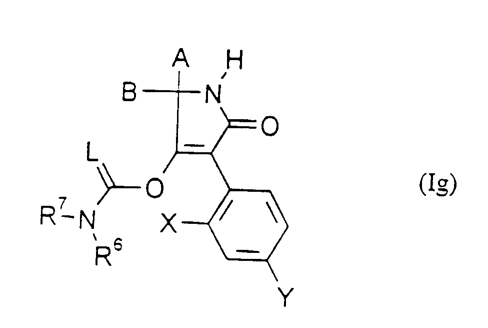 in which
A, B, E, L, M, X, Y, R¹, R², R³, R⁴, R⁵, R⁶ and R⁷ have the meanings given in Claim 1.

5. Process for the preparation of substituted 1H-3-aryl-pyrrolidine-2,4-dione derivatives of the formula (I) according to Claim 1, characterized in that,
(A) N-acylamino acid esters of the formula (II) 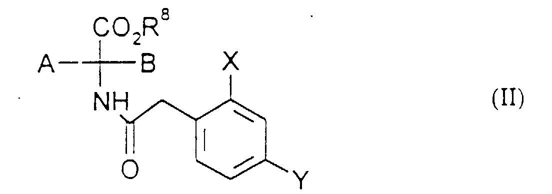 in which
A, B, X and Y have the meanings given in Claim 1
and
R⁸ represents alkyl
are subjected to an intramolecular condensation reaction in the presence of a diluent and in the presence of a base;
and subsequently
the resulting compounds of the formula (Ia) 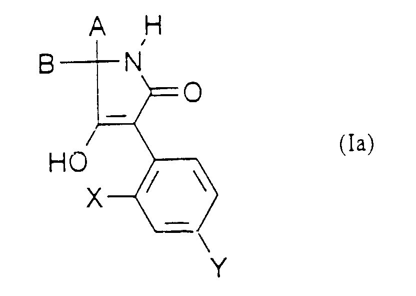 in which
A, B, X and Y have the abovementioned meanings
(B)
α) are reacted with acid halides of the formula (III) 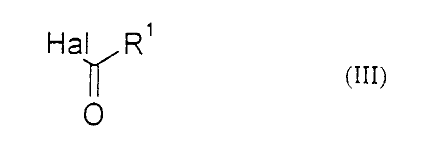 in which
R¹ has the meaning given in Claim 1 and
Hal represents halogen,
if appropriate in the presence of a diluent and
if appropriate in the presence of an acid-binding agent,
or
β) are reacted with carboxylic anhydrides of the formula (IV)
R¹-CO-O-CO-R¹ (IV)
in which
R¹ has the abovementioned meaning,
if appropriate in the presence of a diluent and
if appropriate in the presence of an acid-binding agent;
or
(C) are reacted with chloroformic esters or chloroformic thioesters of the formula (V)
R²-M-CO-Cl (V)
in which
R² and M have the meanings given in Claim 1,
if appropriate in the presence of a diluent and
if appropriate in the presence of an acid-binding agent;
or
(D)
α) are reacted with chloromonothioformic esters or chlorodithioformic esters of the formula (VI) 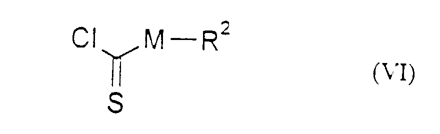 (VI)
in which
M and R² have the abovementioned meanings,
if appropriate in the presence of a diluent and
if appropriate in the presence of an acid-binding agent,
or
β) are reacted with carbon disulphide and subsequently with alkyl halides of the formula (VII)
R²-Hal (VII)
in which
R² has the abovementioned meaning
and
Hal represents chlorine, bromine or iodine;
or
(E) are reacted with sulphonyl chlorides of the formula (VIII)
R³-SO₂-Cl (VIII)
in which
R³ has the meaning given in Claim 1,
if appropriate in the presence of a diluent and
if appropriate in the presence of an acid-binding agent;
or
(F) are reacted with phosphorus compounds of the formula (IX) 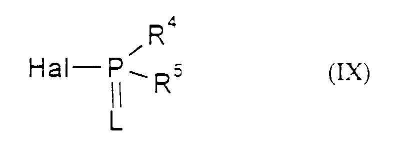 in which
L, R⁴ and R⁵ have the meanings given in Claim 1
and
Hal represents halogen,
if appropriate in the presence of a diluent and
if appropriate in the presence of an acid-binding agent;
or
(G) are reacted with metal hydroxides, metal alkoxides or amines of the formulae (X) and (XI)
Me(OR¹⁰)ₜ (X) 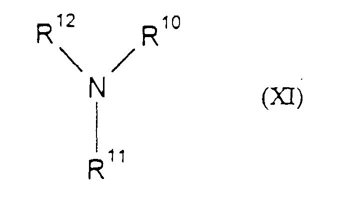
in which
Me represents mono- or divalent metal ions,
t represents the number 1 or 2 and
R¹⁰, R¹¹ and R¹² independently of one another represent hydrogen and/or alkyl,
if appropriate in the presence of a diluent,
or
(H)
α) are reacted with isocyanates or isothiocyanates of the formula (XII)
R⁶-N=C=L (XII)
in which
L and R⁶ have the meaning given in Claim 1,
if appropriate in the presence of a diluent and
if appropriate in the presence of a catalyst
or
β) with carbamoyl chlorides or thiocarbamoyl chlorides of the formula (XIII) 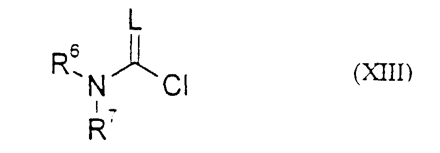 in which
L, R⁶ and R⁷ have the meanings given in Claim 1,
if appropriate in the presence of a diluent and
if appropriate in the presence of an acid-binding agent.

6. Compound of the formula (II) 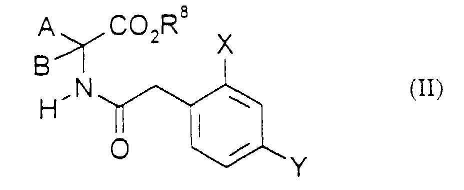 in which
A, B, X and Y have the meanings given in Claim 1 and
R⁸ represents alkyl.

7. Process for the preparation of the acyl-amino acid esters of the formula (II) according to Claim 6, characterized in that amino acid derivatives of the formula (XIV) 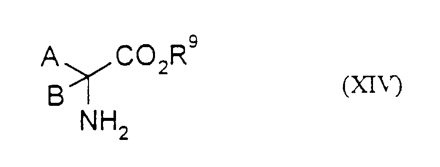 in which
R⁹ represents hydrogen (XIVa) or alkyl (XIVb)
and
A and B have the meanings given in Claim 1,
are acylated with phenylacetyl halides of the formula (XV) 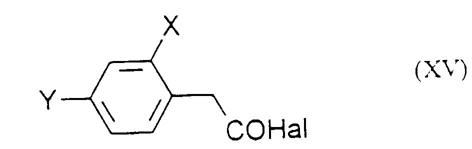 in which
X and Y have the abovementioned meanings and
Hal represents chlorine or bromine,
and the acylamino acids which have been obtained if R⁹ = hydrogen, of the formula (IIa) 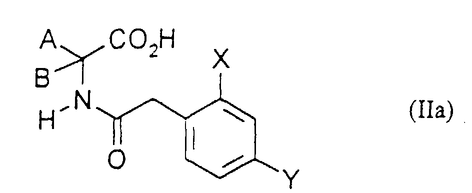 in which
A, B, X and Y have the abovementioned meanings, are esterified,
or in that aminonitriles of the formula (XVI) 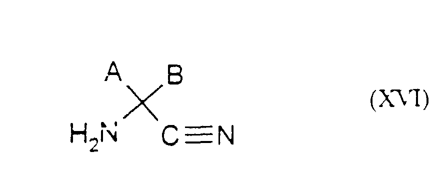 in which
A and B have the abovementioned meanings
are reacted with phenylacetyl halides of the formula (XV) 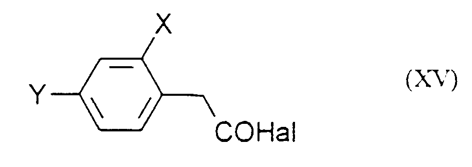 in which
X and Y have the abovementioned meanings and
Hal represents chlorine or bromine,
to give compounds of the formula (XVII) 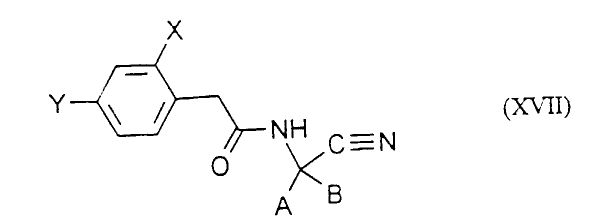 in which
A, B, X and Y have the abovementioned meanings,
and these compounds are subsequently subjected to alcoholysis in the presence of sulphuric acid.

8. Compound of the formula (XVII) 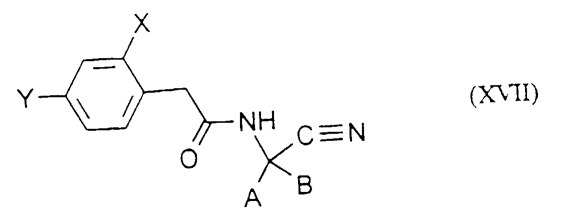 in which
A, B, X and Y have the meanings given in Claim 1.

9. Process for the preparation of compounds of the formula (XVII) according to Claim 8, characterized in that aminonitriles of the formula (XVI) 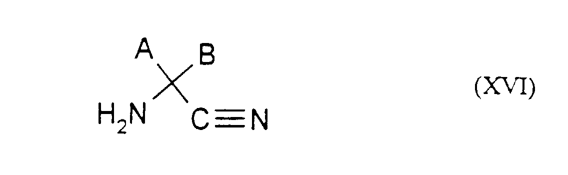 in which
A and B have the meanings given in Claim 1
are reacted with phenylacetyl halides of the formula (XV) 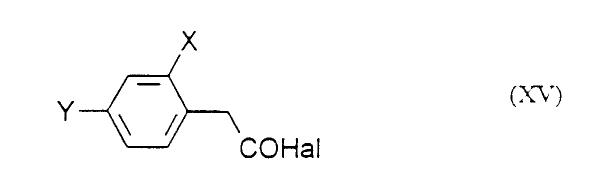 in which
X and Y have the meanings given in Claim 1
and
Hal represents chlorine or bromine.

10. Pesticide or herbicide, characterized in that it comprises at least one 1H-3-aryl-pyrrolidine-2,4-dione derivative of the formula (I) according to Claim 1.

11. Use of 1H-3-aryl-pyrrolidine-2,4-dione derivatives of the formula (I) according to Claim 1 for combating pests and undesired vegetation.

12. A method of combating pests, characterized in that 1H-3-aryl-pyrrolidine-2,4-dione derivatives of the formula (I) according to Claim 1 are allowed to act on pests, undesired vegetation and/or their environment.

13. Process for the preparation of pesticides and herbicides, characterized in that 1H-3-aryl-pyrrolidine-2,4-dione derivatives of the formula (I) according to Claim 1 are mixed with extenders and/or surface-active agents.

## Revendications

1. Dérivés de 1H-3-aryl-pyrrolidine-2,4-diones de formule (I) 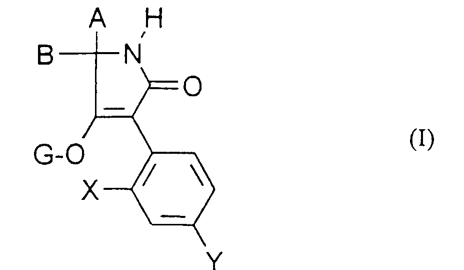 dans laquelle
A représente de l'hydrogène, un groupe alkyle en C₁ à C₁₀, alcényle en C₃ à C₆, (alkoxy en C₁ à C₈)-(alkyle en C₁ à C₆), (polyalkoxy en C₁ à C₆) - (alkyle en C₁ à C₆), (alkylthio en C₁ à C₈)-(alkyle en C₁ à C₆), dont chacun est substitué le cas échéant par du fluor et/ou du chlore, un groupe cycloalkyle à noyau de 3 à 7 atomes éventuellement substitué par du fluor, du chlore, un radical alkyle en C₁ à C₃, alkoxy en C₁ à C₃, qui peut être interrompu par un ou deux atomes d'oxygène et/ou de soufre, ou un groupe phényle, furannyle, pyridyle, imidazolyle, triazolyle, pyrazolyle, indolyle, thiazolyle, thiényle ou phényl-(alkyle en C₁ à C₄) dont chacun est substitué le cas échéant par du fluor, du chlore, du brome, un radical alkyle en C₁ à C₄, halogénalkyle en C₁ à C₄, alkoxy en C₁ à C₄ et/ou nitro,
B représente de l'hydrogène, un groupe alkyle en C₁ à C₁₂ ou alkoxyalkyle en C₁ à C₈, ou bien
A, B et l'atome de carbone auquel ils sont liés forment ainsi le cycle spiro en C₃ à C₁₀ saturé ou non saturé, éventuellement interrompu par de l'oxygène ou du soufre, qui est substitué le cas échéant une ou plusieurs fois par un radical alkyle en C₁ à C₁₀ cycloalkyle en C₃ à C₁₀, halogénalkyle en C₁ à C₆, alkoxy en C₁ à C₁₀, alkylthio en C₁ à C₁₀ halogéno ou phényle, ou bien
A, B et l'atome de carbone auquel ils sont liés représentent un cycle spiro en C₃ à C₆, qui est substitué par un groupe alkylène-diyle éventuellement interrompu par un ou deux atomes d'oxygène et/ou de soufre, ou par un groupe alkylène-dioxyle ou par un groupe alkylène-dithioyle, qui forme avec l'atome de carbone auquel il est lié un autre cycle spiro de 5 à 8 chaînons, ou bien
A, B et l'atome de carbone auquel ils sont liés représentent un cycle spiro en C₃ à C₈ dans lequel deux substituants forment conjointement un cycle de 3 à 8 chaînons saturé ou non saturé, éventuellement substitué par un radical alkyle en C₁ à C₆, alkoxy en C₁ à C₆ ou halogéno, qui peut être interrompu par de l'oxygène ou du soufre,
X représente un halogène ou un groupe alkyle en C₁ à C₆,
Y représente un halogène ou un groupe alkyle en C₁ à C₆,
G est de l'hydrogène (a) ou l'un des groupes 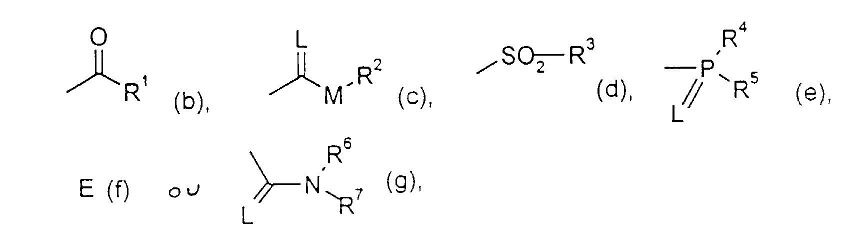 dans lesquels
E représente un équivalent d'ion métallique ou un ion ammonium et
L et M représentent chacun de l'oxygène ou du soufre,
R¹ est un groupe alkyle en C₁ à C₁₆, alcényle en C₂ à C₁₆, (alkoxy en C₁ à C₆)-(alkyle en C₁ à C₆), (alkylthio en C₁ à C₁₆)-(alkyle en C₁ à C₆), (polyalkoxy en C₁ à C₆)-(alkyle en C₁ à C₆) dont chacun est éventuellement substitué par du fluor et/ou du chlore, ou un groupe cycloalkyle à noyau de 3 à 7 atomes éventuellement substitué par un halogène ou un radical alkyle en C₁ à C₅, qui peut être interrompu par 1-2 atomes d'oxygène et/ou de soufre,
un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
un groupe pyrazolyle, thiazolyle, pyridyle, pyrimidyle, furannyle ou thiényle éventuellement substitué par du fluor, du chlore, du brome et/ou un radical alkyle en C₁ à C₄,
un groupe phénoxy-(alkyle en C₁ à C₅) éventuellement substitué par du fluor, du chlore, du brome et/ou un radical alkyle en C₁ à C₄, ou bien
un groupe pyridyloxy-(alkyle en C₁ à C₅), pyrimidyloxy-(alkyle en C₁ à C₅) ou thiazoiyioxy-(alkyle en C₁ à C₅) éventuellement substitué par du fluor, du chlore, du brome, un radical amino et/ou un radical alkyle en C₁ à C₄,
R² représente un groupe alkyle en C₁ à C₂₀, alcényle en C₃ à C₂₀, (alkoxy en C₁ à C₈)-(alkyle en C₁ à C₈), (polyalkoxy en C₁ à C₈)-(alkyle en C₁ à C₈) dont chacun est éventuellement substitué par un halogène,
un groupe cycloalkyle en C₃ à C₈ éventuellement substitué par un halogène, un radical alkyle en C₁ à C₄ et/ou un radical alkoxy en C₁ à C₄,
un groupe phényle ou un groupe benzyle dont chacun est éventuellement substitué par un halogène, un radical nitro, alkyle en C₁ à C₆, alkoxy en C₁ à C₆ et/ou halogénalkyle en C₁ à C₆,
R³, R⁴ et R⁵ représentent, indépendamment les uns des autres, un groupe alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₈, cycloalkylthio en C₃ à C₇, dont chacun est éventuellement substitué par un halogène, un groupe phényle, phénoxy ou phénylthio dont chacun est éventuellement substitué par un halogène, un radical nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄, et
R⁶ et R⁷ représentent, indépendamment l'un de l'autre, de l'hydrogène, un groupe alkyle en C₁ à C₈, cycloalkyle en C₃ à C₈, alkoxy en C₁ à C₈, alcényle en C₃ à C₈, (alkoxy en C₁ à C₈)-(alkyle en C₂ à C₈), dont chacun est éventuellement substitué par un halogène, un groupe phényle éventuellement substitué par un halogène, un radical halogénalkoxy en C₁ à C₈, alkyle en C₁ à C₈ et/ou alkoxy en C₁ à C₈, un groupe benzyle éventuellement substitué par un halogène, un radical alkyle en C₁ à C₈, halogénalkyle en C₁ à C₈ et/ou alkoxy en C₁ à C₈ ou forment ensemble un reste alkylène en C₃ à C₆ éventuellement interrompu par de l'oxygène ou du soufre,
sous réserve que X et Y ne soient pas en même temps un groupe alkyle et ne soient pas en même temps un halogène.

2. Dérivés de 1H-aryl-pyrrolidine-2,4-diones de formule (I) suivant la revendication 1,
dans laquelle
A représente de l'hydrogène, un groupe alkyle en C₁ à C₁₀, alcényle en C₃ à C₆, (alkoxy en C₁ à C₈)-(alkyle en C₁ à C₆), (polyalkoxy en C₁ à C₆)-(alkyle en C₁ à C₆), (alkylthio en C₁ à C₈)-(alkyle en C₁ à C₆) dont chacun est substitué le cas échéant par du fluor et/ou du chlore, un groupe cycloalkyle à noyau de 3 à 7 atomes, éventuellement substitué par du fluor, du chlore, un radical alkyle en C₁ à C₃, alkoxy en C₁ à C₃, qui peut être interrompu par un ou deux atomes d'oxygène et/ou de soufre, ou un groupe phényle, furannyle, pyridyle, imidazolyle, triazolyle, pyrazolyle, indolyle, thiazolyle, thiényle ou phényl-(alkyle en C₁ à C₄) dont chacun est substitué le cas échéant par du fluor, du chlore, du brome, un radical alkyle en C₁ à C₄, halogénalkyle en C₁ à C₄, alkoxy en C₁ à C₄ et/ou nitro,
B représente de l'hydrogène, un groupe alkyle en C₁ à C₁₀ ou alkoxyalkyle en C₁ à C₆, ou bien
A, B et l'atome de carbone auquel ils sont liés représentent un cycle spiro en C₃ à C₉ saturé ou non saturé, éventuellement interrompu par de l'oxygène ou du soufre, qui est substitué le cas échéant une ou plusieurs fois par un radical alkyle en C₁ à C₆, cycloalkyle en C₃ à C₈, halogénalkyle en C₁ à C₃, alkoxy en C₁ à C₆, alkylthio en C₁ à C₆, du fluor, du chlore ou un radical phényle, ou bien
A, B et l'atome de carbone auquel ils sont liés représentent un cycle spiro en C₃ à C₆, qui est substitué par un groupe alkylène-diyle éventuellement interrompu par un ou deux atomes d'oxygène ou de soufre, ou par un groupe alkylène-dioxyle ou par un groupe alkylènedithiol, qui forme avec l'atome de carbone auquel il est lié un autre cycle spiro de 5 à 7 chaînons,
A, B et l'atome de carbone auquel ils sont liés représentent un cycle spiro en C₃ à C₆ dans lequel deux substituants forment ensemble un cycle de 5 à 8 chaînons saturés ou non saturés, éventuellement substitués par un radical alkyle en C₁ à C₃, alkoxy en C₁ à C₃, du fluor, du chlore ou du brome, qui peut être interrompu par de l'oxygène ou du soufre,
X représente du fluor, du chlore, du brome ou un groupe alkyle en C₁ à C₄,
Y représente du fluor, du chlore, du brome ou un groupe alkyle en C₁ à C₄,
G représente de l'hydrogène (a) ou l'un des groupes 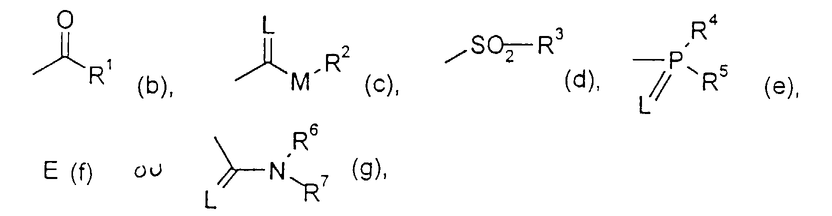 dans lesquels
E représente un équivalent d'ion métallique ou un ion ammonium
et
L et M représentent chacun de l'oxygène ou du soufre,
R¹ représente un groupe alkyle en C₁ à C₁₆, alcényle en C₂ à C₁₆, (alkoxy en C₁ à C₆)-(alkyle en C₁ à C₆), (alkylthio en C₁ à C₁₆)-(alkyle en C₁ à C₆), (polyalkoxy en C₁ à C₆)-(alkyle en C₁ à C₆) dont chacun est éventuellement substitué par du fluor et/ou du chlore, ou un groupe cycloalkyle à noyau de 3 à 7 atomes, éventuellement substitué par un halogène ou un radical alkyle en C₁ à C₅, qui peut être interrompu par 1-2 atomes d'oxygène et/ou de soufre,
un groupe phényle éventuellement substitué par un halogène, un radical nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un groupe phényl-(alkyle en C₁ à C₄) éventuellement substitué par un halogène, un radical alkyle en C₁ à C₄, alkoxy en C₁ à C₄, halogénalkyle en C₁ à C₃, halogénalkoxy en C₁ à C₃,
un groupe pyrazolyle, thiazolyle, pyridyle, pyrimidyle, furannyle ou thiényle éventuellement substitué par du fluor, du chlore, du brome et/ou un radical alkyle en C₁ à C₄,
un groupe phénoxy-(alkyle en C₁ à C₅) éventuellement substitué par du fluor, du chlore, du brome et/ou un radical alkyle en C₁ à C₄, ou bien
un groupe pyridyloxy-(alkyle en C₁ à C₅), pyrimidyloxy-(alkyle en C₁ à C₅) ou thiazolyloxy-(alkyle en C₁ à C₅) éventuellement substitué par du fluor, du chlore, du brome, un radical amino et/ou alkyle en C₁ à C₄,
R² est un groupe alkyle en C₁ à C₁₆, alcényle en C₃ à C₁₆, (alkoxy en C₁ à C₆)-(alkyle en C₁ à C₆), (polyalkoxy en C₁ à C₆)-(alkyle en C₁ à C₆) dont chacun est éventuellement substitué par un halogène,
un groupe cycloalkyle en C₃ à C₇ éventuellement substitué par un halogène, un radical alkyle en C₁ à C₃ et/ou alkoxy en C₁ à C₃, ou bien
un groupe phényle ou benzyle éventuellement substitué par un halogène, un radical nitro, alkyle en C₁ à C₄, alkoxy en C₁ à C₃ et/ou halogénalkyle en C₁ à C₃,
R³, R⁴ et R⁵ représentent, indépendamment les uns des autres, un groupe alkyle en C₁ à C₆, alkoxy en C₁ à C₆, alkylamino en C₁ à C₆; di(alkyle en C₁ à C₆)-amino, alkylthio en C₁ à C₆, alcénylthio en C₃ ou C₄, cycloalkylthio en C₃ à C₆ dont chacun est substitué le cas échéant par un halogène, un groupe phényle, phénoxy ou phénylthio dont chacun est substitué le cas échéant par du fluor, du chlore, du brome, un radical nitro, cyano, alkoxy en C₁ à C₃, halogénalkoxy en C₁ a C₃, alkylthio en C₁ à C₃, halogénalkylthio en C₁ à C₃, alkyle en C₁ à C₃, halogénalkyle en C₁ à C₃ et
R⁶ et R⁷ représentent, indépendamment l'un de l'autre, de l'hydrogène, un groupe alkyle en C₁ à C₆, cycloalkyle en C₃ à C₆, alkoxy en C₁ à C₆, alcényle en C₃ à C₆, (alkoxy en C₁ à C₆)-(alkyle en C₁ à C₆) dont chacun est substitué le cas échéant par un halogène, un groupe phényle éventuellement substitué par un halogène, un radical halogénalkyle en C₁ à C₅, alkyle en C₁ à C₅ et/ou alkoxy en C₁ à C₅, un groupe benzyle éventuellement substitué par un halogène, un radical alkyle en C₁ à C₅, halogénalkyle en C₁ à C₅ et/ou alkoxy en C₁ à C₅, ou forment ensemble un reste alkylène en C₃ à C₆ éventuellement interrompu par de l'oxygène ou du soufre,
sous réserve que X et Y ne représentent pas en même temps un groupe alkyle et ne représentent pas en même temps un halogène.

3. Dérivés de 1H-3-aryl-pyrrolidine-2,4-diones de formule (I) suivant la revendication 1,
dans laquelle
A représente de l'hydrogène, un groupe alkyle en C₁ à C₈ éventuellement substitué par du fluor et/ou du chlore, un groupe alcényle en C₃ ou C₄, (alkoxy en C₁ à C₆)-(alkyle en C₁ à C₄), (polyalkoxy en C₁ à C₄)-(alkyle en C₁ à C₄), (alkylthio en C₁ à C₆)-(alkyle en C₁ à C₄), un groupe cycloalkyle à noyau de 3 à 6 atomes éventuellement substitué par du fluor, du chlore, un radical méthyle, éthyle, méthoxy ou éthoxy, qui peut être interrompu par un ou deux atomes d'oxygène et/ou de soufre, ou représente un groupe phényle, furannyle, pyridyle, imidazolyle, pyrazolyle, triazolyle, indolyle, thiazolyle, thiényle ou benzyle dont chacun est substitué le cas échéant par du fluor, du chlore, du brome, un radical méthyle, éthyle, propyle, isopropyle, méthoxy, éthoxy, trifluorométhyle et/ou nitro,
B représente de l'hydrogène, un groupe alkyle en C₁ à C₈ ou alkoxyalkyle en C₁ à C₄, ou bien
A, B et l'atome de carbone auquel ils sont liés représentent un cycle spiro en C₃ à C₈ saturé ou non saturé, éventuellement interrompu par de l'oxygène ou du soufre, qui est substitué le cas échéant une ou plusieurs fois par un radical méthyle, éthyle, propyle, isopropyle, butyle, isobutyle, sec.-butyle, tertio-butyle, cyclohexyle, trifluorométhyle, méthoxy, éthoxy, propoxy, isopropoxy, butoxy, isobutoxy, sec.-butoxy, tertio-butoxy, méthylthio, éthylthio, fluoro, chloro ou phényle, ou bien
A, B et l'atome de carbone auquel ils sont liés représentent un cycle spiro en C₃ à C₆ qui est substitué le cas échéant par un groupe alkylène-diyle éventuellement interrompu par un atome d'oxygène ou de soufre, ou par un groupe alkylènedioxyle, qui forme un autre cycle spiro de 5 à 7 chaînons avec l'atome de carbone auquel il est lié, ou bien
A, B et l'atome de carbone auquel ils sont liés forment un cycle spiro en C₃ à C₆ dans lequel deux substituants forment ensemble un cycle pentagonal ou hexagonal saturé ou non saturé qui peut être interrompu par de l'oxygène ou du soufre,
X représente du chlore, du brome, un groupe méthyle, éthyle, propyle ou isopropyle,
Y représente du chlore, du brome, un groupe méthyle, éthyle, propyle ou isopropyle,
G représente de l'hydrogène (a) ou l'un des groupes 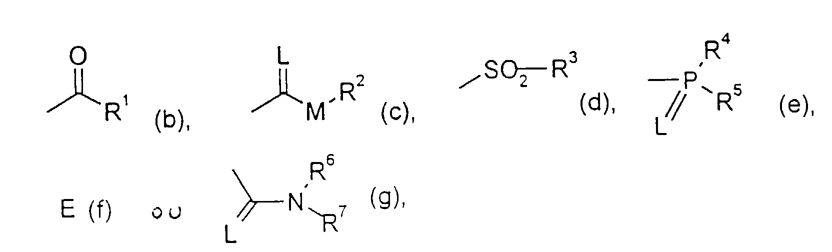 dans lesquels
E représente un équivalent d'ion métallique ou un ion ammonium,
L et M représentent chacun de l'oxygène ou du soufre,
R¹ est un groupe alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), (polyalkoxy en C₁ à C₄)-(alkyle en C₁ à C₄) dont chacun est substitué le cas échéant par du fluor et/ou du chlore, ou un groupe cycloalkyle à noyau de 3 à 6 chaînons éventuellement substitué par du fluor, du chlore, un radical méthyle, éthyle, propyle, isopropyle, butyle, isobutyle ou tertio-butyle, qui peut être interrompu par un ou deux atomes d'oxygène et/ou de soufre,
un groupe phényle éventuellement substitué par du fluor, du chlore, du brome, un radical nitro, méthyle, éthyle, propyle, isopropyle, méthoxy, éthoxy, trifluorométhyle, trifluorométhoxy, méthylthio, éthylthio, méthylsulfonyle, éthylsulfonyle,
un groupe phényl-(alkyle en C₁ à C₃) éventuellement substitué par du fluor, du chlore, du brome, un radical méthyle, éthyle, propyle, isopropyle, méthoxy, éthoxy, trifluorométhyle ou trifluorométhoxy,
un groupe furannoyle, thiényle, pyridyle, pyrimidinyle, thiazolyle ou pyrazolyle éventuellement substitué par du fluor, du chlore, du brome, un radical méthyle ou éthyle,
un groupe phénoxy-(alkyle en C₁ à C₄) éventuellement substitué par du fluor, du chlore, un radical méthyle et/ou éthyle, ou bien
un groupe pyridyloxy-(alkyle en C₁ à C₄), pyrimidyloxy-(alkyle en C₁ à C₄) ou thiazolyloxy-(alkyle en C₁ à C₄) éventuellement substitué par du fluor, du chlore, un radical amino, méthyle, éthyle,
R² est un groupe alkyle en C₁ à C₁₄, alcényle en C₃ à C₁₄, (alkoxy en C₁ à C₄)-(alkyle en C₁ à C₆), (polyalkoxy en C₁ à C₄)-(alkyle en C₁ à C₆) dont chacun est substitué le cas échéant par du fluor et/ou du chlore,
un groupe cycloalkyle en C₃ à C₆ éventuellement substitué par du fluor, du chlore, un radical méthyle, éthyle, propyle, isopropyle ou méthoxy,
ou bien un radical phényle ou benzyle dont chacun est substitué le cas échéant par du fluor, du chlore, un radical nitro, méthyle, éthyle, propyle, isopropyle, méthoxy, éthoxy, trifluorométhyle,
R³, R⁴ et R⁵ représentent, indépendamment les uns des autres, un groupe alkyle en C₁ à C₄, alkoxy en C₁ à C₄, alkylamino en C₁ à C₄, di-(alkyle en C₁ à C₄)-amino, alkylthio en C₁ à C₄ dont chacun est substitué le cas échéant par du fluor et/ou du chlore, un groupe phénylthio, phénoxy ou phényle éventuellement substitué par du fluor, du chlore, du brome, un radical nitro, cyano, alkoxy en C₁ ou C₂, fluoralkoxy en C₁ à C₄, alkylthio en C₁ ou C₂, fluoralkylthio en C₁ ou C₂, alkyle en C₁ à C₃, et
R⁶ et R⁷ représentent, indépendamment l'un de l'autre, de l'hydrogène, un groupe alkyle en C₁ à C₄, cycloalkyle en C₃ à C₆, alkoxy en C₁ à C₄, alcényle en C₃ ou C₄, (alkoxy en C₁ à C₄)-(alkyle en C₁ à C₄) dont chacun est substitué le cas échéant par du fluor, du chlore, du brome, un groupe phényle éventuellement substitué par du fluor, du chlore, du brome, un radical halogénalkyle en C₁ à C₄, alkyle en C₁ à C₄ et/ou alkoxy en C₁ à C₄, un groupe benzyle éventuellement substitué par du fluor, du chlore, du brome, un radical alkyle en C₁ à C₄, halogénalkyle en C₁ à C₄ et/ou alkoxy en C₁ à C₄, ou forment ensemble un reste alkylène en C₄ à C₆ éventuellement interrompu par de l'oxygène ou du soufre,
sous réserve que X et Y ne soient pas en même temps un groupe alkyle et ne soient pas en même temps un halogène.

4. Dérivés de 1H-3-aryl-pyrrolidine-2,4-diones de formule (I) suivant la revendication 1, caractérisés en ce qu'il s'agit de l'une des structures (Ia) à (Ig) suivantes : 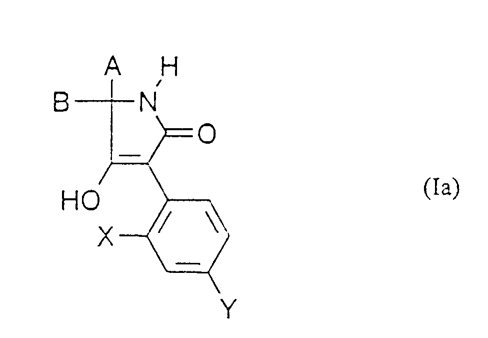 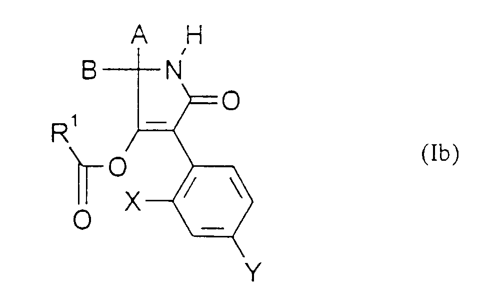 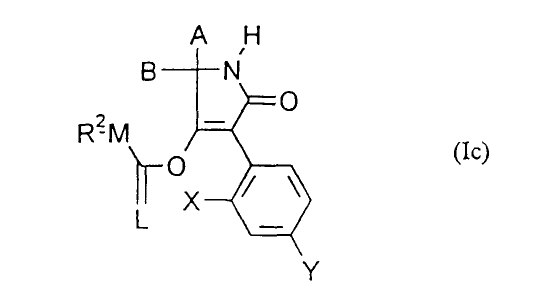 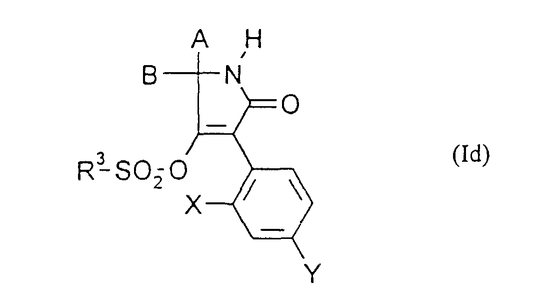 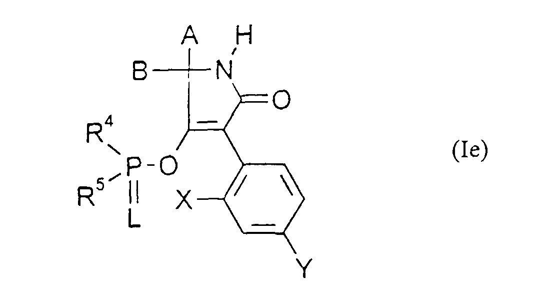 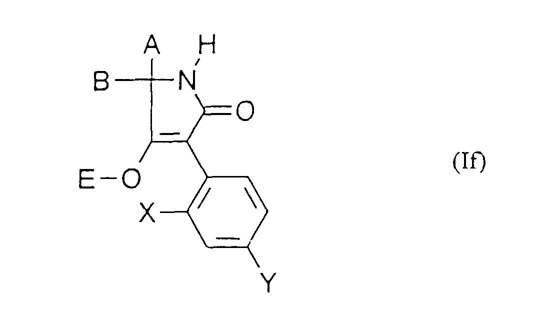 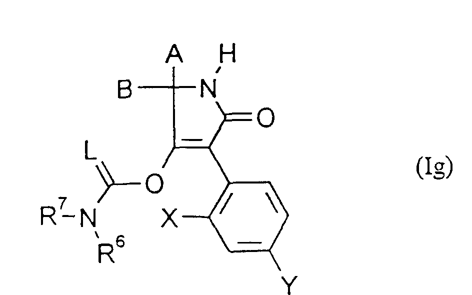 dans lesquelles
A, B, E, L, M, X, Y, R¹, R², R³, R⁴, R⁵, R⁶ et R⁷ ont les définitions indiquées dans la revendication 1.

5. Procédé de production de dérivés de 1H-3-aryl-pyrrolidine-2,4-diones substitués de formule (I) suivant la revendication 1, caractérisé en ce que :
(A) On effectue la condensation intramoléculaire d'esters de N-acylaminoacides de formule (II) 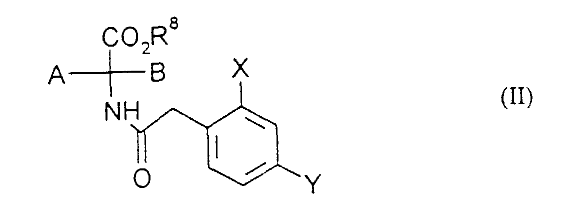 dans laquelle
A, B, X et Y ont la définition indiquée dans la revendication 1,
et
R⁸ est un groupe alkyle,
en présence d'un diluant et en présence d'une base ;
puis
on fait réagir les composés obtenus de formule (Ia) ; 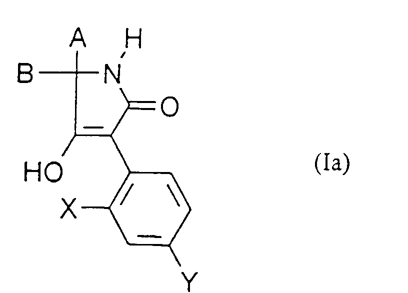 dans laquelle
A, B, X et Y ont la définition indiquée ci-dessus,
(B)
α) avec des halogénures d'acides de formule (III) 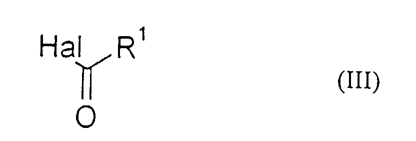 dans laquelle
R¹ a la définition indiquée ci-dessus et
Hal est un halogène,
le cas échéant en présence d'un diluant et en la présence éventuelle d'un accepteur d'acide,
ou bien
β) avec des anhydrides d'acides carboxyliques de formule (IV)
R¹-CO-O-CO-R¹ (IV)
dans laquelle
R¹ a la définition indiquée ci-dessus,
le cas échéant en présence d'un diluant et en la présence éventuelle d'un accepteur d'acide ;
ou bien
(C) on les fait réagir avec des esters d'acide chloroformique ou des thiolesters d'acide chloroformique de formule (V)
R²-M-CO-Cl (V)
dans laquelle
R² et M ont la définition indiquée dans la revendication 1,
le cas échéant en présence d'un diluant et en la présence éventuelle d'un accepteur d'acide, ou bien
(D)
α) on les fait réagir avec des esters d'acide chloromonothioformique ou des esters d'acide chlorodithioformique de formule (VI) 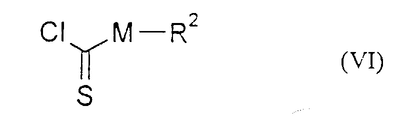 dans laquelle
M et R² ont la définition indiquée ci-dessus,
le cas échéant en présence d'un diluant et en la présence éventuelle d'un accepteur d'acide,
ou bien
β) avec du sulfure de carbone puis avec des halogénures d'alkyle de formule (VII)
R²-Hal (VII)
dans laquelle
R² a la définition indiquée ci-dessus,
et
Hal représente le chlore, le brome ou l'iode ;
ou bien
(E) on les fait réagir avec des chlorures d'acide sulfonique de formule (VIII)
R³-SO₂-Cl (VIII)
dans laquelle
R³ a la définition indiquée dans la revendication 1,
le cas échéant en présence d'un diluant et en la présence éventuelle d'un accepteur d'acide,
ou bien
(F) on les fait réagir avec des composés phosphorés de formule (IX) 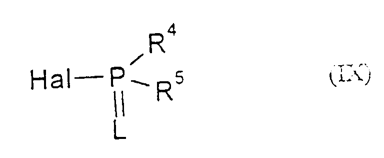 dans laquelle
L, R⁴ et R⁵ ont la définition indiquée dans la revendication 1,
et
Hal représente un halogène,
le cas échéant en présence d'un diluant et en la présence éventuelle d'un accepteur d'acide ;
ou bien
(G) on les fait réagir avec des hydroxydes de métaux, des alcoolates de métaux ou des amines de formules (X) et (XI)
Me(OR¹⁰)ₜ (X) 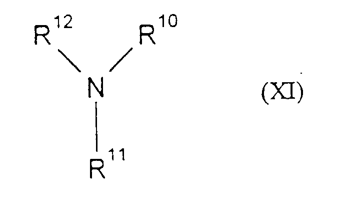
dans lesquelles
Me représente un ion de métal monovalent ou divalent
t représente le nombre 1 ou 2 et
R¹⁰, R¹¹ et R¹² représentent, indépendamment les uns des autres, de l'hydrogène et/ou un groupe alkyle,
le cas échéant en présence d'un diluant,
ou bien on les fait réagir
(H)
α) avec des isocyanates ou des isothiocyanates de formule (XII)
R⁶-N=C=L (XII)
dans laquelle
L et R⁶ ont la définition donnée dans la revendication 1,
le cas échéant en présence d'un diluant et en la présence éventuelle d'un catalyseur,
ou bien
β) avec des chlorures d'acide carbamique ou des chlorures d'acide thiocarbamique de formule (XIII) 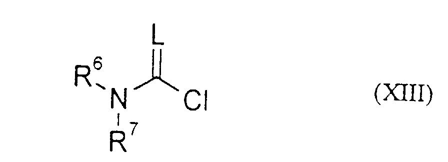 dans laquelle
L, R⁶ et R⁷ ont la définition indiquée dans la revendication 1,
le cas échéant en présence d'un diluant et en la présence éventuelle d'un accepteur d'acide.

6. Composés de formule (II) 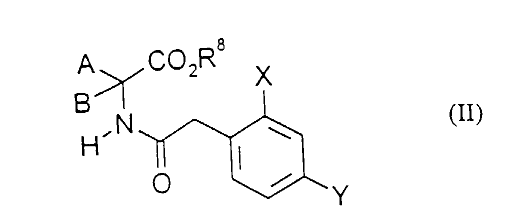 dans laquelle
A, B, X et Y ont la définition indiquée dans la revendication 1 et
R⁸ est un groupe alkyle.

7. Procédé de production des esters d'acylaminoacides de formule (II) suivant la revendication 6, caractérisé en ce qu'on acyle des dérivés d'aminoacides de formule (XIV), 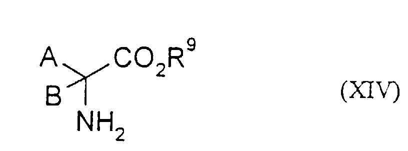 dans laquelle
R⁹ représente de l'hydrogène (XIVa) ou un groupe alkyle (XIVb)
et
A et B ont la définition indiquée dans la revendication 1,
avec des halogénures d'acide phénylacétique de formule (XV) 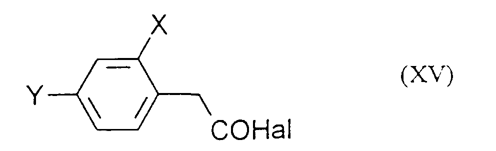 dans laquelle
X et Y ont la définition indiquée ci-dessus et
Hal représente du chlore ou du brome,
et on estérifie les acylaminoacides obtenus en l'occurrence pour R⁹ = l'hydrogène, de formule (IIa), 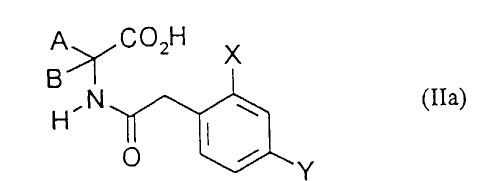 dans laquelle
A, B, X et Y ont la définition indiquée ci-dessus,
ou bien on fait réagir des aminonitriles de formule (XVI) 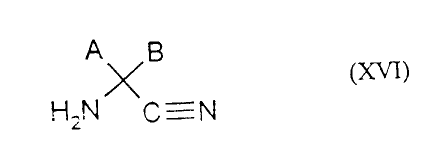 dans laquelle
A et B ont la définition indiquée ci-dessus,
avec des halogénures d'acide phénylacétique de formule (XV) 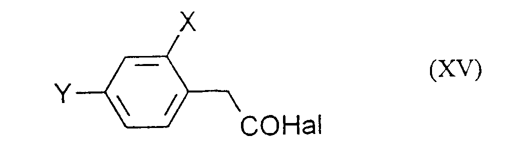 dans laquelle
X et Y ont la définition indiquée ci-dessus et
Hal représente du chlore ou du brome,
pour obtenir des composés de formule (XVII) 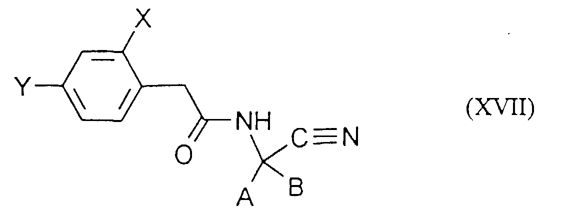 dans laquelle
A, B, X et Y ont la définition indiquée ci-dessus,
et on soumet ensuite ces composés à une alcoolyse sulfurique.

8. Composés de formule (XVII) 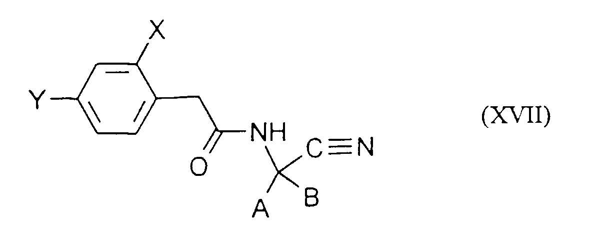 dans laquelle
A, B, X et Y ont la définition indiquée dans la revendication 1.

9. Procédé de production de composés de formule (XVII) suivant la revendication 8, caractérisé en ce qu'on fait réagir des aminonitriles de formule (XVI) 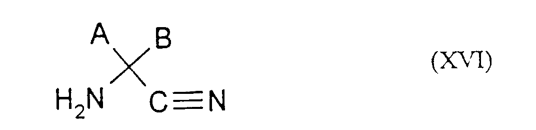 dans laquelle
A et B ont la définition indiquée dans la revendication 1,
avec des halogénures d'acide phénylacétique de formule (XV) 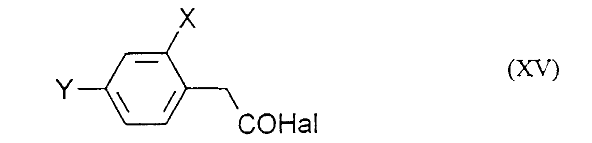 dans laquelle
X et Y ont la définition indiquée dans la revendication 1
et
Hal représente du chlore ou du brome.

10. Compositions pesticides et herbicides, caractérisées par une teneur en au moins un dérivé de 1H-3-aryl-pyrrolidine-2,4-dione de formule (I) suivant la revendication 1.

11. Utilisation de dérivés de 1H-3-aryl-pyrrolidine-2,4-diones de formule (I) suivant la revendication 1 pour combattre des parasites et la croissance non désirée de plantes.

12. Procédé pour combattre des parasites, caractérisé en ce qu'on fait réagir des dérivés de 1H-3-aryl-pyrrolidine-2,4-diones de formule (I) suivant la revendication 1 sur les parasites, sur une végétation non désirée et/ou sur leur milieu.

13. Procédé de préparation de compositions pesticides et d'herbicides, caractérisé en ce qu'on mélange des dérivés de 1H-3-aryl-pyrrolidine-2,4-diones de formule (I) suivant la revendication 1 avec des diluants et/ou des agents tensio-actifs.
